# EUROPEAN PATENT APPLICATION

(11) **EP 1 690 863 A1**
(43) Date of publication of application: **16.08.2006**
(21) Application number: 04799888.5
(22) Date of filing: 24.11.2004
(51) Int. Cl.: C07D 471/04, A61K 31/4545, A61K 31/5377, A61P 3/10, A61P 43/00

(54) **NOVEL CONDENSED IMIDAZOLE DERIVATIVE**

(30) Priority: 26.11.2003 JP 2003396242; 17.12.2003 JP 2003419427
(71) Applicant: Dainippon Sumitomo Pharma Co., Ltd., Osaka 541-8524 (JP)
(72) Inventor: NAKAHIRA, Hiroyuki c/o Dainippon Sumitomomo Pharma, Osaka-shi, Osaka 5540022 (JP); KIMURA, Hidenori c/o Dainippon Sumimoto Pharma, Osaka-shi, Osaka 5540022 (JP); KOBAYASHI, Tomonori c/o Dainippon Sumimoto Pharma, Osaka-shi, Osaka 5540022 (JP); HOCHIGAI, Hitoshi c/o Dainippon Sumimoto Pharma, Osaka-shi, Osaka 5540022 (JP)
(74) Representative: Duckworth, Timothy John
(86) International application number: PCT/JP2004/017828
(87) International publication number: WO 2005/051949

(57) **Abstract**

Disclosed is a compound represented by the formula (1) below which has a high DPP-IV inhibitory activity and is improved in safety, toxicity and the like. Also disclosed is a prodrug of such a compound and pharmaceutically acceptable salts of them. 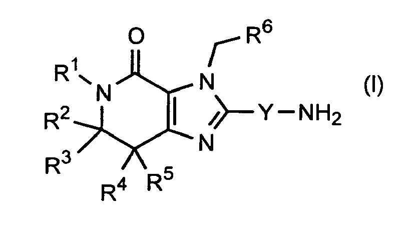 (In the formula, R¹ represents a hydrogen atom, an optionally substituted alkyl group or the like; R² and R³ independently represent a hydrogen atom, an optionally substituted alkyl group or the like; R⁴ and R⁵ independently represent a hydrogen atom, an optionally substituted alkyl group or the like: R⁶ represents a hydrogen atom, an optionally substituted aryl group or the like; and -Y-NH₂, represents a group represented by the following formula (A): 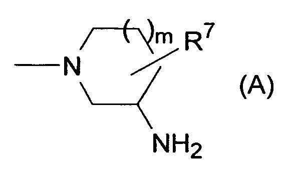 (wherein m is 0, 1 or 2; and R⁷ may not exist or one or two R⁷ may exist and independently represent an optionally substituted alkyl group or the like) or the like.]

## Description

### TECHNICAL FIELD

The present invention relates to novel cyclic imidazole derivatives useful as medicines. More particularly, it relates to novel cyclic imidazole derivatives effective as a dipeptidyl peptidase IV (DPP-IV) inhibitor. Furthermore, it relates to a pharmaceutical composition for the treatment of diabetes containing a novel cyclic imidazole derivative effective as a dipeptidyl peptidase IV (DPP-IV) inhibitor, as an active ingredient.

### BACKGROUND ART

DPP-IV is a serine protease widely present in the body, is one of dipeptidyl aminopeptidases capable of hydrolyzing and releasing a N-terminal dipeptide, and markedly acts on, in particular, peptides containing proline as the second amino acid from the N-terminal. Therefore, DPP-IV is referred to also prolyl endopeptidase. DPP-IV is known to accept, as substrates, various biological peptides concerned in the endocrine system, the neuroendocrine system, immune functions and the like. It is known that many physiologically active peptides such as the pancreatic polypeptide family represented by pancreatic polypeptides (PP), neuropeptide Y (NPY) and the like; the glucagon/VIP family represented by vasoactive intestinal polypeptides (VIP), glucagon-like peptide-1 (GLP-1), glucose-dependent insulinotropic polypeptides (GIP), growth hormone release accelerating factor (GRF) and the like; and the chemocaine family are substrates for DPP-IV and feel the influences of DPP-IV, such as activation/inactivation, metabolism acceleration and the like (J. Langner and S. Ansorge, "Cellular Peptidases in Immune Functions and Disease 2", Advances in Experimental Medicine and Biology Vol. 477).

DPP-IV severs two amino acids (His-Ala) from the N-terminal of GLP-1. It is known that although the severed peptide binds weekly to a GLP-1 receptor, it has no activating effect on the receptor and acts as an antagonist (L.B. Knudsen et al., European Journal of Pharmacology, Vol. 318, p429-435, 1996). The metabolism of GLP-1 by DPP-IV in blood is known to be very rapid, and the concentration of active GLP-1 in blood is increased by the inhibition of DPP-IV (T.J. Kieffer et al., Endocrinology, Vol. 136, p3585-3596, 1995). GLP-1 is a peptide secreted from intestinal tract by the ingestion of sugars and is a main accelerating factor for the glucose-responsive secretion of insulin by pancreas. In addition, GLP-1 is known to have accelerating effect on insulin synthesis in pancreatic β cells and accelerating effect on β cell proliferation. Moreover, it is known that GLP-1 receptors appear also in digestive tracts, liver, muscle, adipose tissue and the like, and it is also known that in these tissues, GLP-1 affects working of digestive tracts, the secretion of acid in stomach, the synthesis and degradation of glycogen, insulin-dependent glucose uptake, and the like. Accordingly, there is expected the development of a DPP-IV inhibitor effective against type 2 diabetes (non-insulin-dependent diabetes) which brings about effects such as the acceleration of insulin secretion dependent on blood sugar level, the improvement of pancreas function, the improvement of a high postprandial blood sugar level, the improvement of glucose tolerance abnormality, the improvement of insulin resistance, and the like, by increasing the concentration of GLP-1 in blood (R.A. Pederson et al., Diabetes Vol. 47, p1253-1258, 1998).

Various DPP-IV inhibitors have been reported. For example, International Publication No. WO02/0256 pamphlet reports that xanthine derivatives having a piperazine ring or the like are effective as a DPP-IV inhibitor. International Publication No. WO02/068420 pamphlet and International Publication No. WO03/004496 pamphlet report that xanthine derivatives having a piperidine ring or the like are effective as a DPP-IV inhibitor. International Publication No. WO03/024965 pamphlet reports that xanthine derivatives containing a 2-aminocyclohexylamino group are effective as a DPP-IV inhibitor. International Publication No. WO02/024698 pamphlet reports that xanthine derivatives are effective as a phosphodiesterase V inhibitor.

### DISCLOSURE OF THE INVENTION

An object of the present invention is to provide a novel compound having an excellent DPP-IV inhibitory activity.

The present inventors earnestly investigated in order to achieve the above object, and consequently found that the following compound, a prodrug thereof or a pharmaceutically acceptable salt of the compound or prodrug (if necessary, they are hereinafter abbreviated as the present inventive compound in some cases) has an excellent DPP-IV inhibitory effect, whereby the present invention has been accomplished.

That is, the present invention relates to the following:
[1] A compound represented by the formula (I): 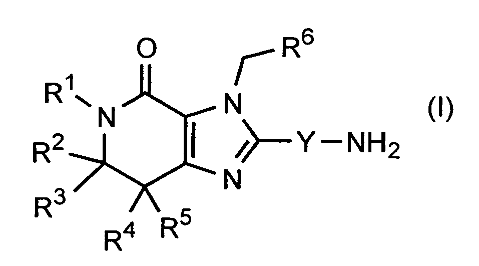 wherein R¹ is a hydrogen atom, an optionally substituted alkyl group, an optionally substituted cycloalkyl group, an optionally substituted aryl group, or an optionally substituted heteroaryl group;
   R² and R³ are independently a hydrogen atom, a halogen atom, a cyano group, a formyl group, an optionally substituted alkyl group, an optionally substituted cycloalkyl group, an optionally substituted cycloalkyloxy group, an optionally substituted alkenyl group, an optionally substituted alkynyl group, an optionally substituted amino group, an optionally substituted carbamoyl group, a carboxyl group, an optionally substituted alkoxy group, an optionally substituted alkoxycarbonyl group, an optionally substituted aryl group, an optionally substituted aryloxy group, an optionally substituted aryloxycarbonyl group, an optionally substituted aralkyl group, an optionally substituted aralkyloxy group, an optionally substituted aroyl group, an optionally substituted arylthio group, an optionally substituted arylsulfinyl group, an optionally substituted arylsulfonyl group, an optionally substituted alkylthio group, an optionally substituted alkylsulfinyl group, an optionally substituted alkylsulfonyl group, an optionally substituted heteroaryl group, an optionally substituted heteroarylalkyl group, an optionally substituted heteroarylcarbonyl group, an optionally substituted heteroaryloxy group, an optionally substituted alkylcarbonyl group, an optionally substituted nitrogen-containing saturated heterocyclic group, an optionally substituted aralkyloxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ is a hydrogen atom, an alkyl group, an alkenyl group, a cycloalkyl group or an alkoxy group, and R¹⁹ is an optionally substituted alkyl group, an optionally substituted alkenyl group, a cycloalkyl group, a cycloalkyloxy group, an optionally substituted alkoxy group, an optionally substituted alkenyloxy group, a 2-indanyloxy group, a 5-indanyloxy group or an optionally substituted aryloxy group, or R² and R³ may be taken together to form an oxo group on the ring;
   R⁴ and R⁵ are independently a hydrogen atom, a halogen atom, an optionally substituted alkyl group or an alkoxycarbonylmethyl group;
   R³ and R⁵ may be taken together to form a double bond on the ring;
   R², R³, R⁴ and R⁵ may form an optionally substituted benzene ring, an optionally substituted cycloalkene ring or an optionally substituted 5-or 6-membered heteroaromatic ring together with the adjacent carbon atoms;
   R⁶ is a hydrogen atom, an optionally substituted alkyl group, an optionally substituted cycloalkyl group, an optionally substituted aryl group, an optionally substituted vinyl group, an optionally substituted nitrogen-containing saturated heterocyclic group or an optionally substituted heteroaryl group; and
   -Y-NH₂ is a group represented by the following formula (A) or a group represented by the following formula (B): 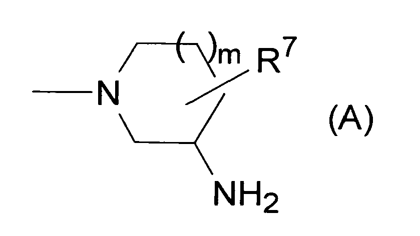 wherein m is 0, 1 or 2, and R⁷ is absent or one or two R⁷s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁷s, when taken together, represent methylene or ethylene and may bind to two carbon atoms constituting the ring, to form a new ring, or 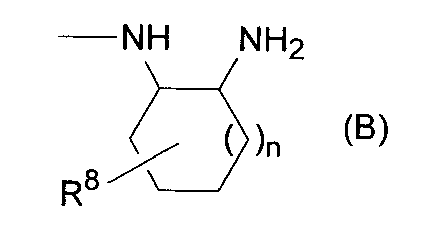 wherein n is 0, 1 or 2, and R⁸ is absent or one or two R⁸s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁸s, when taken together, represent methylene or ethylene and may bind to two carbon atoms constituting the ring, to form a new ring,
   a prodrug of said compound, or a pharmaceutically acceptable salt of said compound or prodrug.
[2] A compound, a prodrug thereof or a pharmaceutically acceptable salt of the compound or prodrug according to [1], wherein -Y-NH₂ is a group represented by the formula (A) and m is 1 or 2, or -Y-NH₂ is a group represented by the formula (B) and n is 1 or 2.
[3] A compound, a prodrug thereof or a pharmaceutically acceptable salt of the compound or prodrug according to [1] or [2], wherein R² and R³ are taken together to form an oxo group on the ring.
[4] A compound, a prodrug thereof or a pharmaceutically acceptable salt of the compound or prodrug according to [1] or [2], wherein R³ and R⁵ are taken together to form a double bond on the ring.
[5] A compound, a prodrug thereof or a pharmaceutically acceptable salt of the compound or prodrug according to [1] or [2], wherein R², R³, R⁴ and R⁵ form an optionally substituted benzene ring, an optionally substituted cycloalkene ring or an optionally substituted 5-or 6-membered heteroaromatic ring together with the adjacent carbon atoms.
[6] A compound, a prodrug thereof or a pharmaceutically acceptable salt of the compound or prodrug according to [4], wherein R² is a hydrogen atom, a cyano group, an optionally substituted alkyl group, a carboxyl group, an optionally substituted alkoxy group, an optionally substituted alkoxycarbonyl group, an optionally substituted cycloalkyloxycarbonyl group, an optionally substituted aryl group, an optionally substituted heteroaryl group, an optionally substituted aryloxy group, an optionally substituted aryloxycarbonyl group, an optionally substituted aralkyl group, an optionally substituted aralkyloxy group, an optionally substituted aroyl group, an optionally substituted alkylcarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in [1].
[7] A compound, a prodrug thereof or a pharmaceutically acceptable salt of the compound or prodrug according to [4], wherein R⁴ is a hydrogen atom or a methyl, ethyl or alkoxycarbonylmethyl group.
[8] A compound, a prodrug thereof or a pharmaceutically acceptable salt of the compound or prodrug according to [4], wherein R² is a hydrogen atom, a cyano group, an optionally substituted alkyl group, a carboxyl group, an optionally substituted alkoxy group, an optionally substituted alkoxycarbonyl group, an optionally substituted cycloalkyloxycarbonyl group, an optionally substituted aryl group, an optionally substituted heteroaryl group, an optionally substituted aryloxy group, an optionally substituted aryloxycarbonyl group, an optionally substituted aralkyl group, an optionally substituted aralkyloxy group, an optionally substituted aroyl group, an optionally substituted alkylcarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in [1]; and R⁴ is a hydrogen atom or a methyl, ethyl or alkoxycarbonylmethyl group.
[9] A compound, a prodrug thereof or a pharmaceutically acceptable salt of the compound or prodrug according to any one of [1] to [8], wherein R⁶ is a group represented by the following formula (C), (D) or (E): 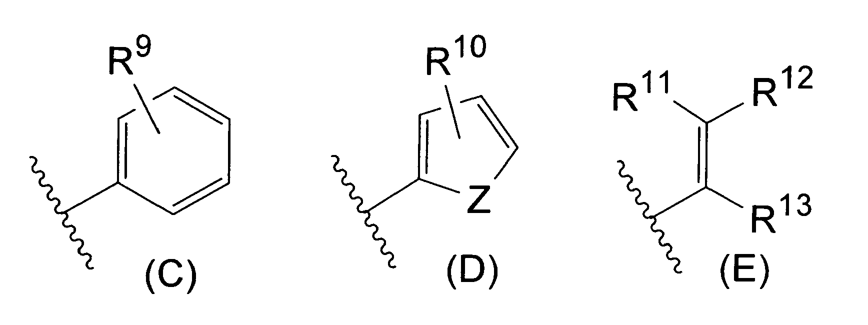 wherein Z is an oxygen atom, -S(O)p- or -N(R¹⁴)-,
   R⁹ is absent or one or two R⁹s are present and are independently a halogen atom, a hydroxyl group, a formyl group, a carboxyl group, a cyano group, an alkylthio group, an alkylsulfinyl group, an alkylsulfonyl group, an alkyl group, a haloalkyl group, a cycloalkyl group, an alkoxy group, a haloalkoxy group, an optionally substituted amino group, an optionally substituted carbamoyl group, an alkoxycarbonyl group, an optionally substituted alkylcarbonyl group, a cycloalkylcarbonyl group, an optionally substituted aryl group, or an optionally substituted heteroaryl group, or two R⁹s, when taken together, represent a C₁₋₃ alkylenedioxy group,
   R¹⁰ is absent or one or two R¹⁰s are present and are independently a halogen atom, a cyano group, an alkyl group, a haloalkyl group, a cycloalkyl group, an alkoxy group or a haloalkoxy group,
   R¹¹ is methyl, ethyl, a chlorine atom or a bromine atom,
   R¹² is a hydrogen atom, methyl, ethyl, a chlorine atom or a bromine atom,
   R¹³ is a hydrogen atom, methyl or ethyl,
   p is 0, 1 or 2, and
   R¹⁴ is a hydrogen atom or an alkyl group.
[10] A compound, a prodrug thereof or a pharmaceutically acceptable salt of the compound or prodrug according to [9], wherein R⁶ is the formula (C) or the formula (E).
[11] A compound, a prodrug thereof or a pharmaceutically acceptable salt of the compound or prodrug according to [10], wherein R⁶ is the formula (C), and R⁹ is absent or one or two R⁹s are present and are independently a halogen atom, a cyano group, an alkylthio group, an alkylsulfonyl group, a C₁₋₃ alkylenedioxy group, an alkyl group, a haloalkyl group, a cycloalkyl group, an alkoxy group, a haloalkoxy group, an alkoxycarbonyl group, an alkylcarbonyl group, a haloalkylcarbonyl group or a cycloalkylcarbonyl group.
[12] A compound, a prodrug thereof or a pharmaceutically acceptable salt of the compound or prodrug according to any one of [1] to [8], wherein R⁶ is the following formula (F): 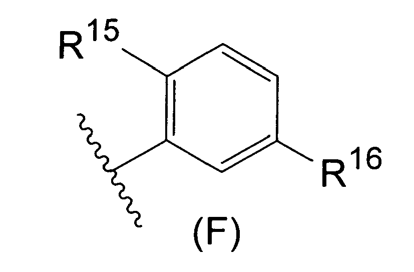 wherein R¹⁵ is a halogen atom, a cyano group, an alkyl group, a haloalkyl group, a cycloalkyl group, an alkoxy group or a haloalkoxy group, and R¹⁶ is a hydrogen atom or a fluorine atom.
[13] A compound, a prodrug thereof or a pharmaceutically acceptable salt of the compound or prodrug according to any one of [1] to [12], wherein R¹ is a hydrogen atom or an optionally substituted alkyl group of 1 to 3 carbon atoms whose substituent(s) is selected from fluorine atom, optionally substituted aroyl groups, carboxyl group, optionally substituted alkoxycarbonyl groups, optionally substituted aryl groups and optionally substituted aryloxy groups.
[14] A compound, a prodrug thereof or a pharmaceutically acceptable salt of the compound or prodrug according to any one of [1] to [12], wherein R¹ is a group represented by the formula: -Ra-Rb-Rc in which
   Ra is an alkylene chain,
   Rb is a single bond or a carbonyl group, and
   Rc is an optionally substituted alkyl group, an optionally substituted alkoxy group, an optionally substituted aryl group, an optionally substituted heteroaryl group, an optionally substituted aryloxy group or an optionally substituted heteroaryloxy group.
[15] A compound, a prodrug thereof or a pharmaceutically acceptable salt of the compound or prodrug according to any one of [1] to [12], wherein R¹ is a hydrogen atom, methyl or ethyl.
[16] A compound according to [1], which is represented by the formula (II): 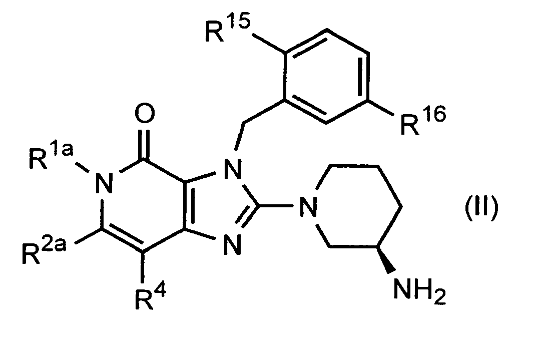 wherein R⁴ is as defined in [1]; R¹⁵ and R¹⁶ are as defined in [12]; R^{1a} is a hydrogen atom, methyl or the formula: -Ra-Rb-Rc wherein Ra, Rb and Rc are as defined in [14]; and R^{2a} is a cyano group, a carboxyl group, an oxazolyl group, an optionally substituted alkoxycarbonyl group, an optionally substituted cycloalkyloxycarbonyl group, a tetrahydrofuranyloxycarbonyl group, an optionally substituted aryloxycarbonyl group, a cinnamyloxycarbonyl group, or a group represented by the formula: -C(O)OCH(R¹⁸)OC(O)R¹⁹ wherein R¹⁸ and R¹⁹ are as defined in [1],
   a prodrug of said compound, or a pharmaceutically acceptable salt of said compound or prodrug.
[17] A compound according to [1], which is represented by the formula (III): 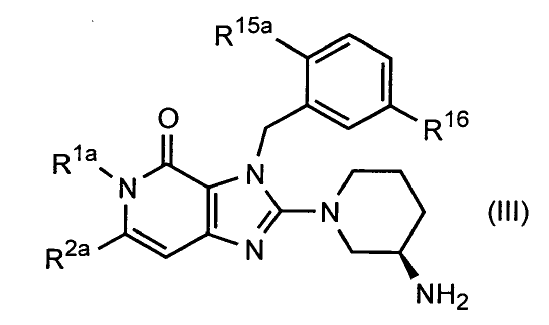 wherein R¹⁶ is as defined in [12]; R^{1a} and R^{2a} are as defined in [16]; and R^{15a} is a chlorine atom, a bromine atom, an iodine atom, a cyano group, methyl, difluoromethyl, trifluoromethyl, methoxy, fluoromethoxy, difluoromethoxy or trifluoromethoxy,
   a prodrug of said compound, or a pharmaceutically acceptable salt of said compound or prodrug.
[18] A compound, a prodrug thereof or a pharmaceutically acceptable salt of the compound or prodrug according to [17], wherein R^{1a} is a hydrogen atom.
[19] A compound, a prodrug thereof or a pharmaceutically acceptable salt of the compound or prodrug according to [17] or [18], wherein R^{2a} is a carboxyl group, an optionally substituted alkoxycarbonyl group, or a group represented by the formula: -
   C(O)OCH(R¹⁸)OC(O)R¹⁹ wherein R¹⁸ and R¹⁹ are as defined in [1].
[20] A compound according to [1], which is represented by the formula (IV): 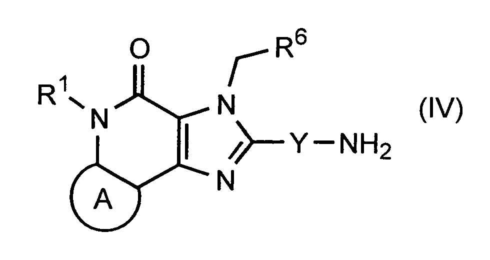 wherein R¹, R⁶ and Y are as defined in [1]; and the ring A is an optionally substituted benzene ring, an optionally substituted cycloalkene ring or an optionally substituted 5-or 6-membered heteroaromatic ring,
   a prodrug of said compound, or a pharmaceutically acceptable salt of said compound or prodrug.
[21] A compound according to [1], which is represented by the formula (V): 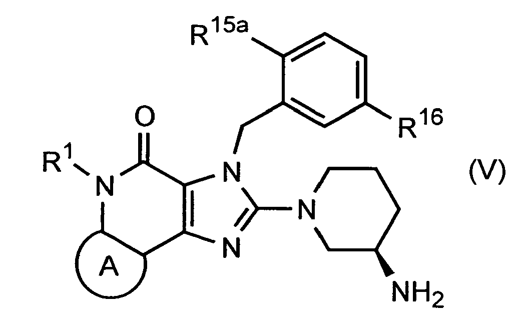 wherein R¹ is as defined in [1]; R¹⁶ is as defined in [12]; R^{15a} is as defined in [17]; and A is as defined in [20],
   a prodrug of said compound, or a pharmaceutically acceptable salt of said compound or prodrug.
[22] A compound, a prodrug thereof or a pharmaceutically acceptable salt of the compound or prodrug according to [21], wherein R¹ is a hydrogen atom or methyl.
[23] A compound according to [1], which is represented by the formula (VI): 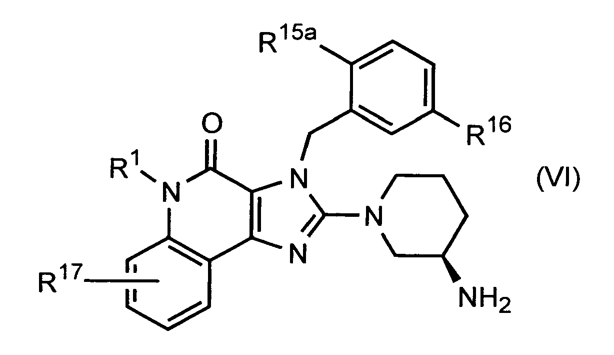 wherein R¹ is as defined in [1] ; R¹⁶ is as defined in [12]; R^{15a} is as defined in [17]; and R¹⁷ is absent or one to four R¹⁷ s are present and are independently a hydroxyl group, a halogen atom, a cyano group, a carboxyl group, an optionally substituted alkyl group, an optionally substituted cycloalkyl group, an optionally substituted cycloalkyloxy group, an optionally substituted alkenyl group, an optionally substituted carbamoyl group, an optionally substituted alkoxy group, an optionally substituted alkoxycarbonyl group, an optionally substituted aryloxycarbonyl group, an optionally substituted alkylcarbonyl group, an optionally substituted cycloalkyloxycarbonyl group, an optionally substituted aralkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in [1],
   a prodrug of said compound, or a pharmaceutically acceptable salt of said compound or prodrug.
[24] A compound, a prodrug thereof or a pharmaceutically acceptable salt of the compound or prodrug according to [23], wherein R¹ is a hydrogen atom, methyl or the formula: -Ra-Rb-Rc wherein Ra, Rb and Rc are as defined in [14].
[25] A compound, a prodrug thereof or a pharmaceutically acceptable salt of the compound or prodrug according to [23], wherein R¹ is methyl.
[26] A compound, a prodrug thereof or a pharmaceutically acceptable salt of the compound or prodrug according to [25], wherein R¹⁷ is a fluorine atom, a chlorine atom, a cyano group, a carboxyl group, acetyl, dimethylcarbamoyl, diethylcarbamoyl, methyl, ethyl, isopropyl, cyclopropyl, difluoromethyl, trifluoromethyl, methoxy, ethoxy, isopropoxy, difluoromethoxy, trifluoromethoxy, an alkoxyalkyl group optionally substituted by a halogen atom or a hydroxyl group, an optionally substituted alkoxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in [1].
[27] A compound, a prodrug thereof or a pharmaceutically acceptable salt of the compound or prodrug according to [25], wherein R¹⁷ is a fluorine atom, a cyano group, a carboxyl group, an alkoxymethyl group optionally substituted by a halogen atom, an optionally substituted alkoxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in [1].
[28] A dipeptidyl peptidase IV inhibitor comprising a compound, a prodrug thereof or a pharmaceutically acceptable salt of the compound or prodrug according to any one of [1] to [27] as an active ingredient.
[29] A pharmaceutical composition for the treatment of diabetes comprising a compound, a prodrug thereof or a pharmaceutically acceptable salt of the compound or prodrug according to any one of [1] to [27] as an active ingredient.
[30] Use of a compound, a prodrug thereof or a pharmaceutically acceptable salt of the compound or prodrug according to any one of [1] to [27] in the manufacture of a dipeptidyl peptidase IV inhibitor.
[31] Use of a compound, a prodrug thereof or a pharmaceutically acceptable salt of the compound or prodrug according to any one of [1] to [27] in the manufacture of a pharmaceutical composition for the treatment of diabetes.
[32] A method for treating diabetes comprising administering an effective amount of a compound, a prodrug thereof or a pharmaceutically acceptable salt of the compound or prodrug according to any one of [1] to [27] to a patient who needs treatment.

The present inventive compound has an excellent DPP-IV inhibitory activity and is useful as a therapeutic agent for diabetes.

### BEST MODE FOR CARRYING OUT THE INVENTION

The present invention is explained below in further detail.

In the present specification, the number of substituents of each group defined by the term "optionally substituted" or "substituted" is not particularly limited so long as the substitution is possible, and it is 1 or more. Unless otherwise specified, the explanation of each group applies also to the case where the group is a portion or the substituent of another group.

The "halogen atom" includes, for example, fluorine atom, chlorine atom, bromine atom and iodine atom.

The "alkyl group" includes, for example, linear or branched alkyl groups of 1 to 6 carbon atoms. Specific examples thereof are methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, etc. Preferable examples thereof are linear or branched alkyl groups of 1 to 4 carbon atoms. Specific examples of such groups are methyl, ethyl, propyl, isopropyl, butyl, tert-butyl, etc.

The "alkenyl group" includes, for example, alkenyl groups of 2 to 6 carbon atoms. Specific examples thereof are vinyl, propenyl, methylpropenyl, butenyl, methylbutenyl, etc.

The "alkynyl group" includes, for example, alkynyl groups of 2 to 6 carbon atoms. Specific examples thereof are ethynyl, 1-propynyl, 2-propynyl, butynyl, pentynyl, hexynyl, etc.

The "cycloalkyl group" includes, for example, cycloalkyl groups of 3 to 10 carbon atoms. Specific examples thereof are cyclopropyl, cyclobutyl, cyclopentyl, cyclohexyl, cycloheptyl, adamantyl, norbornyl, etc. Preferable examples thereof are cycloalkyl groups of 3 to 6 carbon atoms. Specific examples of such groups are cyclopropyl, cyclobutyl, cyclopentyl, cyclohexyl, etc.

The "aryl group" includes, for example, aryl groups of 6 to 10 carbon atoms. Specific examples thereof are phenyl, 1-naphthyl, 2-naphthyl, etc.

The "aralkyl group" includes, for example, groups formed by bonding of an aryl group to an alkylene chain. Specific examples thereof are benzyl, 2-phenylethyl, 1-naphthylmethyl, etc.

The "alkylene chain" includes, for example, alkylene chains of 1 to 3 carbon atoms. Specific examples thereof are methylene, ethylene, trimethylene, etc.

The "heteroaryl group" includes, for example, 5-to 10-membered monocyclic or polycyclic groups containing one or more (for example, 1 to 4) heteroatoms selected from nitrogen atom, sulfur atom and oxygen atom. Specific examples thereof are pyrrolyl, thienyl, benzothienyl, benzofuranyl, benzoxazolyl, benzothiazolyl, furyl, oxazolyl, thiazolyl, isoxazolyl, imidazolyl, pyrazolyl, pyridyl, pyrazyl, pyrimidyl, pyridazyl, quinolyl, isoquinolyl, triazolyl, triazinyl, tetrazolyl, indolyl, imidazo[1,2-a]pyridyl, dibenzofuranyl, benzimidazolyl, quinoxalyl, cinnolyl, quinazolyl, indazolyl, naphthyridyl, quinolinolyl, isoquinolinolyl, etc. Preferable examples thereof are 5-or 6-membered groups containing a heteroatom selected from nitrogen atom, sulfur atom and oxygen atom. Specific examples of such groups are pyridyl, thienyl, furyl, etc.

The heteroaryl portion of the "heteroarylalkyl group" includes the groups exemplified above as the heteroaryl group.

The "alkylcarbonyl group" includes, for example, alkylcarbonyl groups of 2 to 4 carbon atoms. Specific examples thereof are acetyl, propionyl, butyryl, etc.

The "cycloalkylcarbonyl group" includes, for example, cycloalkylcarbonyl groups of 4 to 11 carbon atoms. Specific examples thereof are cyclopropylcarbonyl, cyclobutylcarbonyl, cyclopentylcarbonyl, cyclohexylcarbonyl, adamantylcarbonyl, norbornylcarbonyl, etc. Preferable examples thereof are cycloalkylcarbonyl groups of 4 to 7 carbon atoms. Specific examples of such groups are cyclopropylcarbonyl, cyclobutylcarbonyl, cyclopentylcarbonyl, cyclohexylcarbonyl, etc

The "aroyl group" includes, for example, aroyl groups of 7 to 11 carbon atoms. Specific examples thereof are benzoyl, 1-naphthoyl, 2-naphthoyl, etc.

The heteroaryl portion of the "heteroarylcarbonyl group" includes the groups exemplified above as the heteroaryl group.

The "alkoxycarbonyl group" includes, for example, alkoxycarbonyl groups of 2 to 5 carbon atoms. Specific examples thereof are methoxycarbonyl, ethoxycarbonyl, propoxycarbonyl, 2-propoxycarbonyl, tert-butoxycarbonyl, etc.

The "aryloxycarbonyl group" includes, for example, aryloxycarbonyl groups of 7 to 11 carbon atoms. Specific examples thereof are phenyloxycarbonyl, 2-naphthyloxycarbonyl, 1-naphthyloxycarbonyl group, etc.

The "alkoxy group" includes, for example, alkoxy groups of 1 to 4 carbon atoms. Specific examples thereof are methoxy, ethoxy, propoxy, isopropoxy, butoxy, isobutoxy, sec-butoxy, tert-butoxy, etc.

The "cycloalkyloxy group" includes, for example, cycloalkyloxy groups of 3 to 10 carbon atoms. Specific examples thereof are cyclopropyloxy, cyclobutoxy, cyclopentyloxy, cyclohexyloxy, cycloheptyloxy, adamantyloxy, norbornyloxy, etc. Preferable examples thereof are cycloalkyloxy groups of 3 to 6 carbon atoms. Specific examples of such groups are cyclopropyloxy, cyclobutoxy, cyclopentyloxy, cyclohexyloxy, etc.

The cycloalkyloxy portion of the "cycloalkyloxycarbonyl group" includes the groups exemplified above as the cycloalkyloxy group.

The "aryloxy group" includes, for example, aryloxy groups of 6 to 10 carbon atoms. Specific examples thereof are phenoxy, 1-naphthyloxy, 2-naphthyloxy, etc.

The aralkyl portion of the "aralkyloxy group" includes the groups exemplified above as the aralkyl group. Specific examples thereof are benzyloxy, 2-phenylethyloxy, etc.

The aralkyl portion of the "aralkyloxycarbonyl group" includes the groups exemplified above as the aralkyl group.

The heteroaryl portion of the "heteroaryloxy group" includes the groups exemplified above as the heteroaryl group.

The "alkylthio group" includes, for example, alkylthio groups of 1 to 6 carbon atoms. Specific examples thereof are methylthio, ethylthio, propylthio, isopropylthio, butylthio, sec-butylthio, tert-butylthio, pentylthio, hexylthio, etc. Preferable examples thereof are alkylthio groups of 1 to 4 carbon atoms. Specific examples of such groups are methylthio, ethylthio, propylthio, isopropylthio, butylthio, sec-butylthio, tert-butylthio, etc.

The "alkylsulfinyl group" includes, for example, alkylsulfinyl groups of 1 to 6 carbon atoms. Specific examples thereof are methylsulfinyl, ethylsulfinyl, propylsulfinyl, isopropylsulfinyl, butylsulfinyl, pentylsulfinyl, hexylsulfinyl, etc. Preferable examples thereof are alkylsulfinyl groups of 1 to 4 carbon atoms. Specific examples of such groups are methylsulfinyl, ethylsulfinyl, propylsulfinyl, isopropylsulfinyl, butylsulfinyl, etc.

The "alkylsulfonyl group" includes, for example, alkylsulfonyl groups of 1 to 6 carbon atoms. Specific examples thereof are methylsulfonyl, ethylsulfonyl, propylsulfonyl, isopropylsulfonyl, butylsulfonyl, pentylsulfonyl, hexylsulfonyl, etc. Preferable examples thereof are alkylsulfonyl groups of 1 to 4 carbon atoms. Specific examples of such groups are methylsulfonyl, ethylsulfonyl, propylsulfonyl, isopropylsulfonyl, butylsulfonyl, etc.

The "arylthio group" includes, for example, arylthio groups of 6 to 10 carbon atoms. Specific examples thereof are phenylthio, 1-naphthylthio, 2-naphthylthio, etc.

The "arylsulfinyl group" includes, for example, arylsulfinyl groups of 6 to 10 carbon atoms. Specific examples thereof are phenylsulfinyl, 1-naphthylsulfinyl, 2-naphthylsulfinyl, etc.

The "arylsulfonyl group" includes, for example, arylsulfonyl groups of 6 to 10 carbon atoms. Specific examples thereof are phenylsulfonyl, tosyl, 1-naphthylsulfonyl, 2-naphthylsulfonyl, etc.

The "nitrogen-containing saturated heterocyclic group" includes, for example, 5-or 6-membered saturated heterocyclic groups which have one or two nitrogen atoms and may further have an oxygen atom or a sulfur atom. Specific examples thereof are pyrrolidinyl, imidazolidinyl, piperidinyl, morpholinyl, thiomorpholinyl, dioxothiomorpholinyl, hexamethyleniminyl, oxazolidinyl, thiazolidinyl, imidazolidinyl, oxoimidazolidinyl, dioxoimidazolidinyl, oxooxazolidinyl, dioxooxazolidinyl, dioxothiazolidinyl, tetrahydrofuranyl, tetrahydropyridinyl, etc.

The substituent(s) of the "optionally substituted alkyl group" includes, for example, (1) halogen atoms, (2) hydroxyl group, (3) cyano group, (4) carboxyl group, (5) optionally substituted cycloalkyl groups, (6) optionally substituted aryl groups, (7) optionally substituted heteroaryl groups, (8) optionally substituted aroyl groups, (9) optionally substituted heteroarylcarbonyl groups, (10) optionally substituted arylaminocarbonyl groups, (11) optionally substituted heteroarylaminocarbonyl groups, (12) optionally substituted aryloxy groups, (13) optionally substituted arylsulfonyl groups, (14) optionally substituted aralkylsulfonyl groups, (15) optionally substituted alkoxy groups, (16) optionally substituted cycloalkyloxy groups, (17) optionally substituted alkoxycarbonyl groups, (18) optionally substituted aryloxycarbonyl groups, (19) optionally substituted amino groups, (20) optionally substituted carbamoyl groups, (21) alkylsulfonyl groups, (22) optionally substituted alkylcarbonyl groups, (23) cycloalkyloxycarbonyl groups, (24) tetrahydrofuranyloxycarbonyl group, and (25) tetrahydrofuranyl group.

Here, the above items (1) to (25) are explained below.

The substituents of the "optionally substituted cycloalkyl groups" of the above item (5) include, for example, alkyl groups, aralkyl groups, alkoxy groups, alkoxycarbonyl groups and fluorine atom.

The substituents of the "optionally substituted aryl groups" of the above item (6) include those exemplified hereinafter as the substituent(s) of the "optionally substituted aryl group".

The substituents of the "optionally substituted heteroaryl groups" of the above item (7) include, for example,
(a) hydroxyl group,
(b) halogen atoms,
(c) alkyl groups,
(d) alkyl groups substituted by a halogen atom(s) or an alkoxy group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethoxy, ethoxymethoxy, methoxyethoxy, ethoxyethoxy, methoxypropoxy and ethoxypropoxy),
(e) alkoxy groups,
(f) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(g) cyano group,
(h) carboxyl group,
(i) alkoxycarbonyl groups,
(j) carbamoyl groups which may be substituted by an alkyl group(s) (for example, carbamoyl, methylcarbamoyl, dimethylcarbamoyl, ethylcarbamoyl and diethylcarbamoyl),
(k) aryl groups,
and (l) amino group.

The substituents of the "optionally substituted aroyl groups" of the above item (8) include those exemplified as the substituents of the "optionally substituted aryl groups" of the above item (6).

The substituents of the "optionally substituted heteroarylcarbonyl groups" of the above item (9) include those exemplified as the substituents of the "optionally substituted heteroaryl groups" of the above item (7).

The substituents of the "optionally substituted arylaminocarbonyl groups" of the above item (10) include those exemplified as the substituents of the "optionally substituted aryl groups" of the above item (6).

The substituents of the "optionally substituted heteroarylaminocarbonyl groups" of the above item (11) include those exemplified as the substituents of the "optionally substituted heteroaryl groups" of the above item (7).

The substituents of the "optionally substituted aryloxy groups" of the above item (12) and the "optionally substituted arylsulfonyl groups" of the above item (13) include those exemplified as the substituents of the "optionally substituted aryl groups" of the above item (6).

The aralkyl portion of each of the "optionally substituted aralkylsulfonyl groups" of the above item (14) includes the groups exemplified above as the aralkyl group.

The substituents of the "optionally substituted aralkylsulfonyl groups" include those exemplified as the substituents of the "optionally substituted aryl groups" of the above item (6).

The substituents of the "optionally substituted alkoxy groups" of the above item (15) include, for example,
(a) hydroxyl group,
(b) carboxyl group,
(c) alkoxy groups,
(d) alkylcarbonyloxy groups (for example, methylcarbonyloxy, ethylcarbonyloxy, propylcarbonyloxy, isopropylcarbonyloxy, butylcarbonyloxy and tert-butylcarbonyloxy),
(e) alkoxycarbonyl groups,
(f) amino groups which may be substituted by an alkyl group(s) (for example, amino, dimethylamino and diethylamino),
(g) carbamoyl groups substituted by an alkyl group(s),
(h) sulfamoyl groups substituted by an alkyl group(s),
(i) ureido groups substituted by an alkyl group(s),
(j) alkoxycarbonyloxy groups (for example, methoxy-carbonyloxy, ethoxycarbonyloxy, 2-propoxycarbonyloxy and tert-butoxycarbonyloxy),
(k) cycloalkyloxycarbonyloxy groups (for example, cyclopentyloxycarbonyloxy, cyclohexyloxycarbonyloxy and cycloheptyloxycarbonyloxy),
(l) phenyl groups which may be substituted by a halogen atom or an alkoxy group (for example, phenyl, 2-fluorophenyl, 3-fluorophenyl, 4-fluorophenyl, 2-chlorophenyl, 3-chlorophenyl, 4-chlorophenyl, 2-methoxyphenyl, 3-methoxyphenyl, 4-methoxyphenyl, 2-ethoxyphenyl, 3-ethoxyphenyl, 4-ethoxyphenyl, 2-isopropoxyphenyl and 3-isopropoxyphenyl),
(m) 5-methyl-2-oxo-1,3-dioxolen-4-yl,
(n) 5-oxo-2-tetrahydrofuranyl,
(o) 1,3-dihydro-3-oxo-1-isobenzofuranyl,
(p) tetrahydrofuranyl,
(q) nitrogen-containing saturated heterocyclic groups,
(r) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(s) cycloalkyl groups,
(t) cycloalkyl groups substituted by a halogen atom or an alkoxy group (for example, 2-fluorocyclopropyl, 2-methoxycyclopropyl, 2-fluorocyclobutyl, 3-fluorocyclobutyl and 3-methoxycyclobutyl), and
(u) halogen atoms.

The substituents of the "optionally substituted cycloalkyloxy groups" of the above item (16) and the "optionally substituted alkoxycarbonyl groups" of the above item (17) include those exemplified as the substituents of the "optionally substituted alkoxy groups" of the above item (15).

The substituents of the "optionally substituted aryloxycarbonyl groups" of the above item (18) include those exemplified as the substituents of the "optionally substituted aryl groups" of the above item (6).

The substituents of the "optionally substituted amino groups" of the above item (19) include, for example,
(a) alkyl groups,
(b) alkylcarbonyl groups,
(c) aroyl groups,
(d) alkylsulfonyl groups,
(e) arylsulfonyl groups,
(f) optionally substituted aryl groups (whose substituent(s) includes, for example, halogen atoms, alkyl groups and alkoxy groups),
(g) alkoxycarbonylmethyl groups (the carbon atom of the methyl portion may be substituted by one or two alkyl groups, and the two alkyl groups on the carbon atom of the methyl portion may bind to each other to form cyclopropyl, cyclobutyl or cyclopentyl together with the carbon atom of the methyl portion),
and (h) aralkyl groups.

As the optionally substituted amino groups, (i) imides are also exemplified.

The substituents of the "optionally substituted carbamoyl groups" of the above item (20) include, for example, alkyl groups and cycloalkyl groups. The two substituents of the carbamoyl group may bind to each other to form an aliphatic heterocyclic ring which may contain carbon, nitrogen or oxygen, such as pyrrolidine (which may be substituted by a hydroxyl group), piperidine, morpholine, thiomorpholine, thiomorpholine oxide, thiomorpholine dioxide, piperazine (whose nitrogen atom may be substituted by methyl or ethyl), or the like.

Specific examples of the "optionally substituted carbamoyl groups" are carbamoyl, methylcarbamoyl, dimethylcarbamoyl, ethylcarbamoyl, diethylcarbamoyl, ethylmethylcarbamoyl, methylpropylcarbamoyl, cyclopropylcarbamoyl, cyclopropylmethylcarbamoyl, pyrrolidinocarbonyl, piperidinocarbonyl, morpholinocarbonyl, etc.

The substituents of the "optionally substituted alkylcarbonyl groups" of the above item (22) include, for example,
(a) halogen atoms,
(b) alkoxy groups,
(c) cycloalkyl groups,
(d) alkoxycarbonyl groups,
(e) optionally substituted aryl groups (whose substituent(s) include, for example, halogen atoms, alkyl groups, alkoxy groups and alkoxycarbonyl groups), and (f) hydroxyl group.

The substituent(s) of each of the "optionally substituted alkylthio group", "optionally substituted alkylsulfinyl group" and "optionally substituted alkylsulfonyl group" includes those exemplified as the substituent(s) of the above-mentioned "optionally substituted alkyl group".

The substituent(s) of the "optionally substituted alkenyl group" or the "optionally substituted alkynyl group" includes
(1) hydroxyl group,
(2) halogen atoms,
(3) alkyl groups,
(4) alkyl groups substituted by a halogen atom(s) or an alkoxy group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethyl, ethoxymethyl, methoxyethyl, ethoxyethyl, methoxypropyl and ethoxypropyl),
(5) alkoxy groups,
(6) alkoxy groups substituted by a halogen atom(s) or an alkoxy group (for example, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
(7) phenyl groups or aroyl groups, which may be substituted by the following (aa), (bb) or (cc):
   (aa) an alkoxy group(s) which may be substituted by a halogen atom(s) or an alkoxy group (for example, methoxy, ethoxy, propoxy, isopropoxy, butoxy, isobutoxy, sec-butoxy, tert-butoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
   (bb) an alkyl group(s) which may be substituted by a halogen atom(s) (for example, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl and 1-(difluoromethyl)-2,2-difluoroethyl), or
   (cc) a halogen atom(s),
(8) cyano group,
(9) carboxyl group,
(10) optionally substituted alkoxycarbonyl groups (whose substituent(s) includes those exemplified as the substituents of (15) the "optionally substituted alkoxy groups" as the substituent(s) of the above-mentioned "optionally substituted alkyl group"),
(11) carbamoyl groups which may be substituted by an alkyl group(s) (for example, carbamoyl, methylcarbamoyl, dimethylcarbamoyl, ethylcarbamoyl and diethylcarbamoyl),
(12) alkylsulfonyl groups,
and (13) phenyloxy group.

The substituent(s) of the "optionally substituted vinyl group" includes, for example, halogen atoms and alkyl groups.

Specific examples of the substituted vinyl groups are 1-propylene, 2-methyl-1-propylene, 2-chloro-1-propylene, etc.

The substituent(s) of the "optionally substituted ethynyl group" includes, for example, alkyl groups and cycloalkyl groups.

Specific examples of the substituted ethynyl groups are ethylidyne, propylidyne, 2-cyclopropyl-1-ethylidyne, etc.

The substituent(s) of the "optionally substituted cycloalkyl group" includes those exemplified as the substituents of (5) the "optionally substituted cycloalkyl groups" as the substituent(s) of the above-mentioned "optionally substituted alkyl group".

The substituent(s) of the "optionally substituted aryl group" includes, for example,
(1) hydroxyl group,
(2) halogen atoms,
(3) alkyl groups,
(4) alkyl groups substituted by a halogen atom(s), an alkoxy group or a cycloalkyl group (for example, fluoromethyl, difluoromethyl, trifluoromethyl, 2,2-difluoroethyl, 2,2,2-trifluoroethyl, perfluoroethyl, 2-fluoro-1-(fluoromethyl)ethyl, 1-(difluoromethyl)-2,2-difluoroethyl, methoxymethyl, ethoxymethyl, methoxyethyl, ethoxyethyl, methoxypropyl and ethoxypropyl),
(5) phenyl groups which may be substituted by the following (aa), (bb) or (cc):
   (aa) an alkoxy group(s) which may be substituted by a halogen atom(s) or an alkoxy group (for example, methoxy, ethoxy, propoxy, isopropoxy, butoxy, isobutoxy, sec-butoxy, tert-butoxy, fluoromethoxy, difluoromethoxy, trifluoro-methoxy, 2,2-difluoroethoxy, 2,2,2-trifluoroethoxy, perfluoroethoxy, 2-fluoro-1-(fluoromethyl)ethoxy, 1-(difluoromethyl)-2,2-difluoroethoxy, methoxymethoxy, ethoxymethoxy, methoxyethoxy, ethoxyethoxy, methoxypropoxy and ethoxypropoxy),
   (bb) an alkyl group(s) which may be substituted by a halogen atom(s) (for example, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl and 1-(difluoromethyl)-2,2-difluoroethyl), or
   (cc) a halogen atom(s),
(6) cyano group,
(7) carboxyl group,
(8) alkoxycarbonyl groups which may be substituted by a halogen atom(s) (for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, fluoromethoxycarbonyl, difluoromethoxycarbonyl, 2,2-difluoroethoxycarbonyl, 2,2,2-trifluoroethoxycarbonyl, methoxycarbonyl and ethoxycarbonyl),
(9) carbamoyl groups which may be substituted by an alkyl group(s) (for example, carbamoyl, methylcarbamoyl, dimethylcarbamoyl, ethylcarbamoyl and diethylcarbamoyl),
(10) alkylsulfonyl groups,
(11) C₁₋₃ alkylenedioxy groups,
(12) formyl group,
(13) optionally substituted phenyloxy groups (whose substituent(s) includes, for example, halogen atoms, alkyl groups and alkoxy groups),
(14) nitrogen-containing saturated heterocyclic groups (for example, pyrrolidinyl, piperidinyl, morpholinyl and piperazinyl (whose nitrogen atoms may be substituted, for example, by methyl, ethyl or propyl)),
(15) cycloalkyloxy groups which may be substituted by a hydroxyl group, an oxo group, a carboxyl group, a carboxymethyl group, an alkoxycarbonyl group, an alkoxycarbonylalkyl group (e.g. methoxycarbonylmethyl, ethoxycarbonylmethyl or isopropoxycarbonylmethyl), an alkyl group, a fluoroalkyl group (e.g. fluoromethyl, difluoromethyl, trifluoromethyl, 2,2-difluoroethyl, 2,2,2-trifluoroethyl or perfluoroethyl), an alkoxyalkyl group (e.g. methoxymethyl, ethoxymethyl or isopropoxymethyl), a cycloalkyloxyalkyl group (e.g. cyclopropyloxymethyl, cyclopropyloxyethyl or cyclobutoxy), an alkoxy group, a cycloalkyloxy group or a halogen atom(s) (for example, 3-carboxycyclobutoxy, 3-methoxycarbonylcyclobutoxy, 3-ethoxycarbonylbutoxy, 2-methylcyclopropyloxy, 2-fluorocyclopropyloxy, 3-methoxycyclobutoxy, 3-fluorocyclobutoxy, 3,3-difluorocyclobutoxy and 3-(2-fluoroethyl)cyclobutoxy),
(16) alkoxy groups which may be substituted by a hydroxyl group, an oxo group, a carboxyl group, an alkoxycarbonyl group, a cycloalkyl group, an alkoxy group, a cycloalkyloxy group, an optionally substituted oxygen-containing heterocyclic group (e.g. a 5-or 6-membered saturated heterocyclic group having an oxygen atom(s), specific examples of which are tetrahydrofuranyl, tetrahydropyranyl, etc.; its substituent(s) includes, for example, halogen atoms, oxo group and alkoxy groups), or a halogen atom(s) (for example, methoxy, ethoxy, propoxy, isopropoxy, butoxy, isobutoxy, sec-butoxy, tert-butoxy, 2-hydroxyethoxy, carboxymethoxy, methoxycarbonylmethoxy, ethoxycarbonylmethoxy, tert-butoxycarbonylmethoxy, cyclopropylmethoxy, cyclobutylmethoxy, methoxymethoxy, ethoxymethoxy, methoxyethoxy, ethoxyethoxy, isopropoxymethoxy, cyclopropyloxymethoxy, cyclobutoxymethoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy and 1-(difluoromethyl)-2,2-difluoroethoxy),
(17) difluoromethylenedioxy,
(18) alkenyl groups which may be substituted by a halogen atom(s) (for example, vinyl, propenyl, methylpropenyl, butenyl and methylbutenyl),
(19) amino groups which may be substituted by an alkyl group(s) (for example, amino, methylamino, ethylamino, propylamino, dimethylamino, methylethylamino and diethylamino),
(20) optionally substituted alkylcarbonyl groups (whose substituent(s) includes, for example, halogen atoms, alkoxy groups and cycloalkyl groups),
(21) alkylcarbonyloxy groups (for example, methylcarbonyloxy, ethylcarbonyloxy and isopropylcarbonyloxy),
(22) cycloalkyl groups which may be substituted by a fluorine atom (for example, cyclopropyl, cyclobutyl, cyclopentyl, 2-fluorocyclopropyl, 2-fluorocyclobutyl, 3-fluorocyclobutylcyclobutyl, adamantyl and norbornyl),
(23) cycloalkylcarbonyl groups which may be substituted by a fluorine atom (for example, cyclopropylcarbonyl, 2-fluorocyclopropylcarbonyl, cyclobutylcarbonyl and cyclopentylcarbonyl),
(24) alkylthio groups,
(25) alkylsulfinyl groups,
(26) optionally substituted heteroaryl groups (whose substituent(s) includes, for example, halogen atoms, alkyl groups, alkoxy groups, haloalkyl groups and haloalkoxy groups),
(27) groups represented by the following formulas (T1) to (T16): 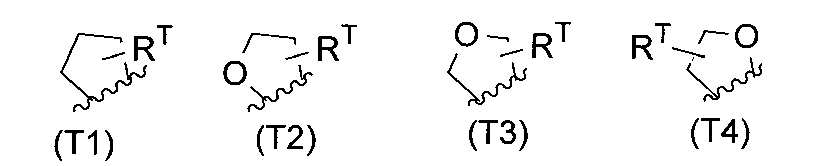 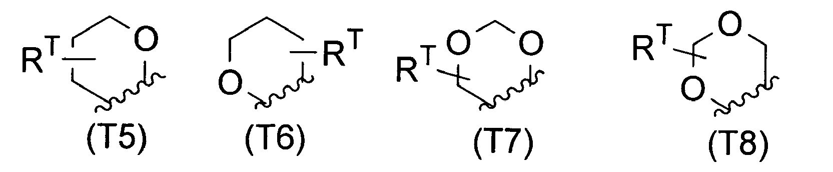 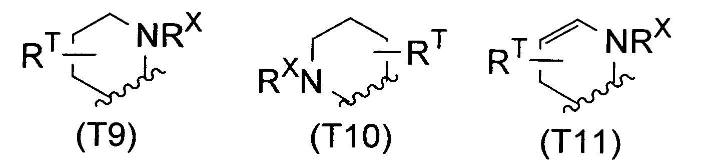 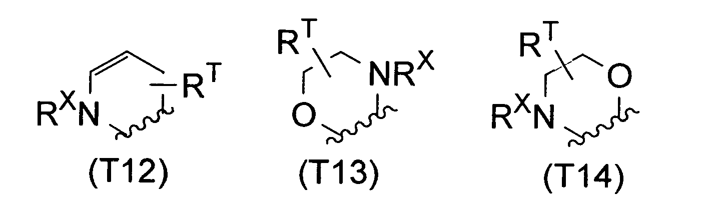 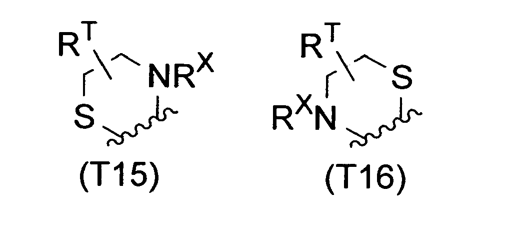 wherein R^{T} is absent or one or more R^{T}s are present and are independently a halogen atom, a hydroxyl group, an oxo group, a carboxyl group, an optionally substituted alkyl group (whose substituent(s) includes, for example, halogen atoms and alkoxy groups), an optionally substituted alkoxycarbonyl group (whose substituent(s) includes, for example, halogen atoms and alkoxy groups), an optionally substituted alkoxy group (whose substituent(s) includes, for example, halogen atoms and alkoxy groups), an optionally substituted carbamoyl group (whose substituent(s) includes, for example, alkyl groups), or a saturated heterocyclic group oxycarbonyl group (the saturated heterocyclic group includes, for example, 5-or 6-membered saturated heterocyclic groups having one or two oxygen atoms, nitrogen atoms and/or sulfur atoms, specific examples of which are tetrahydrofuranyl, tetrahydropyranyl, dihydrofuranyl, tetrahydrothiopyranyl, tetrahydrodioxothiopyranyl, pyrrolidinyl, piperidyl, piperazyl, imidazolidinyl, oxazolidinyl, thiazolidinyl, etc.), or two R^{T}_{S}, when taken together, represent methylene, ethylene, trimethylene, tetramethylene or butenylene and may bind to one or more carbon atoms constituting the ring, to form a new ring; and R^{X} is a hydrogen atom or an alkyl group, and (28) aroyl groups.

The substituent(s) of each of the "optionally substituted heteroaryl group", "optionally substituted aralkyl group", "optionally substituted heteroarylalkyl group", "optionally substituted aroyl group", "optionally substituted heteroarylcarbonyl group", "optionally substituted aryloxycarbonyl group", "optionally substituted aryloxy group", "optionally substituted aralkyloxy group", "optionally substituted aralkyloxycarbonyl group", "optionally substituted heteroaryloxy group", "optionally substituted arylthio group", "optionally substituted arylsulfinyl group" and "optionally substituted arylsulfonyl group" includes those exemplified as the substituent(s) of the above-mentioned "optionally substituted aryl group".

The substituent(s) of the "optionally substituted alkylcarbonyl group" includes those exemplified as the substituents of (22) the "optionally substituted alkylcarbonyl groups" as the substituent(s) of the above-mentioned "optionally substituted alkyl group".

The substituent(s) of the "optionally substituted cycloalkylcarbonyl group" includes, for example, halogen atoms and alkoxy groups.

The substituent(s) of each of the "optionally substituted alkoxy group" and the "optionally substituted alkoxycarbonyl group" includes those exemplified as the substituents of (15) the "optionally substituted alkoxy groups" as the substituent(s) of the above-mentioned "optionally substituted alkyl group".

The substituent(s) of each of the "optionally substituted cycloalkyloxy group" and the "optionally substituted cycloalkyloxycarbonyl group" includes those exemplified as the substituents of (16) the "optionally substituted cycloalkyloxy groups" as the substituent(s) of the above-mentioned "optionally substituted alkyl group".

The substituent(s) of the "optionally substituted amino group" includes those exemplified as the substituents of (19) the "optionally substituted amino groups" as the substituent(s) of the above-mentioned "optionally substituted alkyl group".

The substituent(s) of the "optionally substituted carbamoyl group" includes, for example,
(1) alkyl groups,
(2) cycloalkyl groups,
(3) aryl groups which may be substituted by the following (aa), (bb), (cc) or (dd):
   (aa) a halogen atom(s),
   (bb) an alkoxy group(s) which may be substituted by a halogen atom(s) (for example, methoxy, ethoxy, propoxy, isopropoxy, butoxy, isobutoxy, sec-butoxy, tert-butoxy, fluoromethoxy, difluoromethoxy, trifluoromethoxy, 2,2-difluoroethoxy, 2,2,2-trifluoroethoxy, perfluoroethoxy, 2-fluoro-1-(fluoromethyl)ethoxy and 1-(difluoromethyl)-2,2-difluoroethoxy),
   (cc) an alkyl group(s) which may be substituted by a halogen atom(s) (for example, methyl, ethyl, propyl, isopropyl, butyl, methyl, ethyl, propyl, isopropyl, butyl, fluoromethyl, difluoromethyl, trifluoromethyl, 2,2-difluoroethyl, 2,2,2-trifluoroethyl, perfluoroethyl, 2-fluoro-1-(fluoromethyl)ethyl and 1-(difluoromethyl)-2,2-difluoroethyl), or
   (dd) a C₁₋₃ alkylenedioxy group,
(4) alkylsulfonyl groups,
(5) cycloalkylsulfonyl groups,
(6) optionally substituted arylsulfonyl groups (whose substituent(s) includes, for example, halogen atoms, alkyl groups, haloalkyl groups, alkoxy groups and haloalkoxy groups),
(7) alkylcarbonyl groups,
(8) alkoxycarbonyl groups,
and (9) optionally substituted aroyl groups (whose substituent(s) includes, for example, halogen atoms, alkyl groups, haloalkyl groups, alkoxy groups, haloalkoxy groups, alkoxycarbonyl groups and C₁₋₃ alkylenedioxy groups).

Specific examples of the "optionally substituted carbamoyl group" are carbamoyl, methylcarbamoyl, dimethylcarbamoyl, ethylcarbamoyl, diethylcarbamoyl, ethylmethylcarbamoyl, phenylcarbamoyl, phenylmethylcarbamoyl, etc.

The two substituents of the carbamoyl group may bind to each other to form an aliphatic heterocyclic ring which may contain carbon, nitrogen, oxygen or sulfur, such as pyrrolidine, piperidine, morpholine, thiomorpholine, thiomorpholine oxide, thiomorpholine dioxide, piperazine (whose nitrogen atom may be substituted, for example, by methyl, ethyl or propyl), or the like. Specific examples of such a carbamoyl group are pyrrolidinocarbamoyl, piperidinocarbamoyl, morpholinocarbamoyl, etc.

The substituent(s) of the "optionally substituted nitrogen-containing saturated heterocyclic group" includes, for example,
(1) halogen atoms,
(2) alkyl groups,
(3) alkyl groups substituted by a halogen atom(s) or an alkoxy group (for example, fluoromethyl, difluoromethyl, trifluoromethyl, 2-fluoroethyl, 2,2-difluoroethyl, perfluoroethyl and methoxyethyl),
(4) alkoxy groups,
(5) alkoxy groups substituted by a halogen atom(s) or an alkoxy group (for example, fluoromethoxy, difluoromethoxy, trifluoromethoxy, methoxymethoxy, ethoxymethoxy, methoxyethoxy, ethoxyethoxy, methoxypropoxy and ethoxypropoxy),
(6) cyano group,
and (7) oxo group.

When two R⁷s or R⁸s are present, they may be present either on one and the same carbon atom or on different carbon atoms, respectively.

The phrase "two R⁷s or R⁸s, when taken together, represent methylene or ethylene and bind to one or more carbon atoms constituting the ring, to form a new ring" means that they form a spiro ring or a bicyclo ring through one and the same carbon atom or different carbon atoms.

The phrase "two R^{T}s, when taken together, represent methylene, ethylene, trimethylene, tetramethylene or butenylene and bind to one or two carbon atoms constituting the ring, to form a new ring" means that they form a spiro ring or a bicyclo ring through one and the same carbon atom or different carbon atoms.

The "haloalkoxy group" includes, for example, alkoxy groups of 1 to 4 carbon atoms substituted by a halogen atom(s). Specific examples thereof are fluoromethoxy, difluoromethoxy, trifluoromethoxy, etc.

The "haloalkyl group" includes, for example, alkyl groups of 1 to 4 carbon atoms substituted by a halogen atom(s). Specific examples thereof are fluoromethyl, difluoromethyl, trifluoromethyl, 2-fluoroethyl, perfluoroethyl, etc.

The "C₁₋₃ alkylenedioxy group" includes, for example, methylenedioxy, ethylenedioxy and trimethylenedioxy.

The "substituted alkyl group" for R¹⁹ includes, for example, alkyl groups of 1 to 3 carbon atoms substituted by a cycloalkyl group of 3 to 7 carbon atoms (for example, cyclopentyl, cyclohexyl or cycloheptyl) or an optionally substituted aryl group (for example, phenyl). Specific examples thereof are benzyl, p-chlorobenzyl, p-methoxybenzyl, p-fluorobenzyl, cyclopentylmethyl, cyclohexylmethyl, etc.

The "substituted alkenyl group" for R¹⁹ includes, for example, alkenyl groups of 2 or 3 carbon atoms substituted by a cycloalkyl group of 5 to 7 carbon atoms (for example, cyclopentyl, cyclohexyl or cycloheptyl) or an aryl group (for example, phenyl). Examples thereof are vinyl, propenyl, allyl, isopropenyl and the like, which are substituted by phenyl, cyclopropyl, cyclobutyl, cyclopentyl, cyclohexyl or the like.

The "alkenyloxy group" for R¹⁹ includes, for example, linear or branched alkenyloxy groups of 2 to 8 carbon atoms. Specific examples thereof are allyloxy, isobutenyloxy, etc.

The "substituted alkoxy group" for R¹⁹ includes, for example, alkoxy groups of 1 to 3 carbon atoms substituted by a cycloalkyl group of 3 to 7 carbon atoms (for example, cyclopropyl, cyclopentyl, cyclohexyl or cycloheptyl) or an optionally substituted aryl group (for example, phenyl). Specific examples thereof are benzyloxy, phenethyloxy, cyclopropylmethyloxy, cyclopropylethyloxy, cyclopentylmethyloxy, etc.

The "substituted alkenyloxy group" for R¹⁹ includes, for example, alkenyloxy groups of 2 or 3 carbon atoms substituted by a cycloalkyl group of 3 to 7 carbon atoms (for example, cyclopropyl, cyclopentyl, cyclohexyl or cycloheptyl) or an optionally substituted aryl group (for example, phenyl). Examples thereof are vinyloxy, propenyloxy, allyloxy and isopropenyloxy which are substituted by phenyl, cyclopropyl, cyclobutyl, cyclopentyl, cyclohexyl or the like.

Specific examples of the "optionally substituted aryloxy group" for R¹⁹ are phenoxy, p-nitrophenoxy, p-methoxyphenoxy, p-fluorophenoxy, naphthoxy, etc.

Specific examples of each of the "substituted alkoxycarbonyl group" and the group represented by the formula: -C(O)OCH(R¹⁸)OC(O)R¹⁹ (wherein R¹⁸ and R¹⁹ are as defined above) are pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethyloxycarbonyl, propyloxymethoxycarbonyl, n-butoxymethoxycarbonyl, isobutoxymethoxycarbonyl, 1-(ethoxycarbonyloxy)ethoxycarbonyl, 1-(acetyloxy)ethoxycarbonyl, 1-(isobutoxy)ethoxycarbonyl, cyclohexylcarbonyloxymethoxycarbonyl, cyclopentylcarbonyloxymethoxycarbonyl, etc.

The substituent(s) of each of the "optionally substituted alkyl group" and the "optionally substituted alkoxy group" for Rc includes, for example, halogen atoms, alkoxy groups and cycloalkyl groups.

The substituent(s) of each of the "optionally substituted heteroaryl group" and the "optionally substituted heteroaryloxy group" for Rc includes those exemplified as the substituents of (7) the "optionally substituted heteroaryl groups" as the substituent(s) of the above-mentioned "optionally substituted alkyl group".

The phrase "R², R³, R⁴ and R⁵ may form an optionally substituted benzene ring, an optionally substituted cycloalkene ring or an optionally substituted 5-or 6-membered heteroaromatic ring together with the adjacent carbon atoms" means that the formula (I): 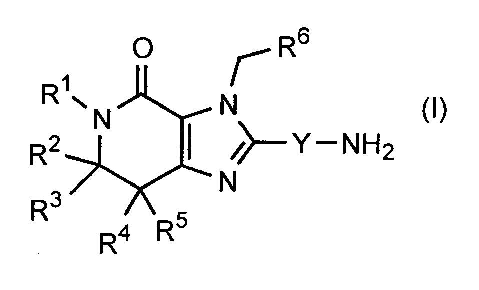 is shown as the formula (IV): 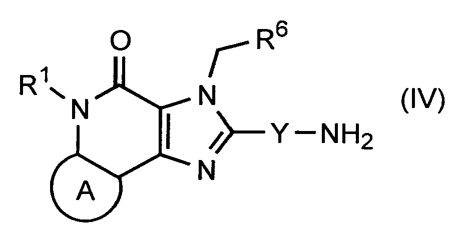 wherein the ring A is an optionally substituted benzene ring, an optionally substituted cycloalkene ring or an optionally substituted 5-or 6-membered heteroaromatic ring. The substituent of the ring A may be a carboxyl group or a group for obtaining a prodrug of a compound of the formula (IV) in which the substituent of the ring A is a carboxyl group. The substituent of the ring A may be one which is biologically or chemically convertible in a living body.

Specifically, the phrase "the ring A forms a benzene ring" means that the formula (IV) is shown as the formula (IVa):

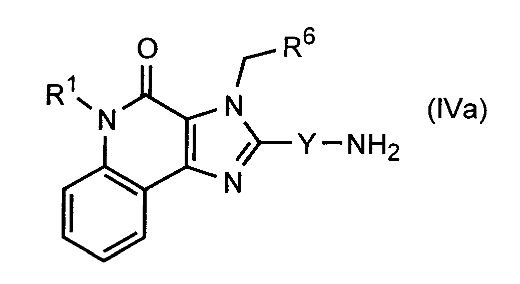

The substituent of the "optionally substituted benzene ring" portion as the ring A includes those exemplified as the substituent(s) of the "optionally substituted aryl group", besides the above-mentioned groups represented by R¹⁷.

The cycloalkene ring in the case of the "optionally substituted cycloalkene ring" as the ring A includes, for example, cycloalkene rings of 4 to 10 carbon atoms. Specific examples thereof are cyclobutene, cyclopentene, cyclohexene, cycloheptene, norbornylene, etc.

Specifically, the phrase "the ring A forms a cycloalkene ring" means, for example, that the formula (IV) becomes, for instance, the formula (IVb): 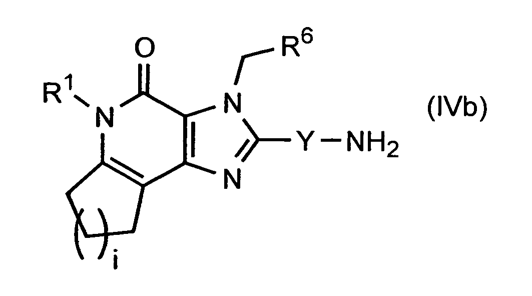 wherein i is an integer of 0 to 6. In addition, the aforesaid cycloalkene ring may contain an oxygen atom. Specific examples of such a compound are compounds of the formula (IVb-1): 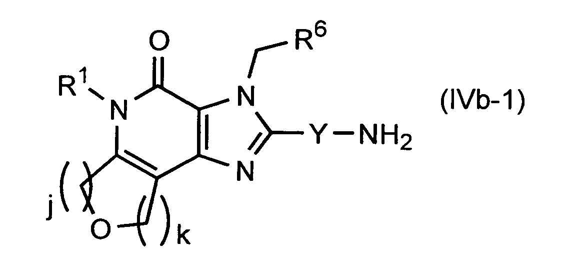 wherein each of j and k is an integer of 0 to 3, provided that when one of j and k is 0, the other is 2 or 3.

The substituent of the "optionally substituted cycloalkene ring" portion as the ring A includes, for example, alkyl groups, aralkyl groups, alkoxycarbonyl groups, alkoxy groups, oxo group and fluorine atom.

The 5-or 6-membered heteroaromatic ring in the case of the "optionally substituted 5-or 6-membered heteroaromatic ring" as the ring A includes, for example, 5-or 6-membered heteroaromatic rings containing, besides carbon atoms, one to three heteroatoms of one or two kinds selected from nitrogen atom, sulfur atom and oxygen atom. Specific examples thereof are thiophene, furan, pyrrole, imidazole, pyrazole, oxazole, pyridine, pyrazine, pyrimidine, pyridazine, thiazole, isothiazole, oxazole, isoxazole, etc.

Specifically, the phrase "the ring A forms a 5-or 6-membered heteroaromatic ring" means, for example, that the formula (IV) becomes, for instance, the formula (IVc-1), (IVc-2), (IVc-3), (IVc-4), (IVc-5), (IVc-6), (IVc-7), (IVc-8), (IVc-9), (IVc-10), (IVc-11) or (IVc-12):

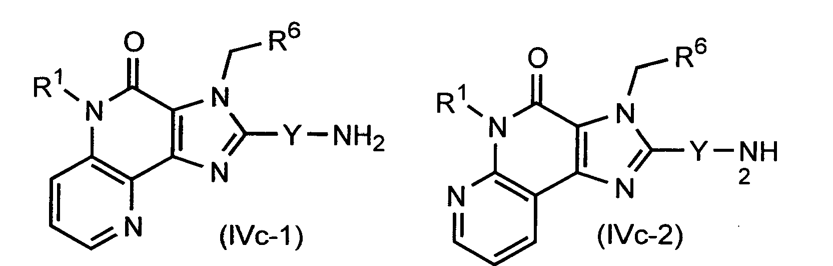

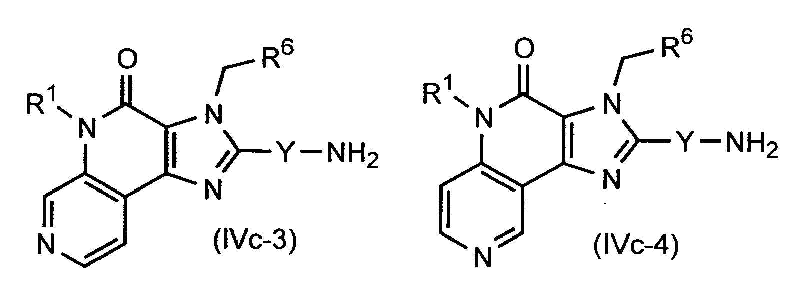

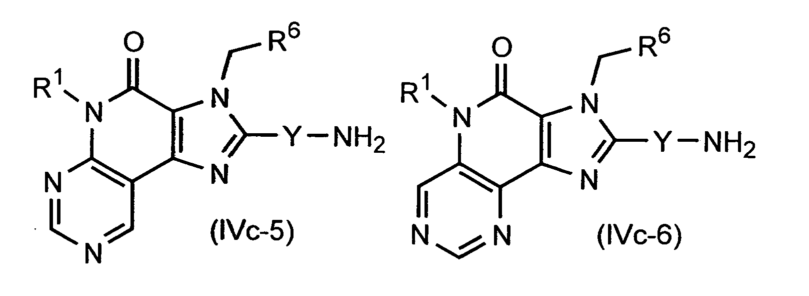

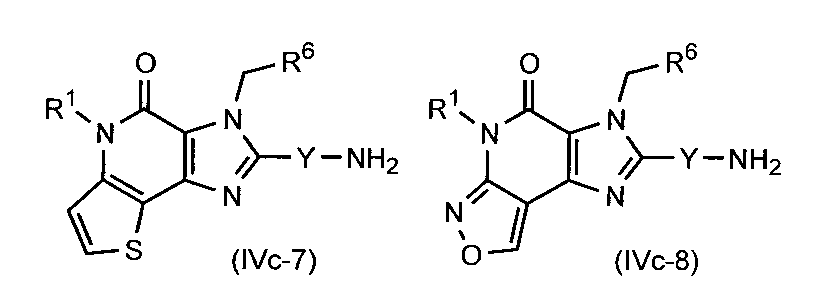

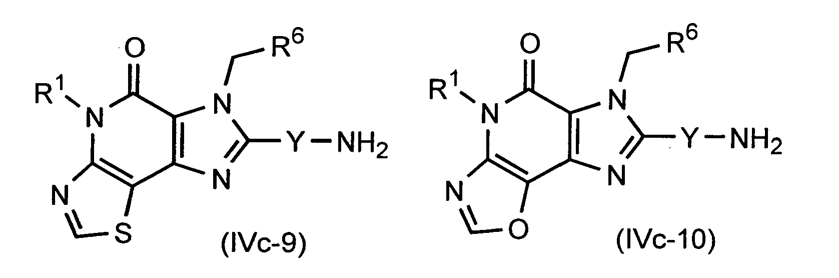

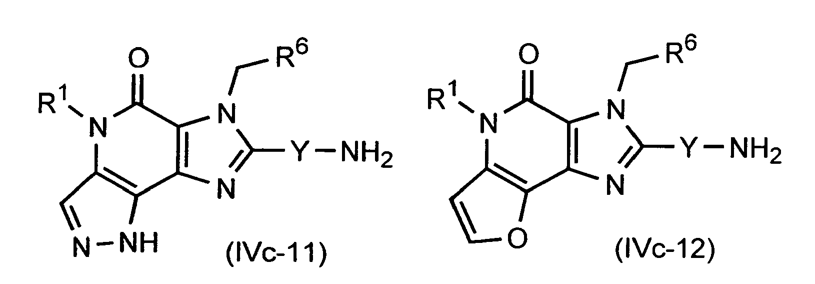

The substituent of the "optionally substituted 5-or 6-membered heteroaromatic ring" portion as the ring A includes, for example, "optionally substituted alkoxycarbonyl groups" and groups of the formula: -C (O)OCH(R¹⁸)OC(O)R¹⁹ wherein R¹⁸ and R¹⁹ are as defined above, besides the substituents exemplified as the substituent(s) of the "optionally substituted heteroaryl group".

When R³ and R⁵ are taken together to form a double bond on the ring, the formula (I) preferably represents the formula (II): 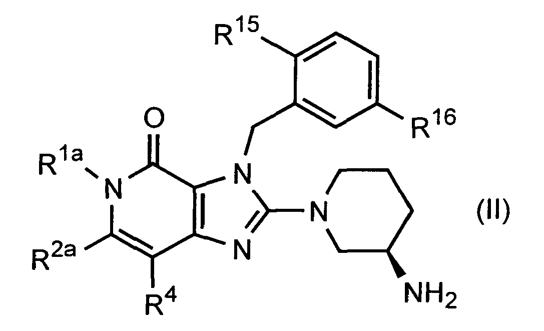 wherein R⁴ is as defined in [1]; R¹⁵ and R¹⁶ are as defined in [12]; R^{1a} is a hydrogen atom, methyl or the formula: -Ra-Rb-Rc wherein Ra, Rb and Rc are as defined in [14]; and R^{2a} is a cyano group, a carboxyl group, an oxazolyl group, an optionally substituted alkoxycarbonyl group, an optionally substituted cycloalkyloxycarbonyl group, a tetrahydrofuranyloxycarbonyl group, an optionally substituted aryloxycarbonyl group, a cinnamyloxycarbonyl group, or a group represented by the formula: -C(O)OCH(R¹⁸)OC(O)R¹⁹ wherein R¹⁸ and R¹⁹ are as defined in [1]. More preferably, the formula (I) represents the formula (III): 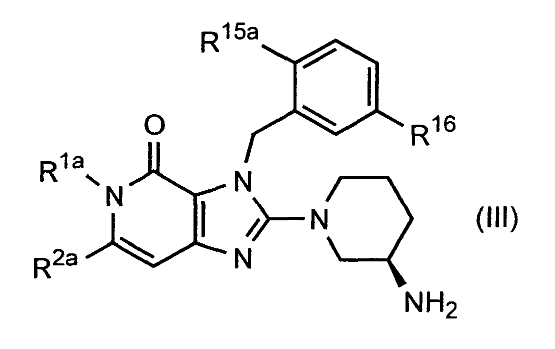 wherein R¹⁶ is as defined in [12]; R^{1a} and R^{2a} are as defined in [16]; and R^{15a} is a chlorine atom, a bromine atom, an iodine atom, a cyano group, methyl, difluoromethyl, trifluoromethyl, methoxy, fluoromethoxy, difluoromethoxy or trifluoromethoxy.

As the "prodrug", there are exemplified those which are easily hydrolyzed in a living body to regenerate the compound (I) of the present invention. Specific examples thereof are compounds obtained by converting the amino group of a compound represented by the formula (I) to -NHQ^{X}. Here, the following are exemplified as Q^{X}:
(1) 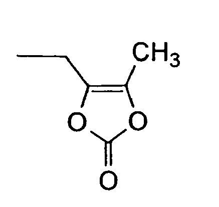
(2) -COR²¹
(3) -COO-CR²²(R²³)-OCOR²⁴
(4) -COOR²⁵
wherein R²¹ is a hydrogen atom, an alkyl group or an optionally substituted aryl group; R²² and R²³ are independently a hydrogen atom or an alkyl group; R²⁴ is a hydrogen atom, an alkyl group, an aryl group or a benzyl group; and R²⁵ is an alkyl group or a benzyl group.

Preferable examples of Q^{X} are the group of (1) and the groups of (3). Preferable examples of the groups of (3) are groups in which R²² is a hydrogen atom, R²³ is a hydrogen atom, methyl or ethyl and R²⁴ is methyl or ethyl. These compounds may be produced according to conventional processes (for example, J. Med. Chem. 35, 4727 (1992) and WO 01/40180). In addition, the prodrug may be one which is converted to the original compound under physiological conditions, such as those described in "Development of Medicines Vol.7, Molecular Design", pp. 163-198, Hirokawa Shoten, 1990.

As the "pharmaceutically acceptable salt", there are exemplified inorganic acid salts such as hydrochloride, hydrobromide, sulfate, phosphate, nitrate, etc., and organic acid salts such as acetate, propionate, oxalate, succinate, lactate, malate, tartrate, citrate, maleate, fumarate, methanesulfonate, benzenesulfonate, p-toluenesulfonate, ascorbate, etc.

In addition, the present invention includes compounds represented by the formula (I), prodrugs thereof and pharmaceutically acceptable salts of the compounds or prodrugs. The present invention also includes their hydrates or solvates (e.g. ethanol solvate). Furthermore, the present invention includes all tautomers, all existing stereoisomers and all crystal forms of the compound (I) of the present invention.

Preferable examples of the compound of the present invention are the following compounds. In the compounds listed in the following tables, the following abbreviations are used in some cases for the simplification of description.

2-Py: 2-pyridyl group, 3-Py: 3-pyridyl group, 4-Py: 4-pyridyl group, Ph: phenyl group, Et: ethyl group, Me: methyl group, n-Pr: n-propyl group, i-Pr: isopropyl group, n-Bu: n-butyl group, t-Bu: tert-butyl group, Bn: benzyl group, Ac: acetyl group, cycpro: cyclopropyl group, cycbu: cyclobutyl group, cychex: cyclohexyl group, etoet: ethoxyethyl group, meoet: methoxyethyl group, f2etoet: 2,2-difluoroethoxyethyl group, f2meoet: difluoromethoxyethyl group, cycprooet: cyclopropyloxyethyl group, isoproet: isopropyloxyethyl group, ms: methanesulfonyl group, etomet: ethoxymethyl group, meomet: methoxymethyl group, f2meomet: difluoromethoxymethyl group, f2etomet: 2,2-difluoroethoxymethyl group.

In addition, the following abbreviations of partial structures are used in some cases.

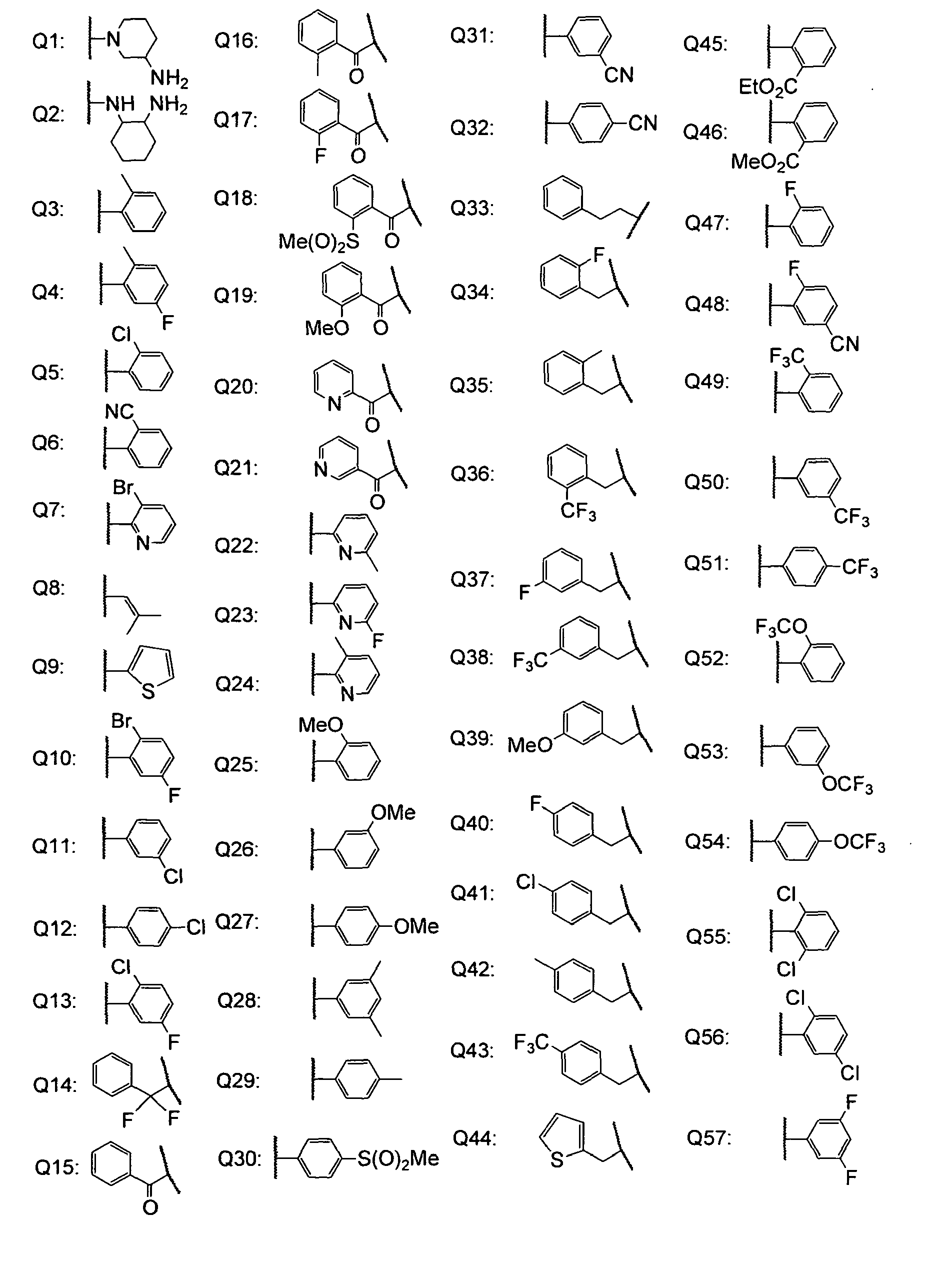

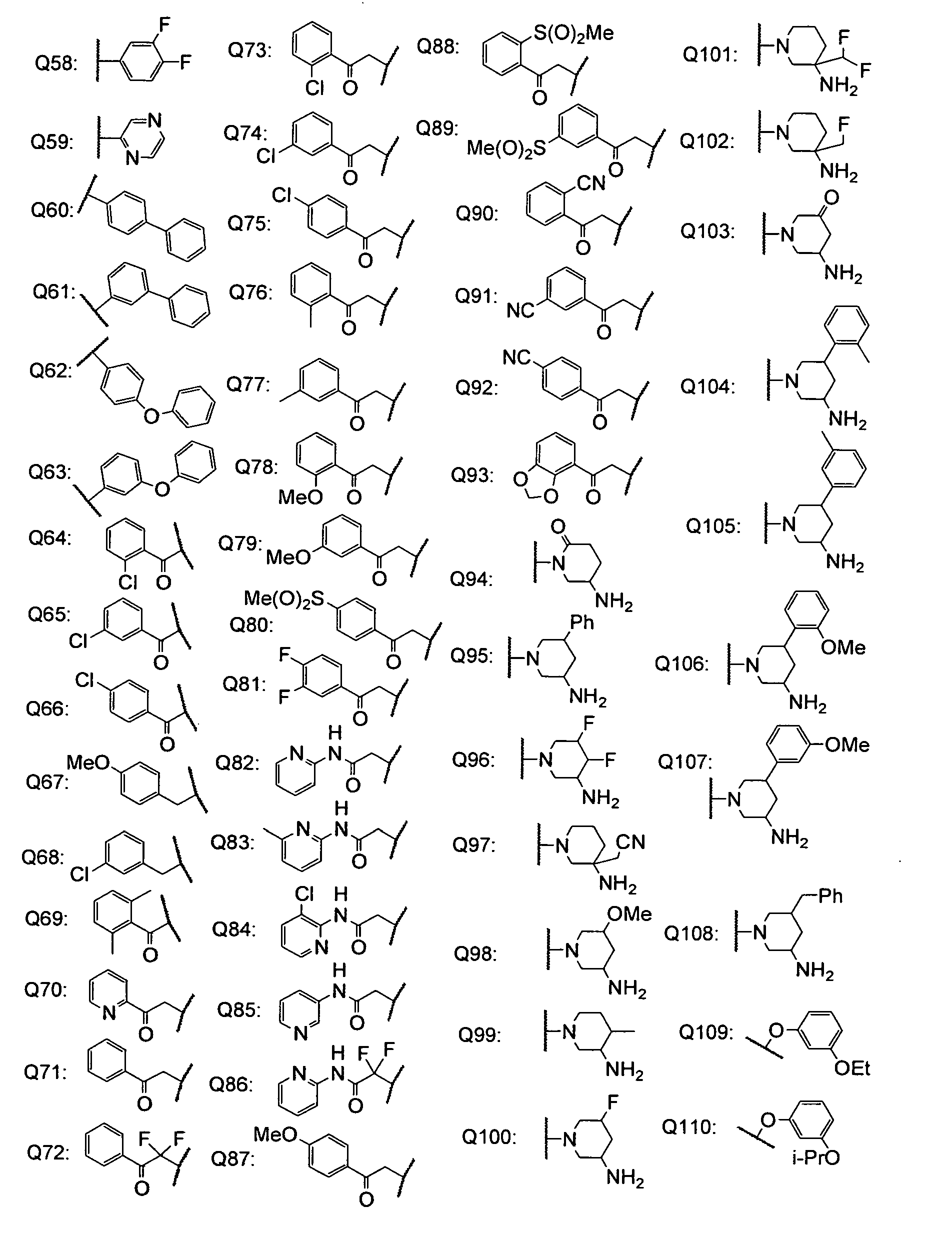

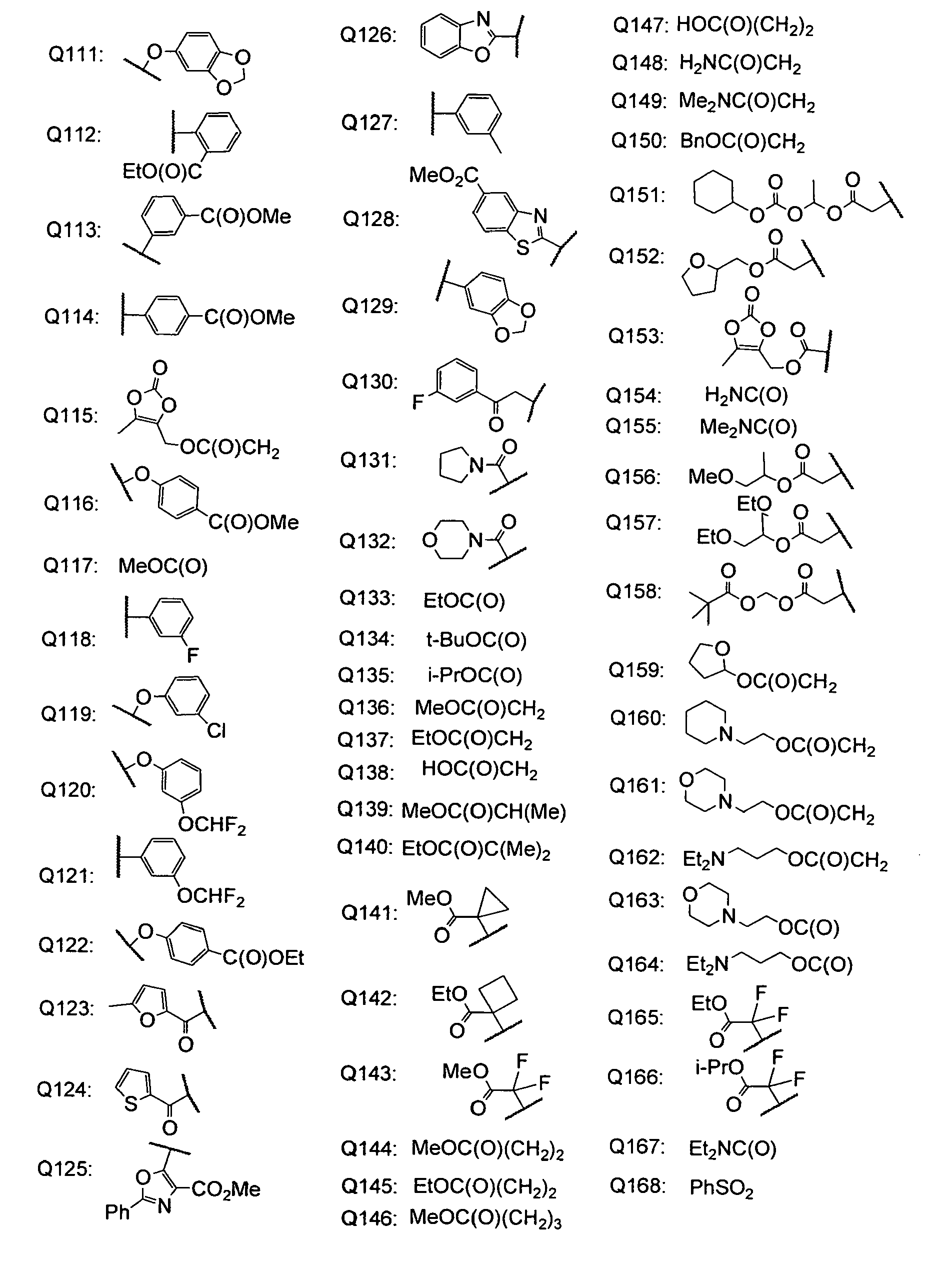

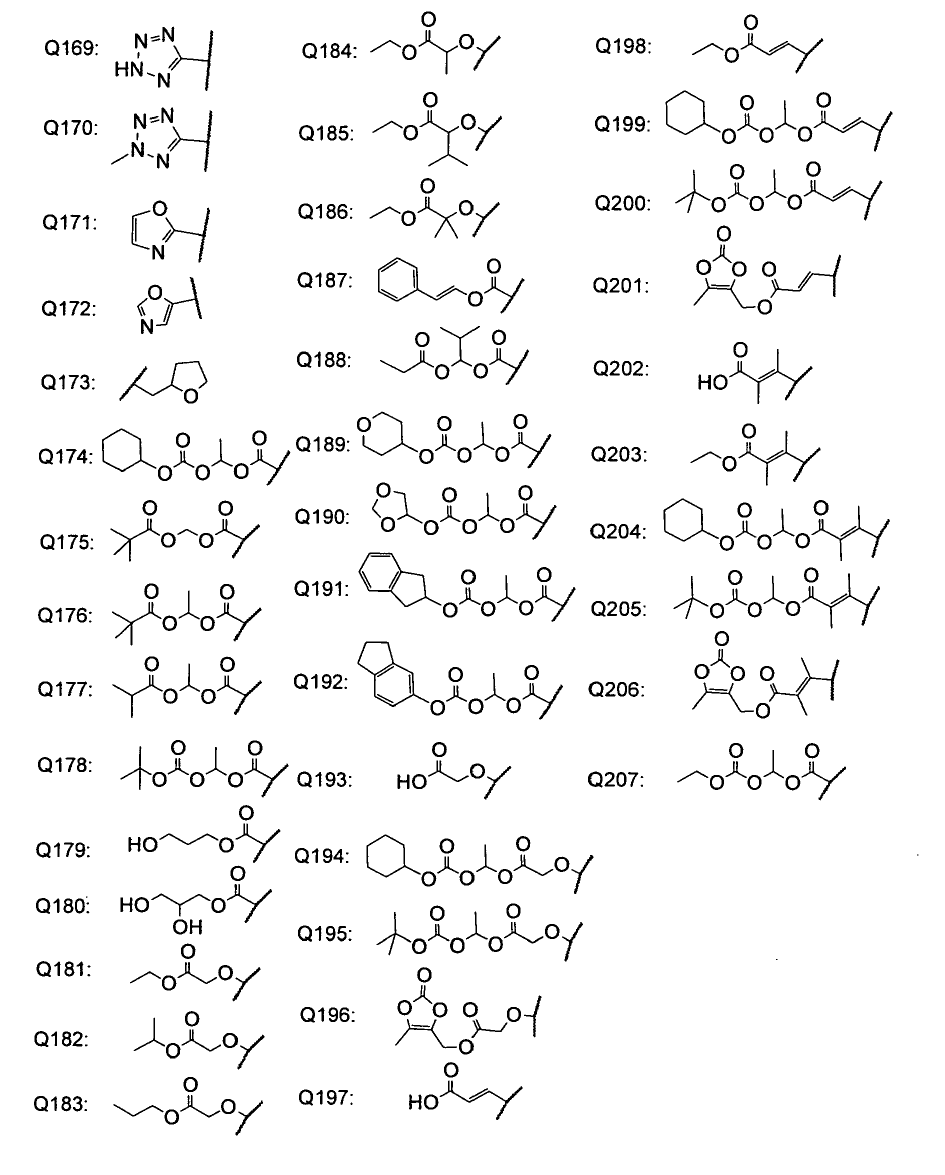

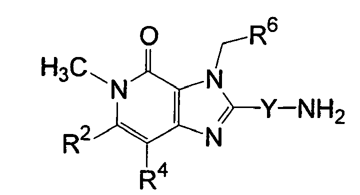

| No. | R⁶ | Y-NH₂ | R² | R⁴ | No. | R⁶ | Y-NH₂ | R² | R⁴ |
|---|---|---|---|---|---|---|---|---|---|
| 1 | Q3 | Q1 | H | Me | 23 | Q8 | Q1 | CN | H |
| 2 | Q4 | Q2 | H | H | 24 | Q8 | Q1 | Ac | H |
| 3 | Q5 | Q1 | H | Me | 25 | Q5 | Q1 | Et | H |
| 4 | Q13 | Q1 | H | Me | 26 | Q13 | Q1 | Et | H |
| 5 | Q6 | Q1 | H | Me | 27 | Q13 | Q1 | Et | H |
| 6 | Q7 | Q1 | H | H | 28 | Q5 | Q1 | i-Pr | H |
| 7 | Q8 | Q1 | H | H | 29 | Q13 | Q1 | cycpro | H |
| 8 | Q9 | Q1 | H | H | 30 | Q8 | Q1 | cycpro | H |
| 9 | Q10 | Q1 | H | H | 31 | Q5 | Q1 | MeO(Me)₂C | H |
| 10 | Q11 | Q1 | H | H | 32 | Q8 | Q1 | MeO(Me)₂C | H |
| 11 | Q12 | Q1 | H | H | 33 | Q4 | Q1 | MeO(Me)₂C | H |
| 12 | Q5 | Q1 | Me | H | 34 | Q8 | Q1 | meomet | H |
| 13 | Q13 | Q1 | Me | H | 35 | Q13 | Q1 | meomet | H |
| 14 | Q5 | Q1 | H | Me | 36 | Q4 | Q1 | meomet | H |
| 15 | Q13 | Q1 | H | Me | 37 | Q5 | Q1 | MsNHCH₂ | H |
| 16 | Q5 | Q1 | Me | Me | 38 | Q8 | Q1 | MsNHCH₂ | H |
| 17 | Q13 | Q1 | Me | Et | 39 | Q13 | Q1 | MsC(Me)₂ | H |
| 18 | Q13 | Q1 | CF₃ | H | 40 | Q13 | Q1 | Q117 | H |
| 19 | Q4 | Q1 | CF₃ | H | 41 | Q8 | Q1 | Q133 | H |
| 20 | Q5 | Q1 | Me | CF₃ | 42 | Q5 | Q1 | Q133 | H |
| 21 | Q13 | Q1 | H | CF₃ | 43 | Q8 | Q1 | Q155 | H |
| 22 | Q8 | Q1 | CF₃ | H | 44 | Q4 | Q1 | Q155 | H |

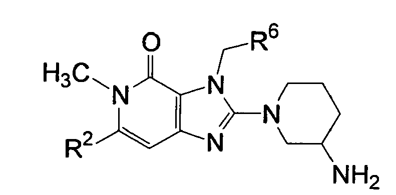

| No. | R⁶ | R² | No. | R⁶ | R² | No. | R⁶ | R² |
|---|---|---|---|---|---|---|---|---|
| 45 | Q8 | Q167 | 67 | Q13 | 3-Py | 89 | Q8 | Q42 |
| 46 | Q13 | Q154 | 68 | Q5 | 4-Py | 90 | Q4 | Q43 |
| 47 | Q4 | Q167 | 69 | Q13 | Q25 | 91 | Q5 | Q44 |
| 48 | Q13 | CN | 70 | Q5 | Q26 | 92 | Q13 | Ph |
| 49 | Q4 | CO₂H | 71 | Q13 | Q27 | 93 | Q4 | 2-Py |
| 50 | Q4 | Q134 | 72 | Q5 | Q3 | 94 | Q5 | 3-Py |
| 51 | Q13 | Q131 | 73 | Q13 | Q28 | 95 | Q5 | Q45 |
| 52 | Q13 | Q132 | 74 | Q5 | Q29 | 96 | Q5 | Q46 |
| 53 | Q8 | MsC(Me)₂ | 75 | Q13 | Q30 | 97 | Q13 | Q26 |
| 54 | Q13 | Q14 | 76 | Q5 | Q6 | 98 | Q8 | Q27 |
| 55 | Q8 | Q14 | 77 | Q13 | Q31 | 99 | Q4 | Q3 |
| 56 | Q4 | Bn | 78 | Q13 | Q32 | 100 | Q5 | Q28 |
| 57 | Q13 | Q15 | 79 | Q5 | Q33 | 101 | Q13 | Q29 |
| 58 | Q5 | Q16 | 80 | Q13 | Q34 | 102 | Q4 | Q30 |
| 59 | Q13 | Q16 | 81 | Q13 | Q35 | 103 | Q5 | Q47 |
| 60 | Q5 | Q17 | 82 | Q5 | Q36 | 104 | Q13 | Q48 |
| 61 | Q13 | Q18 | 83 | Q13 | Q37 | 105 | Q5 | Q32 |
| 62 | Q5 | Q19 | 84 | Q8 | Q38 | 106 | Q13 | Q49 |
| 63 | Q13 | Q19 | 85 | Q5 | Q39 | 107 | Q4 | Q50 |
| 64 | Q5 | Q20 | 86 | Q13 | Q67 | 108 | Q5 | Q51 |
| 65 | Q13 | Q21 | 87 | Q8 | Q40 | 109 | Q13 | Q52 |
| 66 | Q5 | 2-Py | 88 | Q5 | Q41 | 110 | Q4 | Q53 |

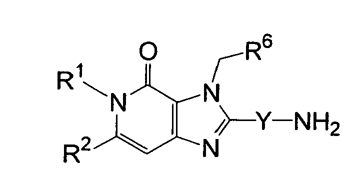

| No. | R¹ | R⁶ | R² | Y-NH₂ | No. | R¹ | R⁶ | R² | Y-NH₂ | No. | R¹ | R⁶ | R² | Y-NH₂ |
|---|---|---|---|---|---|---|---|---|---|---|---|---|---|---|
| 111 | Me | Q5 | Q54 | Q1 | 136 | Q75 | Q4 | CF₃ | Q1 | 161 | Me | Q13 | CN | Q94 |
| 112 | Me | Q13 | Q55 | Q1 | 137 | Q76 | Q5 | H | Q1 | 162 | Me | Q4 | Me | Q94 |
| 113 | Me | Q4 | Q56 | Q1 | 138 | Q77 | Q13 | Me | Q1 | 163 | Me | Q5 | H | Q95 |
| 114 | Me | Q5 | Q22 | Q1 | 139 | Q78 | Q13 | CN | Q1 | 164 | Me | Q13 | CN | Q95 |
| 115 | Me | Q13 | Q23 | Q1 | 140 | Q79 | Q5 | Ac | Q1 | 165 | Me | Q4 | Et | Q96 |
| 116 | Me | Q4 | Q24 | Q1 | 141 | Q80 | Q13 | CN | Q1 | 166 | Me | Q5 | Ac | Q97 |
| 117 | Me | Q5 | Q57 | Q1 | 142 | Q81 | Q4 | CF₃ | Q1 | 167 | Me | Q13 | CN | Q98 |
| 118 | Me | Q13 | Q58 | Q1 | 143 | Q82 | Q8 | H | Q1 | 168 | Me | Q4 | CF₃ | Q97 |
| 119 | Me | Q4 | Q59 | Q1 | 144 | Q83 | Q13 | Me | Q1 | 169 | Me | Q5 | H | Q99 |
| 120 | Me | Q5 | Q60 | Q1 | 145 | Q84 | Q4 | CF₃ | Q1 | 170 | Me | Q13 | Me | Q100 |
| 121 | Me | Q13 | Q61 | Q1 | 146 | Q85 | Q5 | Ac | Q1 | 171 | Me | Q4 | Et | Q101 |
| 122 | Me | Q4 | Q62 | Q1 | 147 | Q86 | Q13 | CN | Q1 | 172 | Me | Q5 | Ac | Q102 |
| 123 | Me | Q5 | Q63 | Q1 | 148 | Q78 | Me | CN | Q1 | 173 | Me | Q13 | CN | Q103 |
| 124 | Me | Q13 | Q15 | Q1 | 149 | Q79 | Q5 | H | Q1 | 174 | Me | Q4 | CF₃ | Q103 |
| 125 | Me | Q4 | Q64 | Q1 | 150 | Q87 | Q13 | Me | Q1 | 175 | Me | Q5 | H | Q103 |
| 126 | Me | Q5 | Q65 | Q1 | 151 | Q88 | Q4 | Me | Q1 | 176 | Me | Q13 | Me | Q103 |
| 127 | Me | Q13 | Q66 | Q1 | 152 | Q89 | Q5 | Ac | Q1 | 177 | Me | Q4 | Me | Q104 |
| 128 | Me | Q4 | Q70 | Q1 | 153 | Q90 | Q13 | CN | Q1 | 178 | Me | Q5 | Ac | Q105 |
| 129 | Me | Q5 | Q68 | Q1 | 154 | Q91 | Q8 | CF₃ | Q1 | 179 | Me | Q13 | CN | Q106 |
| 130 | Me | Q13 | Q69 | Q1 | 155 | Q92 | Q5 | CN | Q1 | 180 | Me | Q4 | CF₃ | Q107 |
| 131 | Q70 | Q5 | CN | Q1 | 156 | Q93 | Q13 | CF₃ | Q1 | 181 | Me | Q5 | CN | Q98 |
| 132 | Q71 | Q13 | Me | Q1 | 157 | Me | Q5 | H | Q94 | 182 | Me | Q13 | CF₃ | Q108 |
| 133 | Q72 | Q8 | CF₃ | Q1 | 158 | Me | Q13 | Me | Q94 | | | | | |
| 134 | Q73 | Q5 | Ac | Q1 | 159 | Me | Q4 | Et | Q94 | | | | | |
| 135 | Q74 | Q13 | CN | Q1 | 160 | Me | Q5 | Ac | Q94 | | | | | |

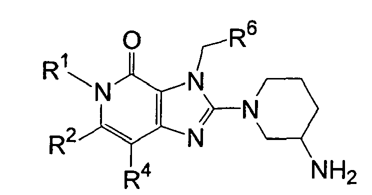

| No. | R¹ | R⁶ | R² | R⁴ | No. | R¹ | R⁶ | R² | R⁴ | No. | R¹ | R⁶ | R² | R⁴ |
|---|---|---|---|---|---|---|---|---|---|---|---|---|---|---|
| 183 | H | Q5 | Q117 | Me | 209 | Me | Q5 | Q109 | H | 235 | Q144 | Q5 | Ph | H |
| 184 | Me | Q13 | Q117 | Me | 210 | Me | Q5 | Q110 | H | 236 | Q144 | Q5 | CN | H |
| 185 | H | Q4 | Q133 | Me | 211 | Me | Q5 | Q111 | H | 237 | Q145 | Q5 | CN | H |
| 186 | Me | Q13 | Q133 | Me | 212 | Q136 | Q5 | CF₃ | H | 238 | Q144 | Q5 | CF₃ | H |
| 187 | H | Q5 | Q133 | Me | 213 | Q137 | Q5 | Ac | H | 239 | Q144 | Q5 | Ac | H |
| 188 | H | Q13 | Q134 | Me | 214 | Q138 | Q5 | H | H | 240 | Q144 | Q5 | CN | Me |
| 189 | H | Q5 | Q135 | Me | 215 | Q138 | Q5 | Me | H | 241 | Q146 | Q5 | CN | H |
| 190 | H | Q13 | Q135 | Me | 216 | Q139 | Q5 | CN | H | 242 | Q136 | Q5 | Q15 | H |
| 191 | Q79 | Q5 | Q133 | Me | 217 | Q140 | Q5 | CN | H | 243 | Q147 | Q5 | Ph | H |
| 192 | Q71 | Q13 | Q117 | Me | 218 | Q141 | Q5 | CN | H | 244 | Q138 | Q5 | Ph | H |
| 193 | Q80 | Q5 | Q133 | Me | 219 | Q142 | Q5 | CN | H | 245 | Me | Q5 | Q45 | H |
| 194 | Q81 | Q13 | Q117 | Me | 220 | Q143 | Q5 | CN | H | 246 | Me | Q5 | Q113 | H |
| 195 | Q89 | Q5 | Q133 | Me | 221 | Q144 | Q5 | Q58 | H | 247 | Me | Q5 | Q114 | H |
| 196 | Q87 | Q5 | Q135 | Me | 222 | Q136 | Q5 | Q5 | H | 248 | Q136 | Q5 | Q117 | Me |
| 197 | Q78 | Q13 | Q117 | Me | 223 | Q136 | Q5 | Q12 | Me | 249 | Q148 | Q5 | Q117 | Me |
| 198 | Q85 | Q5 | Q133 | Me | 224 | Q136 | Q5 | Q11 | H | 250 | Q149 | Q5 | Q113 | Me |
| 199 | Q86 | Q13 | Q117 | Me | 225 | Q136 | Q5 | Ph | Me | 251 | Q136 | Q5 | Q150 | Me |
| 200 | Q78 | Q5 | Q135 | Me | 226 | Q137 | Q5 | Ph | H | 252 | Q136 | Q5 | Q135 | Me |
| 201 | Q72 | Q13 | Q133 | Me | 227 | Me | Q5 | PhO | H | 253 | Q136 | Q5 | Q134 | Me |
| 202 | Q73 | Q5 | Q117 | Me | 228 | Q136 | Q5 | PhO | H | 254 | Q150 | Q5 | Q117 | Me |
| 203 | Q74 | Q4 | Q135 | Me | 229 | Me | Q5 | PhO | Me | 255 | Q136 | Q5 | Q154 | H |
| 204 | Q75 | Q5 | Q117 | Me | 230 | Q136 | Q5 | PhS | H | 256 | Q136 | Q5 | Q155 | H |
| 205 | Q90 | Q13 | Q135 | Me | 231 | Me | Q5 | Q168 | H | 257 | H | Q5 | Q153 | Me |
| 206 | Q91 | Q5 | Q117 | Me | 232 | Q136 | Q5 | Q168 | H | 258 | Q115 | Q5 | CN | H |
| 207 | Q76 | Q5 | Q133 | Me | 233 | Q143 | Q5 | Ac | H | 259 | Q151 | Q5 | CN | H |
| 208 | Q79 | Q13 | Q135 | Me | 234 | Q137 | Q5 | Ac | Me | 260 | Q152 | Q5 | CN | Me |

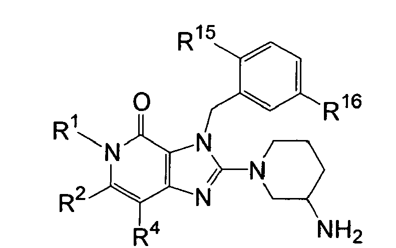

| No. | R¹ | R² | R⁴ | R¹⁵ | R¹⁶ | No. | R¹ | R² | R⁴ | R¹⁵ | R¹⁶ |
|---|---|---|---|---|---|---|---|---|---|---|---|
| 261 | Q156 | Ph | H | Me | H | 287 | Me | Ph | Me | Me | H |
| 262 | Q157 | CN | H | Cl | H | 288 | Me | Me | Me | Cl | F |
| 263 | Q158 | CN | H | Me | F | 289 | Me | CN | Me | Me | F |
| 264 | Q159 | CN | H | Cl | F | 290 | Me | CF₃ | Me | Cl | H |
| 265 | Q160 | Ac | H | Me | H | 291 | Me | Ac | Me | Me | H |
| 266 | Q161 | CN | H | Cl | H | 292 | Me | H | Me | Cl | F |
| 267 | Q162 | Ph | H | Me | F | 293 | Me | PhO | Me | Me | H |
| 268 | Q162 | CN | H | Cl | F | 294 | Me | PhO | Me | Cl | H |
| 269 | Q162 | CN | H | Me | H | 295 | Me | PhO | Me | Cl | F |
| 270 | Q160 | CN | H | Cl | H | 296 | Me | Q117 | Me | Cl | F |
| 271 | Q161 | CN | H | Me | F | 297 | Me | Q133 | Me | Me | F |
| 272 | H | Q156 | Me | Cl | F | 298 | Me | Q135 | Me | Cl | H |
| 273 | H | Q157 | Me | Me | H | 299 | Me | CN | Me | Me | H |
| 274 | Me | Q158 | Me | Me | H | 300 | Me | Q15 | Me | Me | H |
| 275 | Me | Q159 | Me | Cl | H | 301 | Me | Q116 | Me | Cl | F |
| 276 | Me | Q161 | Me | Me | F | 302 | Me | Ph | Me | Me | F |
| 277 | Me | Q162 | Me | Cl | F | 303 | Me | Q26 | Me | Cl | H |
| 278 | Q143 | Q117 | Me | Me | H | 304 | Me | Q25 | Me | Me | H |
| 279 | Q165 | Q117 | Me | Cl | H | 305 | Me | Q26 | Me | Cl | H |
| 280 | Q143 | Q133 | Me | Me | F | 306 | Me | Q111 | Me | Me | H |
| 281 | Q166 | Q117 | Me | Cl | F | 307 | Me | Q118 | Me | Cl | H |
| 282 | Q143 | Ph | Me | Me | H | 308 | Me | Q57 | Me | Me | H |
| 283 | Q165 | Et | Me | Cl | H | 309 | Me | Q119 | Me | Cl | H |
| 284 | Q136 | CN | Me | Me | F | 310 | Me | Q120 | Me | Me | F |
| 285 | Q166 | CF₃ | H | Cl | F | 311 | Me | Q121 | Me | Cl | H |
| 286 | Q143 | Ac | H | Me | H | 312 | Me | Q122 | Me | Cl | F |

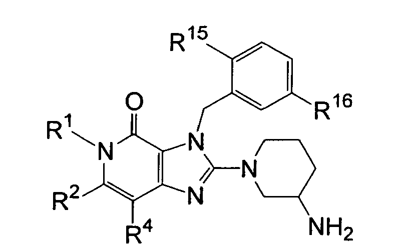

| No. | R¹ | R² | R⁴ | R¹⁵ | R¹⁶ | I No. | R¹ | R² | R⁴ | R¹⁵ | R¹⁶ |
|---|---|---|---|---|---|---|---|---|---|---|---|
| 313 | Me | Q123 | H | Me | H | 339 | Me | Q169 | H | Me | H |
| 314 | Me | Q124 | H | Cl | H | 340 | Me | Q170 | H | Cl | F |
| 315 | Me | Q125 | H | Me | H | 341 | Me | Q171 | Me | Me | F |
| 316 | Q79 | Q126 | H | Cl | H | 342 | Me | Q172 | Me | Cl | H |
| 317 | Q118 | Ac | H | Me | H | 343 | Me | Q172 | Me | Cl | F |
| 318 | Q127 | Ac | H | Cl | H | 344 | Me | Q172 | Q173 | Cl | F |
| 319 | Q118 | CN | H | Me | H | 345 | Me | Q172 | f2etoet | Cl | F |
| 320 | Q127 | CN | H | Cl | H | 346 | Me | Q172 | f2meoet | Cl | F |
| 321 | Me | Q128 | H | Me | H | 347 | Me | Q172 | etoet | Cl | F |
| 322 | Me | Q136 | H | Cl | H | 348 | Me | Q172 | Q137 | Cl | F |
| 323 | Me | Q137 | H | Me | H | 349 | Me | Q172 | isoproet | Me | F |
| 324 | Q79 | Q136 | H | Cl | H | 350 | H | Q172 | H | Cl | H |
| 325 | Q130 | Q136 | H | Me | H | 351 | Me | CN | Me | Me | H |
| 326 | Q26 | CN | H | Me | H | 352 | Me | Q15 | Me | Me | H |
| 327 | Q53 | CN | H | Cl | H | 353 | Me | Q116 | Me | Cl | F |
| 328 | Q26 | Ac | H | Me | H | 354 | Me | Ph | Me | Me | F |
| 329 | Q121 | CN | H | Cl | H | 355 | Me | Q26 | Me | Cl | H |
| 330 | Q26 | Ac | Me | Me | H | 356 | Me | Q25 | Me | Me | H |
| 331 | Q129 | CN | H | Cl | H | 357 | Me | Q26 | Me | Cl | H |
| 332 | 3-Py | CN | Me | Me | H | 358 | Me | Q111 | Me | Me | H |
| 333 | Q79 | H | Q136 | Cl | H | 359 | Me | Q118 | Me | Cl | H |
| 334 | H | H | Q136 | Me | H | 360 | Me | Q57 | Me | Me | H |
| 335 | Me | H | Q136 | Cl | H | 361 | Me | Q119 | Me | Cl | H |
| 336 | Me | CN | Q136 | Me | H | 362 | Me | Q120 | Me | Me | F |
| 337 | Q79 | Ac | Q136 | Cl | H | 363 | Me | Q121 | Me | Cl | H |
| 338 | Q79 | CN | Q136 | Me | H | 364 | Me | Q122 | Me | Cl | F |

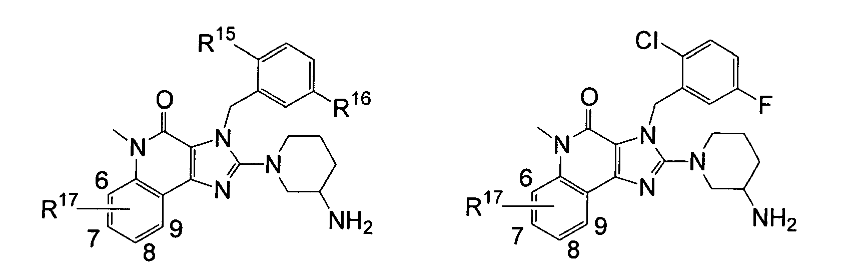

| No. | R¹⁷ | R¹⁵ | R¹⁶ | No. | R¹⁷ | No. | R¹⁷ |
|---|---|---|---|---|---|---|---|
| 365 | 6-OMe | Cl | H | 391 | 7-CN/8-Q137 | 417 | 7-CN/9-CN |
| 366 | 8-Cl | Me | H | 392 | 7-CN/8-Q140 | 418 | 8-CN/9-CN |
| 367 | 6-Q117 | Cl | H | 393 | 7-CN/8-Q138 | 419 | 8-Q133/7-CN |
| 368 | 6-CN | Cl | F | 394 | 7-CN/8-Q143 | 420 | 8-Q133/9-CN |
| 369 | 7-Q117 | Cl | H | 395 | 7-CN/8-Q115 | 421 | 8-Q133/7-OEt |
| 370 | 6-Me | Cl | H | 396 | 7-CN/8-Q151 | 422 | 8-Q133/9-OEt |
| 371 | 8-Q117 | Cl | H | 397 | 7-Q115/8-CN | 423 | 8-Q133/9-etomet |
| 372 | 7-OEt | Cl | H | 398 | 7-Q151/8-CN | 424 | 7-Q135/9-CO₂H |
| 373 | 6-Me/8-CN | Me | H | 399 | 7-Q138/8-CN | 425 | 7-Q133/9-CO₂H |
| 374 | 374 8-Me/7-OMe | Cl | H | 400 | 7-Q140/8-CN | 426 | 7-Q135/9-etomet |
| 375 | 6-Ac | Cl | H | 401 | 7-Q137/8-CN | 427 | 7-Q135/9-f2meoet |
| 376 | 6-Q117/8-F | Cl | H | 402 | 7-Q115/9-CN | 428 | 7-CN/8-Q174 |
| 377 | 9-Q117 | Cl | H | 403 | 7-Q151/9-CN | 429 | 7-CN/8-Q153 |
| 378 | 6-OPh | Cl | H | 404 | 7-Q138/9-CN | 430 | 7-CN/8-Q158 |
| 379 | 9-OMe | Cl | H | 405 | 7-Q140/9-CN | 431 | 7-CN/8-CO₂H |
| 380 | 7-CN/8-F | Cl | F | 406 | 7-Q137/9-CN | 432 | 8-CN/7-Q174 |
| 381 | 9-CN/8-F | Cl | H | 407 | 9-Q115/7-CN | 433 | 8-CN/7-Q153 |
| 382 | 7-CN/9-F | Cl | H | 408 | 9-Q151/7-CN | 434 | 8-CN/7-Q158 |
| 383 | 7-CN/8-OMe | Cl | H | 409 | 9-Q138/7-CN | 435 | 8-CN/7-CO₂H |
| 384 | 9-CN/8-OMe | Cl | F | 410 | 9-Q140/7-CN | 436 | 9-CN/7-Q174 |
| 385 | 7-CN/8-meoet | Cl | H | 411 | 9-Q137/7-CN | 437 | 9-CN/7-Q153 |
| 386 | 7-CN/8-f2etoet | Cl | F | 412 | 9-Q175/7-CN | 438 | 9-CN/7-Q158 |
| 387 | 9-CN/7-OMe | Cl | H | 413 | 7-Q175/9-CN | 439 | 9-CN/7-CO₂H |
| 388 | 9-CN/7-meomet | Cl | F | 414 | 7-Q175/8-CN | 440 | 7-CN/9-Q174 |
| 389 | 9-CN/8-meomet | Cl | H | 415 | 8-Q175/9-CN | 441 | 7-CN/9-Q153 |
| 390 | 7-CN/8-O(i-Pr) | Cl | F | 416 | 7-CN/8-CN | 442 | 7-CN/9-Q158 |

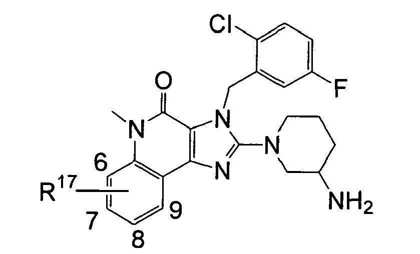

| No. | R¹⁷ | No. | R¹⁷ |
|---|---|---|---|
| 443 | 7-CN/9-CO₂H | 469 | 7-Q136 |
| 444 | 7-CN/8-Q174 | 470 | 7-Q137 |
| 445 | 7-CN/8-Q153 | 471 | 7-Q138 |
| 446 | 7-CN/8-Q158 | 472 | 7-Q181 |
| 447 | 7-CN/8-CO₂H | 473 | 7-Q182 |
| 448 | 7-Q174 | 474 | 7-Q183 |
| 449 | 7-Q175 | 475 | 7-Q184 |
| 450 | 7-Q176 | 476 | 7-Q185 |
| 451 | 7-Q177 | 477 | 7-Q186 |
| 452 | 7-Q178 | 478 | 8-Q136 |
| 453 | 7-Q179 | 479 | 8-Q137 |
| 454 | 7-Q180 | 480 | 8-Q138 |
| 455 | 8-Q174 | 481 | 8-Q181 |
| 456 | 8-Q175 | 482 | 8-Q182 |
| 457 | 8-Q176 | 483 | 8-Q183 |
| 458 | 8-Q177 | 484 | 8-Q184 |
| 459 | 8-Q178 | 485 | 8-Q185 |
| 460 | 8-Q179 | 486 | 8-Q186 |
| 461 | 8-Q180 | 487 | 7-CN/9-f2etoet |
| 462 | 9-Q174 | 488 | 7-CN/9-OEt |
| 463 | 9-Q175 | 489 | 7-Q158/9-OCHF₂ |
| 464 | 9-Q176 | 490 | 7-Q174/9-OCHF₂ |
| 465 | 9-Q177 | 491 | 7-CO₂H/9-OCHF₂ |
| 466 | 9-Q178 | 492 | 7-OCHF₂/9-CO₂H |
| 467 | 9-Q179 | 493 | 7-cycpro-CH₂O/9-CO₂H |
| 468 | 9-Q180 | 494 | 7-CN/9-cycpro-CH₂O |

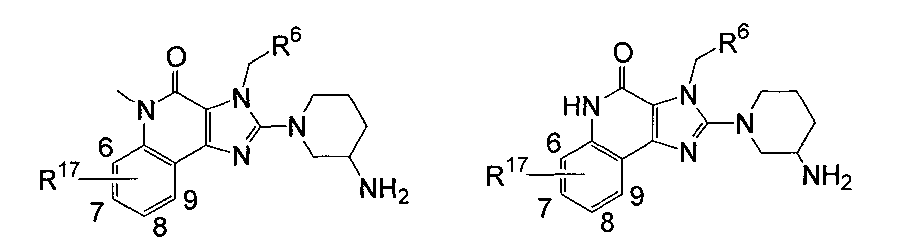

| No. | R¹⁷ | R⁶ | No. | R¹⁷ | R⁶ |
|---|---|---|---|---|---|
| 495 | 6-OMe | Q3 | 521 | 6-OMe | Q3 |
| 496 | 8-Cl | Q4 | 522 | 8-Cl | Q4 |
| 497 | 6-Q117 | Q6 | 523 | 6-Q117 | Q6 |
| 498 | 6-CN | Q8 | 524 | 6-CN | Q8 |
| 499 | 7-Q117 | Q10 | 525 | 7-Q117 | Q10 |
| 500 | 6-Me | Q25 | 526 | 6-Me | Q25 |
| 501 | 8-Q117 | Q49 | 527 | 8-Q117 | Q49 |
| 502 | 7-OEt | Q6 | 528 | 7-OEt | Q6 |
| 503 | 6-Me/8-CN | Q3 | 529 | 6-Me/8-CN | Q3 |
| 504 | 8-Me/7-OMe | Q10 | 530 | 8-Me/7-OMe | Q10 |
| 505 | 6-Ac | Q4 | 531 | 6-Ac | Q4 |
| 506 | 6-Q117/8-F | Q6 | 532 | 6-Q117/8-F | Q6 |
| 507 | 9-Q117 | Q8 | 533 | 9-Q117 | Q8 |
| 508 | 7-OCHF₂ | Q10 | 534 | 6-OPh | Q10 |
| 509 | 9-OMe | Q25 | 535 | 9-OMe | Q25 |
| 510 | 7-CN/8-F | Q49 | 536 | 7-CN/8-F | Q49 |
| 511 | 9-CN/8-F | Q6 | 537 | 9-CN/8-F | Q6 |
| 512 | 7-CN/9-F | Q3 | 538 | 7-CN/9-F | Q3 |
| 513 | 7-CN/8-OMe | Q4 | 539 | 7-CN/8-OMe | Q4 |
| 514 | 9-CN/8-OMe | Q6 | 540 | 9-CN/8-OMe | Q6 |
| 515 | 7-CN/8-meoet | Q8 | 541 | 7-CN/8-meoet | Q8 |
| 516 | 7-CN/8-f2etoet | Q10 | 542 | 7-CN/8-f2etoet | Q10 |
| 517 | 9-CN/7-OMe | Q25 | 543 | 9-CN/7-OMe | Q25 |
| 518 | 9-CN/7-meomet | Q49 | 544 | 9-CN/7-meomet | Q49 |
| 519 | 9-CN/8-meomet | Q6 | 545 | 9-CN/8-meomet | Q6 |
| 520 | 7-CN/8-O(i-Pr) | Q6 | 546 | 7-CN/8-O(i-Pr) | Q6 |

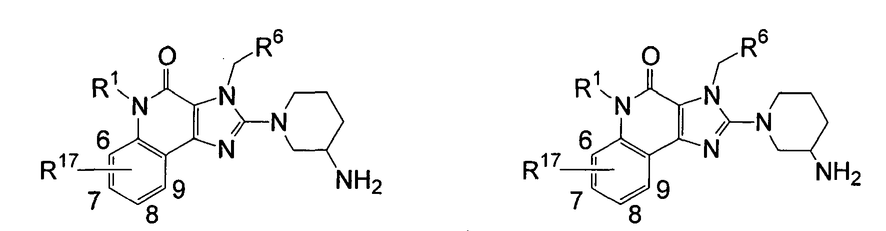

| No. | R¹⁷ | R¹ | R⁶ | No. | R¹⁷ | R¹ | R⁶ |
|---|---|---|---|---|---|---|---|
| 547 | 6-CO₂H | Q70 | Q3 | 573 | 6-OMe | Q70 | Q3 |
| 548 | 7-CO₂H | Q71 | Q4 | 574 | 8-Cl | Q71 | Q4 |
| 549 | 7-CO₂H/8-OH | Me | Q4 | 575 | 6-Q117 | Q79 | Q6 |
| 550 | 7-CO₂H/8-OMe | Q82 | Q4 | 576 | 6-CN | Q82 | Q8 |
| 551 | 7-CO₂H/8-OEt | Me | Q4 | 577 | 7-Q117 | Q152 | Q10 |
| 552 | 7-CO₂H/8-F | Me | Q6 | 578 | 6-Me | Q156 | Q25 |
| 553 | 7-CO₂H/9-F | Me | Q6 | 579 | 8-Q117 | Q159 | Q49 |
| 554 | 7-Q117/8-OH | Q173 | Q6 | 580 | 7-OEt | Q173 | Q6 |
| 555 | 7-Q133/8-OMe | meomet | Q6 | 581 | 6-Me/8-CN | meomet | Q3 |
| 556 | 7-Q133/8-F | etomet | Q13 | 582 | 8-Me/7-OMe | etomet | Q10 |
| 557 | 7-Q135/9-F | meoet | Q13 | 583 | 6-Ac | meoet | Q4 |
| 558 | 7-Q 138 | etoet | Q13 | 584 | 6-Q117/8-F | etoet | Q6 |
| 559 | 7-Q136 | f2etoet | Q3 | 585 | 9-Q117 | f2etoet | Q8 |
| 560 | 7-Q137 | Q70 | Q3 | 586 | 6-OPh | Q70 | Q10 |
| 561 | 8-CO₂H | Q71 | Q4 | 587 | 9-OMe | Q71 | Q25 |
| 562 | 8-CO₂H/7-OH | Q79 | Q4 | 588 | 7-CN/8-F | Q79 | Q49 |
| 563 | 8-CO₂H/7-OMe | Me | Q6 | 589 | 9-CN/8-F | Q82 | Q6 |
| 564 | 8-CO₂H/7-F | Q152 | Q6 | 590 | 7-CN/9-F | Q152 | Q3 |
| 565 | 8-Q133/8-OMe | Me | Q6 | 591 | 7-CN/8-OMe | Q156 | Q4 |
| 566 | 8-Q133/8-F | Q159 | Q6 | 592 | 9-CN/8-OMe | Q159 | Q6 |
| 567 | 8-Q136 | Me | Q6 | 593 | 7-CN/8-meoet | Q173 | Q8 |
| 568 | 8-Q137 | meomet | Q4 | 594 | 7-CN/8-f2etoet | meomet | Q10 |
| 569 | 9-CO₂H | etomet | Q13 | 595 | 9-CN/7-OMe | etomet | Q25 |
| 570 | 9-CO₂H/6-F | meoet | Q10 | 596 | 9-CN/7-meomet | meoet | Q49 |
| 571 | 9-Q117/7-F | etoet | Q13 | 597 | 9-CN/8-meomet | etoet | Q6 |
| 572 | 9-Q133/6F | Me | Q6 | 598 | 7-CN/8-O(i-Pr) | f2etoet | Q6 |

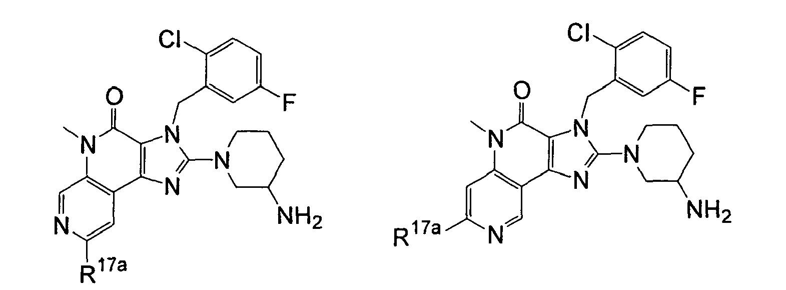

| No. | R^{17a} | No. | R^{17a} |
|---|---|---|---|
| 599 | CO₂H | 606 | CO₂H |
| 600 | Q133 | 607 | Q133 |
| 601 | Q135 | 608 | Q135 |
| 602 | Q174 | 609 | Q174 |
| 603 | Q175 | 610 | Q175 |
| 604 | Q207 | 611 | Q207 |
| 605 | CN | 612 | CN |

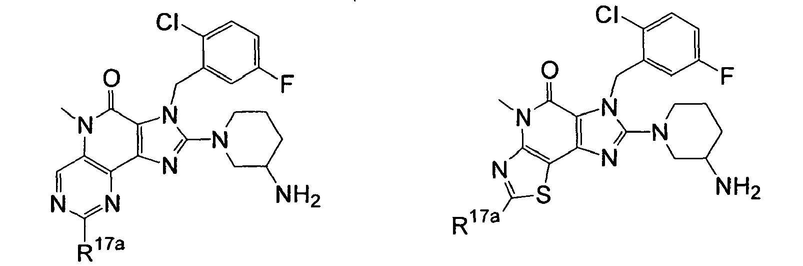

| No. | R^{17a} | No. | R^{17a} |
|---|---|---|---|
| 613 | CO₂H | 620 | CO₂H |
| 614 | Q133 | 621 | Q133 |
| 615 | Q135 | 622 | Q135 |
| 616 | Q174 | 623 | Q174 |
| 617 | Q175 | 624 | Q175 |
| 618 | Q207 | 625 | Q207 |
| 619 | CN | 626 | CN |

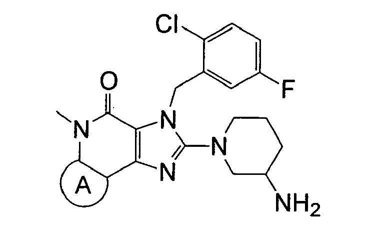

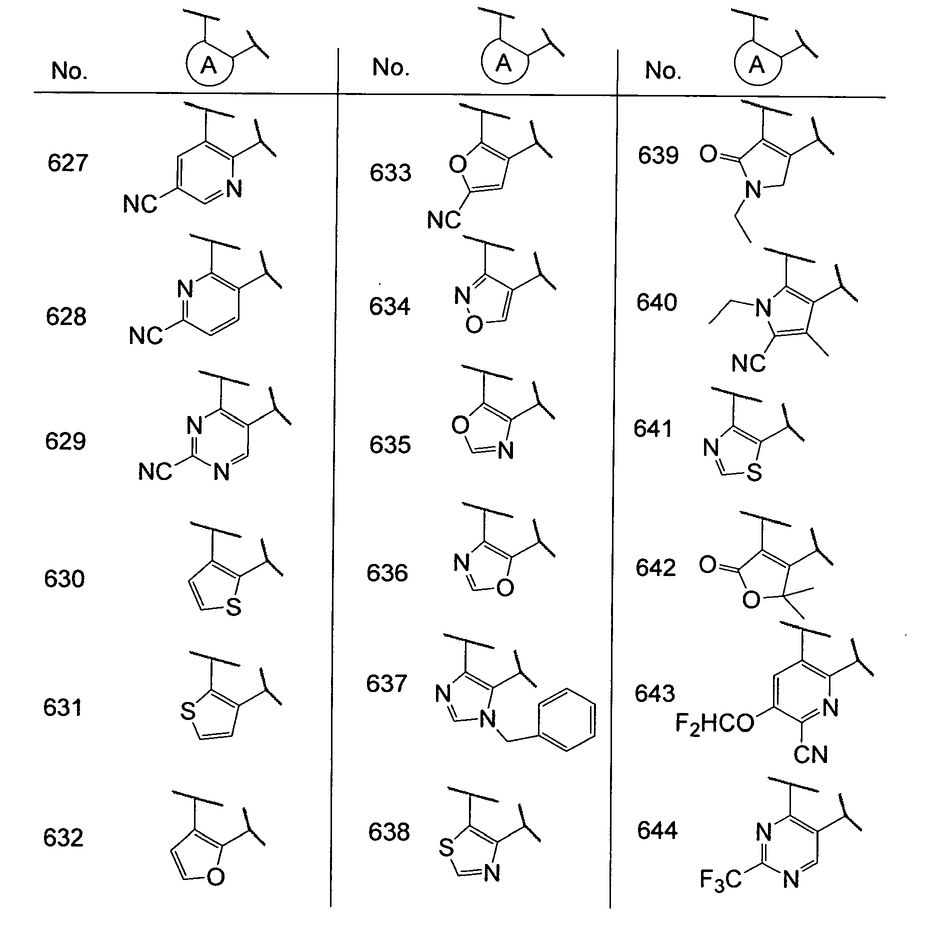

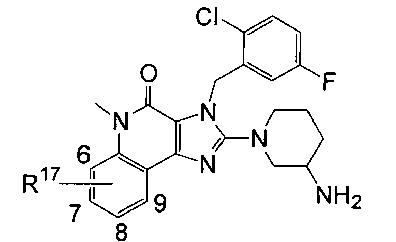

| No. | R¹⁷ | No. | R² |
|---|---|---|---|
| 645 | 7-f2etoet/9-CO₂H | 670 | 7-CN/9-Q193 |
| 646 | 7-O(i-Pr)/9-CO₂H | 671 | 7-Q135/9-Q193 |
| 647 | 7-f2meomet/9-CO₂H | 672 | 7-CF₃/9-Q193 |
| | | 673 | 7-Q194 |
| 648 | 7-f2etomet/9-CO₂H | 674 | 7-Q195 |
| 649 | 7-OEt/9-CO₂H | 675 | 7-Q196 |
| 650 | 7-etomet/9-CO₂H | 676 | 7-Q194/9-CN |
| 651 | 7-OMe/9-CO₂H | 677 | 7-Q195/9-CN |
| 652 | 7-Q187 | 678 | 7-Q196/9-CN |
| 653 | 8-Q187 | 679 | 7-Q197 |
| 654 | 7-Q188 | 680 | 7-Q198 |
| 655 | 7-Q189 | 681 | 7-Q199 |
| 656 | 7-Q190 | 682 | 7-Q200 |
| 657 | 7-Q172 | 683 | 7-Q201 |
| 658 | 8-Q172 | 684 | 7-Q202 |
| 659 | 7-CF₃/9-CO₂H | 685 | 7-Q203 |
| 660 | 9-CF₃/7-CO₂H | 686 | 7-Q204 |
| 661 | 8-CF₃/7-CO₂H | 687 | 7-Q205 |
| 662 | 6-F/8-F/9-F/7-CO₂H | 688 | 7-Q206 |
| 663 | 7-Q134/9-CO₂H | 689 | 7-Q198/9-CO₂H |
| 664 | 7-Q191 | 690 | 7-Q203/9-CO₂H |
| 665 | 7-Q192 | | |
| 666 | 8-Q191 | 691 | 7-Q199/9-CN |
| 667 | 8-Q192 | 692 | 7-Q200/9-CN |
| 668 | 7-Q182/9-CO₂H | 693 | 7-Q201/9-CN |
| 669 | 9-Q182/7-CN | 694 | 7-Q206/9-CN |

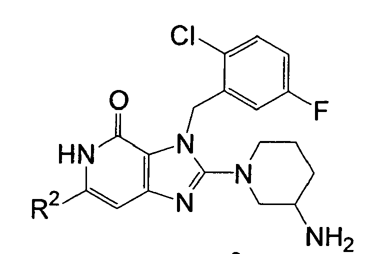

| No. | R² | No. | R² | No. | R² |
|---|---|---|---|---|---|
| 695 | Q153 | 701 | Q179 | 707 | Q189 |
| 696 | Q174 | 702 | Q180 | 708 | Q190 |
| 697 | Q175 | 703 | Q163 | 709 | Q191 |
| 698 | Q176 | 704 | Q164 | 710 | Q192 |
| 699 | Q177 | 705 | Q187 | 711 | Q207 |
| 700 | Q178 | 706 | Q188 | | |

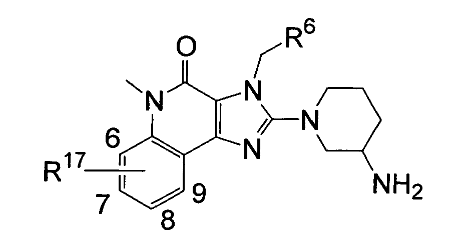

| No. | R¹⁷ | R⁶ | No. | R¹⁷ | R⁶ |
|---|---|---|---|---|---|
| 712 | 7-Q138/6-F | Q3 | 735 | 8-Q140/9-F | Q5 |
| 713 | 7-Q138/8-F | Q4 | 736 | 8-Q159/7-CN | Q10 |
| 714 | 7-Q159/8-OMe | Q6 | 737 | 9-Q137/6-F | Q13 |
| 715 | 7-Q162/8-CN | Q8 | 738 | 9-Q137/8-OMe | Q4 |
| 716 | 7-Q137/8-F | Q10 | 739 | 9-Q151/7-OMe | Q4 |
| 717 | 7-Q137/8-OMe | Q13 | 740 | 9-Q152/7-F | Q10 |
| 718 | 7-Q137/6,8-F | Q3 | 741 | 9-Q158/8-F | Q13 |
| 719 | 7-Q140/8-F | Q4 | 742 | 7-Q207/8-F | Q13 |
| 720 | 7-Q140/9-F | Q5 | 743 | 7-Q207/9-F | Q13 |
| 721 | 7-Q151/6-F | Q10 | 744 | 7-Q207/9-CN | Q13 |
| 722 | 7-Q151/9-CN | Q13 | 745 | 7-Q207/9-CO₂H | Q13 |
| 723 | 7-Q152/8-OMe | Q4 | 746 | 7-Q207/9-OMe | Q13 |
| 724 | 7-Q152/9-F | Q4 | 747 | 8-Q207/7-F | Q13 |
| 725 | 7-Q158/8-F | Q10 | 748 | 8-Q207/9-F | Q13 |
| 726 | 7-Q158/9-F | Q13 | 749 | 8-Q207/9-CN | Q13 |
| 727 | 8-Q138/6-F | Q3 | 750 | 8-Q207/9-OMe | Q13 |
| 728 | 8-Q138/7-F | Q4 | 751 | 7-CO₂H/9-CO₂H | Q13 |
| 729 | 8-Q137/7-OMe | Q6 | 752 | 8-CO₂H/7-F | Q13 |
| 730 | 8-Q140/7-CN | Q8 | 753 | 8-CO₂H/9-F | Q13 |
| 731 | 8-Q165/7-F | Q10 | 754 | 8-CO₂H/9-OMe | Q13 |
| 732 | 8-Q151/7-OMe | Q13 | 755 | 8-CO₂H/9-CN | Q13 |
| 733 | 8-Q152/6-F | Q3 | 756 | 7-CO₂H/9-OMe | Q13 |
| 734 | 8-Q158/8-F | Q4 | 757 | 8-Q207/7-CN | Q13 |

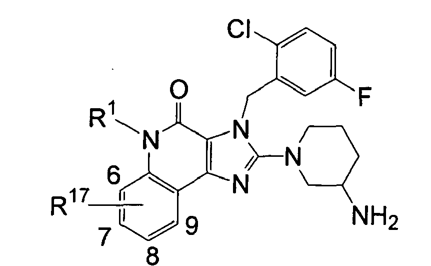

| No. | R¹⁷ | R¹ | No. | R¹⁷ | R¹ |
|---|---|---|---|---|---|
| 758 | 7-CN | Q138 | 764 | 7-CN | Q151 |
| 759 | 7-CN/8-F | Q138 | 765 | 7-CN/8-F | Q158 |
| 760 | 7-CN/9-OMe | Q138 | 766 | 7-CN/9-OMe | Q151 |
| 761 | 7-CN/8-F | Q136 | 767 | 7-CN/9-OMe | Q158 |
| 762 | 7-CN | Q137 | 768 | 7-CN/9-OMe | Q115 |
| 763 | 7-CN/8-F | Q115 | | | |

When in each of the above compounds having compound numbers 1 to 768, the portion corresponding to Y-NH₂ described in the item [1] is an unsubstituted or substituted 3-aminopyrrolidin-1-yl group, an unsubstituted or substituted 3-aminopiperidin-1-yl group or an unsubstituted or substituted (3-amino)hexahydroazepin-1-yl group, bicyclic pyrazole derivatives whose amino group at the 3-position has an absolute configuration represented by the following formula (F₁) are more preferable. 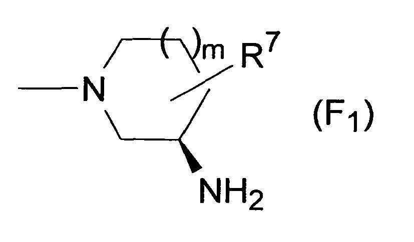 wherein m and R⁷ are as defined in the item [1].

When in each of the above compounds having compound numbers 1 to 768, the portion corresponding to Y-NH₂ described in the item [1] is an unsubstituted or substituted (2-aminocycloalkyl)amino group, compounds whose amino groups at the 1-position and 2-position have an absolute configuration represented by the following formula (F₂) or (F₃) are more preferable. 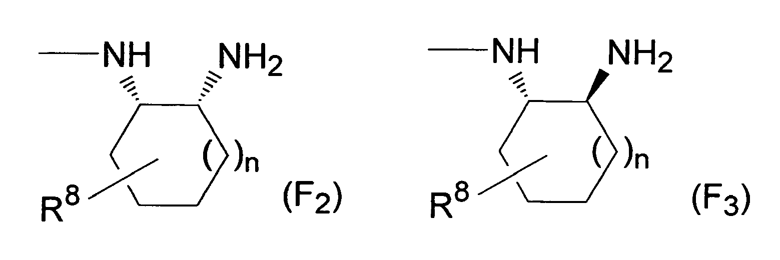 wherein n and R⁸ are as defined in the item [1].

Compounds whose amino groups at the 1-position and 2-position have an absolute configuration represented by the following formula (F₄) are still more preferable. 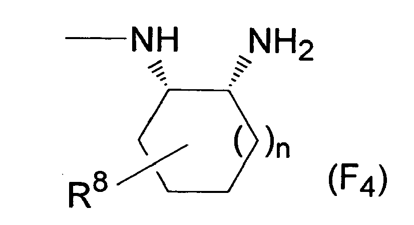 wherein n and R⁸ are as defined in the item [1].

In the following description, a bond shown by a wedge-shaped solid or broken line as in the formula (J₁) and formula (J₂) indicates the absolute configuration of an amino group, and a bond shown by a thick line as in the formula (J₃) indicates the relative configuration of an amino group (for example, the formula (J₃) represents a (+)-cis form). 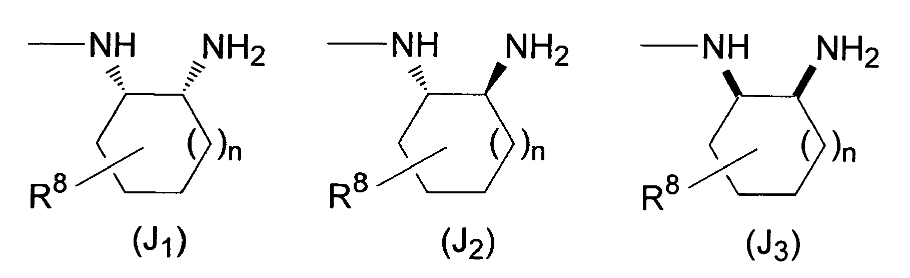 wherein n and R⁸ are as defined in the item [1].

In the compounds, among the above compounds having compound numbers 1 to 768, in which each of the portions corresponding to R¹, R² and R⁴, respectively, described in the item [1], or its partial structure is "an optionally substituted alkoxycarbonyl group", "an optionally substituted cycloalkoxycarbonyl group", "an optionally substituted aryloxycarbonyl group" or "an optionally substituted aralkyloxycarbonyl group", each of the substituents described above is converted to "a carboxyl group" in some cases under physiological conditions in a living body by oxidation, reduction, hydrolysis or the like by an enzyme, or hydrolysis by acid in the stomach, or the like.

A process for producing the compound represented by the formula (I) of the present invention is explained below with reference to examples, which should not be construed as limiting the scope of the invention. In the present specification, the following abbreviations are used in some cases for the simplification of description.
Boc: tert-butoxycarbonyl group
Cbz: benzyloxycarbonyl group
TMS: trimethylsilyl group
TBS: tert-butyldimethylsilyl group
SEM: 2-[(trimethylsilyl)ethoxy]methyl group
Ac: acetyl group
Me: methyl group
Et: ethyl group
Pr: propyl group
i-Pr: isopropyl group
Bu: butyl group
i-Bu: isobutyl group
t-Bu: t-butyl group
Ph: phenyl group
Bn: benzyl group
Ms: methanesulfonyl group
TFA: trifluoroacetic acid

The compound represented by the formula (I) may be synthesized from a well-known compound by a combination of well-known synthesis processes. It may be synthesized, for example, by any of the following processes.

### Production Process 1

A compound of the formula (1-17) as the compound of the formula (I), or a salt thereof is produced, for example, by the following process: 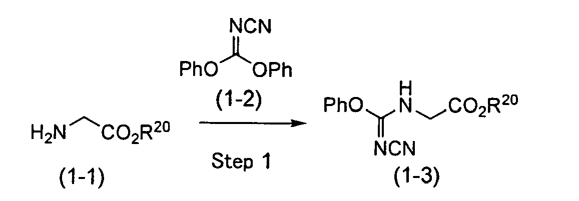 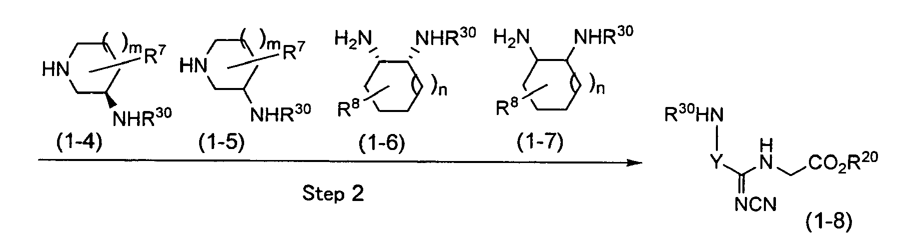 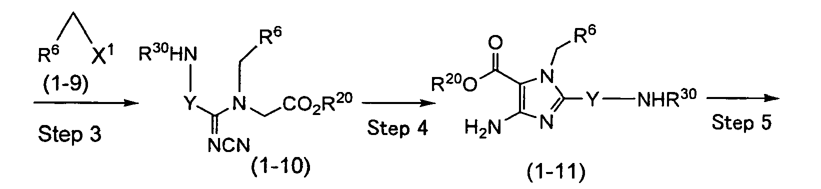 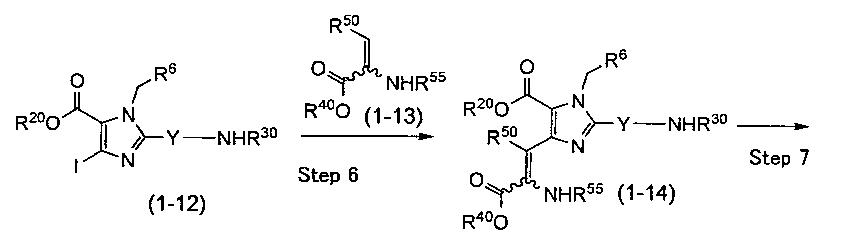 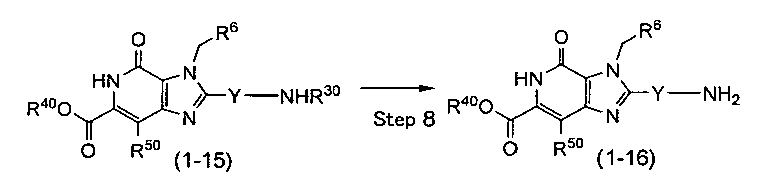 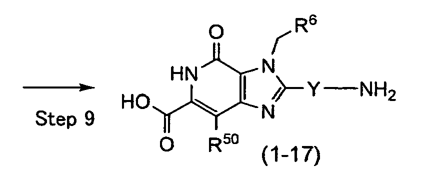 wherein m, n, R⁶, R⁷, R⁸ and Y are as defined above; X¹ is a leaving group (for example, a bromine atom, a chlorine atom, an iodine atom, methanesulfonyloxy, trifluoromethanesulfonyloxy or p-toluenesulfonyloxy); R²⁰ is methyl, ethyl, propyl, 2-propyl or benzyl; R³⁰ is Boc or Cbz; R⁴⁰ is methyl or ethyl; R⁵⁰ is a hydrogen atom, methyl or ethyl; and R⁵⁵ is acetyl or benzoyl.

### 1) Step 1

A compound (1-3) may be produced from a compound (1-1) by the same production process as described in literature (for example, Bioorg. Med. Chem. Lett. 12, 653 (2002), Chem. Pharm. Bull. 45, 2005 (1997), Tetrahedron Letters 39, 7983 (1998), Tetrahedron 46, 7803 (1990), Tetrahedron Letters 32, 691 (1991), Tetrahedron 51, 5369 (1995), J. Med. Chem. 38, 3236 (1995) and J. Heterocycl. Chem. 24, 275 (1987)).

### 2) Step 2

A compound (1-8) may be produced from the compound (1-3) by the following process A or B.
A: A compound (1-8) may be produced by reacting the compound (1-3) with a compound (1-4) or a compound (1-5) in an inert solvent in the presence or absence of an additive and in the presence or absence of a base. The additive includes, for example, 4-(dimethylamino)pyridine. The base includes, for example, diisopropylethylamine, triethylamine, pyridine, N-methylmorpholine and 1-methylpiperidine. Preferable examples thereof are diisopropylethylamine and triethylamine. The amount of the base used is usually chosen in the range of 1 to 10 equivalents per equivalent of the compound (1-3). The inert solvent includes, for example, alcohol solvents (e.g. ethanol, methanol and 2-propanol), ether solvents (e.g. 1,4-dioxane), and mixed solvents thereof. The reaction temperature may be chosen in the range of about 50°C to about 200°C. It is also possible to carry out the reaction in a closed reaction vessel such as an autoclave.
B: A compound (1-8) may be produced by reacting the compound (1-3) with a compound (1-6) or a compound (1-7) in an inert solvent in the presence or absence of an additive and in the presence or absence of a base. The additive includes, for example, 4-(dimethylamino)pyridine. The base includes, for example, diisopropylethylamine, triethylamine, pyridine and N-methylmorpholine. A preferable example thereof is diisopropylethylamine. The amount of the base used is usually chosen in the range of 1 to 10 equivalents per equivalent of the compound (1-3). The inert solvent includes, for example, N-methyl-2-piperidone, N-methyl-2-pyrrolidinone, alcohol solvents (e.g. ethanol, methanol and 2-propanol), N,N-dimethylformamide, toluene, and mixed solvents thereof. Preferable examples thereof are N-methyl-2-piperidone and N-methyl-2-pyrrolidinone. The reaction temperature may be chosen in the range of about 50°C to about 200°C. It is also possible to carry out the reaction in a closed reaction vessel such as an autoclave.

### 3) Step 3

A compound (1-10) may be produced by reacting the compound (1-8) with a compound (1-9) in an inert solvent in the presence or absence of a base (see, for example, J. Heterocycl. Chem. 37, 1033 (2000), J. Chem. Soc., Perkin Trans. 1, 13, 1833 (1999) and J. Med. Chem. 38, 3838 (1995)). The amount of the compound (1-9) used is usually chosen in the range of 1 to 3 equivalents per equivalent of the compound of the formula (1-8). The base includes, for example, alkali carbonates (e.g. potassium carbonate, sodium carbonate, potassium hydrogencarbonate and sodium hydrogencarbonate), alkali hydrides (e.g. sodium hydride and potassium hydride) and alkali hydroxides (e.g. potassium hydroxide and sodium hydroxide). A preferable example thereof is potassium carbonate. The amount of the base used is usually chosen in the range of 1 to 5 equivalents per equivalent of the compound (1-8). The inert solvent includes, for example, aprotic solvents (e.g. N,N-dimethylformamide and dimethyl sulfoxide), ether solvents (e.g. diethyl ether, tetrahydrofuran and 1,4-dioxane), ketones (e.g. acetone), and mixed solvents thereof. Preferable examples thereof are N,N-dimethylformamide and dimethyl sulfoxide. The reaction temperature may be chosen in the range of about 10°C to about 120°C.

In general, compounds having a R⁶CH₂ group introduced into a different nitrogen atom can also be produced as by-products in the production of the compound (1-10). The by-products can easily be removed by a conventional purification method.

### 4) Step 4

A compound (1-11) may be produced from the compound (1-10) by the same production process as described in literature (for example, WO02/068420).

### 5) Step 5

A compound (1-12) may be produced from the compound (1-11) by the same production process as described in literature (for example, W099/8, Tetrahedron Letters 38, 7963 (1997), Bioorg. Med. Chem. Lett. 12, 543 (2002), Heterocycles 57, 123 (2002), Tetrahedron Letters 41, 9957 (2000) and Tetrahedron Letters 42, 2201 (2001)).

### 6) Step 6

A compound (1-14) may be produced from the compound (1-12) by the same production process as described in literature (for example, Tetrahedron Letters 43, 5079 (2002)).

### 7) Step 7

A compound (1-15) may be produced by reacting the compound (1-14) in an inert solvent in the presence or absence of an additive and in the presence or absence of a base. The additive includes, for example, 4-(dimethylamino)pyridine. The base includes, for example, alkali hydroxides (e.g. potassium hydroxide and sodium hydroxide), alkali hydrides (e.g. sodium hydride and potassium hydride) and alkoxy alkalis (sodium methoxide, sodium ethoxide and potassium t-butoxide). Preferable examples thereof are sodium methoxide and sodium ethoxide. The amount of the base used is usually chosen in the range of 1 equivalent to large excess equivalents per equivalent of the compound (1-14). The inert solvent includes, for example, alcohol solvents (e.g. ethanol, methanol and 2-propanol), ether solvents (e.g. tetrahydrofuran), and mixed solvents thereof. The reaction temperature may be chosen in the range of about 10°C to about 100°C.

### 8) Step 8

When R³⁰ is Boc in the compound (1-15), a compound (1-16) may be produced by removing the Boc group of the compound (1-15) to effect deprotection, in an inert solvent in the presence of an acid. The acid includes, for example, hydrochloric acid, sulfuric acid and trifluoroacetic acid. Preferable examples thereof are hydrochloric acid and trifluoroacetic acid. The amount of the acid used is usually chosen in the range of 1 equivalent to large excess equivalents per equivalent of the compound (1-15). The inert solvent includes, for example, halogenated hydrocarbon solvents (e.g. dichloromethane, dichloroethane and chloroform), ether solvents (e.g. 1,4-dioxane), and mixed solvents thereof. The reaction temperature may be chosen in the range of about -20°C to about 30°C.

When R³⁰ is Cbz, a compound (1-16) may be produced from the compound (1-15) by the same production process as described in literature (for example, J. Am. Chem. Soc. 85, 2149 (1963), Tetrahedron Lett. 41, 3029 (2000) and Tetrahedron Lett. 36, 8677 (1995)).

### 9) Step 9

The compound (1-17) may be produced from the compound (1-16) by the same production process as described in literature (for example, J. Org. Chem. 61, 215 (1996), J. Org. Chem. 61, 9437 (1996) and J. Org. Chem. 59, 6147 (1994)).

### Production Process 2

Compounds of the formula (2-3), formula (2-4) and formula (2-7) as the compound of the formula (I), or salts thereof are produced, for example, by the following processes: 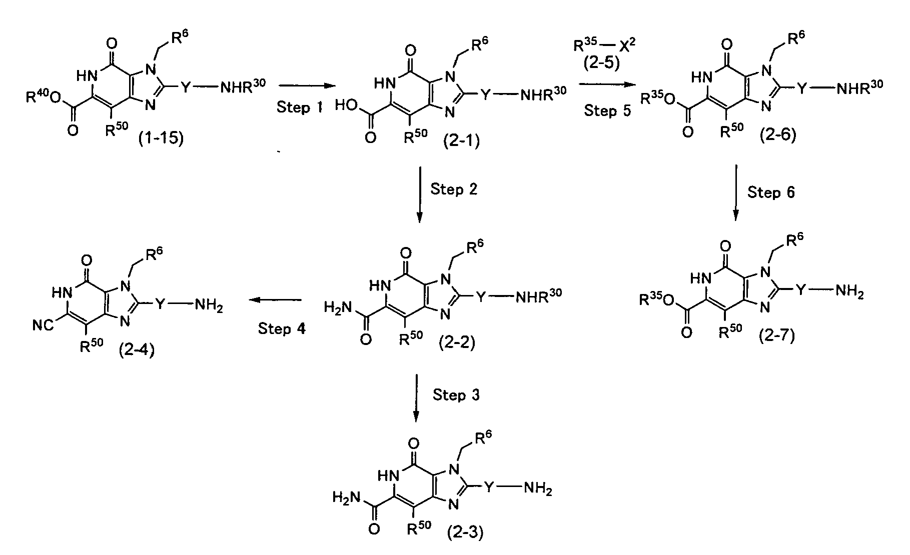 wherein R⁶, R³⁰, R⁴⁰, R⁵⁰ and Y are as defined above; R³⁵OC(O) is "an optionally substituted alkoxycarbonyl group", "an optionally substituted aryloxycarbonyl group", "an optionally substituted aralkyloxycarbonyl group", "an optionally substituted cycloalkyloxycarbonyl group" or "an esterified carboxyl group"; and X² is a leaving group (for example, a bromine atom, a chlorine atom, an iodine atom, methanesulfonyloxy, trifluoromethanesulfonyloxy or p-toluenesulfonyloxy).

### 1) Step 1

A compound (2-1) may be produced from a compound (1-15) by the same production process as described in the step 9 in the production process 1.

### 2) Step 2

A compound (2-2) may be produced from the compound (2-1) by the same production process as described in literature (for example, Heterocycles 53, 797 (2000), Bioorg. Med. Chem. Lett. 7, 739 (1997) and Org. Prep. Proced. Int. 26, 429 (1994)).

When the compound (2-3) is produced by deprotection by the removal of the Boc group of the compound (2-2) in this step, the compound (2-2) may be produced from the compound (2-3) by the same process as that described in literature (for example, Protective Groups in Organic Synthesis 2nd Edition (John Wiley & Sons, Inc.)), or the like.

### 3) Step 3

The compound (2-3) may be produced from the compound (2-2) by the same production process as described in the step 8 in the production process 1.

### 4) Step 4

The compound (2-4) may be produced from the compound (2-2) by the same production process as described in literature (for example, Tetrahedron Lett. 38, 1241 (1997) and Synth. Commun. 22, 2811 (1992)).

When a compound (2-5) represented by the formula: 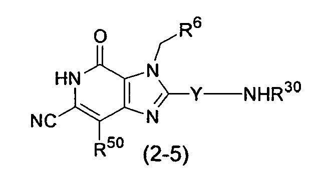 wherein R⁶, R³⁰, R⁵⁰ and Y are as defined above, is produced by the protection of the compound (2-4) by R³⁰ in this step, the compound (2-4) may be produced from the compound (2-5) by the same production process as described in the step 8 in the production process 1.

### 5) Step 5

A compound (2-6) may be produced from a compound (2-1) by the same production process as described in the step 1 in the production process 3. As a compound (2-5), a commercial reagent may be used, or the compound (2-5) may be produced by the same production process as described in literature (for example, WO03/027098, WO00/06581, and R.C. Ralock, "Comprehensive Organic transformation", VCH publisher Inc., 1989).

### 6) Step 6

The compound (2-7) may be produced from the compound (2-6) by the same production process as described in the step 8 in the production process 1.

### Production Process 3

Compounds of the formula (3-4) and the formula (3-6) as the compound of the formula (I), or salts thereof are produced, for example, by the following processes: 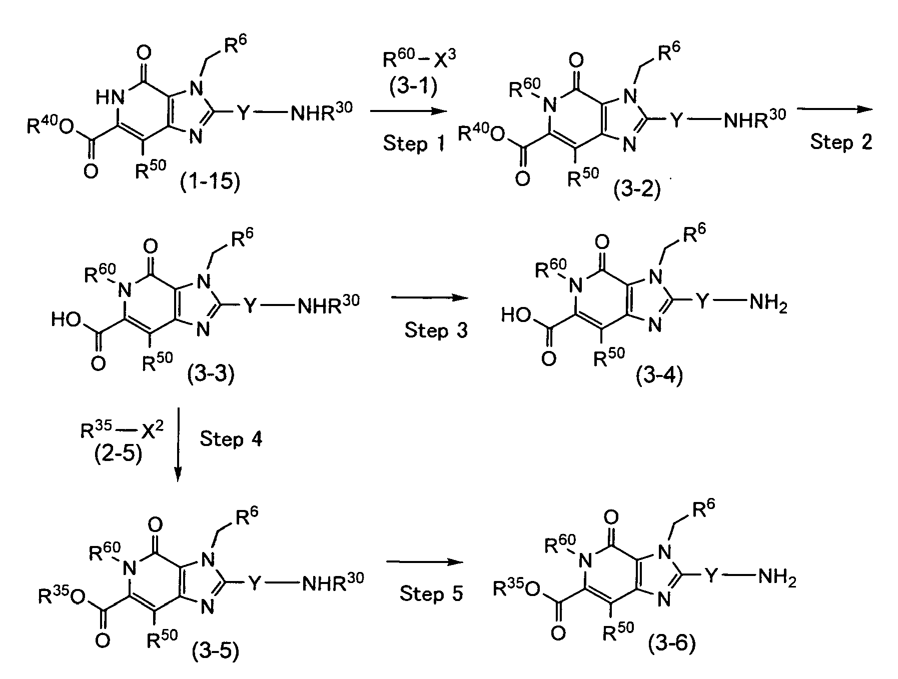 wherein R⁶, R³⁰, R³⁵, R⁴⁰, R⁵⁰, Y and X² are as defined above; X³ is a leaving group (for example, a bromine atom, a chlorine atom, an iodine atom, methanesulfonyloxy, trifluoromethanesulfonyloxy or p-toluenesulfonyloxy); and R⁶⁰ is "an optionally substituted alkyl group" or "an optionally substituted cycloalkyl group".

### 1) Step 1

A compound (3-2) may be produced by reacting a compound (1-15) with a compound (3-1) in an inert solvent in the presence of a base. The amount of the compound (3-1) used is usually chosen in the range of 1 to 3 equivalents per equivalent of the compound (1-15). The base includes, for example, alkali carbonates (e.g. potassium carbonate, sodium carbonate, potassium hydrogencarbonate and sodium hydrogencarbonate), alkali hydroxides (e.g. potassium hydroxide and sodium hydroxide), alkali hydrides (e.g. sodium hydride and potassium hydride) and alkoxy alkalis (e.g. potassium t-butoxide). Preferable examples thereof are potassium carbonate and sodium hydride. The amount of the base used is usually chosen in the range of 1 to 5 equivalents per equivalent of the compound (1-15). The inert solvent includes, for example, aprotic solvents (e.g. N,N-dimethylformamide and dimethyl sulfoxide), ether solvents (e.g. diethyl ether, tetrahydrofuran and 1,4-dioxane), ketones (e.g. acetone), and mixed solvents thereof. A preferable example thereof is N,N-dimethylformamide. The reaction temperature may be chosen in the range of about 10°C to about 100°C.

### 2) Step 2

A compound (3-3) may be produced from the compound (3-2) by the same production process as described in the step 9 in the production process 1.

### 3) Step 3

The compound (3-4) may be produced from the compound (3-3) by the same production process as described in the step 8 in the production process 1.

### 4) Step 4

A compound (3-5) may be produced from the compound (3-3) by the same production process as described in the step 5 in the production process 2.

### 5) Step 5

The compound (3-6) may be produced from the compound (3-5) by the same production process as described in the step 8 in the production process 1.

### Production Process 4

A compound of the formula (4-1) as the compound of the formula (I), or a salt thereof is produced, for example, by the following process: 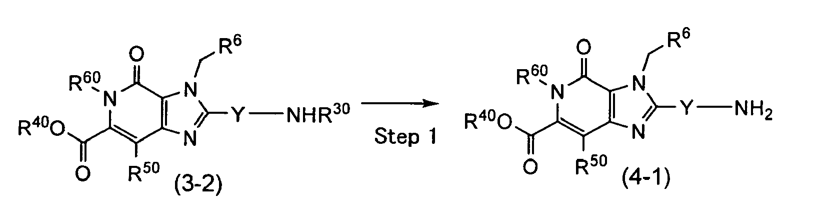 wherein R⁶, R³⁰, R⁴⁰, R⁵⁰, R⁶⁰ and Y are as defined above.

### 1) Step 1

The compound (4-1) may be produced from a compound (3-2) by the same production process as described in the step 8 in the production process 1.

### Production Process 5

A compound of the formula (5-3) as the compound of the formula (I), or a salt thereof is produced, for example, by the following process: 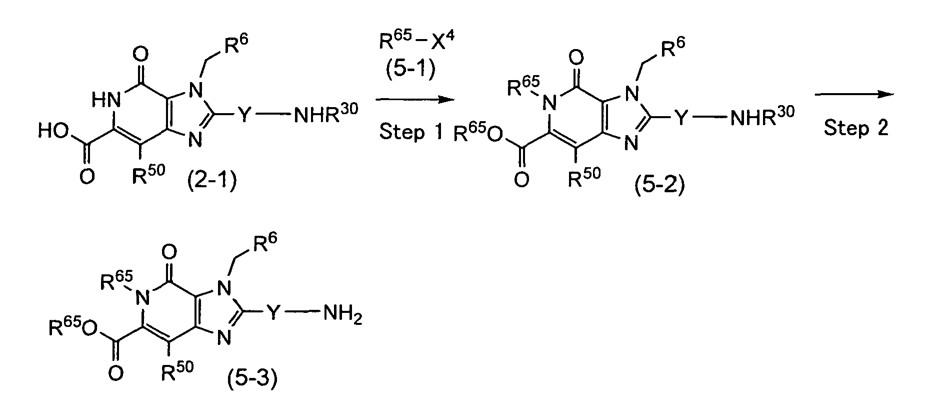 wherein R⁶, R³⁰, R⁵⁰ and Y are as defined above; R⁶⁵ is "an optionally substituted alkyl group" or "an optionally substituted cycloalkyl group"; and X⁴ is a leaving group (for example, a bromine atom, a chlorine atom, methanesulfonyloxy, trifluoromethanesulfonyloxy or p-toluenesulfonyloxy).

### 1) Step 1

A compound (5-2) may be produced from a compound (2-1) by the same production process as described in the step 1 in the production process 3.

### 2) Step 2

The compound (5-3) may be produced from the compound (5-2) by the same production process as described in the step 8 in the production process 1.

### Production Process 6

A compound of the formula (6-3) as the compound of the formula (I), or a salt thereof is produced, for example, by the following process: 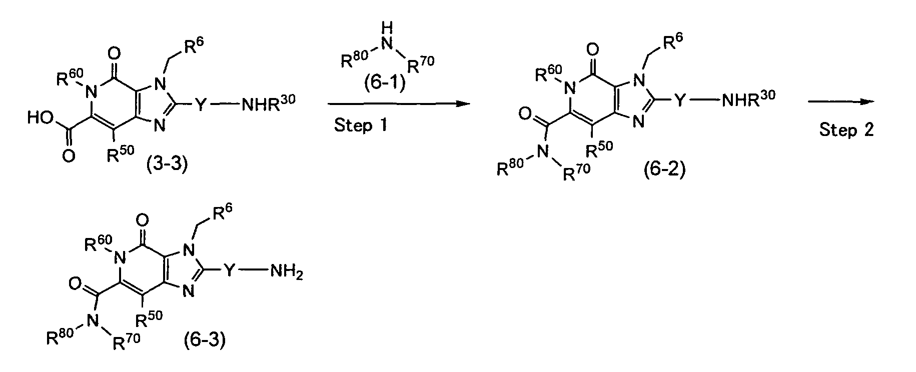 wherein R⁶, R³⁰, R⁵⁰, R⁶⁰ and Y are as defined above; and R⁸⁰R⁷⁰NC(O) is "an optionally substituted carbamoyl group".

### 1) Step 1

A compound (6-2) may be produced from a compound (3-3) by the same production process as described in literature (for example, R.C. Ralock, "Comprehensive Organic transformation", VCH publisher Inc., 972-976 (1989)).

### 2) Step 2

The compound (6-3) may be produced from the compound (6-2) by the same production process as described in the step 8 in the production process 1.

### Production Process 7

A compound of the formula (7-4) as the compound of the formula (I), or a salt thereof is produced, for example, by the following process: 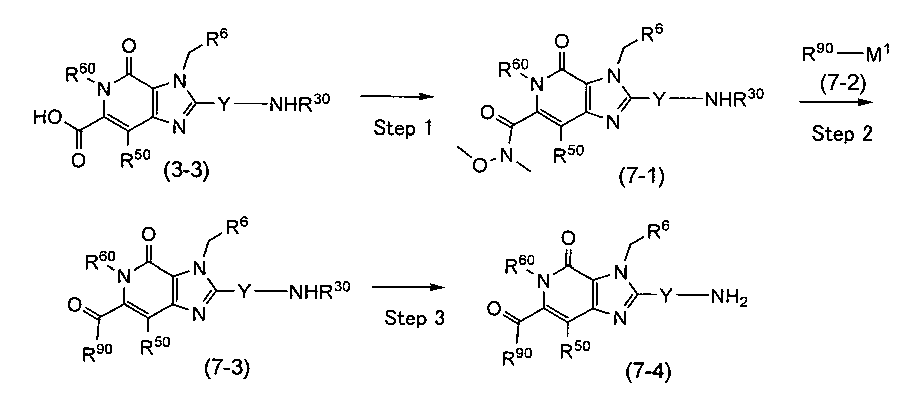 wherein R⁶, R³⁰, R⁵⁰, R⁶⁰ and Y are as defined above; M¹ is lithium, magnesium chloride or magnesium bromide; and C(O)R⁹⁰ is "an optionally substituted alkylcarbonyl group", "an optionally substituted aroyl group" or "an optionally substituted heteroarylcarbonyl group".

### 1) Step 1 to Step 2

A compound (7-3) may be produced from a compound (3-3) by the same production process as described in literature (for example, Bioorg. Med. Chem. Lett. 11, 2951 (2001), Tetrahedron Letters 42, 8955 (2001), Synthesis 1852 (2000), Organic Letters 2, 4091 (2000), Tetrahedron Letters 42, 5609 (2001), Synthesis 2239 (2001), Synlett 5, 715 (2002), J. Org. Chem. 67, 5032 (2002), Bioorg. Med. Chem. Lett. 11, 287 (2001) and Tetrahedron Letters 42, 3763 (2001)). As a compound (7-2), a commercial one may be used, or the compound (7-2) may be produced by the process described, for example, in Japanese Chemical Association, "Jikken Kagaku Koza (Experimental Chemistry)" Vol. 25, Maruzen Co., Ltd.

### 2) Step 3

The compound (7-4) may be produced from the compound (7-3) by the same production process as described in the step 8 in the production process 1.

### Production Process 8

A compound of the formula (8-5) as the compound of the formula (I), or a salt thereof is produced, for example, by the following process: 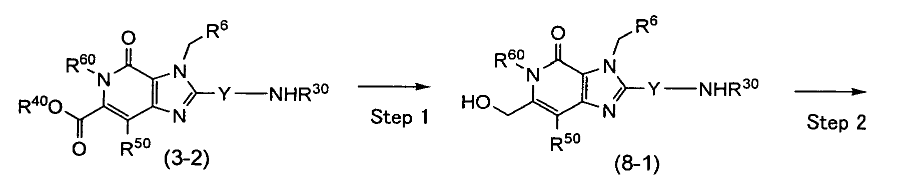 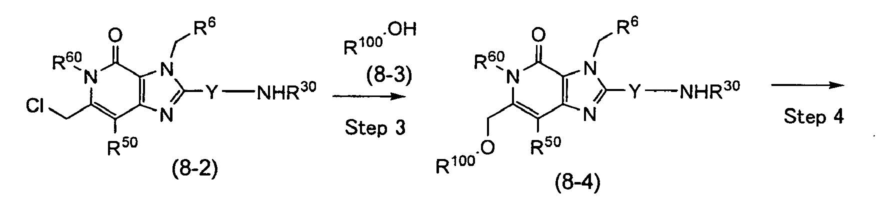 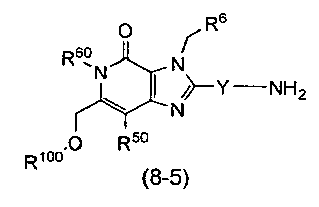 wherein R⁶, R³⁰, R⁴⁰, R⁵⁰, R⁶⁰ and Y are as defined above; and R¹⁰⁰O is the "optionally substituted aryloxy group" or "optionally substituted alkoxy group" exemplified as the substituent(s) of the "optionally substituted alkyl group".

### 1) Step 1

A compound (8-1) may be produced from a compound (3-2) by the same production process as described, for example, in Japanese Chemical Association, "Jikken Kagaku Koza (Experimental Chemistry)" Vol. 20 and Vol. 22, Maruzen Co., Ltd.

### 2) Step 2

A compound (8-2) may be produced from the compound (8-1) by the same production process as described, for example, in Japanese Chemical Association, "Jikken Kagaku Koza (Experimental Chemistry)" Vol. 19, Maruzen Co., Ltd.

### 3) Step 3

A compound (8-4) may be produced from the compound (8-2) by the same production process as described, for example, in Japanese Chemical Association, "Jikken Kagaku Koza (Experimental Chemistry)" Vol. 20, Maruzen Co., Ltd.

### 4) Step 4

The compound (8-5) may be produced from the compound (8-4) by the same production process as described in the step 8 in the production process 1.

### Production Process 9

A compound of the formula (9-6) as the compound of the formula (I), or a salt thereof is produced, for example, by the following process: 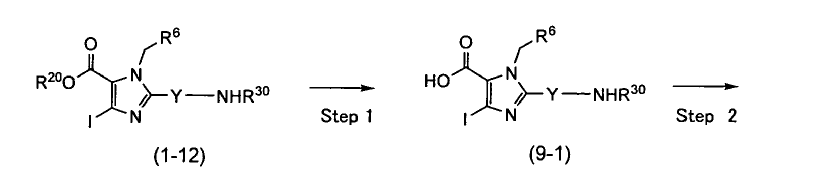 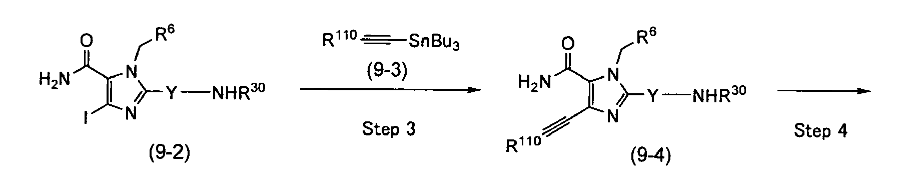 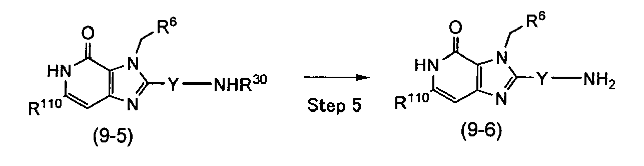 wherein R⁶, R²⁰, R³⁰ and Y are as defined above; and R¹¹⁰ is a hydrogen atom, "an optionally substituted alkyl group", "an optionally substituted cycloalkyl group", "an optionally substituted aryl group", "an optionally substituted aralkyl group", "an optionally substituted heteroaryl group" or "an optionally substituted heteroarylalkyl group", or a trimethylsilyl group.

### 1) Step 1

A compound (9-1) may be produced from a compound (1-12) by the same production process as described in the step 9 in the production process 1.

### 2) Step 2

A compound (9-2) may be produced from the compound (9-1) by the same production process as described in the step 2 in the production process 2.

### 3) Steps 3 to 4

A compound (9-5) may be produced from the compound (9-2) by the same production process as described in literature (for example, Chem. Pharm. Bull. 44, 288 (1996), J. Med. Chem. 34, 778 (1991) and Tetrahedron 49, 557 (1993)). The step 3 may be carried out with reference to the production process described in literature (for example, Chem. Rev. 103, 1979 (2003) and Chem. Rev. 103, 1875 (2003)).

### 4) Step 5

The compound (9-6) may be produced from the compound (9-5) by the same production process as described in the step 8 in the production process 1.

### Production Process 10

Compounds of the formula (10-3), formula (10-6) and formula (10-8) as the compound of the formula (I), or salts thereof are produced, for example, by the following processes: 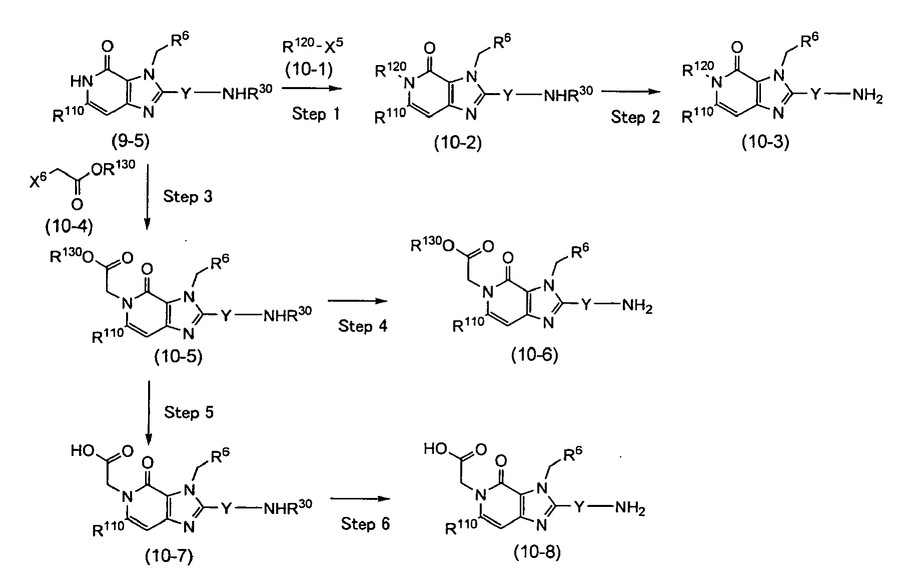 wherein R⁶, R³⁰, R¹¹⁰ and Y are as defined above; R¹²⁰ is methyl, ethyl, propyl or 2-propyl; R¹³⁰ is methyl or ethyl; and each of X⁵ and X⁶ is a leaving group (for example, a bromine atom, a chlorine atom, methanesulfonyloxy, trifluoromethanesulfonyloxy or p-toluenesulfonyloxy).

### 1) Step 1

A compound (10-2) may be produced from a compound (9-5) by the same production process as described in the step 1 in the production process 3.

### 2) Step 2

The compound (10-3) may be produced from the compound (10-2) by the same production process as described in the step 8 in the production process 1.

### 3) Step 3

A compound (10-5) may be produced from a compound (9-5) by the same production process as described in the step 1 in the production process 3.

### 4) Step 4

The compound (10-6) may be produced from the compound (10-5) by the same production process as described in the step 8 in the production process 1.

### 5) Step 5

A compound (10-7) may be produced from the compound (10-5) by the same production process as described in the step 9 in the production process 1.

### 6) Step 6

The compound (10-8) may be produced from the compound (10-7) by the same production process as described in the step 8 in the production process 1.

### Production Process 11

A compound of the formula (11-3) as the compound of the formula (I), or a salt thereof is produced, for example, by the following process: 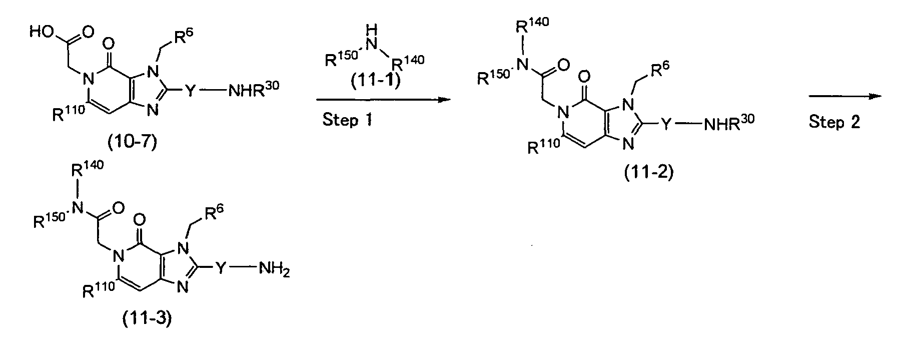 wherein R⁶, R³⁰, R¹¹⁰ and Y are as defined above; and R¹⁴⁰R¹⁵⁰NC(O) is the "optionally substituted carbamoyl group" exemplified as the substituent(s) of the "optionally substituted alkyl group".

### 1) Step 1

A compound (11-2) may be produced from a compound (10-7) by the same production process as described in the step 1 in the production process 6.

### 2) Step 2

The compound (11-3) may be produced from the compound (11-2) by the same production process as described in the step 8 in the production process 1.

### Production Process 12

A compound of the formula (12-4) as the compound of the formula (I), or a salt thereof is produced, for example, by the following process: 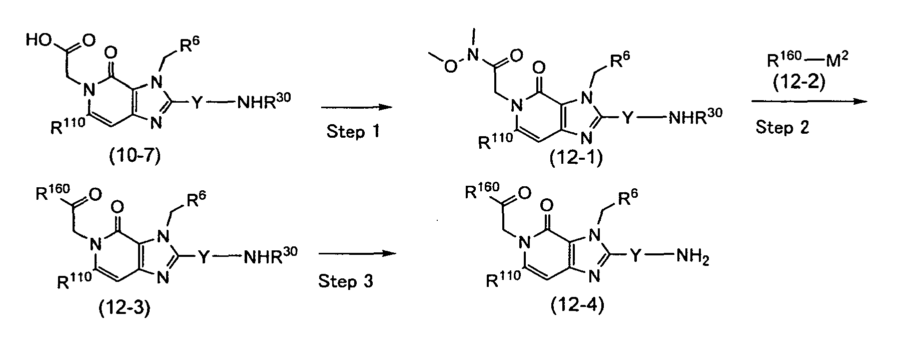 wherein R⁶, R³⁰, R¹¹⁰ and Y are as defined above; M² is lithium, magnesium chloride or magnesium bromide; and C(O)R¹⁶⁰ is the "optionally substituted aroyl group" or "optionally substituted nitrogen-containing heteroarylcarbonyl group" exemplified as the substituent(s) of the "optionally substituted alkyl group".

### 1) Step 1 to Step 2

A compound (12-3) may be produced from a compound (10-7) by the same production process as described in the step 1 to step 2 in the production process 7. As a compound (12-2), a commercial one may be used, or the compound (12-2) may be produced by the process described, for example, in Japanese Chemical Association, "Jikken Kagaku Koza (Experimental Chemistry)" Vol. 25, Maruzen Co., Ltd.

### 2) Step 3

The compound (12-4) may be produced from the compound (12-3) by the same production process as described in the step 8 in the production process 1.

### Production Process 13

A compound of the formula (13-5) as the compound of the formula (I), or a salt thereof is produced, for example, by the following process: 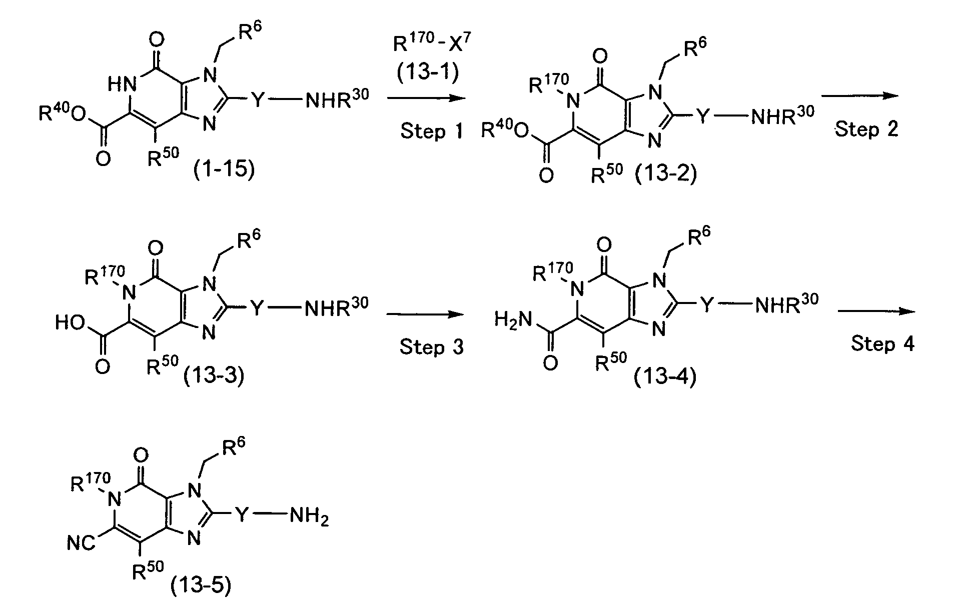 wherein R⁶, R³⁰, R⁴⁰, R⁵⁰ and Y are as defined above; R¹⁷⁰ is "an optionally substituted alkyl group" or "an optionally substituted cycloalkyl group"; and X⁷ is a leaving group (for example, a bromine atom, a chlorine atom, methanesulfonyloxy, trifluoromethanesulfonyloxy or p-toluenesulfonyloxy).

### 1) Step 1

A compound (13-2) may be produced from a compound (1-15) by the same production process as described in the step 1 in the production process 3.

### 2) Step 2

A compound (13-3) may be produced from the compound (13-2) by the same production process as described in the step 9 in the production process 1.

### 3) Step 3

A compound (13-4) may be produced from the compound (13-3) by the same production process as described in the step 2 in the production process 2.

### 4) Step 4

The compound (13-5) may be produced from the compound (13-4) by the same production process as described in the step 4 in the production process 2.

When a compound (13-6) represented by the formula: 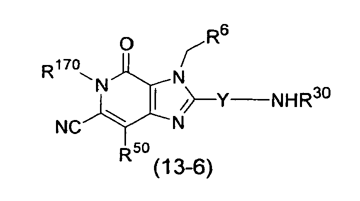 wherein R⁶, R³⁰, R⁵⁰, R¹⁷⁰ and Y are as defined above, is produced by the protection of the compound (13-5) by R³⁰ in this step, the compound (13-5) may be produced from the compound (13-6) by the same production process as described in the step 8 in the production process 1.

### Production Process 14

Compounds of the formula (14-5) and the formula (14-7) as the compound of the formula (I), or salts thereof are produced, for example, by the following processes: 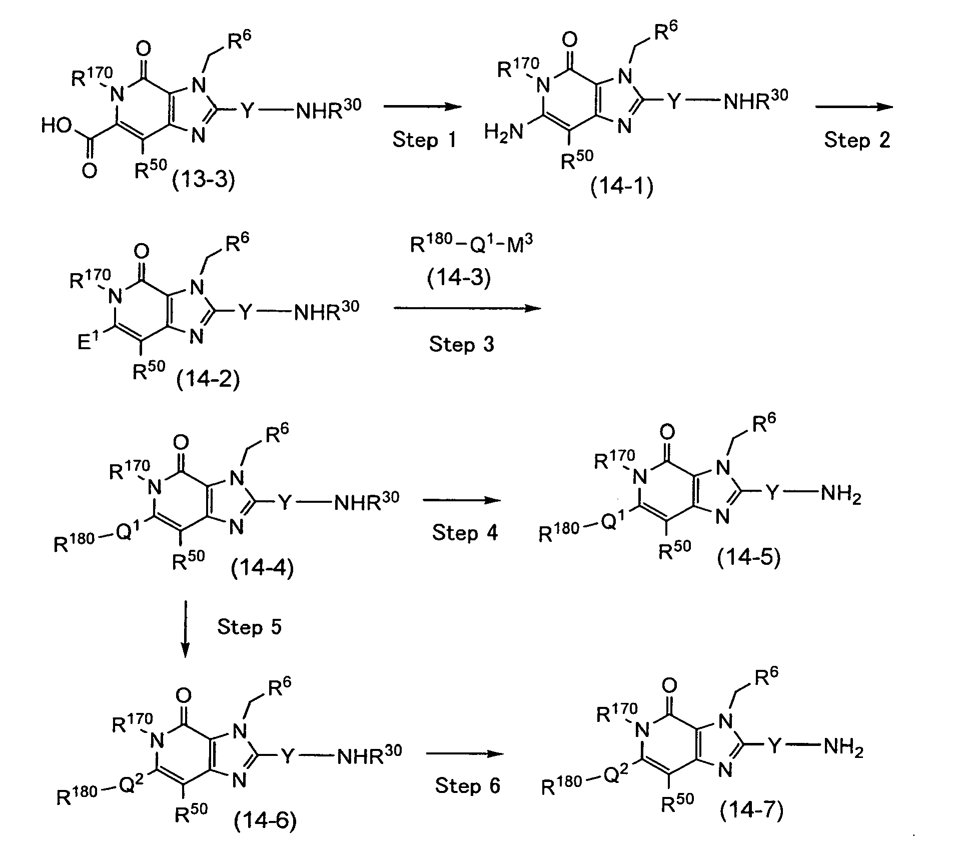 wherein R⁶, R³⁰, R⁵⁰, R¹⁷⁰ and Y are as defined above; R¹⁸⁰-Q¹ is "an optionally substituted aryloxy group", "an optionally substituted arylthio group" or "an optionally substituted heteroaryloxy group"; R¹⁸⁰-Q² is "an optionally substituted arylsulfonyl group"; E¹ is a chlorine atom or a bromine atom; and M³ is lithium, sodium, potassium or cesium.

### 1) Step 1

A compound (14-1) may be produced from a compound (13-3) by the same production process as described in literature (for example, R.C. Ralock, "Comprehensive Organic transformation", VCH publisher Inc., 972-976 (1989) and Eur. J. Org. Chem. 1353 (2000)). When a compound (14-8) represented by the formula: 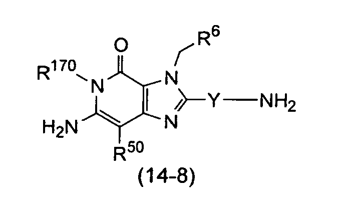 wherein R⁶, R⁵⁰, R¹⁷⁰ and Y are as defined above, is produced by deprotection by the removal of Boc for R³⁰ of the compound (14-1) in this step, the compound (14-1) may be produced by the following process. That is, the compound (14-8) is reacted with di-tert-butyl dicarbonate in an inert solvent in the presence of a base. The amount of di-tert-butyl dicarbonate used is usually chosen in the range of 3 to 6 equivalents per equivalent of the compound (14-8). The base includes, for example, inorganic bases such as sodium hydroxide, potassium carbonate, etc.; and organic bases such as triethylamine, etc. The inert solvent includes, for example, ether solvents (e.g. tetrahydrofuran and 1,4-dioxane). The reaction temperature is chosen in the range of about -10°C to about 40°C.

### 2) Step 2

A compound (14-2) may be produced from the compound (14-1) by the same production process as described in literature (for example, R.C. Ralock, "Comprehensive Organic transformation", VCH publisher Inc., 972-976 (1989) and Eur. J. Org. Chem. 1353 (2000)). When the Boc group of the compound (14-2) is removed, namely, deprotection is caused, the compound (14-2) may be produced by introduction of Boc by the same production process as described in the step 1 in the production process 14.

### 3) Step 3

A compound (14-4) may be produced from the compound (14-2) by the same production process as described in literature (for example, Heterocycles 52, 253 (2000)).

### 4) Step 4

The compound (14-5) may be produced from the compound (14-4) by the same production process as described in the step 8 in the production process 1.

### 5) Step 5

When Q¹ of the compound (14-4) is a sulfur atom, a compound (14-6) may be produced by the conversion of Q¹ to sulfone by the same production process as described in literature (for example, R.C. Ralock, "Comprehensive Organic transformation", VCH publisher Inc., 972-976 (1989) and Eur. J. Org. Chem. 1353 (2000)).

### 6) Step 6

The compound (14-7) may be produced from the compound (14-6) by the same production process as described in the step 8 in the production process 1.

### Production Process 15

A compound of the formula (15-5) as the compound of the formula (I), or a salt thereof is produced, for example, by the following process: 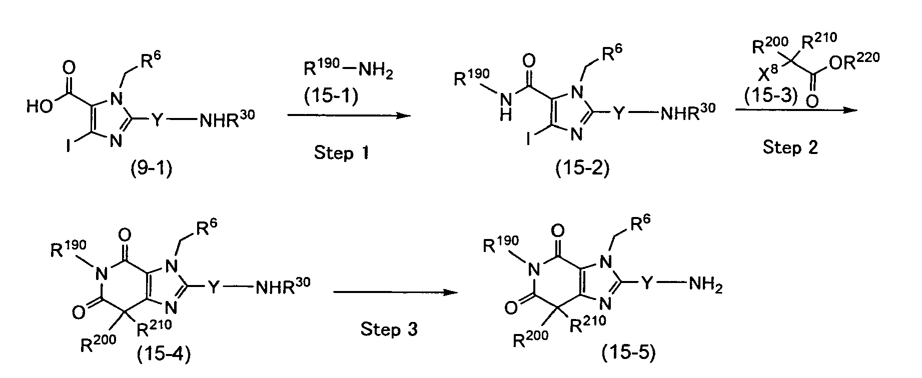 wherein R⁶, R³⁰ and Y are as defined above; R¹⁹⁰ is "an optionally substituted alkyl group" or "an optionally substituted cycloalkyl group"; each of R²⁰⁰ and R²¹⁰, which may be the same or different, is a hydrogen atom, a fluorine atom, methyl or ethyl; and X⁸ is a chlorine atom or a bromine atom.

### 1) Step 1

A compound (15-2) may be produced from a compound (9-1) by the same production process as described in the step 1 in the production process 6.

### 2) Step 2

A compound (15-4) may be produced from the compound (15-2) by the same production process as described in literature (for example, Chem. Pharm. Bull. 40, 982 (2000)).

### 3) Step 3

The compound (15-5) may be produced from the compound (15-4) by the same production process as described in the step 8 in the production process 1.

### Production Process 16

A compound of the formula (16-7) as the compound of the formula (I), or a salt thereof is produced, for example, by the following process: 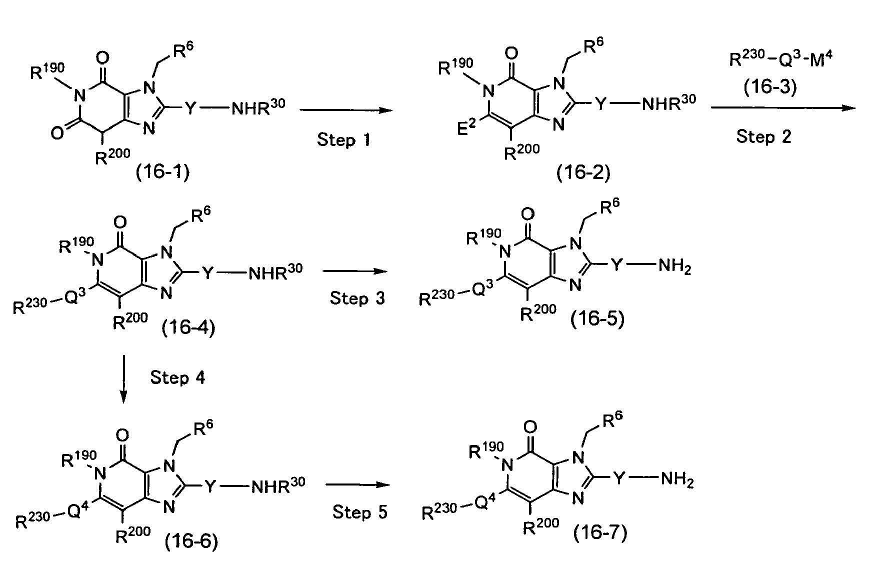 wherein the compound (16-1) corresponds to the compound (15-4) described in the production process 15 when R²¹⁰ is a hydrogen atom; R⁶, R³⁰, R¹⁹⁰, R²⁰⁰ and Y are as defined above; R²³⁰-Q³ is "an optionally substituted aryloxy group", "an optionally substituted arylthio group" or "an optionally substituted heteroaryloxy group"; R²³⁰-Q⁴ is "an optionally substituted arylsulfonyl group"; E² is a chlorine atom or a bromine atom; and M⁴ is lithium, sodium, potassium or cesium.

### 1) Step 1

A compound (16-2) may be produced from the compound (16-1) by the same production process as described in literature (for example, Heterocycles 37, 1147 (1994), J. Heterocycl. Chem. 34, 659 (1997), Tetrahedron 54, 9207 (1998), Chem. Pharm. Bull. 40, 846 (1992), Tetrahedron Lett. 25, 5043 (1984) and Tetrahedron Lett. 25, 4007 (1984)).

### 2) Step 2

A compound (16-4) may be produced from the compound (16-2) by the same production process as described in literature (for example, Heterocycles 52, 253 (2000), Tetrahedron Lett. 33, 2027 (1992) and Synthesis 11, 921 (1980)).

### 3) Step 3

A compound (16-5) may be produced from the compound (16-4) by the same production process as described in the step 8 in the production process 1.

### 4) Step 4

When Q¹ of the compound (16-4) is a sulfur atom, a compound (16-6) may be produced from the compound (16-4) by the same production process as described in the step 5 in the production process 14.

### 5) Step 5

The compound (16-7) may be produced from the compound (16-6) by the same production process as described in the step 8 in the production process 1.

### Production Process 17

A compound of the formula (17-6) as the compound of the formula (I), or a salt thereof is produced, for example, by the following process: 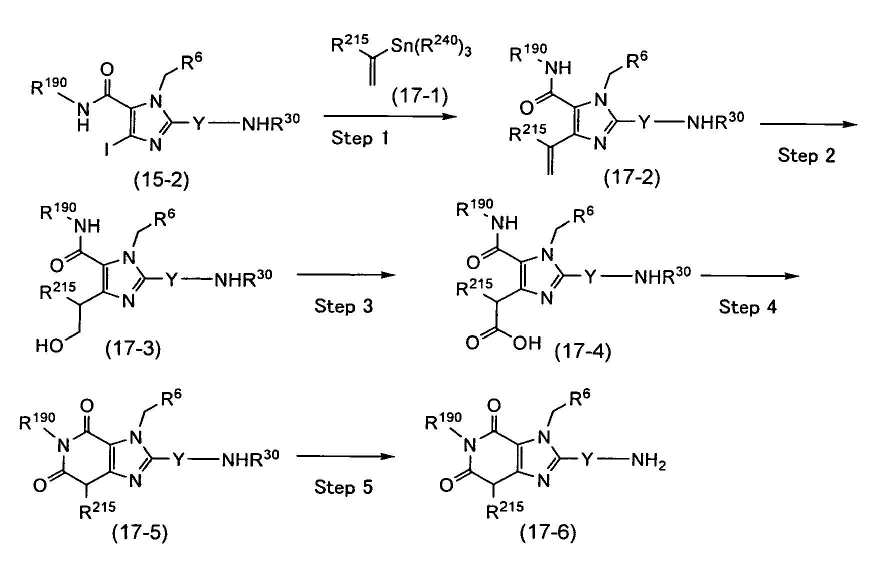 wherein R⁶, R³⁰, R¹⁹⁰ and Y are as defined above; R²¹⁵ is a hydrogen atom, methyl, ethyl or "an alkoxycarbonylmethyl group"; and R²⁴⁰ is methyl, ethyl, propyl, 2-propyl or butyl.

### 1) Step 1 to Step 2

A compound (17-3) may be produced from a compound (15-2) by the same production process as described in literature (for example, Bioorg. Med. Chem. Lett. 12, 827 (2002)).

### 2) Step 3

A compound (17-4) may be produced from the compound (17-3) by the same production process as described in literature (for example, J. Org. Chem. 68, 4999 (2003) and Organic Process Research & Development 7, 614 (2003)).

### 3) Step 4

A compound (17-5) may be produced from the compound (17-4) by the same production process as described in literature (for example, J. Am. Chem. Soc. 121, 975 (1999), Synth. Commun. 30, 341 (2000), Bioorg. Med. Chem. Lett. 9, 1625 (1999) and Sci. Pharm. 69, 161 (2001)).

### 4) Step 5

The compound (17-6) may be produced from the compound (17-5) by the same production process as described in the step 8 in the production process 1.

### Production Process 18

A compound of the formula (18-5) as the compound of the formula (I), or a salt thereof is produced, for example, by the following process: 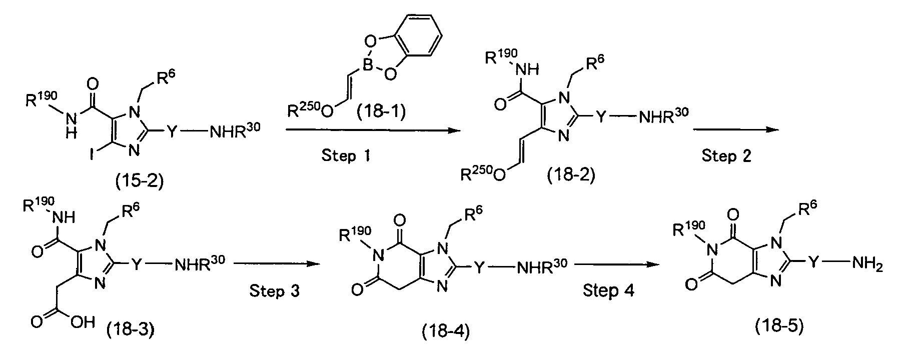 wherein R⁶, R³⁰, R¹⁹⁰ and Y are as defined above; and R²⁵⁰ is methyl, ethyl, propyl, 2-propyl or butyl.

### 1) Step 1

A compound (18-3) may be produced from a compound (15-2) by the same production process as described in literature (for example, J. Org. Chem. 47, 2117 (1982)).

### 2) Step 2

A compound (18-3) may be produced from the compound (18-2) by the same production process as described in literature (for example, J. Org. Chem. 61, 3200 (1996)).

### 3) Step 3

A compound (18-4) may be produced from the compound (18-3) by the same production process as described in the step 4 in the production process 17.

### 4) Step 4

The compound (18-5) may be produced from the compound (18-4) by the same production process as described in the step 8 in the production process 1.

### Production Process 19

The compound (18-3) described in the production process 18 is produced, for example, by the following process: 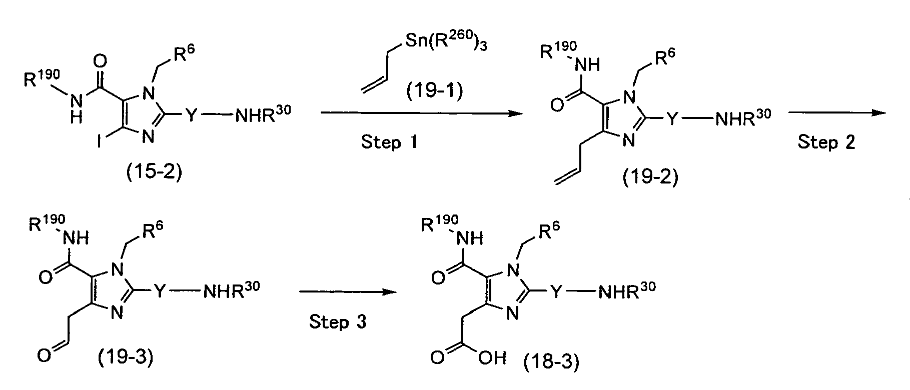 wherein R⁶, R³⁰, R¹⁹⁰ and Y are as defined above; and R²⁶⁰ is methyl, ethyl, propyl, 2-propyl or butyl.

### 1) Step 1

A compound (19-2) may be produced from a compound (15-2) by the same production process as described in literature (for example, Angew. Chem. Int Ed. Engl. 25, 508 (1986), Tetrahedron Lett. 31, 5877 (1990) and J. Org. Chem. 66, 9033 (2001)).

### 2) Steps 2 to 3

The compound (18-3) may be produced from the compound (19-2) by the same production process as described, for example, in Jikken Kagaku Koza (Experimental Chemistry) Vols. 20 to 23, Maruzen Co., Ltd. (published in 1992) and literature (for example, Tetrahedron Lett. 44, 5991 (2003)).

### Production Process 20

The compound (1-8) described in the production process 1 may be produced also according to, for example, the following process: 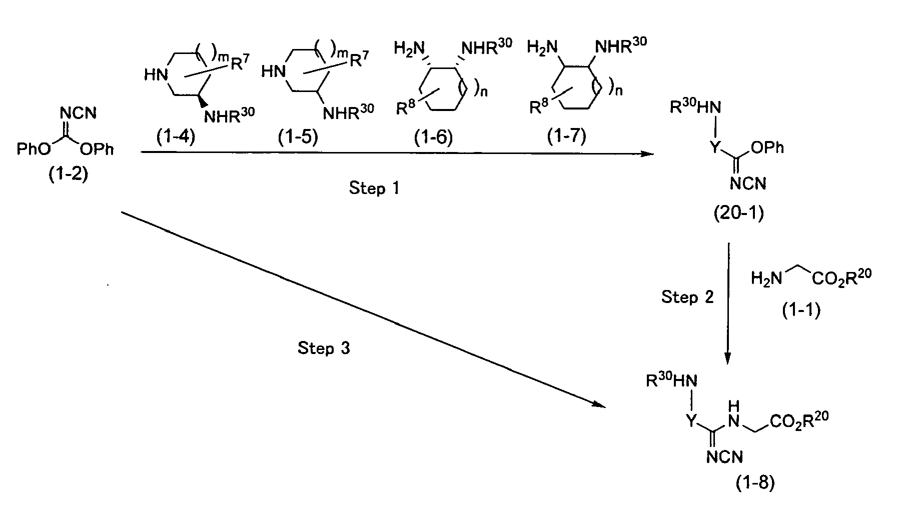 wherein m, n, R⁷, R⁸, R²⁰, R³⁰ and Y are as defined above.

### 1) Step 1

A compound (20-1) may be produced from a compound (1-2) by the same process as in the step 2 described in the production process 1.

### 2) Step 2

The compound (1-8) may be produced from the compound (20-1) by the same process as in the step 1 described in the production process 1.

### 3) Step 3

It is also possible to produce the compound (1-8) from a compound (1-2) by carrying out the following reactions A and B.
A: The compound (1-2) is reacted with a compound (1-4), a compound (1-5), a compound (1-6) or a compound (1-7) in an inert solvent. The inert solvent includes, for example, alcohol solvents such as methanol, ethanol, 2-propanol, etc. The reaction temperature may be chosen in the range of about 0°C to about 50°C.
B: A base and a compound (1-1) are added to the reaction mixture obtained in the item A and the reaction is carried out. The base includes, for example, organic bases such as imidazole, triethylamine, diisopropylethylamine, tributylamine, 1,5-diazabicyclo[4,3,0]non-5-ene, 1,4-diazabicyclo[2,2,2]octane, 1,8-diazabicyclo[5,4,0]undec-7-ene, 4-(dimethylamino)pyridine, picoline, etc. A preferable example thereof is triethylamine. The amount of the compound (1-1) used is usually chosen in the range of 3 to 10 equivalents per equivalent of the compound (1-2). The amount of the base used is usually chosen in the range of 5 to 15 equivalents per equivalent of the compound (1-2). The reaction temperature may be chosen in the range of about 50°C to about 150°C.

### Production Process 21

A compound of the formula (21-3) as the compound of the formula (I), or a salt thereof is produced, for example, by the following process: 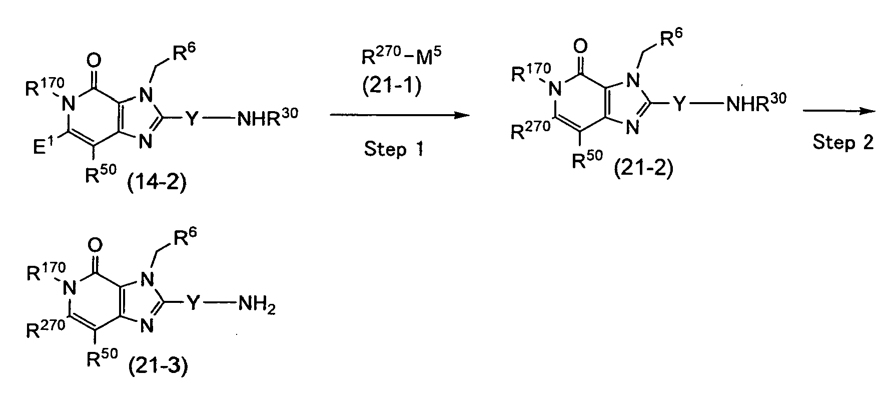 wherein R⁶, R³⁰, R⁵⁰, R¹⁷⁰, E¹ and Y are as defined above; R²⁷⁰ is "an optionally substituted alkenyl group", "an optionally substituted aryl group" or "an optionally substituted heteroaryl group"; and M⁵ is trimethyltin, triethyltin, tributyltin, catechol borane or B(OR²⁸⁰)₂ wherein R²⁸⁰ is a hydrogen atom, methyl, ethyl or isopropyl.

### 1) Step 1

A compound (21-2) may be produced from a compound (14-2) by the same production process as described in literature (for example, Angew. Chem. Int Ed. Engl. 25, 508 (1986), Chem. Rev. 95, 2457 (1995), Org. Lett. 26, 4263 (2001), Tetrahedron 58, 10137 (2002) and J. Org. Chem. 66, 9033 (2001)).

### 2) Step 2

The compound (21-3) may be produced from the compound (21-2) by the same process as in the step 8 described in the production process 1.

### Production Process 22

A compound of the formula (22-2) as the compound of the formula (I), or a salt thereof is produced, for example, by the following process: 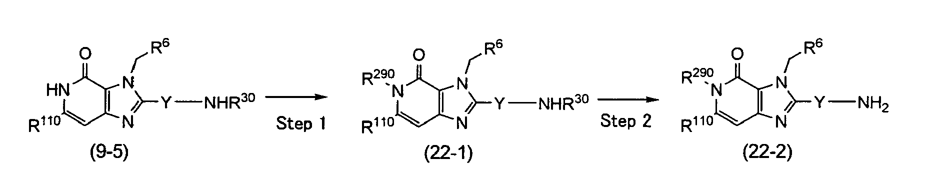 wherein R⁶, R³⁰, R¹¹⁰ and Y are as defined above; and R²⁹⁰ is "an optionally substituted aryl group" or "an optionally substituted heteroaryl group".

### 1) Step 1

A compound (22-1) may be produced from a compound (9-5) by the same production process as described in literature (for example, Tetrahedron 55, 12757 (1999), Tetrahedron Lett. 43, 3091 (2002) and Chem. Pharm. Bull. 45, 719 (1997)).

### 2) Step 2

The compound (22-2) may be produced from the compound (22-1) by the same process as in the step 8 described in the production process 1.

### Production Process 23

The compound (1-15) described in the production process I may be produced also according to, for example, the following production process: 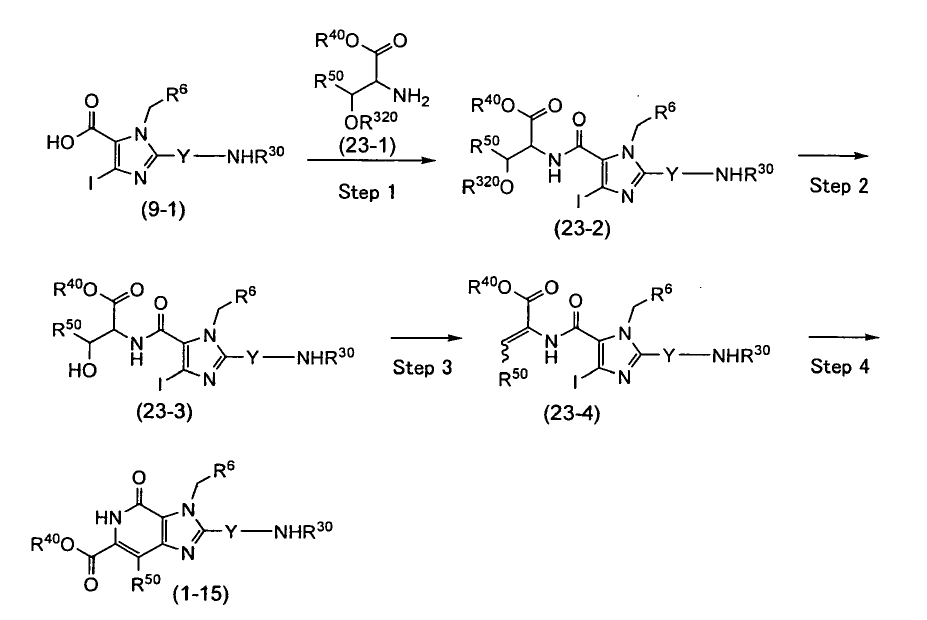 wherein R⁶, R³⁰, R⁴⁰, R⁵⁰ and Y are as defined above; and R³²⁰ is benzyl, acetyl or benzoyl.

### 1) Step 1

A compound (23-2) may be produced from a compound (9-1) by the same process as in the step 1 described in the production process 6.

### 2) Step 2

A compound (23-3) may be produced from the compound (23-2) by the same production process as described in literature (for example, T.W. Greene and P.G.M. Wuts, "Protective Groups in Organic Synthesis" 2nd Edition, John Wiley & Sons, Inc. (1991)).

### 3) Step 3

A compound (23-4) may be produced from the compound (23-3) by the same production process as described in literature (for example, Eur. J. Org. Chem. 45 (2001), Tetrahedron Letters 43, 8679 (2002), Synthesis 201 (2003), J. Am. Chem. Soc. 121, 6100 (1999), Tetrahedron Letters 33, 8145 (1992), Tetrahedron Letters 22, 4817 (1981) and J. Org. Chem. 45, 3131 (1980)).

### 4) Step 4

The compound (1-15) may be produced from the compound (23-4) by the same production process as described in literature (for example, Org. React. 27, 345 (1982), Heterocycles 48, 2543 (1998) and Tetrahedron, 58, 6673 (2002)).

### Production Process 24

A compound (1-5) may be produced according to, for example, the following process. 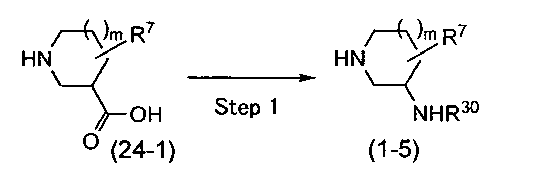 wherein R⁷, R³⁰ and m are as defined above.

### 1) Step 1

The compound (1-5) may be produced from a compound (23-1) by the same production process as described in literature (for example, J. Org. Chem. 58, 879 (1993)).

### Production Process 25

A compound (1-4) may be produced according to, for example, the following process. 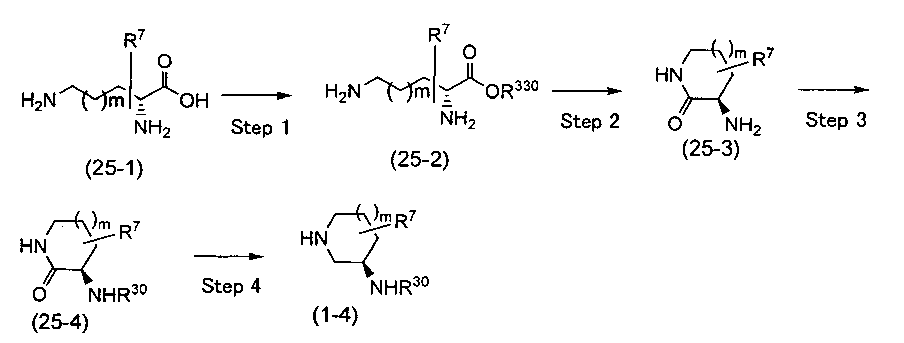 wherein R⁷, R³⁰ and m are as defined above; and R³³⁰ is methyl or ethyl.

### 1) Step 1

A compound (25-2) may be produced by reacting a compound (25-1) with thionyl chloride or the like in an alcohol solvent. The alcohol solvent includes, for example, methanol and ethanol. The amount of thionyl chloride used is usually chosen in the range of 2 to 10 equivalents per equivalent of the compound (25-1). The reaction temperature may be chosen in the range of about -90°C to about 30°C.

### 2) Step 2

A compound (25-3) may be produced by reacting the compound (25-2) with a base in water solvent. The base includes, for example, sodium hydrogencarbonate, potassium hydrogencarbonate, sodium carbonate and potassium carbonate. The reaction temperature may be chosen in the range of about 30°C to about 100°C.

### 3) Step 3

A compound (25-4) may be produced from the compound (25-3) by the same process as that described in literature (for example, Protective Groups in Organic Synthesis 2nd Edition (John Wiley & Sons, Inc.)), or the like.

### 4) Step 4

The compound (1-4) may be produced by reacting the compound (25-4) with a reducing agent in an inert solvent. The reducing agent includes, for example, aluminum lithium hydride, and diborane. The inert solvent includes, for example, tetrahydrofuran, 1,4-dioxane, mixed solvents thereof. When aluminum lithium hydride is used, the reaction temperature is chosen in the range of about -20°C to about 40°C. When diborane is used, the reaction temperature is chosen in the range of about 50°C to about 80°C.

### Production Process 26

Examples of synthesis of compounds (1-5a) to (1-5j) as specific examples of the compound (1-5) are given below. The compounds (1-5a) to (1-5j) include pharmaceutically acceptable salts thereof.

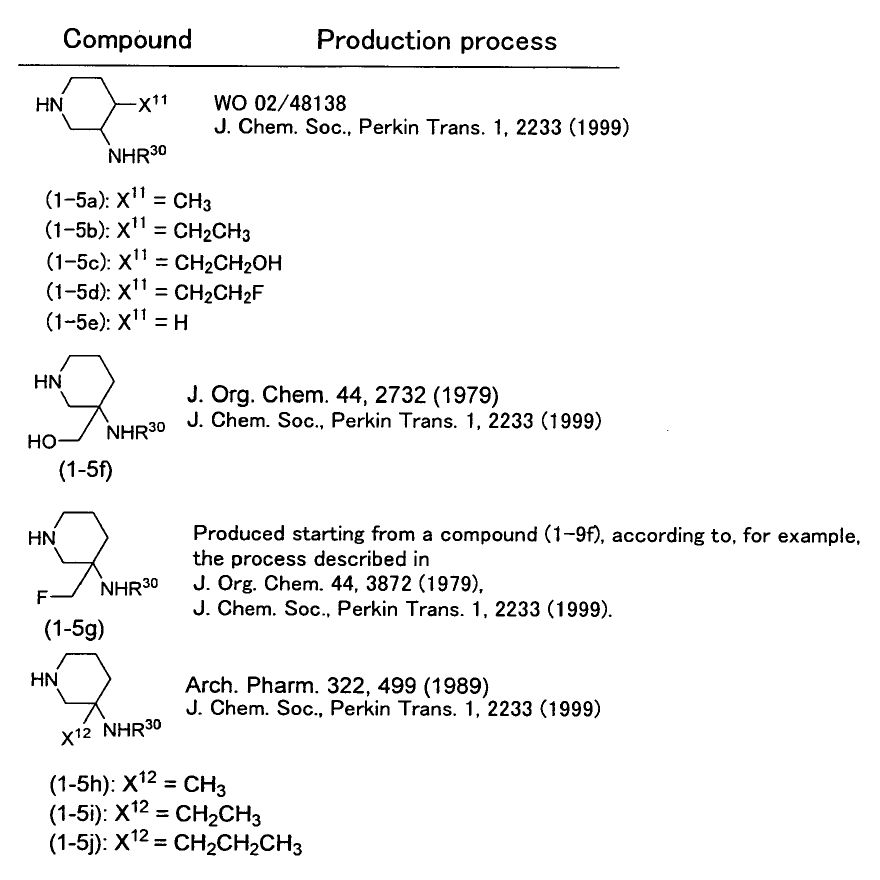

As hydrochloride of the compound (1-5e), a commercial one may also be used. It is also possible to synthesize the compound (1-5) from a substituted DL-ornithine by a well-known process. A specific example of the well-known process is the process described in literature (for example, R.C. Ralock, "Comprehensive Organic transformation", VCH publisher Inc., (1989)).

### Production Process 27

Examples of synthesis of compounds (1-4a) to (1-4i) as specific examples of the compound (1-4) are given below. The compounds (1-4a) to (1-4i) include pharmaceutically acceptable salts thereof.

| Compound | Production process |
|---|---|
| 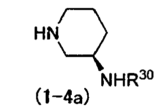 | WO 01/27082 J. Chem. Soc., Perkin Trans. 1, 2233 (1999) |
| 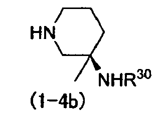 | Int. J. Peptide Protein Res. 40, 119 (1992) WO 01/27082 J. Chem. Soc., Perkin Trans. 1, 2233 (1999) |
| 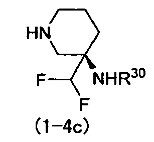 | US 4413141 WO 01/27082 J. Chem. Soc., Perkin Trans. 1, 2233 (1999) |
| 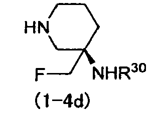 | Tetrahedron: Asymmetry 8, 327 (1997) WO 01/27082 J. Chem. Soc., Perkin Trans. 1, 2233 (1999) |
| 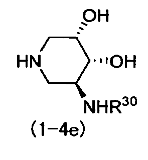 | Tetrahedron: Asymmetry 11, 567 (2000) J. Chem. Soc., Perkin Trans. 1, 2233 (1999) |

| Compound | Production process |
|---|---|
| 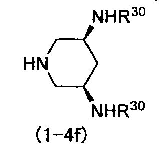 | Chem. Eur. J. 6, 2830 (2000) WO 00/26332 J. Chem. Soc., Perkin Trans. 1,2233 (1999) |
| 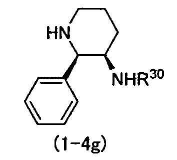 | JP-T-2002-525325 J. Chem. Soc., Perkin Trans. 1, 2233 (1999) |
| 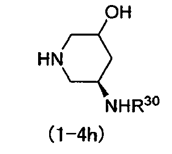 | Bull. Chem. Soc. Jpn. 53, 2605 (1980) J. Chem. Soc., Perkin Trans. 1, 2233 (1999) |
| 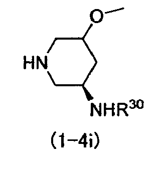 | Produced starting from a compound (1-4h), according to, for example, the process described in J. Am. Chem. Soc. 80, 2584 (1958), J. Chem. Soc. PT1 499 (1972),J. Chem. Soc., Perkin Trans. 1, 2233 (1999). |

### Production Process 28

Examples of synthesis of compounds (1-4j) to (1-4v) as specific examples of the compound (1-4) are given below. The compounds (1-4j) to (1-4v) include pharmaceutically acceptable salts thereof.

| Compound | Production process |
|---|---|
| 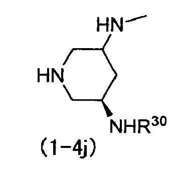 | Produced starting from compound (1-4f) in which R³⁰ is a hydrogen atom, according to, for example, the process described in J. Chem. Soc. Chem. Commun. 611 (1981), J. Chem. Soc., Perkin Trans. 1, 2233 (1999). |
| 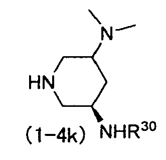 | Produced starting from compound (1-4f) in which R²⁰⁰ is a hydrogen atom, according to, for example, the process described in J. Chem. Soc. Chem. Commun. 611 (1981), J. Chem. Soc., Perkin Trans. 1, 2233 (1999). |
| 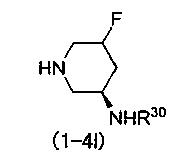 | Produced starting from a compound (1-4h), according to, for example, the process described in J. Org. Chem. 44, 3872 (1979), J. Chem. Soc., Perkin Trans. 1, 2233 (1999). |
| 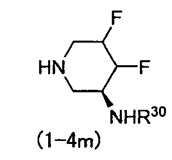 | Produced starting from a compound (1-4e), according to, for example, the process described in J. Org. Chem. 44, 3872 (1979), J. Chem. Soc., Perkin Trans. 1, 2233 (1999). |
| 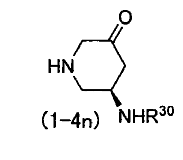 | Produced starting from a compound (1-4h), according to, for example, the process described in Bull. Chem. Soc. Jpn. 64, 2857 (1991), J. Chem. Soc., Perkin Trans. 1,2233 (1999). |
| 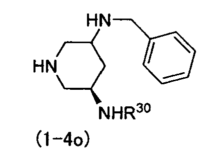 | Produced starting from compound (1-4f) in which R³⁰ is a hydrogen atom, according to, for example, the process described in Tetrahedron Lett. 40, 5609(1999), J. Chem. Soc., Perkin Trans. 1, 2233 (1999). |
| 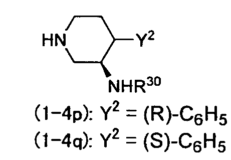 | J. Med. Chem. 35. 833 (1992), R. C. Ralock, "Comprehensive Organic transformation", VCH publisher Inc., 1989, J. Chem. Soc., Perkin Trans. 1, 2233 (1999) |
| 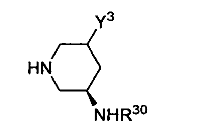 (1-4r): Y³ = NHS(O)₂CH₃ (1-4s): Y³ = NHC(O)CH₃ (1-4t): Y³ = NHC(O)C₆H₅ (1-4u): Y³ = N(CH₃)C(O)CH₃ | Produced starting from compound (1-4f) in which R³⁰ is a hydrogen atom, according to, for example, the process described in R. C. Ralock, "Comprehensive Organic transformation", VCH publisher Inc., 1989, J. Chem. Soc., Perkin Trans. 1, 2233 (1999). |
| 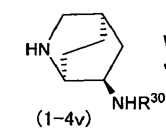 | WO 02/068420 J. Chem. Soc., Perkin Trans. 1, 2233 (1999) |

### Production Process 29

Examples of synthesis of compounds (1-4w) to (1-4dd) as specific examples of the compound (1-4) are given below. The compounds (1-4w) to (1-4dd) include pharmaceutically acceptable salts thereof.

| Compound | Production process |
|---|---|
| 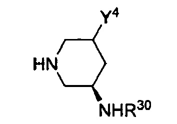 (1-4w): Y⁴ = 2-CH₃-C₆H₅ (1-4x): Y⁴ = 3-CH₃-C₆H₅ (1-4y): Y⁴ = 4-CH₃-C₆H₅ (1-4z): Y⁴ = 2-CH₃O-C₆H₅ (1-4aa): Y⁴ = 3-CH₃O-C₆H₅ (1-4bb): Y⁴ = 4-CH₃O-C₆H₅ (1-4cc): Y⁴ =C₆H₅ (1-4dd): Y⁴ = CH₂C₆H₅ | Produced starting from a compound (1-4n), according to, for example, the process described in R.C. Ralock, "Comprehensive Organic transformation", VCH publisher Inc., 1989, J. Org.. Chem. 66, 3593 (2001), J. Prakt Chem. 342, 421 (2000), Tetrahedron Lett 36, 5611 (1994), J. Org.. Chem. 53, 5143 (1988), Bioorg. Med. Chem. Lett. 11, 1281 (2001), J. Chem. Soc., Perkin Trans. 1, 2233 (1999). |

The compound (1-4) may be synthesized from a substituted D-ornithine by a well-known process. A specific example of the well-known process is the process described in literature (for example, R.C. Ralock, "Comprehensive Organic transformation", VCH publisher Inc., (1989)).

### Production Process 30

A compound (1-6) may be produced according to, for example, the following process. 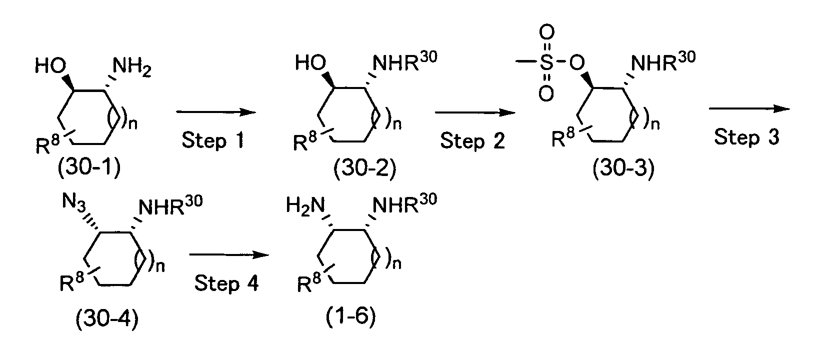 wherein R⁸, R³⁰ and n are as defined above.

### 1) Step 1

A compound (30-2) may be produced from a compound (30-1) by the same process as that described in literature (for example, Protective Groups in Organic Synthesis 2nd Edition (John Wiley & Sons, Inc.)), or the like. The compound (30-1) may be produced by the same production process as described in literature (for example, J. Org. Chem. 50, 4154 (1985)).

### 2) Steps 2 to 4

The compound (1-6) may be produced from the compound (30-2) by the same process as described in literature (for example, R.C. Ralock, "Comprehensive Organic transformation", VCH publisher Inc., (1989)).

### Production Process 31

Examples of synthesis of compounds (1-6a) to (1-6aa) as specific examples of the compound (1-6) are given below. The compounds (1-6a) to (1-6aa) include pharmaceutically acceptable salts thereof. The compounds (1-6a) to (1-6aa) may be produced according to the processes described in literature (for example, WO01/74774, and R.C. Ralock, "Comprehensive Organic transformation", VCH publisher Inc., (1989)).

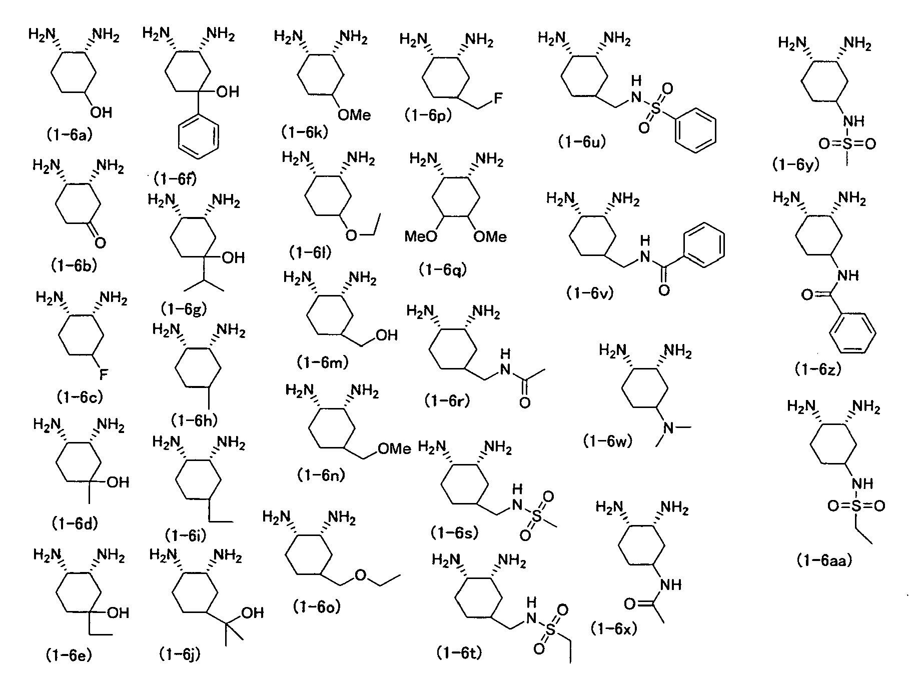

### Production Process 32

Examples of synthesis of compounds (1-6bb) to (1-6tt) as specific examples of the compound (1-6) are given below. The compounds (1-6bb) to (1-6tt) include pharmaceutically acceptable salts thereof. The compounds (1-6bb) to (1-6tt) may be produced according to the processes described in literature (for example, WO01/74774, and R.C. Ralock, "Comprehensive Organic transformation", VCH publisher Inc., (1989)).

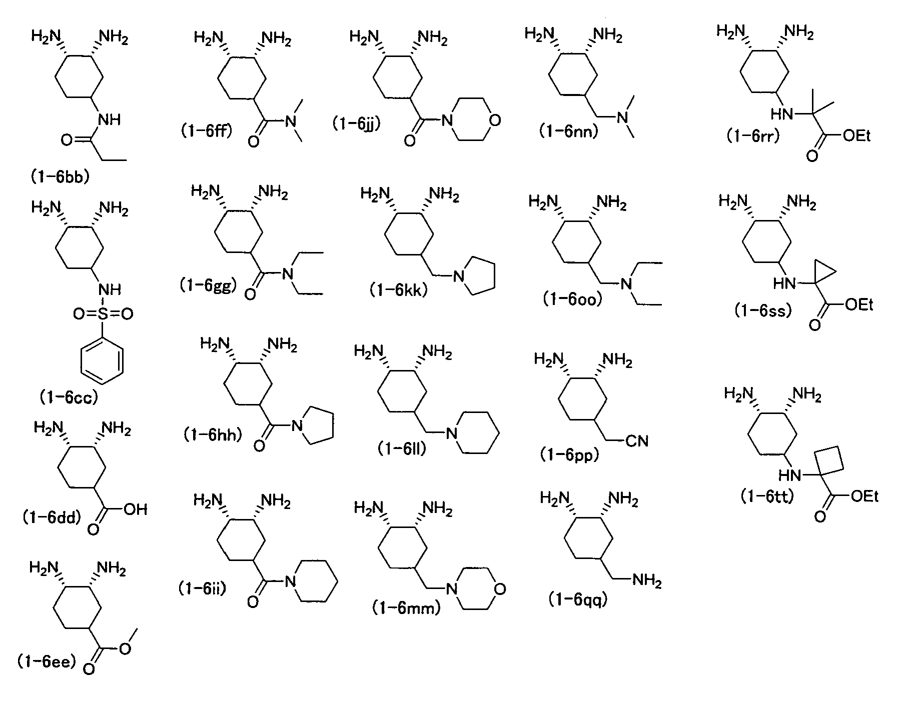

### Production Process 33

Compounds of the formula (33-4) and the formula (33-6) as the compound of the formula (I), or salts thereof are produced, for example, by the following processes: 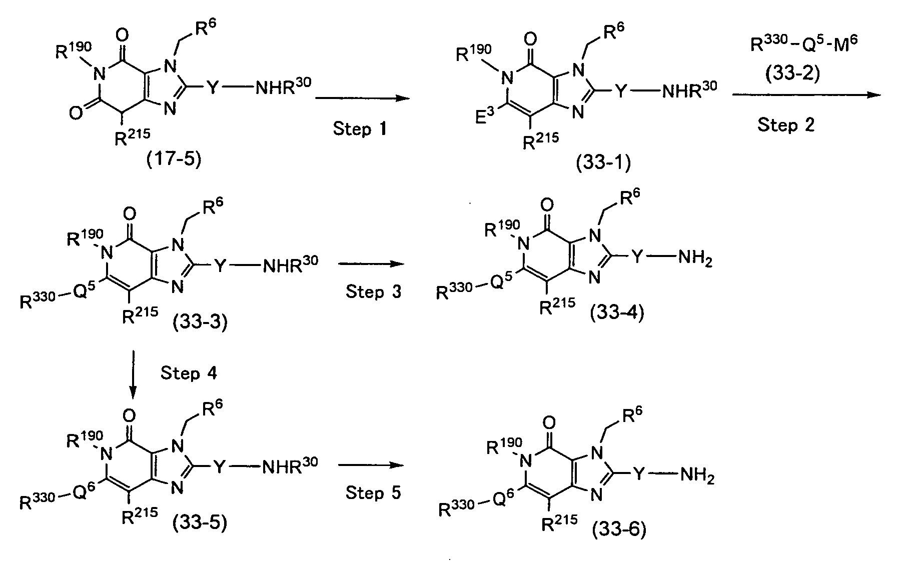 wherein R⁶, R³⁰, R¹⁹⁰, R²¹⁵ and Y are as defined above; R³³⁰-Q⁵ is "an optionally substituted aryloxy group", "an optionally substituted arylthio group" or "an optionally substituted heteroaryloxy group"; R³³⁰-Q⁶ is "an optionally substituted arylsulfonyl group"; E³ is a chlorine atom or a bromine atom; and M⁶ is lithium, sodium, potassium or cesium.

### 1) Step 1

A compound (33-1) may be produced from a compound (17-5) by the same production process as described in the step 1 in the production process 16.

### 2) Step 2

A compound (33-3) may be produced from the compound (33-1) by the same production process as described in the step 2 in the production process 16.

### 3) Step 3

The compound (33-4) may be produced from the compound (33-3) by the same production process as described in the step 8 in the production process 1.

### 4) Step 4

A compound (33-5) may be produced from the compound (33-3) by the same production process as described in the step 4 in the production process 16.

### 5) Step 5

The compound (33-6) may be produced from the compound (33-5) by the same production process as described in the step 8 in the production process 1.

### Production Process 34

A compound of the formula (34-3) as the compound of the formula (I), or a salt thereof is produced, for example, by the following process: 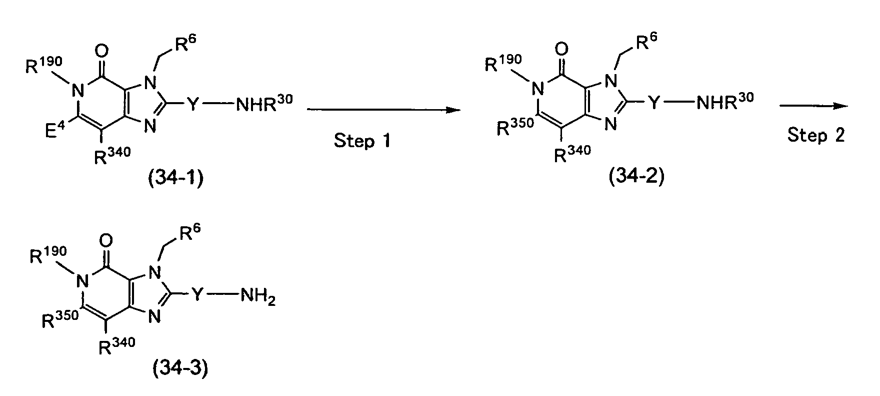 wherein the compound (34-1) corresponds to the compound (16-1) described in the production process 16 or the compound (33-1) described in the production process 33; R⁶, R³⁰, R¹⁹⁰ and Y are as defined above; R³⁵⁰ is "an optionally substituted carbamoyl group", "an optionally substituted aryl group", "an optionally substituted alkoxycarbonyl group", "an optionally substituted aryloxycarbonyl group", "an optionally substituted aroyl group" or "an optionally substituted heteroaryl group"; R³⁴⁰ is a hydrogen atom, a fluorine atom, methyl, ethyl or "an alkoxycarbonylmethyl group"; and E⁴ is a chlorine atom or a bromine atom.

### 1) Step 1

A compound (34-2) may be produced from the compound (34-1) by the same production process as described in literature (for example, Chem. Rev. 103, 1979 (2003) and Chem. Rev. 103, 1875 (2003)).

### 2) Step 2

The compound (34-3) may be produced from the compound (34-2) by the same production process as described in the step 8 in the production process 1.

### Production Process 35

A compound of the formula (35-4) as the compound of the formula (I), or a salt thereof is produced, for example, by the following process: 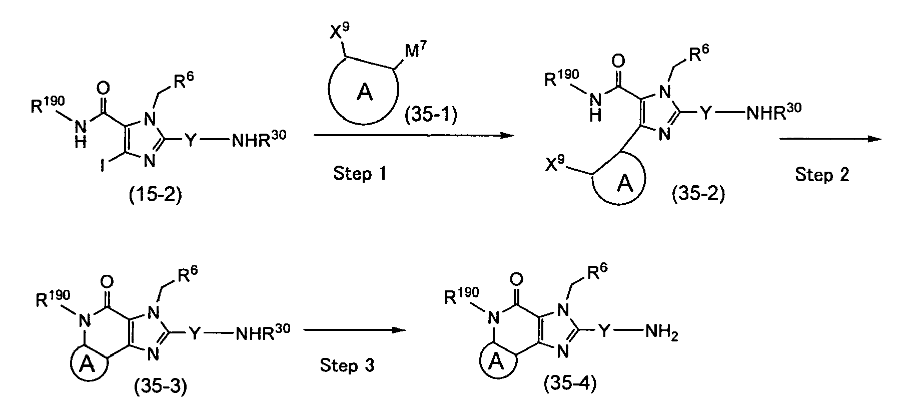 wherein R⁶, R³⁰, R¹⁹⁰ and Y are as defined above; M⁷ is trimethyltin, triethyltin, tributyltin, catechol borane, B(OR³⁶⁰)₂ (wherein R³⁶⁰ is a hydrogen atom, methyl, ethyl or isopropyl), or a group represented by the following formula (35-5): 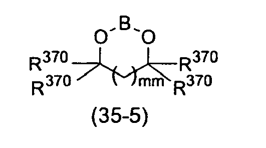 wherein R³⁷⁰ is a hydrogen atom or methyl and mm is an integer of 0 or 1; the ring A is "an optionally substituted benzene ring, an optionally substituted cycloalkene ring or an optionally substituted 5-or 6-membered heteroaromatic ring"; and X⁹ is an iodine atom, a chlorine atom or a bromine atom.

### 1) Step 1

A compound (35-2) may be produced from a compound (15-2) by the same production process as described in the step 1 in the production process 21.

As a compound (35-1), a commercial one may be used, or the compound (35-1) may be produced by the process described in literature (for example, R.C. Ralock, "Comprehensive Organic transformation", VCH publisher Inc., (1989)).

### 2) Step 2

A compound (35-3) may be produced from the compound (35-2) by the same production process as described in literature (for example, Bioorg. Med. Chem. Lett. 13, 273 (2003), Synlett 231 (2002), J. Chem. Soc. Perkin Trans. 1, 733 (2002), Tetrahedron 52, 7525 (1996) and Chem. Rev. 103, 1875 (2003)).

### 3) Step 3

The compound (35-4) may be produced from the compound (35-3) by the same production process as described in the step 8 in the production process 1.

### Production Process 36

Compounds of the formula (36-4) and the formula (36-7) as the compound of the formula (I), or salts thereof are produced, for example, by the following processes: 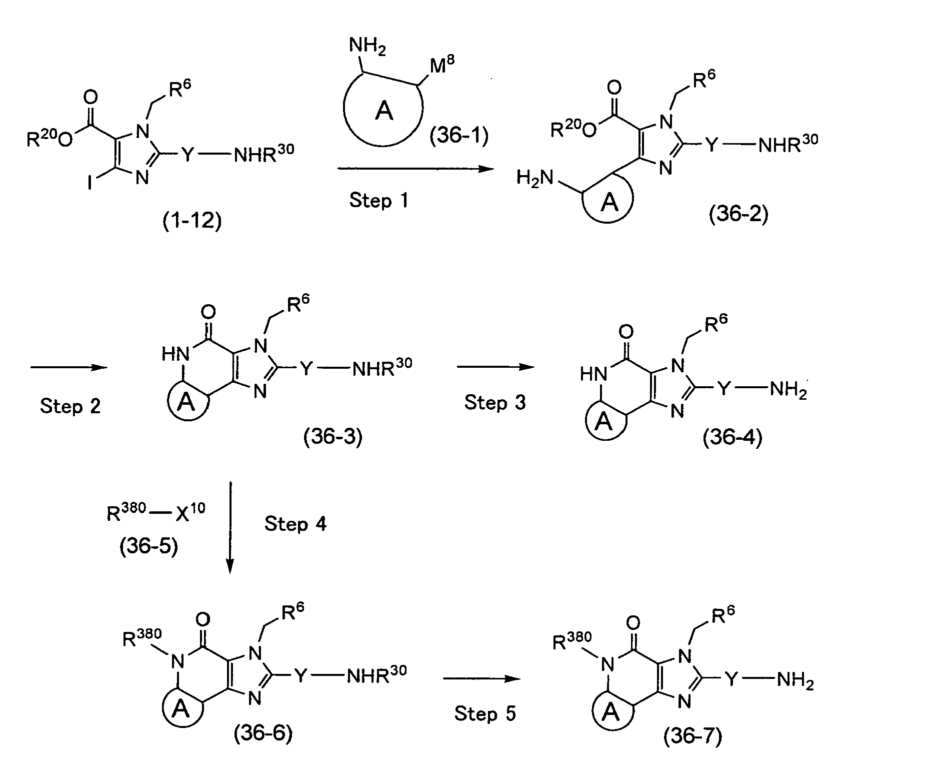 wherein R⁶, R²⁰, R³⁰, R⁶, Y and A are as defined above; M⁸ is trimethyltin, triethyltin, tributyltin, catechol borane, B(OR³⁹⁰)₂ (wherein R³⁹⁰ is a hydrogen atom, methyl, ethyl or isopropyl), or a group represented by the following formula (36-8): 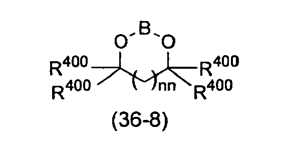 wherein R⁴⁰⁰ is a hydrogen atom or methyl and nn is an integer of 0 or 1; R³⁸⁰ is "an optionally substituted alkyl group" or "an optionally substituted cycloalkyl group"; and X¹⁰ is a leaving group (for example, a bromine atom, a chlorine atom, methanesulfonyloxy, trifluoromethanesulfonyloxy or p-toluenesulfonyloxy).

### 1) Step 1

A compound (36-2) may be produced from a compound (1-12) by the same production process as described in the step 1 in the production process 21.

As a compound (36-1), a commercial one may be used, or the compound (36-1) may be produced by the process described in literature (for example, R.C. Ralock, "Comprehensive Organic transformation", VCH publisher Inc., (1989), Japanese Chemical Association, "Jikken Kagaku Koza (Experimental Chemistry)" Vol. 24, Maruzen Co., Ltd., J. Org. Chem. 67, 5394 (2002), J. Org. Chem. 65, 9268 (2000), Method of Element-Organic Chemistry, vol. 1, North-Holland (1967) and J. Am. Chem. Soc. 116, 11723 (1994)).

### 2) Step 2

A compound (36-3) may be produced by treating the compound (36-2) with a base in an inert solvent. The base includes, for example, alkoxy alkalis (sodium methoxide, sodium ethoxide and potassium t-butoxide). The amount of the base used is usually chosen in the range of 1 equivalent to large excess equivalents per equivalent of the compound (36-2). The inert solvent includes, for example, alcohol solvents (e.g. ethanol, methanol and 2-propanol), ether solvents (e.g. 1,4-dioxane), and mixed solvents thereof. The reaction temperature may be chosen in the range of about 50°C to about 150°C.

### 3) Step 3

The compound (36-4) may be produced from the compound (36-3) by the same production process as described in the step 8 in the production process 1.

### 4) Step 4

A compound (36-6) may be produced from the compound (36-3) by the same production process as described in the step 1 in the production process 3.

### 5) Step 5

The compound (36-7) may be produced from the compound (36-6) by the same production process as described in the step 8 in the production process 1.

### Production Process 37

The compounds (36-3) and (36-6) described in the production process 36 may be produced also according to, for example, the following production processes: 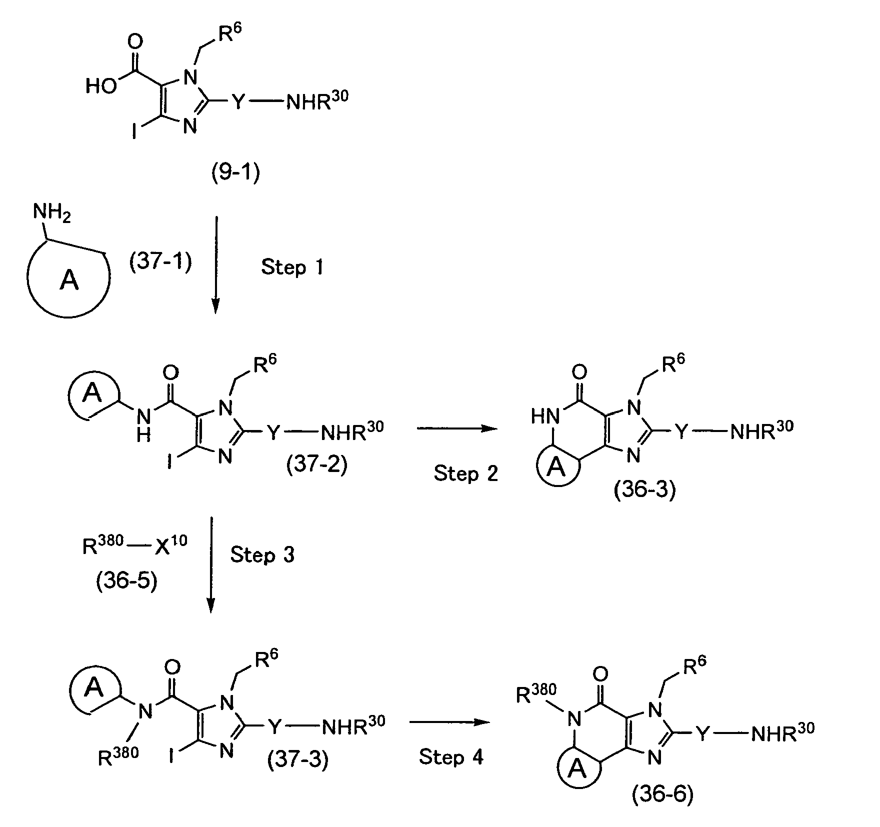 wherein R⁶, R³⁰, R³⁸⁰, Y, A and X¹⁰ are as defined above.

### 1) Step 1

The following production process (A) or production process (B) may be adopted in the step 1. Production process (A):

A compound (37-2) may be produced by condensing a compound (9-1) with a compound (37-1) in an inert solvent by the use of a dehydrating-condensation agent (e.g. dicyclohexylcarbodiimide or carbonyldiimidazole) optionally in the presence of an additive (e.g. 4-(dimethylamino)pyridine). The inert solvent includes, for example, ether solvents such as diethyl ether, tetrahydrofuran, 1,4-dioxane, etc.; aprotic solvents such as N,N-dimethylformamide, etc.; and halogenated hydrocarbon solvents such as dichloromethane, dichloroethane, etc. Mixed solvents of these solvents may also be used. A preferable example of the inert solvent is N,N-dimethylformamide. The reaction temperature is usually chosen in the range of about 0°C to about 50°C.

### Production process (B):

A compound (37-2) may be produced from a compound (9-1) by carrying out the following reactions (1) and (2).
(1) The compound (9-1) is reacted with oxalyl chloride or the like in an inert solvent in the presence or absence of an additive. The additive includes, for example, dimethylformamide. The amount of oxaly chloride used is usually chosen in the range of 1 to 3 equivalents (molar ratio). The inert solvent includes, for example, halogenated hydrocarbon solvents such as dichloromethane, dichloroethane, chloroform, etc. The reaction temperature is usually chosen in the range of about -10°C to about 50°C.
(2) The reaction solution obtained in the above item (1) is concentrated in the presence or absence of a hydrocarbon solvent such as toluene or benzene. The residue after the concentration is reacted with a compound (37-1) in an inert solvent in the presence of an organic base. The inert solvent includes, for example, halogenated hydrocarbon solvents such as dichloromethane, dichloroethane, chloroform, etc.; and hydrocarbon solvents such as toluene, benzene, etc. The organic base includes, for example, N-methylmorpholine, triethylamine, diisopropylethylamine, tributylamine, 1,8-diazabicyclo[5,4,0]undec-7-ene (DBU), 1,5-diazabicyclo[4,3,0]non-5-ene (DBN), 1,4-diazabicyclo[5,4,0]undec-7-ene (DABCO), pyridine, dimethylaminopyridine and picoline. When these bases are liquid, they may be used also as a solvent.

A preferable example of the organic base is diisopropylethylamine. The amount of the compound (37-1) used is usually chosen in the range of 1 to 3 equivalents (molar ratio) per equivalent of the compound (9-1). The amount of the organic base used is usually chosen in the range of 1 to 20 equivalents (molar ratio) per equivalent of the compound (9-1).
The reaction temperature is usually chosen in the range of about 10°C to about 150°C.

### 2) Step 2

The compound (36-3) may be produced from the compound (37-2) by the same production process as described in literature (for example, Synthesis 444 (2001)).

### 3) Step 3

A compound (37-3) may be produced from the compound (37-2) by the same production process as described in the step 1 in the production process 3.

### 4) Step 4

The compound (36-6) may be produced from the compound (37-3) by the same production process as described in the above step 2.

### Production Process 38

Compounds of the formula (38-3) and the formula (38-5) as the compound of the formula (I), or salts thereof are produced, for example, by the following processes: 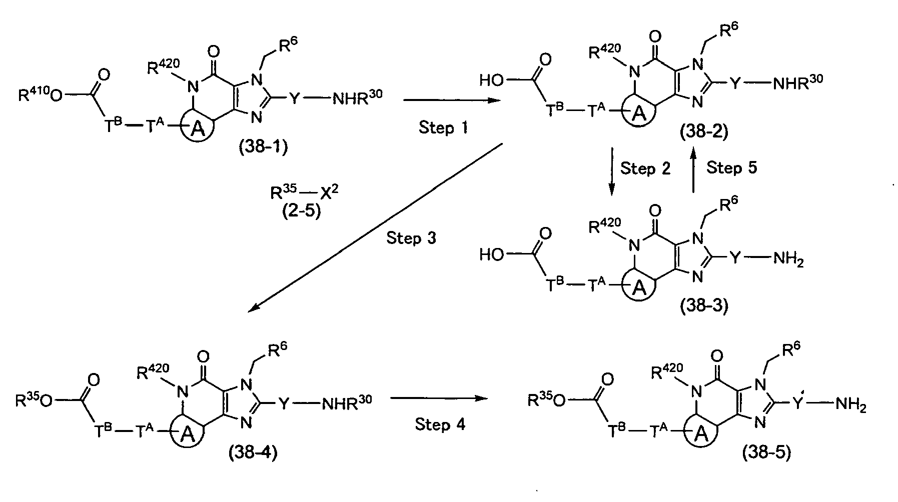 wherein R⁶, R³⁰, R³⁵, Y and A are as defined above; R⁴²⁰ is "a hydrogen atom", "an optionally substituted alkyl group" or "an optionally substituted cycloalkyl group"; R⁴¹⁰ is "an alkyl group"; T^{A} is a single bond or an oxygen atom; and T^{B} is a single bond or an optionally substituted alkylene chain.

### 1) Step 1

A compound (38-2) may be produced from a compound (38-1) by the same production process as described in literature (for example, T.W. Greene and P.G.M. Wuts, "Protective Groups in Organic Synthesis" 2nd Edition, John Wiley & Sons, Inc. (1991)). In such a reaction, a compound in which the protective group for the primary amino group in Y-NH₂ has been removed is produced in some cases. The primary amino group in Y-NH₂ may be protected again with the protective group (e.g. Boc or Cbz) by the same method as in the production process described in literature (for example, T.W. Greene and P.G.M. Wuts, "Protective Groups in Organic Synthesis" 2nd Edition, John Wiley & Sons, Inc. (1991)).

### 2) Step 2

The compound (38-3) may be produced from the compound (38-2) by the same production process as described in the step 8 in the production process 1 or literature (for example, T.W. Greene and P.G.M. Wuts, "Protective Groups in Organic Synthesis" 2nd Edition, John Wiley & Sons, Inc. (1991)).

### 3) Step 3

A compound (38-4) may be produced from the compound (38-2) by the same production process as described in the step 1 in the production process 3.

### 4) Step 4

The compound (38-5) may be produced from the compound (38-4) by the same production process as described in the above step 2.

### 5) Step 5

The compound (38-2) may be produced from the compound (38-3) by the same production process as described in literature (for example, T.W. Greene and P.G.M. Wuts, "Protective Groups in Organic Synthesis" 2nd Edition, John Wiley & Sons, Inc. (1991)).

### Production Process 39

Compounds of the formula (39-3) and the formula (39-5) as the compound of the formula (I), or salts thereof are produced, for example, by the following processes: 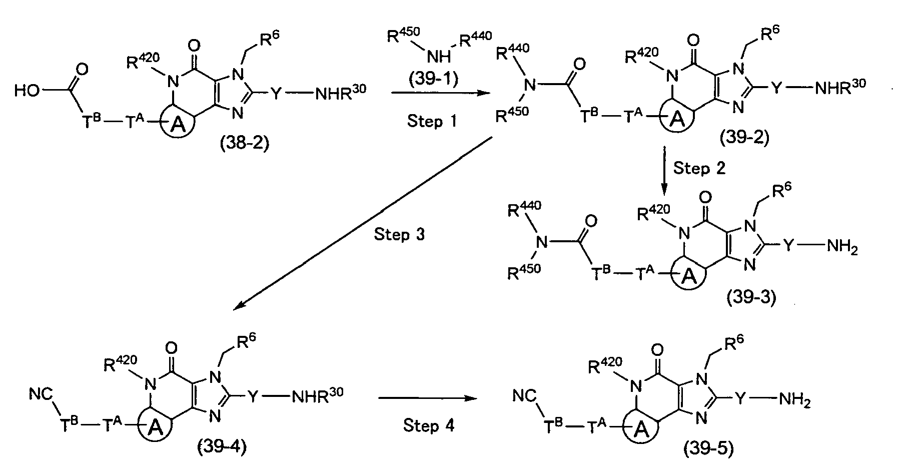 wherein R⁶, R³⁰, R⁴²⁰, Y, T^{A}, T^{B} and A are as defined above; and R⁴⁴⁰R⁴⁵⁰NC(O) is "an optionally substituted carbamoyl group".

### 1) Step 1

A compound (39-2) may be produced from a compound (38-2) by the same production process as described in the step 1 in the production process 6.

### 2) Step 2

The compound (39-3) may be produced from the compound (39-2) by the same production process as described in the step 2 in the production process 38.

### 3) Step 3

A compound (39-4) may be produced from a compound (39-2) in which each of R⁴⁴⁰ and R⁴⁵⁰ is a hydrogen atom, by the same production process as described in literature (for example, Synth Commun 32, 2535 (2002), and R. C. Ralock, "Comprehensive Organic transformation", VCH publisher Inc., (1989)).

### 4) Step 4

The compound (39-5) may be produced from the compound (39-4) by the same production process as described in the step 2 in the production process 38.

### Production Process 40

A compound of the formula (40-3) as the compound of the formula (I), or a salt thereof is produced, for example, by the following process: 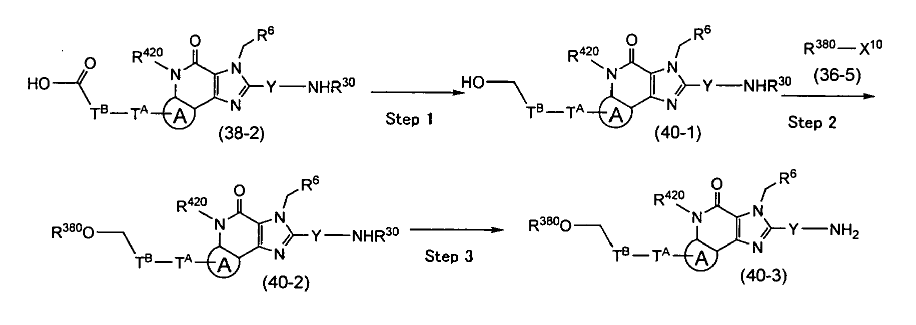 wherein R⁶, R³⁰, R³⁸⁰, R⁴²⁰, Y, X¹⁰, A, T^{A} and T^{B} are as defined above.

### 1) Step 1

A compound (40-1) may be produced from a compound (38-2) by carrying out the following reactions (1) and (2).
(1) The compound (38-2) is reacted with an alkyl chloroformate in an inert solvent in the presence of an organic base. The organic base includes, for example, N-methylmorpholine, triethylamine, diisopropylethylamine, tributylamine, 1,8-diazabicyclo[5,4,0]undec-7-ene (DBU), 1,5-diazabicyclo[4,3,0]non-5-ene (DBN), 1,4-diazabicyclo[5,4,0]undec-7-ene (DABCO), pyridine, dimethylaminopyridine and picoline. The amount of the organic base used is usually chosen in the range of 1 to 3 equivalents (molar ratio) per equivalent of the compound (38-2). The alkyl chloroformate includes, for example, isopropyl chloroformate, isobutyl chloroformate and n-butyl chloroformate. Preferable examples thereof are isopropyl chloroformate and isobutyl chloroformate. The amount of the alkyl chloroformate used is usually chosen in the range of 1 to 3 equivalents (molar ratio). The inert solvent includes, for example, ether solvents (e.g. diethyl ether, tetrahydrofuran and 1,4-dioxane). The reaction temperature is usually chosen in the range of about -10°C to about 50°C.
(2) A reducing agent is added to the reaction solution obtained in the above item (1) and the reaction is carried out. The reducing agent includes, for example, hydrides such as lithium aluminum hydride, sodium borohydride, sodium cyanoborohydride, etc. A preferable example thereof is sodium borohydride. The amount of the reducing agent used is usually chosen in the range of 1 to 3 equivalents (molar ratio) per equivalent of the compound (38-2). The reaction temperature is usually chosen in the range of about -10°C to about 50°C.

### 2) Step 2

A compound (40-2) may be produced from the compound (40-1) by the same production process as described in the step 1 in the production process 3.

### 3) Step 3

The compound (40-3) may be produced from the compound (40-2) by the same production process as described in the step 2 in the production process 38.

### Production Process 41

Compounds of the formula (41-3), formula (41-5) and formula (41-7) as the compound of the formula (I), or salts thereof are produced, for example, by the following processes: 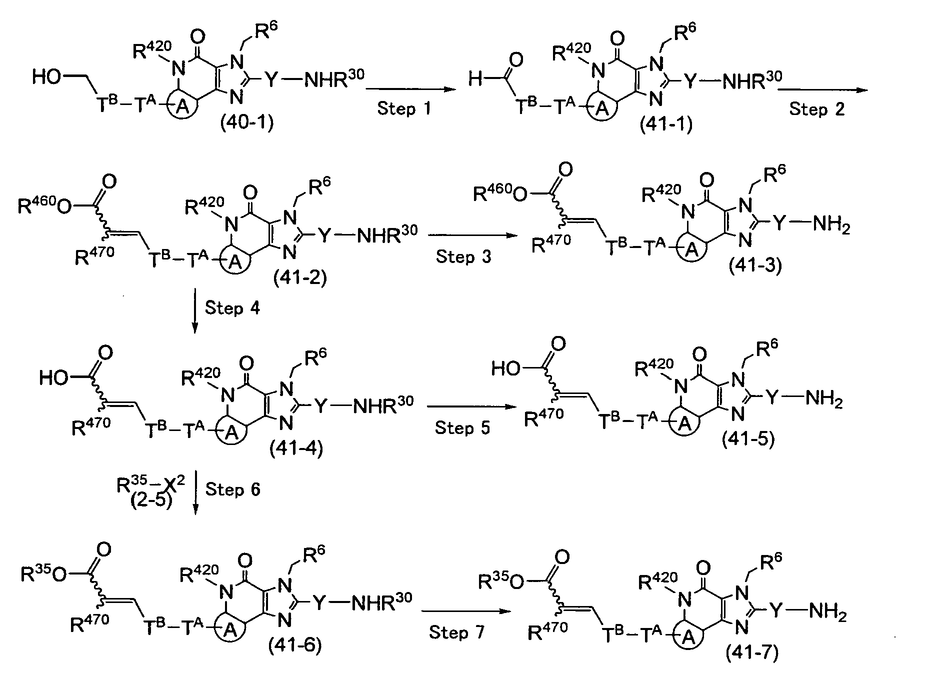 wherein R⁶, R³⁰, R³⁵, R⁴²⁰, Y, T^{A}, T^{B} and A are as defined above; R⁴⁶⁰ is "an alkyl group"; and R⁴⁷⁰ is "an optionally substituted alkyl group".

### 1) Step 1

A compound (41-1) may be produced from a compound (40-1) by the same production process as described in literature (for example, R. C. Ralock, "Comprehensive Organic transformation", VCH publisher Inc., 972-976 (1989), Tetrahedron 59, 6739 (2003), Tetrahedron Letters 44, 2553 (2003), Synlett 1735 (2001) and J. Org. Chem. 66, 7907 (2001)).

### 2) Step 2

A compound (41-2) may be produced from the compound (41-1) by the same production process as described in literature (for example, R. C. Ralock, "Comprehensive Organic transformation", VCH publisher Inc., 972-976 (1989), J. Org. Chem. 68, 6440 (2003), Eur. J. Med. Chem. 36, 673 (2001), Synth. Commun. 31, 89 (2001) and Synth. Commun. 26, 1921 (1996)).

### 3) Step 3

The compound (41-3) may be produced from the compound (41-2) by the same production process as described in the step 2 in the production process 38.

### 4) Step 4

A compound (41-4) may be produced from the compound (41-2) by the same production process as described in the step 9 in the production process 1.

### 5) Step 5

The compound (41-5) may be produced from the compound (41-4) by the same production process as described in the step 2 in the production process 38.

### 6) Step 6

A compound (41-6) may be produced from the compound (41-4) by the same production process as described in the step 3 in the production process 38.

### 7) Step 7

The compound (41-7) may be produced from the compound (41-6) by the same production process as described in the step 2 in the production process 38.

### Production Process 42

Compounds of the formula (42-5), formula (42-10) and formula (42-9) as the compound of the formula (I), or salts thereof are produced, for example, by the following processes: 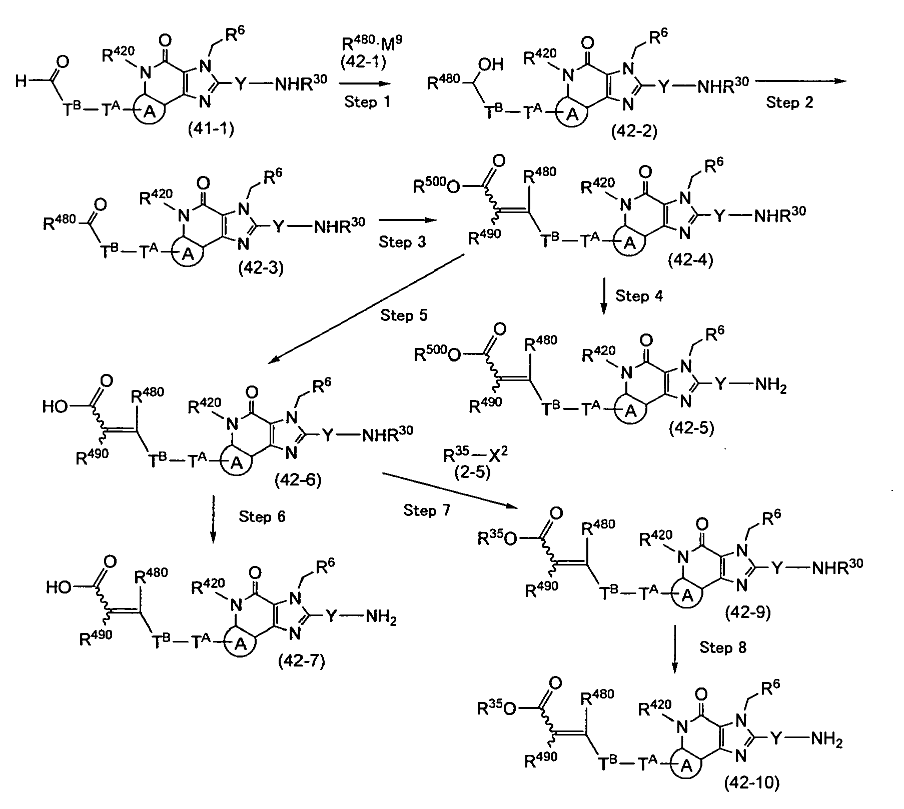 wherein R⁶, R³⁰, R³⁵, R⁴²⁰, Y, T^{A}, T^{B} and A are as defined above; R⁵⁰⁰ is "an alkyl group"; each of R⁴⁸⁰ and R⁴⁹⁰ is "an optionally substituted alkyl group"; and M⁹ is lithium, magnesium chloride or magnesium bromide.

### 1) Step 1

A compound (42-2) may be produced from a compound (41-1) by the same production process as described in literature (for example, R. C. Ralock, "Comprehensive Organic transformation", VCH publisher Inc., 972-976 (1989)).

### 2) Step 2

A compound (42-3) may be produced from the compound (42-2) by the same production process as described in literature (for example, R. C. Ralock, "Comprehensive Organic transformation", VCH publisher Inc., 972-976 (1989), Org. Lett. 4, 3935 (2002), Org. Lett. 5, 4425 (2003) and Tetrahedron Letters 44, 2553 (2003)).

### 3) Step 3

A compound (42-4) may be produced from the compound (42-3) by the same production process as described in literature (for example, R. C. Ralock, "Comprehensive Organic transformation", VCH publisher Inc., 972-976 (1989), Tetrahedron 59, 9433 (2003) and Bioorg. Med. Chem. Lett. 13, 2227 (2003)).

### 4) Step 4

The compound (42-5) may be produced from the compound (42-4) by the same production process as described in the step 2 in the production process 38.

### 5) Step 5

A compound (42-6) may be produced from the compound (42-4) by the same production process as described in the step 9 in the production process 1.

### 6) Step 6

The compound (42-7) may be produced from the compound (42-6) by the same production process as described in the step 2 in the production process 38.

### 7) Step 7

A compound (42-9) may be produced from the compound (42-6) by the same production process as described in the step 3 in the production process 38.

### 8) Step 8

The compound (42-10) may be produced from the compound (42-9) by the same production process as described in the step 2 in the production process 38.

### Production Process 43

A compound of the formula (IV) is produced, for example, by the following process: 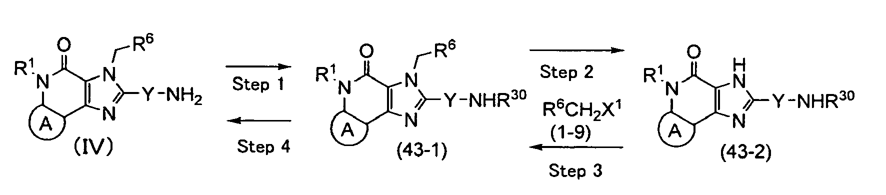 wherein R¹, R⁶, R³⁰, X¹ and A are as defined above.

### 1) Step 1

A compound (43-1) may be produced from the compound of the formula (IV) by the same production process as described in literature (for example, T.W. Greene and P.G.M. Wuts, "Protective Groups in Organic Synthesis" 2nd Edition, John Wiley & Sons, Inc. (1991)).

### 2) Step 2

A compound (43-2) may be produced by hydrogenating the compound (43-1) in an inert solvent in the presence of a catalyst and in the presence or absence of an additive. The catalyst includes, for example, platinum catalysts such as platinum carbon, etc.; and palladium catalysts such as palladium carbon, palladium hydroxide carbon, etc. The additive includes ammonium formate and the like. The inert solvent includes, for example, alcohol solvents (e.g. ethanol, methanol and 2-propanol), ether solvents (tetrahydrofuran and 1,4-dioxane), and mixed solvents thereof. The reaction temperature may be chosen in the range of about 20°C to about 100°C.

### 3) Step 3

The compound (43-1) may be produced from the compound (43-2) by the same production process as described in the step 3 in the production process 1.

### 4) Step 4

The compound of the formula (IV) may be produced from the compound (43-1) by the same production process as described in literature (for example, T.W. Greene and P.G.M. Wuts, "Protective Groups in Organic Synthesis" 2nd Edition, John Wiley & Sons, Inc. (1991)).

In each of the production processes described above, when the starting compound in each reaction has a reactive group such as hydroxyl group, amino group or carboxyl group, the reactive group in a site other than a site where the reaction is desired is previously protected with a suitable protective group if necessary, and the protective group is removed after carrying out each reaction or after carrying out several reactions, whereby a desired compound may be obtained. As the protective group for protecting the hydroxyl group, amino group, carboxyl group or the like, conventional protective groups used in the field of organic synthetic chemistry may be used. The introduction and removal of such a protective group may be carried out according to a conventional method (for example, the method described in T.W. Greene and P.G.M. Wuts, "Protective Groups in Organic Synthesis" 2nd Edition, John Wiley & Sons, Inc. (1991)).

For example, the protective group for the hydroxyl group includes tert-butyldimethylsilyl group, methoxymethyl group, tetrahydropyranyl group and the like. The protective group for the amino group includes tert-butoxycarbonyl group, benzyloxycarbonyl group and the like. Such a protective group for the hydroxyl group may be removed by reaction in a solvent such as aqueous methanol, aqueous ethanol or aqueous tetrahydrofuran in the presence of an acid such as hydrochloric acid, sulfuric acid or acetic acid. In the case of tert-butyldimethylsilyl group, it is also possible to carry out the removal in a solvent such as tetrahydrofuran in the presence of, for example, tetrabutylammonium fluoride. In the case of tert-butoxycarbonyl group, the protective group for the amino group may be removed, for example, by reaction in a solvent such as aqueous tetrahydrofuran, dichloromethane, chloroform or aqueous methanol in the presence of an acid such as hydrochloric acid or trifluoroacetic acid. In the case of benzyloxycarbonyl group, the removal may be carried out, for example, by reaction in a solvent such as acetic acid in the presence of an acid such as hydrobromic acid.

As a form in which the carboxyl group is protected, tert-butyl esters, orthoesters and acid amides are exemplified. Such a protective group is removed as follows. In the case of the tert-butyl esters, the removal is carried out, for example, by reaction in an aqueous solvent in the presence of hydrochloric acid. In the case of the orthoesters, the removal is carried out, for example, by treatment with an acid and then an alkali such as sodium hydroxide in a solvent such as aqueous methanol, aqueous tetrahydrofuran or aqueous 1,2-dimethoxyethane. In the case of the acid amides, the removal may be carried out, for example, by reaction in a solvent such as water, aqueous methanol or aqueous tetrahydrofuran in the presence of an acid such as hydrochloric acid or sulfuric acid.

The compound of the formula (I) includes those having a center of optical activity. The compound having a center of optical activity may be obtained as a racemic modification, or it may be obtained as an optically active substance when an optically active starting material is used. If necessary, the racemic modification obtained may be physically or chemically resolved into optical antipodes by a well-known method. Preferably, diastereomers are formed from the racemic modification by a reaction using a reagent for optical resolution. The diastereomers different in form may be resolved by a well-known method such as fractional crystallization.

The compound of the present invention or the prodrug thereof may be converted to a salt, for example, by mixing with a pharmaceutically acceptable acid in a solvent such as water, methanol, ethanol or acetone. The pharmaceutically acceptable acid includes, for example, inorganic acids such as hydrochloric acid, hydrobromic acid, sulfuric acid, phosphoric acid, nitric acid, etc.; and organic acids such as acetic acid, propionic acid, oxalic acid, succinic acid, lactic acid, malic acid, tartaric acid, citric acid, maleic acid, fumaric acid, methanesulfonic acid, p-toluenesulfonic acid, ascorbic acid, etc.

The agents of the present invention are expected to be usable for the treatment of various diseases because of their inhibitory effect on DPP-IV. The compounds disclosed in the present specification are useful for the suppression of postcibal hyperglycemia in a prediabetic, the treatment of non-insulin-dependent diabetes mellitus, the treatment of autoimmune diseases such as arthritis and articular rheumatism, the treatment of intestinal mucosa diseases, growth acceleration, the inhibition of rejection of a transplantate, the treatment of corpulence, the treatment of eating disorder, the treatment of HIV infection, the suppression of cancer metastasis, the treatment of prostatomegaly, the treatment of periodontitis, and the treatment of osteoporosis.

When used for the treatment, the present inventive compound may be administered as a pharmaceutical composition orally or parenterally (for example, by intravenous, subcutaneous or intramuscular injection, locally, intrarectally, percutaneously, or through nose). Compositions for the oral administration include, for example, tablets, capsules, pills, granules, powders, solutions and suspensions. Compositions for the parenteral administration include, for example, aqueous or oily preparations for injection, ointments, creams, lotions, aerosols, suppositories and patches. These pharmaceutical compositions are prepared by conventional techniques and may contain non-toxic and inactive carriers or excipients conventionally used in the field of formulation.

Although the dose is varied depending on the individual compounds, the disease, age, body weight and sex of a patient, symptom, administration route and the like, the present inventive compound is usually administered to an adult (body weight: 50 kg) in a dose of 0.1 to 1000 mg/day, preferably 1 to 300 mg/day in one portion or two or three portions a day. It is also possible to administer the present inventive compound at intervals of several days to several weeks.

The present inventive compound may be used in combination with drugs such as remedies for diabetes, remedies for diabetic complications, antilipemics, hypotensors, anti-corpulence drugs, diuretics, etc. (these drugs are hereinafter abbreviated as concomitant drugs) in order to enhance its effect. The timing of administration of the compound and the concomitant drugs is not limited. They may be administered to an object of administration either at the same time or at different times. The dose of the concomitant drugs may be properly chosen on the basis of a dose clinically employed. It is also possible to prepare a mixture of the compound and the concomitant drug(s). The proportions of the compound and the concomitant drug(s) may be properly chosen depending on an object of administration, administration route, a disease to be treated, symptom, a combination of the compound and the concomitant drug(s), and the like. For example, when the object of administration is a human being, the concomitant drug(s) is used in an amount of 0.01 to 100 parts by weight per part by weight of the compound.

The remedies for diabetes include insulin products (e.g. animal insulin products extracted from bovine or porcine pancreas; and human insulin products synthesized by a genetic engineering technique by the use of Escherichia coli or yeast), insulin resistance improving agents (e.g. pioglitazone or its hydrochloride, troglitazone, rosiglitazone or its maleate, GI-262570, JTT-501, MCC-555, YM-440, KRP-297 and CS-011), α-glucosidase inhibitors (e.g. voglibose, acarbose, miglitol and emiglitate), biguanide preparations (e.g. metformin), insulin secretion accelerators (e.g. sulfonylurea preparations such as tolbutamide, glibenclamide, gliclazide, chlorpropamide, tolazamide, acetohexamide, glyclopyramide, glimepiride, etc.; repaglinide, senaglinide, nateglinide and mitiglinide), GLP-1, GLP-1 analogs (exenatide, liraglutide, SUN-E7001, AVE010, BIM-51077 and CJC1131), protein tyrosine phosphatase inhibitors (e.g. vanadic acid), and β3 agonists (e.g. GW-427353B and N-5984).

The remedies for diabetic complications includes aldose reductase inhibitors (e.g. tolrestat, epalrestat, zenarestat, zopolrestat, minarestat, fidarestat, SK-860 and CT-112), neurotrophic factors (e.g. NGF, NT-3 and BDNF), PKC inhibitors (e.g. LY-333531), AGE inhibitors (e.g. ALT946, pimagezine, pyratoxathine and N-phenacylthiazolium bromide (ALT766)), active oxygen removers (e.g. thioctic acid), and cerebrovasodilators (e.g. tiapride and mexiletine). The antilipemics include HMG-CoA reductase inhibitors (e.g. pravastatin, simvastatin, lovastatin, atorvastatin, fluvastatin, itavastatin, and their sodium salts), squalene synthetase inhibitors, ACAT inhibitors, and the like. The hypotensors include angiotensin converting enzyme inhibitors (e.g. captopril, enalapril, alacepril, delapril, lisinopril, imidapril, benazepril, cilazapril, temocapril and trandlapril), angiotensin II antagonists (e.g. candesartan cilexetil, losartan, eprosartan, valsartan, telmisartan, irbesartan and tasosartan), calcium antagonists (e.g. nicardipine hydrochloride, manidipine hydrochloride, nisoldipine, nitrandipine, nilvadipine and amlodipine), and the like.

The anti-corpulence drugs include, for example, central anti-corpulence drugs (e.g. phentermine, sibutramine, amfepramone, dexamphetamine, mazindol and SR-141716A), pancreas lipase inhibitors (e.g. orlistat), peptidergic anorexiants (e.g. leptin and CNTF (ciliary nerve trophic factor)) and cholecystokinin agonists (e.g. lintitript and FPL-15849). The diuretics include, for example, xanthine derivatives (e.g. sodium salicylate theobromine and potassium salicylate theobromine), thiazide preparations (e.g. ethiazide, cyclopenthiazide, trichlormethiazide, hydrochlorothiazide, hydroflumethiazide, benzylhydrochlorothiazide, penflutizide, polythiazide and methyclothiazide), anti-aldosterone preparations (e.g. spironolactone and triamteren), carbonate dehydratase inhibitors (e.g. acetazolamide), chlorobenzenesulfonamide preparations (e.g. chlorthalidone, mefruside and indapamide), azosamide, isosorbide, ethacrynic acid, piretanide, bumetanide and furosemide.

The concomitant drugs are preferably GLP-1, GLP-1 analogs, α-glucosidase inhibitors, biguanide preparations, insulin secretion accelerators, insulin resistance improving agents, and the like. The above-exemplified concomitant drugs may be used in combination of two or more thereof in proper proportions.

When the compound is used in combination with the concomitant drug(s), the amount of the drug(s) used may be reduced so as to be within a safe range in view of the side effects of the drug(s). In particular, the dose of the biguanide preparations may be reduced as compared with a conventional dose. Therefore, side effects causable by these drugs are safely preventable. In addition, the doses of the remedies for diabetic complications, antilipemics, hypotensors and the like may be reduced. As a result, side effects causable by these drugs are effectively preventable.

### EXAMPLES

The present invention is more concretely illustrated below with reference examples, working examples and test examples, which should not be construed as limiting the scope of the invention. The nomenclature of compounds shown in the reference examples and working examples mentioned below is not always based on IUPAC.

### Example 1

Methyl 2-[(3R)-3-aminopiperidin-1-yl]-7-chloro-3-(2-chlorobenzyl)-4-oxo-4,5-dihydro-3H-imidazo[4,5-c]pyridine-6-carboxylate hydrochloride

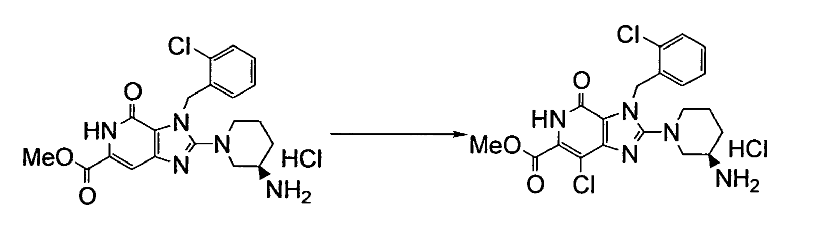

The compound of Example 12 (12.2 mg) was dissolved in N,N-dimethylformamide (0.5 mL), followed by adding thereto N-chlorosuccinimide (8.0 mg), and the resulting mixture was stirred at room temperature for 3 hours. After the solvent was removed, the residue was purified by a preparative thin-layer silica gel chromatography (developing solvent: chloroform/methanol = 10/1), followed by adding thereto 4N hydrochloric acid/1,4-dioxane, and the resulting mixture was concentrated to obtain the title compound (5.8 mg) as a white solid.
¹H NMR (400 MHz, CDCl₃) δ 7.41(dd, J = 1.2, 7.8 Hz, 1H), 7.26-7.15 (m, 2H), 6.74 (dd, J= 1.2, 7.6 Hz, 1H), 5.73 (d, J = 17.0 Hz, 1H), 5.66 (d, J = 17.0 Hz, 1H), 4.02 (s, 3H), 3.48-3.39 (m, 1H), 3.32-3.25 (m, 1H), 3.02-2.87 (m, 2H), 2.82-2.75 (m, 1H), 1.97-1.88 (m, 1H), 1.78-1.70 (m, 1H), 1.65-1.53 (m, 1H), 1.35-1.23 (m, 1H) .
MS (ESI+) 450(M⁺ +1, 100%)

### Example 2

2-[(3R)-3-Aminopiperidin-1-yl]-3-(2-chlorobenzyl)-5-methyl-4-oxo-4,5-dihydro-3H-imidazo[4,5-c]pyridine-6-carbonitrile hydrochloride

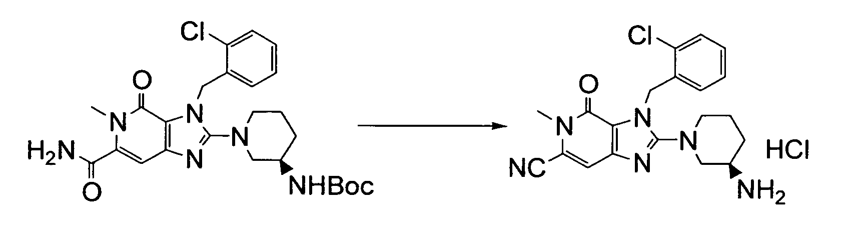

Trifluoroacetic anhydride (851 µL) was added dropwise to a solution of the compound of Reference Example 52 (851 mg) in tetrahydrofuran (20 mL), and the resulting mixture was stirred at room temperature for 2 hours. After the reaction, the reaction mixture was concentrated under reduced pressure and the residue was dissolved in methanol (20 mL). Potassium carbonate (323 mg) and water (0.3 mL) were added thereto and the resulting mixture was stirred at room temperature. After 1 hour, water was poured into the reaction solution, followed by extraction with ethyl acetate. The organic layer was dried over sodium sulfate and filtered, and the filtrate was concentrated under reduced pressure. The resulting residue was purified by a silica gel column chromatography (developing solvent: hexane/ethyl acetate = 1/1), whereby a product (645 mg) was isolated and purified as a white solid. To this product was added 4N hydrochloric acid/1,4-dioxane (10 mL), and the resulting mixture was stirred at 25°C for 1 hour and concentrated under reduced pressure. A saturated aqueous sodium hydrogencarbonate solution was added to the residue, followed by extraction with chloroform. The organic layer was washed with a saturated aqueous sodium chloride solution, dried over sodium sulfate and then filtered, and the filtrate was concentrated under reduced pressure. To the concentrate was added 4N hydrochloric acid/1,4-dioxane (5 mL) and the resulting mixture was concentrated under reduced pressure to obtain the title compound (473 mg) as a white solid.
¹H NMR (400 MHz, CD₃OD) δ 7.49-7.43 (m, 1H), 7.36 (s, 1H), 7.32-7.18 (m, 2H), 6.76-6.73(m, 1H), 5.74-5.64 (m, 2H), 3.68 (s, 3H), 3.68-3.63 (m, 1H), 3.48-3.38 (m, 1H), 3.25-3.14 (m, 2H), 3.00-2.89 (m, 1H), 2.12-2.03 (m, 1H), 1.82-1.70 (m, 1H), 1.69-1.55 (m, 2H).
MS (ESI+) 397 (M⁺+1, 100%).

### Example 3

2-[(3R)-3-Aminopiperidin-1-yl]-3-(2-chlorobenzyl)-6-(carboxymethyl)-5-methyl-3,5-dihydro-4H-imidazo[4,5-c]pyridin-4-one hydrochloride

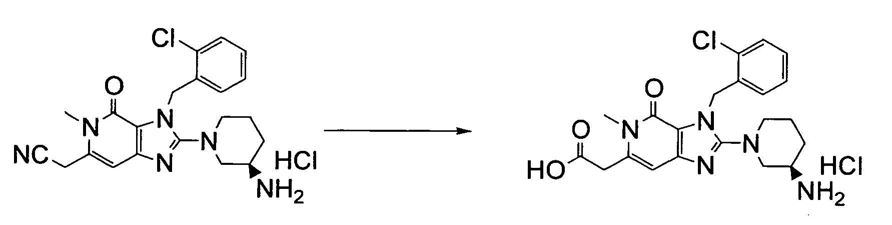

The compound of Example 37 (25 mg) was dissolved in 6N hydrochloric acid (2 mL) and the resulting solution was stirred with heating at 100°C for 8 hours. The reaction solution was cooled to 25°C and then concentrated under reduced pressure, and toluene was added thereto, followed by azeotropic distillation, whereby the title compound (26 mg) was obtained as a light-yellow solid.
¹H NMR (400 MHz, CD₃OD) δ 7.51-7.49 (m, 1H), 7.37-7.28 (m, 2H), 7.10-7.07 (m, 1H), 6.71(s, 1H), 5.74-5.67 (m, 2H), 3.98 (s, 2H), 3.86-3.83 (m, 1H), 3.52 (s, 3H), 3.52-3.11 (m,4H), 2.14-2.08 (m, 1H), 1.86-1.65 (m, 3H). MS (ESI+) 430 (M⁺+1, 100%).

### Example 4

2-[(3R)-3-Aminopiperidin-1-yl]-3-(2-chlorobenzyl)-5-methyl-3,5-dihydro-4H-imidazo[4,5-c]pyridin-4-one

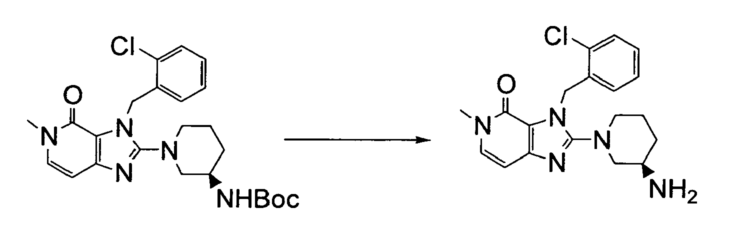

A 4N hydrochloric acid/1,4-dioxane solution (3 mL) was added to the compound of Reference Example 7 (49 mg) and the resulting mixture was stirred at 25°C for 2 hours. Water was poured into the reaction solution and the aqueous layer was washed with chloroform. Then, the aqueous layer was adjusted to pH 8 with a 4N aqueous sodium hydroxide solution and extracted with chloroform. The organic layer was dried over anhydrous sodium sulfate and filtered, and the filtrate was concentrated under reduced pressure to obtain the title compound (26 mg) as a white solid.
¹H NMR (400 MHz, CDCl₃) δ 7.41-7.38 (m, 1H), 7.22-7.13 (m, 2H), 7.09 (d, J = 7.2 Hz, 1H), 6.75-6.73 (m, 1H), 6.59 (d, J = 7.2 Hz, 1H), 5.70 (d, J = 17.0 Hz, 1H), 5.63 (d, J = 17.0 Hz, 1H), 3.57 (s, 3H), 3.35-3.31 (m, 1H), 3.22-3.19 (m, 1H), 2.93-2.88 (m, 2H), 2.71-2.66 (m, 1H), 1.85-1.22 (m, 4H).
MS (ESI+) 372 (M⁺+1, 100%).

The compounds of Examples 5 to 11 were synthesized from corresponding compounds of Reference Examples, respectively, by the same process as in Example 4.

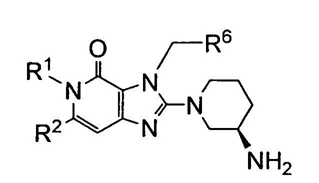

| Example number | R¹ | R² | R⁶ | Reference example number for starting material |
|---|---|---|---|---|
| Example 5 | Me | MeO(O)C | 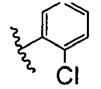 | Reference Example 13 |
| Example 6 | H | Et₂N(CH₂)₃O(O)C | 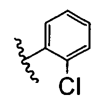 | Reference Example 14 |
| Example 7 | 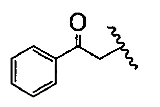 | H | 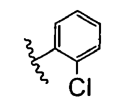 | Reference Example 22 |
| Example 8 | EtOC(O)CH₂ | H | 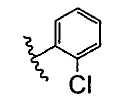 | Reference Example 23 |
| Example 9 | 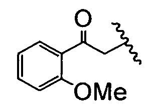 | H | 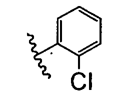 | Reference Example 24 |
| Example 10 | 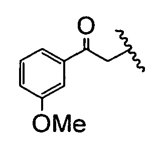 | H | 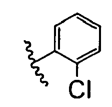 | Reference Example 25 |
| Example 11 | Me | H | 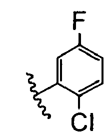 | Reference Example 1 |

### Example 5

¹H NMR(400MHz, CDCl₃) δ 7.42-7.38 (m, 1H), 7.35 (s, 1H), 7.27-7.12 (m, 2H), 6.73-6.70 (m, 1H), 5.71 (d, J = 17.0 Hz, 1H), 5.65 (d, J = 17.0 Hz, 1H), 3.92 (s, 3H), 3.73 (s, 3H), 3.39-3.31 (m, 1H), 3.25-3.16 (m, 1H), 2.97-2.87 (m, 2H), 2.75-2.65 (m, 1H), 1.95-1.85 (m, 1H), 1.78-1.66 (m, 1H), 1.34-1.23 (m, 2H).
MS (ESI+) 430 (M⁺+1, 100%).

### Example 6

¹H NMR(400MHz, CDCl₃) δ 7.44 (s, 1H), 7.42-7.39 (m, 1H), 7.25-7.14 (m, 2H), 6.78-6.76 (m, 1H), 5.72 (d, J = 17.0 Hz, 1H), 5.66 (d, J = 17.0 Hz, 1H), 4.41 (t, J = 6.4 Hz, 2H), 3.39-3.31 (m,1H), 3.26-3.17 (m, 1H), 2.94-2.84 (m, 2H), 2.73-2.65 (m, 1H), 2.60-2.48 (m, 6H), 1.95-1.85 (m, 2H), 1.80-1.59 (m, 2H), 1.31-1.19 (m, 2H), 1.02 (t, J = 7.1 Hz, 6H).
MS (ESI+) 515 (M⁺+1, 100%).

### Example 7

¹H NMR(400MHz, CDCl₃) δ 8.02-7.98 (m, 2H), 7.64-7.57 (m, 1H), 7.50-7.44 (m, 2H), 7.38-7.34 (m, 1H), 7.20-7.13 (m, 2H), 7.03 (d, J = 7.2 Hz, 1H), 6.81-6.78 (m, 1H), 6.69 (d, J = 7.2 Hz, 1H), 5.67 (d, J = 17.0 Hz, 1H), 5.62 (d, J = 17.0 Hz, 1H), 5.42 (s, 2H), 3.38-3.32 (m, 1H), 3.24-3.16 (m, 1H), 2.95-2.84 (m, 2H), 2.72-2.64 (m, 1H), 1.94-1.83 (m, 1H), 1.76-1.65 (m, 1H), 1.33-1.19 (m, 2H).
MS (ESI+) 476 (M⁺+1, 100%).

### Example 8

¹H NMR(400MHz, CDCl₃) δ 7.42-7.35 (m, 1H), 7.22-7.10 (m, 2H), 7.02 (d, J = 7.3 Hz, 1H),6.78-6.72 (m, 1H), 6.64 (d, J = 7.3 Hz, 1H), 5.71-5.59 (m, 2H), 4.67 (s, 2H), 4.22 (q, J = 7. 2Hz, 2H), 3.39-3.31 (m, 1H), 3.21-3.14 (m, 1H), 3.02-2.84 (m, 2H), 2.82-2.72 (m, 1H), 1.87-1.76 (m, 2H), 1.64-1.53 (m, 1H), 1.40-1.28 (m, 1H), 1.25 (t, J = 7.2 Hz, 3H).
MS (ESI+) 444 (M⁺+1, 100%).

### Example 9

¹H NMR(400MHz, CDCl₃) δ 7.95-7.84 (m, 1H), 7.66-7.55 (m, 1H), 7.49-7.35 (m, 1H), 7.30-6.89 (m, 5H), 6.76-6.71 (m, 1H), 6.69-6.59 (m, 1H), 5.70-5.52 (m, 2H), 5.34 (s, 2H), 3.95(s, 3H), 3.41-3.30 (m, 1H), 3.23-3.10 (m, 1H), 3.09-2.73 (m, 3H), 1.95-1.81 (m, 1H), 1.78-1.50 (m, 2H), 1.48-1.30 (m, 1H).
MS (ESI+) 506 (M⁺+1, 100%).

### Example 10

¹H NMR(400MHz, CDCl₃) δ 7.61-7.54 (m, 1H), 7.53-7.45 (m, 1H), 7.42-7.32 (m, 2H), 7.21-7.08 (m, 3H), 7.02 (d, J = 7.2 Hz, 1H), 6.81-6.75 (m, 1H), 6.69 (d, J = 7.2 Hz, 1H), 5.69-5.58 (m, 2H), 5.40 (s, 2H), 3.83 (s, 3H), 3.38-3.30 (m, 1H), 3.23-3.14 (m, 1H), 3.00-2.84 (m, 2H), 2.78-2.68 (m, 1H), 1.95-1.81 (m, 1H), 1.75-1.66 (m, 2H), 1.34-1.22 (m, 1H).
MS (ESI+) 506 (M⁺+1, 100%).

### Example 11

¹H NMR(400MHz, CDCl₃) δ 7.41-7.34 (m, 1H), 7.20 (d, J = 7.2 Hz, 1H), 6.94-6.87 (m, 1H),6.59 (d, J = 7.2 Hz, 1H), 6.51-6.41 (m, 1H), 5.66-5.56 (m, 2H), 3.57 (s, 3H), 3.36-3.28(m, 1H), 3.22-3.12 (m, 1H), 2.98-2.84 (m, 2H), 2.72-2.63 (m, 1H), 1.96-1.87 (m, 1H), 1.78-1.68 (m, 1H), 1.65-1.53 (m, 1H), 1.30-1.20 (m, 1H).
MS (ESI+) 392 (M⁺ +3, 100%).

### Example 12

Methyl 2-[(3R)-3-aminopiperidin-1-yl]-3-(2-chlorobenzyl)-4-oxo-4,5-dihydro-3H-imidazo[4,5-c]pyridine-6-carboxylate hydrochloride

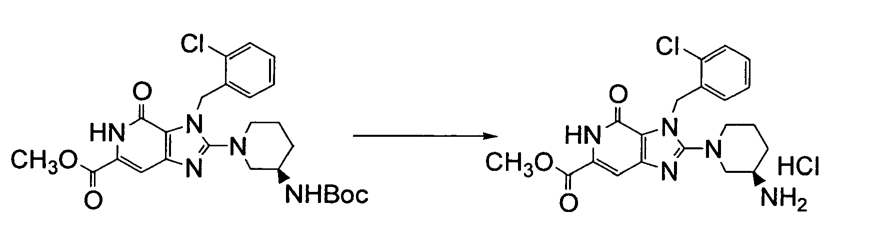

A 4N hydrochloric acid/1,4-dioxane solution (10 mL) was added to the compound of Reference Example 16 (1.01 g) and the resulting mixture was stirred at 25°C for 1 hour. After the reaction solution was concentrated under reduced pressure, toluene was added thereto, followed by azeotropic distillation. Thus, 1,4-dioxane was completely removed to obtain the title compound (870 mg) as a white solid.
¹H NMR (400 MHz, CD₃OD) δ 7.50-7.45 (m, 1H), 7.39 (s, 1H), 7.36-7.24 (m, 2H), 6.99-6.87 (m, 1H), 5.73 (s, 2H), 3.97 (s, 3H), 3.81-3.70 (m, 1H), 3.49-3.38 (m, 1H), 3.34-3.18 (m, 2H), 3.09-2.97 (m, 1H), 2.17-2.05 (m, 1H), 1.89-1.75 (m, 1H), 1.72-1.58 (m, 2H).
MS (ESI+) 416(M⁺ +1, 100%).
The compounds of Examples 13 to 43 were synthesized from corresponding compounds of Reference Examples, respectively, by the same process as in Example 12.

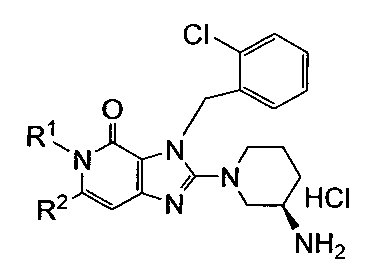

| Example number | R¹ | R² | Reference example number for starting material |
|---|---|---|---|
| Example 13 | H | CO₂H | Reference Example 17 |
| Example 14 | H | EtO(O)C | Reference Example 48 |
| Example 15 | Me | Me₂N(O)C | Reference Example 54 |
| Example 16 | Me | CO₂H | Reference Example 47 |
| Example 17 | H | Me₂CHO(O)C | Reference Example 49 |
| Example 18 | H | BnO(O)C | Reference Example 50 |
| Example 19 | H | H₂N(O)C | Reference Example 51 |
| Example 20 | H | 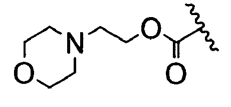 | Reference Example 15 |

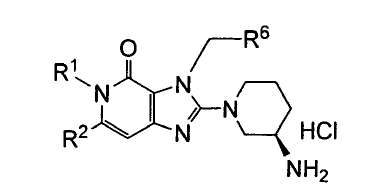

| Example number | R¹ | R² | R⁶ | Reference example number for starting material |
|---|---|---|---|---|
| Example 21 | Me | Me(O)C | 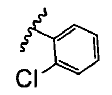 | Reference Example 32 |
| Example 22 | Me | Ph(O)C | 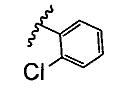 | Reference Example 33 |
| Example 23 | HOC(O)CH₂ | H | 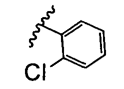 | Reference Example 28 |
| Example 24 | H | MeO(O)C | 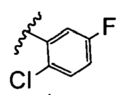 | Reference Example 55 |
| Example 25 | Et | HOCH₂ | 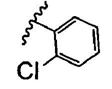 | Reference Example 64 |
| Example 26 | H | CO₂H | 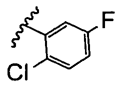 | Reference Example 56 |
| Example 27 | H | MeO(O)C | 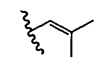 | Reference Example 57 |
| Example 28 | H | CO₂H | 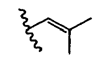 | Reference Example 65 |
| Example 29 | 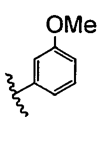 | H | 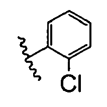 | Reference Example 66 |

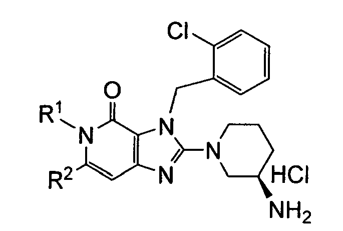

| Example number | R¹ | R² | Reference example number for starting material |
|---|---|---|---|
| Example 30 | 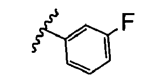 | H | Reference Example 67 |
| Example 31 | PhC(O)CH(Me) | H | Reference Example 26 |
| Example 32 | PhO(CH₂)₂ | H | Reference Example 27 |
| Example 33 | Ph | H | Reference Example 68 |
| Example 34 | 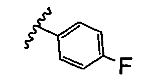 | H | Reference Example 69 |
| Example 35 | Me | CH₂OPh | Reference Example 70 |
| Example 36 | Me | 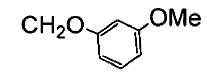 | Reference Example 71 |
| Example 37 | Me | NCCH₂ | Reference Example 72 |
| Example 38 | Me | 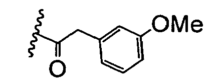 | Reference Example 34 |
| Example 39 | Me | PhCH₂(O)C | Reference Example 35 |
| Example 40 | Me | 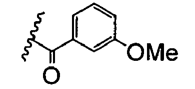 | Reference Example 36 |
| Example 41 | Me | i-Pr(O)C | Reference Example 37 |
| Example 42 | Me | 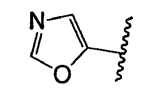 | Reference Example 38 |
| Example 43 | Me | CHO | Reference Example 39 |

### Example 13

¹H NMR (400 MHz, CD₃OD) δ 7.51-7.45 (m, 1H), 7.35 (s, 1H), 7.34-7.22 (m, 2H), 7.03-6.87 (m, 1H), 5.74 (s, 2H), 3.86-3.70 (m, 1H), 3.51-3.39 (m, 1H), 3.37-3.18 (m, 2H), 3.13-2.95 (m, 1H), 2.16-2.05 (m, 1H), 1.89-1.77 (m, 1H), 1.75-1.55 (m, 2H).
MS (ESI+) 402(M⁺ +1, 100%).

### Example 14

¹H NMR (400 MHz, CD₃OD) δ 7.48-7.40 (m, 1H), 7.39 (s, 1H), 7.32-7.18 (m, 2H), 6.81-6.73 (m, 1H), 5.76-5.70 (m, 2H), 4.42 (q, J = 7.1 Hz, 2H), 3.71-3.63 (m, 1H), 3.50-3.37 (m, 1H), 3.23-3.08 (m, 2H), 3.00-2.89 (m, 1H), 2.13-2.02 (m, 1H), 1.86-1.71 (m, 1H), 1.70-1.51 (m, 2H) 1.40 (t, J = 7.1 Hz, 3H).
MS (ESI+) 430(M⁺ +1, 100%).

### Example 15

¹H NMR (400 MHz, CD₃OD) δ 7.45-7.38 (m, 1H), 7.28-7.15 (m, 2H), 6.97-6.90 (m, 1H), 6.62(s, 1H), 5.63 (s, 2H), 3.76-3.68 (m, 1H), 3.42-3.34 (m, 1H), 3.34 (s, 3H), 3.28-3.15 (m, 2H), 3.04 (s, 3H), 3.04-2.97 (m, 1H), 2.94 (s, 3H), 2.08-1.96 (m, 1H), 1.81-1.70 (m, 1H), 1.66-1.54 (m, 2H).
MS (ESI+) 443(M⁺ +1, 100%).

### Example 16

¹H NMR (400 MHz, CD₃OD) δ 7.49-7.47 (m, 1H), 7.33 (s, 1H), 7.33-7.23 (m, 2H), 6.90-6.87 (m, 1H), 5.72 (s, 2H), 3.79-3.71 (m, 1H), 3.70 (s, 3H), 3.50-3.41 (m, 1H), 3.33-3.20 (m, 2H), 3.08-2.99 (m, 1H), 2.14-2.05 (m, 1H), 1.89-1.78 (m, 1H), 1.74-1.55 (m, 2H).
MS (ESI+) 416(M⁺ +1, 100%).

### Example 17

¹H NMR (400 MHz, CD₃OD) δ 7.52-7.45 (m, 1H), 7.35 (s, 1H), 7.37-7.27 (m, 2H), 7.15-7.02 (m, 1H), 5.76 (s, 2H), 5.30-5.22 (m, 1H), 3.99-3.83 (m, 1H), 3.53-3.32 (m, 3H), 3.23-3.08 (m, 1H), 2.20-2.08 (m, 1H), 1.94-1.80 (m, 1H), 1.75-1.61 (m, 2H), 1.40 (s, 3H), 1.38 (s, 3H).
MS (ESI+) 444(M ⁺+1, 100%).

### Example 18

MS (ESI+) 492(M⁺ +1, 100%).

### Example 19

¹H NMR (400 MHz, CD₃OD) δ 7.43-7.38 (m, 1H), 7.30-7.15 (m, 2H), 7.27 (s, 1H), 6.89-6.81 (m, 1H), 5.64 (s, 2H), 3.73-3.63 (m, 1H), 3.51-3.39 (m, 1H), 3.37-3.13 (m, 2H), 3.04-2.92 (m, 1H), 2.08-1.96 (m, 1H), 1.81-1.69 (m, 1H), 1.65-1.49 (m, 2H).
MS (ESI+) 401(M⁺ +1, 100%).

### Example 20

¹H NMR (400 MHz, CD₃OD) δ 7.54 (s, 1H), 7.53-7.46 (m, 1H), 7.38-7.25 (m, 2H), 7.00-6.94 (m, 1H), 5.73 (s, 2H), 4.79-4.71 (m, 2H), 4.09-3.97 (m, 2H), 3.95-3.77 (m, 3H), 3.74-3.55 (m, 5H), 3.53-3.41 (m, 1H), 3.38-3.06 (m, 4H), 2.17-2.05 (m, 1H), 1.91-1.79 (m, 1H), 1.75-1.61 (m, 2H).
MS (ESI+) 515(M⁺ +1, 100%).

### Example 21

¹H NMR (400 MHz, CD₃OD) δ 7.50-7.47 (m, 1H), 7.33 (s, 1H), 7.33-7.28 (m, 1H), 7.26-7.22 (m, 1H), 6.83-6.81 (m, 1H), 5.71 (s, 2H), 3.75-3.66 (m, 1H), 3.56 (s, 3H), 3.50-3.40 (m,1H), 3.31-3.20 (m, 2H), 3.04-2.93 (m, 1H), 2.63 (s, 3H), 2.17-2.05 (m, 1H), 1.88-1.74 (m, 1H), 1.71-1.55 (m, 2H).
MS (ESI+) 414(M⁺ +1, 100%).

### Example 22

¹H NMR (400 MHz, CD₃OD) δ 7.98-7.93 (m, 2H), 7.78-7.70 (m, 1H), 7.62-7.54 (m, 2H), 7.51-7.45 (m, 1H), 7.38-7.23 (m, 2H), 6.95-6.83 (m, 1H) 6.79(s, 1H), 5.74 (s, 2H), 3.78-3.62 (m, 1H), 3.48 (s, 3H), 3.48-3.39 (m, 1H), 3.38-3.18 (m, 2H), 3.09-2.95 (m, 1H), 2.17-2.04 (m, 1H), 1.87-1.75 (m, 1H), 1.74-1.53 (m, 2H).
MS (ESI+) 476(M⁺ +1, 100%).

### Example 23

¹H NMR (400 MHz, CD₃OD) δ 7.56-7.59 (m, 1H), 7.48 (d, J = 7.3 Hz, 1H), 7.37-7.24 (m, 2H), 7.07-7.00 (m, 1H), 6.70(d, J = 7.3 Hz, 1H), 5.68 (s, 2H), 4.76 (s, 2H), 3.88-3.79 (m, 1H), 3.50-3.42 (m, 1H), 3.39-3.23 (m, 2H), 3.17-3.07 (m, 1H), 2.17-2.08 (m, 1H), 1.91-1.79(m, 1H), 1.74-1.61 (m, 2H).
MS (ESI+) 416 (M⁺+1, 100%).

### Example 24

¹H NMR (400 MHz, CD₃OD) δ 7.53-7.46 (m, 1H), 7.39 (s, 1H), 7.14-7.03 (m, 1H), 6.78-6.63 (m, 1H), 5.66 (s, 2H), 3.96 (s, 3H), 3.80-3.69 (m, 1H), 3.55-3.42 (m, 1H), 3.34-3.21 (m, 2H), 3.13-3.01 (m, 1H), 2.17-2.08 (m, 1H), 1.93-1.80 (m, 1H), 1.78-1.51 (m, 2H).
MS (ESI+) 434(M⁺ +1, 100%).

### Example 25

¹H NMR (400 MHz, CD₃OD) δ 7.53-7.48 (m, 1H), 7.40-7.28 (m, 2H), 7.18-7.11 (m, 1H), 6.83 (s, 1H), 5.79-5.69 (m, 2H), 4.66 (s, 2H), 3.97-3.90 (m, 1H), 3.65 (s, 3H), 3.51-3.37 (m, 3H), 3.22-3.12 (m, 1H), 2.19-2.09 (m, 1H), 1.92-1.80 (m, 1H), 1.76-1.64 (m, 2H).
MS (ESI+) 402(M⁺ +1, 100%).

### Example 26

¹H NMR (400 MHz, CD₃OD) δ 7.56-7.48 (m, 1H), 7.38 (s, 1H), 7.16-7.09 (m, 1H), 6.91-6.81 (m, 1H), 5.67 (s, 2H), 3.86-3.78 (m, 1H), 3.59-3.49 (m, 1H), 3.40-3.29 (m, 2H), 3.20-3.08 (m, 1H), 2.19-2.10 (m, 1H), 1.94-1.82 (m, 1H), 1.79-1.64 (m, 2H).
MS (ESI+) 420(M⁺ +1, 100%).

### Example 27

¹H NMR (400 MHz, CD₃OD) δ 7.32 (s, 1H), 5.44-5.39 (m, 1H), 5.07-5.00 (m, 2H), 3.96 (s, 3H), 3.87-3.75 (m, 1H), 3.62-3.50 (m, 2H), 3.37-3.16 (m, 2H), 2.28-2.13 (m, 1H), 2.07-1.93 (m, 1H), 1.91-1.69 (m, 2H), 1.82 (s, 3H), 1.76 (s, 3H).
MS (ESI+) 360 (M⁺+1, 100%).

### Example 28

¹H NMR (400 MHz, CD₃OD) δ 7.32 (s, 1H), 5.48-5.39 (m, 1H), 5.10-4.98 (m, 2H), 3.92-3.75(m, 1H), 3.64-3.48 (m, 2H), 3.39-3.13 (m, 2H), 2.25-2.13 (m, 1H), 2.07-1.93 (m, 1H), 1.91-1.64 (m, 2H), 1.82 (s, 3H), 1.77 (s, 3H).
MS (ESI+) 346 (M⁺+1, 100%).

### Example 29

¹H NMR (400 MHz, CD₃OD) δ 7.50-7.35 (m, 2H), 7.33-7.13 (m, 4H), 7.05-6.85 (m, 3H), 6.67-6.69 (m, 1H), 5.71 (s, 2H), 3.81 (s, 3H), 3.79-3.65 (m, 1H), 3.51-3.36 (m, 1H), 3.35-3.26 (m, 2H), 3.11-2.95 (m, 1H), 2.17-2.03 (m, 1H), 1.90-1.77 (m, 1H), 1.75-1.55 (m, 2H).
MS (ESI+) 464 (M⁺+1, 100%).

### Example 30

¹H NMR (400 MHz, CD₃OD) δ 7.56-7.42 (m, 3H), 7.31-7.15 (m, 5H), 6.97-6.91 (m, 1H), 6.77(d, J = 7.3 Hz, 1H), 5.69 (s, 2H), 3.80-3.68 (m, 1H), 3.49-3.39 (m, 1H), 3.35-3.20 (m, 2H), 3.05-2.95 (m, 1H), 2.15-2.05 (m, 1H), 1.89-1.75 (m, 1H), 1.74-1.55 (m, 2H).
MS (ESI+) 452 (M⁺+1, 100%).

### Example 31

¹H NMR (400 MHz, CD₃OD) δ 7.91-7.84 (m, 2H), 7.61-7.51 (m, 2H), 7.49-7.38 (m, 3H), 7.35-7.28 (m, 1H), 7.24-7.16 (m, 1H), 6.82-6.75 (m, 1H), 6.71 (d, J = 7.4 Hz, 1H), 6.38-6.28 (m, 1H), 5.72-5.58 (m, 2H), 3.76-3.68 (m, 1H), 3.49-3.35 (m, 1H), 3.32-3.16 (m, 2H), 3.08-2.95 (m, 1H), 2.13-2.04 (m, 1H), 1.89-1.75 (m, 1H), 1.70-1.54 (m, 2H), 1.64-1.62 (m, 3H).
MS (ESI+) 490 (M⁺+1, 100%).

### Example 32

¹H NMR (400 MHz, CD₃OD) δ 7.77-7.68 (m, 1H), 7.51-7.45 (m, 1H), 7.38-7.16 (m, 4H), 7.05-6.97 (m, 1H), 6.92-6.77 (m, 3H), 6.66 (d, J = 7.3 Hz, 1H), 5.72 (s, 2H), 4.40 (t, J = 4.9 Hz, 2H), 4.21 (t, J = 4.9 Hz, 2H),3.85-3.74 (m, 1H), 3.50-3.42 (m, 1H), 3.38-3.22 (m, 2H), 3.14-3.03 (m, 1H), 2.17-2.05 (m, 1H), 1.90-1.78 (m, 1H), 1.76-1.55 (m, 2H).
MS (ESI+) 478 (M⁺+1, 100%).

### Example 33

¹H NMR (400 MHz, CD₃OD) δ 7.55-7.40 (m, 5H), 7.36-7.33 (m, 2H), 7.27-7.18 (m, 2H), 6.86-6.84 (m, 1H), 6.75 (d, J = 7.3 Hz, 1H), 5.69 (s, 2H), 3.50-3.15 (m, 4H), 3.05-2.95 (m,1H), 2.15-2.08 (m, 1H), 1.87-1.73 (m, 1H), 1.72-1.53 (m, 2H).
MS (ESI+) 434 (M⁺+1, 100%).

### Example 34

¹H NMR (400 MHz, CD₃OD) δ 7.72-7.54 (m, 1H), 7.48-7.42 (m, 1H), 7.41-7.18 (m, 6H), 7.10-7.00 (m, 1H), 6.79-6.77 (m, 1H), 5.71 (s, 2H), 3.90-3.75 (m, 1H), 3.55-3.42 (m, 1H), 3.40-3.22 (m, 2H), 3.18-3.05 (m, 1H), 2.18-2.05 (m, 1H), 1.90-1.75 (m, 1H), 1.7-1.52 (m, 2H).
MS (ESI+) 452 (M⁺+1, 100%).

### Example 35

¹H NMR (400 MHz, CD₃OD) δ 7.51-7.46 (m, 1H), 7.38-7.25 (m, 4H), 7.10-6.97 (m, 3H), 6.89-6.86 (m, 2H), 5.71 (s, 2H), 5.17 (s, 2H), 3.77-3.68 (m, 1H), 3.63 (s, 3H), 3.50-3.39 (m, 1H), 3.37-3.21 (m, 2H), 3.08-2.98 (m, 1H), 2.14-2.05 (m, 1H), 1.90-1.80 (m, 1H), 1.85-1.72 (m, 2H).
MS (ESI+) 478 (M⁺+1, 100%).

### Example 36

¹H NMR (400 MHz, CD₃OD) δ 7.49-7.46 (m, 1H), 7.38-7.23 (m, 3H), 6.87 (s, 1H), 6.87-6.85(m, 1H), 6.69-6.57 (m, 3H), 5.71 (s, 2H), 5.16 (s, 2H), 3.77 (s, 3H), 3.60 (s, 3H), 3.73-3.12 (m, 4H), 3.10-3.00 (m, 1H), 2.18-2.10 (m, 1H), 1.91-1.80 (m, 1H), 1.81-1.61 (m, 2H).
MS (ESI+) 508 (M⁺+1, 100%).

### Example 37

¹H NMR (400 MHz, CD₃OD) δ 7.51-7.45 (m, 1H), 7.35-7.24 (m, 2H), 6.96-6.94 (m, 1H), 6.86(s, 1H), 5.70 (s, 2H), 4.23 (s, 2H), 3.82-3.56 (m, 3H), 3.57 (s, 3H), 3.49-3.41 (m, 1H),3.12-3.02 (m, 1H), 2.17-2.07 (m, 1H), 1.88-1.62 (m, 3H).
MS (ESI+) 411 (M⁺+1, 100%).

### Example 38

¹H NMR (400 MHz, DMSO-d₆) δ ppm 8.21-8.18 (m, 3H), 7.60 (s, 1H), 7.53-7.50 (m, 1H), 7.32-7.24 (m, 3H), 6.89-6.85 (m, 3H), 6.64 (d, J= 6.4Hz, 1H), 5.59 (s, 2H), 4.35 (s, 2H), 3.74 (s, 3H), 3.62-3.58 (m, 1H), 3.34 (s, 3H), 3.34-3.32 (m, 1H), 3.16-3.05 (m, 2H), 2.85-2.80 (m, 1H), 1.94-1.91 (m, 1H), 1.75-1.71 (m, 1H), 1.56-1.49 (m, 2H).
MS (ESI+) 520 (M⁺+1, 100%).

### Example 39

¹H NMR (400 MHz, DMSO-d₆) δ ppm 8.16-8.10 (m, 3H), 7.60 (s, 1H), 7.52-7.50 (m, 2H), 7.36-7.24 (m, 7H), 6.65-6.62 (m, 1H), 5.58 (s, 2H), 4.38 (s, 2H), 3.45-3.40 (m, 1H), 3.32 (s, 3H), 3.14-3.07 (m, 2H), 2.83-2.80 (m, 1H), 1.92-1.90 (m, 1H), 1.75-1.72 (m, 1H), 1.53-1.48 (m, 2H).
MS (ESI+) 490 (M⁺+1, 100%).

### Example 40

¹H NMR (400 MHz, DMSO-d₆) δ ppm 8.09-8.03 (m, 3H), 7.55-7.53 (m, 2H), 7.46-7.43 (m, 2H), 7.36-7.28 (m, 3H), 6.76 (s, 1H), 6.73-6.71 (m, 1H), 5.62 (s, 2H), 3.85 (s, 3H), 3.59-3.56 (m, 1H), 3.35 (s, 3H), 3.35-3.30 (m, 1H), 3.13-3.06 (m, 2H), 2.68-2.67 (m, 1H), 1.92-1.91 (m, 1H), 1.73-1.70 (m, 1H), 1.53-1.49 (m, 2H).
MS (ESI+) 506 (M⁺+1, 100%).

### Example 41

¹H NMR (400 MHz, CD₃OD) δ ppm 8.25-8.11 (m, 3H), 7.95-7.88 (m, 3H), 7.50-7.49 (m, 1H), 7.36-7.29 (m, 2H), 7.16 (s, 1H), 7.04 (d, J=7.0 Hz, 1H), 5.74 (s, 2H), 3.86-3.83 (m, 1H), 3.58-3.49 (m, 1H), 3.42-3.30 (m, 2H), 3.19-3.10 (m, 1H), 2.13-2.10 (m, 1H), 1.88-1.85 (m, 1H), 1.72-1.67 (m, 2H), 1.22 (d, J= 6.8Hz, 6H).
MS (ESI+) 442 (M⁺ +1, 100%).

### Example 42

MS (ESI+) 439(M⁺ +1, 100%).

### Example 43

MS (ESI+) 400(M⁺ +1, 64%).

### Example 44

Methyl 2-[(3R)-3-aminopiperidin-1-yl]-3-(2-chlorobenzyl)-5,7-dimethyl-4-oxo-4,5-dihydro-3H-imidazo[4,5-c]pyridine-6-carboxylate

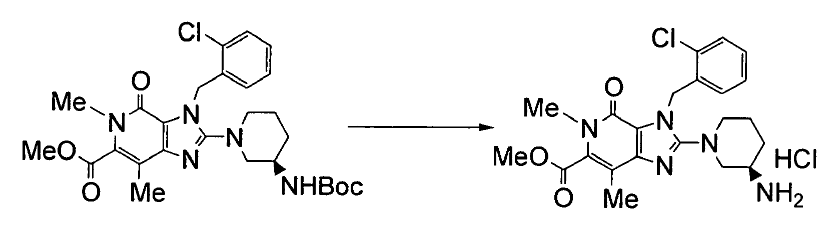

The title compound (260 mg) was synthesized from the compound of Reference Example 40 by the same process as in Example 12.
¹H NMR (400 MHz, CD₃OD) δ 7.49-7.47 (m, 1H), 7.30-7.21 (m, 2H), 6.88-6.86 (m, 1H), 5.71 (s, 2H), 4.00 (s, 3H), 3.82-3.72 (m, 1H), 3.50-3.38 (m, 1H), 3.45 (s, 3H), 3.35-3.21 (m, 2H), 3.08-2.98 (m, 1H), 2.32 (s, 3H), 2.16-2.05 (m, 1H), 1.86-1.75 (m, 1H), 1.71-1.59 (m, 2H).
MS (ESI+) 444(M⁺ +1, 100%).

### Example 45

2-[(3R)-3-aminopiperidin-l-yl]-3-(2-chlorobenzyl)-5,7-dimethyl-3,5-dihydro-4H-imidazo[4,5-c]pyridin-4-one

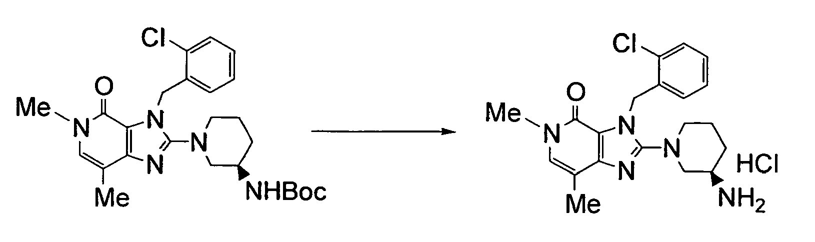

The title compound was synthesized from the compound of Reference Example 43 by the same process as in Example 12.
¹H NMR (400 MHz, CD₃OD) δ 7.51-7.41 (m, 1H), 7.38-7.25 (m, 2H), 7.08-6.98 (m, 1H), 5.80-5.69 (m, 2H), 3.95-3.83 (m, 1H), 3.57 (s, 3H), 3.54-3.30 (m, 3H), 3.20-3.09 (m, 1H), 2.36 (s, 3H), 2.18-2.05 (m, 1H), 1.91-1.77 (m, 1H), 1.75-1.58 (m, 2H).
MS (ESI+) 386(M⁺ +1, 100%).

### Example 46

2-[(3R)-3-Aminopiperidin-1-yl]-3-(2-chlorobenzyl)-3,5-dihydro-4H-imidazo[4,5-c]qquinolin-4-one

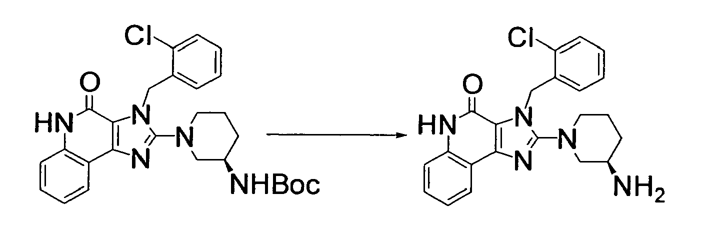

The title compound (33 mg) was synthesized by the same process as in Example 4.
¹H NMR (400 MHz, CDCl₃) δ 8.23-8.18 (m, 1H), 7.47-7.42 (m, 1H), 7.40-7.33 (m, 1H), 7.30-7.24 (m, 1H), 7.23-7.18 (m, 1H), 7.14-7.09 (m, 2H), 6.79-6.75 (m, 1H), 5.76 (d, J = 17 Hz, 1H), 5.70 (d, J = 17 Hz, 1H), 3.42-3.34 (m, 1H), 3.29-3.20 (m, 1H), 3.04-2.90 (m, 2H), 2.85-2.73 (m, 1H), 1.97-1.84 (m, 1H), 1.81-1.69 (m, 1H), 1.35-1.20 (m, 2H).
MS (ESI+) 408(M⁺ +1, 100%).

### Example 47

Methyl 2-[(3R)-3-aminopiperidin-1-yl]-3-(2-chlorobenzyl)-4-oxo-4,5-dihydro-3H-imidazo[4,5-c]quinoline-8-carboxylate hydrochloride

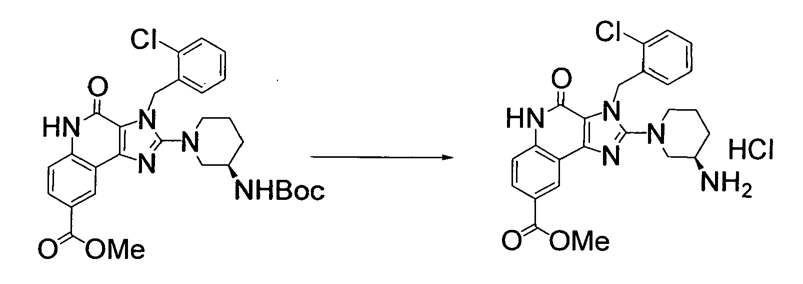

The title compound (5.3 mg) was synthesized by the same process as in Example 12.
¹H NMR (400 MHz, CD₃OD) δ 8.90 (d, J = 1.7 Hz, 1H), 8.12 (dd, J = 1.7, 8.7 Hz, 1H), 7.70-7.61 (m, 1H), 7.57-7.47 (m, 1H), 7.34-7.17 (m, 2H), 6.84 (d, J = 6.7 Hz, 1H), 5.75 (s, 2H), 3.96 (s, 3H), 3.89-3.85 (d, J = 11.8 Hz, 1H), 3.77-3.54 (m, 2H), 3.48-3.32 (m, 1H), 3.13-3.08 (m, 1H), 2.14 (m, 1H), 1.85 (m, 1H), 1.71-1.54 (m, 2H).
MS (ESI+) 466 (M⁺+1, 100%).

### Example 48

2-{(3R)-3-Aminopiperidin-1-yl}-3-(2-chlorobenzyl)-4-oxo-4,5-dihydro-3H-imidazo[4,5-c]quinoline-8-carboxylic acid hydrochloride

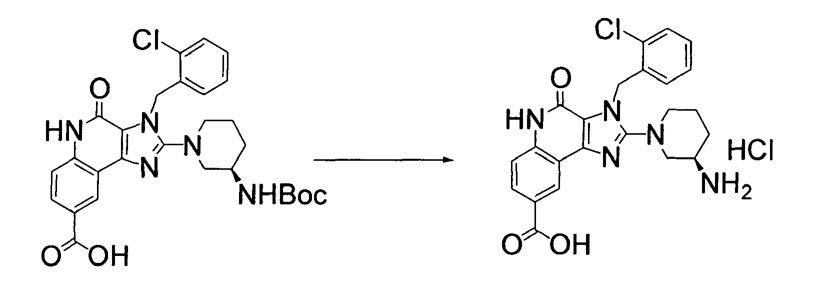

The title compound (2.6 mg) was synthesized by the same process as in Example 12.
¹H NMR (300 MHz, CD₃OD) δ 8.89 (d, J = 1.8 HZ, 1H), 8.12 (dd, J = 1.8, 8.8 Hz, 1H), 7.50-7.46 (m, 2H), 7.33-7.22 (m, 2H), 6.87 (dd, J = 1.3, 7.5 Hz, 1H), 5.75 (brs, 1H), 3.83-3.05 (m, 8H), 2.13-2.11 (m, 1H), 1.85-1.82 (m, 1H), 1.73-1.33 (m, 2H).
MS (ESI+) 452 (M⁺+1, 100%).

The compounds of Examples 49 to 72 were synthesized from corresponding compounds of Reference Examples, respectively, by the same process as in Example 12.

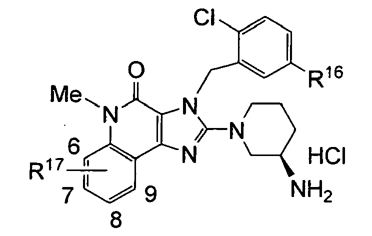

| Example number | R¹⁶ | R¹⁷ | Reference example number for starting material |
|---|---|---|---|
| Example 49 | H | 6-CO₂Me | Reference Example 76 |
| Example 50 | H | 8-CO₂Me | Reference Example 79 |
| Example 51 | H | 8-CO₂H | Reference Example 121 |
| Example 52 | H | 6-CO₂H | Reference Example 123 |
| Example 53 | H | 7-CO₂Me | Reference Example 78 |
| Example 54 | H | 7-CO₂H | Reference Example 120 |
| Example 55 | H | 7,9-CO₂Me | Reference Example 80 |
| Example 56 | H | 7,9-CO₂H | Reference Example 124 |
| Example 57 | H | H | Reference Example 151 |
| Example 58 | F | 7-CO₂H | Reference Example 86 |
| Example 59 | H | 6-MeO/7-CO₂Et | Reference Example 81 |
| Example 60 | H | 6,8-F/7-CO₂Et | Reference Example 82 |
| Example 61 | F | 8-CO₂Me | Reference Example 83 |
| Example 62 | H | 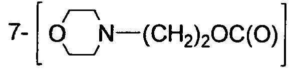 | Reference Example 107 |
| Example 63 | H | 7-[t-BuC(O)OCH₂OC(O)] | Reference Example 115 |
| Example 64 | F | 7-[EtOC(O)OCH(Me)OC(O)] | Reference Example 116 |
| Example 65 | H | 8-CH₂CO₂H | Reference Example 125 |
| Example 66 | H | 8-CH₂CO₂Et | Reference Example 87 |
| Example 67 | F | 7-MeO/8-CO₂H | Reference Example 126 |
| Example 68 | F | 6-MeO/8-CO₂H | Reference Example 127 |
| Example 69 | F | 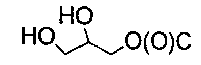 | Reference Example 117 |
| Example 70 | F | Me₂N(CH₂)₂O(O)C | Reference Example 118 |
| Example 71 | F | 7,9-CO₂H | Reference Example 128 |
| Example 72 | F | 7,9-CO₂Me | Reference Example 91 |

### Example 49

¹H NMR (300 MHz, CD₃OD) δ 8.59 (d, J = 7.3 Hz, 1H), 7.85 (d, J = 7.3 Hz, 1H), 7.52-7.47 (m, 2H), 7.37-7.27 (m, 2H), 7.13 (d, J = 6.8 Hz, 1H), 5.78 (s, 2H), 4.05(brd, J = 10.1 Hz, 1H), 3.97 (s, 3H), 3.77-3.71 (m, 1H), 3.68-3.44 (m,3H), 3.64 (s, 3H), 2.18 (m, 1H), 1.89-1.74 (m, 3H).
MS (ESI+) 480 (M⁺+1, 100%).

### Example 50

¹H NMR (300 MHz, DMSO-d₆) δ 8.70 (d, J = 2.0 Hz, 1H), 8.08 (dd, J = 2.0, 8.8 Hz, 1H), 7.70 (d, J = 8.8 Hz, 1H), 7.51 (dd, J = 1.1, 7.7 Hz, 1H), 7.33-7.20 (m, 2H), 6.70 (d, J = 7.7 Hz, 1H), 5.63 (d,J = 18.1 Hz, 1H), 5.57 (d, J = 18.1 Hz, 1H), 3.91 (s, 3H), 3.64 (s, 3H), 3.50-3.37 (m 2H), 3.26-3.19 (m, 1H), 3.11-3.07 (m, 1H), 2.86 -2.82 (m, 1H), 1.95 (m, 1H), 1.75 (m, 1H), 1.60-1.54 (m, 2H).
MS (ESI+) 480 (M⁺+1, 100%).

### Example 51

¹H NMR (300 MHz, CD₃OD) δ 8.96 (d, J = 2.0 HZ, 1H), 8.25 (dd, J = 2.0, 9.0 Hz, 1H), 7.73 (d, J = 9.0 Hz, 1H), 7.49 (dd, J = 1.1, 7.7 Hz, 1H), 7.35-7.23 (m, 2H), 6.98 (d, J = 8.2 Hz, 1H), 5.71 (s, 2H), 3.93 (brd, J = 11.3 Hz, 1H), 3.74-3.37 (m, 3H), 3.64 (s, 3H), 3.21-3.06 (m, 1H), 2.16 (m, 1H), 1.87-1.72 (m, 3H).
MS (ESI+) 466 (M⁺+1, 100%).

### Example 52

¹H NMR (300 MHz, CD₃OD) δ 8.53 (m, 1H), 7.91-7.88 (m, 1H), 7.51-7.46 (m, 2H), 7.36-7.07 (m, 3H), 5.78 (brs, 2H), 3.75-3.52 (m, 5H), 3.62 (s, 3H), 2.16 (m, 1H), 1.87-1.72 (m, 3H).
MS (ESI+) 466 (M⁺+1, 100%).

### Example 53

¹H NMR (300 MHz, CD₃OD) δ 8.36 (d, J = 8.0 Hz, 1H), 8.25 (brs, 1H), 8.01 (d, J = 8.0 Hz, 1H), 7.48 (d, J = 6.1 Hz, 1H), 7.33-7.14 (m, 2H), 6.83 (d, J = 7.0 Hz, 1H), 5.77 (s, 2H), 3.98 (s, 3H), 3.85-3.72 (m, 1H), 3.75 (s, 3H), 3.67-3.65 (m, 1H), 3.59-3.44 (m, 1H), 3.38-3.25 (m, 1H), 3.08-3.06 (m, 1H), 2.16-2.13 (m, 1H), 1.83-1.59 (m, 3H).
MS (ESI+) 480 (M⁺+1, 100%).

### Example 54

¹H NMR (300 MHz, DMSO-d₆) δ 8.21 (d, J = 8.1 Hz, 1H), 8.08 (d, J = 1.1 Hz, 1H), 7.90 (dd, J = 1.1, 8.1 Hz, 1H), 7.51 (dd, J = 1.3, 7.9 Hz, 1H), 7.33-7.19 (m, 2H), 6.68 (d, J = 7.5 Hz, 1H), 5.65 (bs, 2H), 3.67 (s, 3H), 3.51-3.43 (m, 1H), 3.35 (m, 1H), 3.25-3.18 (m, 1H), 3.07 (m, 1H), 2.87-2.81 (m, 1H), 2.01-1.94 (m, 1H), 1.76(m, 1H), 1.59-1.52 (m, 2H).
MS (ESI+) 466 (M⁺+1, 100%).

### Example 55

¹H NMR (300 MHz, CD₃OD) δ 8.22 (s, 1H), 7.87 (s, 1H), 7.47 (d, J = 7.9 Hz, 1H), 7.30-7.17 (m, 2H), 6.70 (d, J = 7.7 Hz, 1H), 5.75 (brs, 2H), 4.05 (s, 3H), 3.98 (s, 3H), 3.72 (s, 3H), 3.67-3.65 (m, 1H), 3.59-3.57 (m, 1H), 3.47 (m, 1H), 3.11 (m, 1H), 2.94 (m, 1H), 2.11 (m, 1H), 1.82-1.66 (m, 3H).
MS (ESI+) 538 (M⁺+1, 100%).

### Example 56

¹H NMR (300 MHz, CD₃OD) δ 8.50 (s, 1H), 8.42 (s, 1H), 7.51 (d, J = 8.0 Hz, 1H), 7.36-7.24 (m, 2H), 7.00 (d, J = 7.7 Hz, 1H), 5.82 (brs, 2H), 3.88-3.18 (m, 5H), 3.81 (s, 3H), 2.14 (m, 1H), 1.84-1.74 (m, 3H).
MS (ESI+) 509 (M⁺+1, 100%).

### Example 57

¹H NMR (300 MHz, DMSO-d₆) δ 8.14 (d, J = 7.3 Hz, 1H), 7.61-7.57 (m, 2H), 7.51 (dd, J = 1.2, 7.9 Hz, 1H), 7.38-7.13 (m, 3H), 6.64 (d, J = 6.2 Hz, 1H), 5.63 (brs, 2H), 3.69-3.62 (m, 4H), 3.33 (m, 1H), 3.24-3.17 (m, 1H), 3.05 (m, 1H), 2.87 (m, 1H), 1.91 (m, 1H), 1.74 (m, 1H), 1.53 (m, 2H).
MS (ESI+) 422 (M⁺+1, 100%).

### Example 58

¹H NMR (400 MHz, DMSO-d₆) δ 8.23 (d. J = 8.1 Hz, 1H), 8.10 (bs, 3H), 7.91, (d, J = 8.1 Hz, 1H), 7.60 (m, 1H), 7.22 (m, 1H), 6.63 (m, 1H), 5.60 (s, 2H), 3.57 (s, 3H), 3.50 (m, 1H), 3.49 (m, 1H), 3.24 (m, 1H), 3.08 (m, 1H), 2.92 (m, 1H), 2.33 (m, 1H), 1.89 (m, 1H), 1.60-1.55 (m, 2H)
MS (ESI+) 484 (M⁺+1, 100%).

### Example 59

¹H NMR (400 MHz, CD₃OD) δ 8.14 (d, J = 8.2 Hz, 1H), 7.71 (d, J = 8.2 Hz, 1H), 7.51 (dd, J = 7.9 and 1.3 Hz, 1H), 7.33 (ddd, J = 7.9, 7.9 and 1.3 Hz, 1H), 7.27 (ddd, J = 7.9, 7.9 and 1.3 Hz, 1H), 6.95 (d, J = 7.9 Hz, 1H), 5.77 (s, 2H), 4.43 (q, J = 7.1 Hz, 2H), 3.90 (s, 3H), 3.85 (brs, 1H), 3.80 (s, 3H), 3.54-3.52 (m, 2H), 3.42-3.39 (m, 1H), 3.14-3.08 (m, 1H), 2.15-2.13 (m, 1H), 1.88-1.84 (m, 1H), 1.74-1.67 (m, 2H), 1.43 (t, J = 7.1 Hz, 3H).
MS (ESI⁺) 524 (M⁺ + 1, 54%), 400 (77%), 125 (100%).

### Example 60

¹H NMR (400 MHz, CD₃OD) δ 7.88 (dd, J = 8.8 and 1.6 Hz, 1H), 7.49 (dd, J = 7.9 and 1.3 Hz, 1H), 7.30 (ddd, J = 7.9, 7.9 and 1.3 Hz, 1H), 7.23 (ddd, J = 7.9, 7.9 and 1.3 Hz, 1H), 6.80 (d, J = 7.9 Hz, 1H), 5.74 (s, 2H), 4.46 (q, J = 7.1 Hz, 2H), 3.88-3.85 (m, 3H), 3.76-3.73 (m, 1H), 3.51 (brs, 1H), 3.20 (brs, 2H), 3.02 (brs, 1H), 2.12 (brs, 1H), 1.81 (brs, 1H), 1.69-1.65 (m, 2H), 1.41 (t, J = 7.1 Hz, 3H).
MS(ESI⁺) 530 (M⁺ + 1, 89%), 406 (69%), 125 (100%).

### Example 61

¹H NMR (400 MHz, DMSO-d₆) δ 8.79 (s, 1H), 8.12-8.09 (m, 3H), 7.74 (m, 1H), 7.60 (m, 1H), 7.23 (m, 1H), 6.65 (m, 1H), 5.58 (s, 2H), 3.92 (s, 3H), 3.69 (m, 1H), 3.66 (s, 3H), 3.42 (m, 1H), 3.22 (m, 1H), 3.11 (m, 1H), 2.91 (m, 1H), 2.45 (m, 1H), 1.80 (m, 1H), 1.59-1.55 (m, 2H)
MS (ESI+) 498 (M⁺+1, 100%).

### Example 62

¹H NMR (300 MHz, DMSO) δ 8.24 (d, J = 8.3 Hz, 1H), 8.19 (brs, 1H), 8.01 (dd, J = 1.5, 8.4 Hz, 1H), 7.52 (dd, J = 1.5, 8.1 Hz, 1H), 7.33-7.20 (m, 2H), 6.69 (d, J = 6.6 Hz, 1H), 5.64 (brs, 2H), 4.73 (brs, 2H), 3.93-3.19 (m, 13H), 3.71 (s, 3H), 3.10-3.05 (m, 1H), 2.88-2.81 (m, 1H), 2.01-1.90 (m, 1H), 1.77 (m 1H), 1.55 (m, 2H).
MS (ESI+) 579 (M⁺ +1, 100%).

### Example 63

¹H NMR (400 MHz, CD₃OD) δ 8.39 (m, 1H), 8.30 (m, 1H), 8.03 (m, 1H), 7.49 (m, 1H), 7.31 (m, 1H), 7.23 (m, 1H), 6.77 (m, 1H), 6.07 (s, 2H), 5.78 (s, 2H), 3.86 (s, 3H), 3.81 (m, 1H), 3.68 (m, 1H), 3.50 (m, 1H), 3.15 (m, 1H), 2.14 (m, 1H), 1.83 (m, 1H), 1.72-1.64 (m, 2H), 1.33 (m, 1H), 1.25 (s, 9H)
MS (ESI+) 580 (M⁺+1, 100%).

### Example 64

¹H NMR (400 MHz, CD₃OD) δ 8.45 (m, 1H), 8.24 (s, 1H), 8.02 (m, 1H), 7.57 (m, 1H), 7.13 (m, 1H), 7.04 (m, 1H), 6.81 (m, 1H), 5.72 (s, 2H), 4.23 (1, J = 7.08 Hz, 2H), 3.93 (m, 1H), 3.78 (s, 3H), 3.63 (m, 1H), 3.52 (m, 1H), 3.35 (m, 1H). 3.18 (m, 1H), 2.18 (m, 1H), 1.95 (m, 1H), 1.75-1.71 (m, 2H), 1.69 (d, J = 5.4Hz, 3H), 1.33 (m, 3H).
MS (ESI+) 600 (M⁺+1, 100%).

### Example 65

¹H NMR (300 MHz, DMSO-d₆) δ 8.32 (bs, 3H), 8.05 (d, J = 1.7Hz, 1H), 7.55-7.43 (m, 3H), 7.31-7.18 (m, 2H), 6.63 (d, J = 7.0Hz, 1H), 5.63 (s, 2H), 3.73 (s, 2H), 3.70-3.67 (m, 1H), 3.60 (s, 3H), 3.40-3.17 (m, 2H), 3.08-3.05 (m, 1H), 2.85-2.81 (m, 1H), 1.96-1.92 (m, 1H), 1.76-1.73 (m, 1H), 1.56-1.51 (m, 2H).
MS (ESI+) 480 (M⁺+1, 100%).

### Example 66

¹H NMR (300 MHz, DMSO-d₆) δ 8.38 (bs, 3H), 8.08 (d, J = 1.9Hz, 1H), 7.56-7.44 (m, 3H), 7.32-7.14 (m, 2H), 6.66 (d, J = 6.8Hz, 1H), 5.63 (s, 2H), 4.09 (dd, J = 7.2, 14.1Hz, 2H), 3.83 (s, 2H), 3.67-3.65 (m, 1H), 3.66 (s, 3H), 3.29-3.22 (m, 2H), 3.07-3.05 (m, 1H), 2.84-2.81 (m, 1H), 1.95-1.93 (m, 1H), 1.76-1.74 (m, 1H), 1.57-1.53 (m, 2H), 1.19 (d, J = 7.0Hz, 3H).
MS (ESI+) 508 (M⁺+1, 100%).

### Example 67

¹H NMR (300 MHz, DMSO-d₆) δ 8.50 (s, 1H), 8.28 (brs, 3H), 7.58 (dd, J = 5.1 and 8.8 Hz, 1H), 7.20 (td, J= 3.0 and 8.5 Hz, 1H), 7.05 (s, 1H), 6.62 (dd, J = 2.9 and 9.3 Hz, 1H), 5.53 (dd, J = 17.9 and 18.1 Hz, 2H), 3.96 (s, 3H), 3.75-3.65 (m, 1H), 3.64 (s, 3H), 3.50-3.40 (m, 1H), 3.30-3.18 (m, 1H), 3.15-3.05 (m, 1H), 2.95-2.85 (m, 1H), 2.00-1.90 (m, 1H), 1.85-1.70 (m, 1H), 1.65-1.45 (m, 2H).
MS (ESI+) 514 (M⁺+1, 100%).

### Example 68

¹H NMR (300 MHz, DMSO-d₆) δ 8.36 (d, J = 1.7 Hz, 1H), 8.29 (brs, 3H), 7.58 (d, J = 1.7 Hz, 1H), 7.57 (dd, J = 5.1 and 8.7 Hz, 1H), 7.20 (td, J = 2.9 and 8.5 Hz, 1H), 6.65 (dd, J = 2.9 and 9.3 Hz, 1H), 5.55 (dd, J = 17.9 and 18.1 Hz, 2H), 3.93 (s, 3H), 3.79 (s, 3H), 3.72-3.62 (m, 1H), 3.45-3.35 (m, 1H), 3.30-3.18 (m, 1H), 3.15-3.05 (m, 1H), 2.95-2.85 (m, 1H), 2.00-1.90 (m, 1H), 1.82-1.70 (m, 1H), 1.68-1.42 (m, 2H).
MS (ESI+) 514 (M⁺+1, 100%).

### Example 69

¹H NMR (400 MHz, CD₃OD) δ 8.32 (m, 1H), 8.26 (s, 1H), 7.96 (m, 1H), 7.42 (m, 1H), 6.99 (m, 1H), 6.53 (m, 1H). 5.61 (s, 2H), 4.38 (m, 1H), 4.31 (m, 1H), 3.95 (m, 1H), 3.69 (s. 3H), 3.59-3.56 (m, 3H), 3.48-3.40 (m, 2H), 3.26-3.40 (m, 2H), 3.26-3.21 (m, 2H), 3.05-2.98 (m, 1H), 2.03 (m, 1H), 1.75 (m, 1H), 1.68-1.55 (m, 2H)
MS (ESI+) 558 (M⁺+1, 100%).

### Example 70

¹H NMR (400 MHz, DMSO-d₆) δ 8.27 (m, 1H), 8.19 (bs, 3H), 8.04 (m, 1H), 7.61 (m, 1H), 7.23 (m, 1H), 6.65 (m, 1H), 5.60 (s, 2H), 4.68-4.66 (m, 2H), 3.72 (s, 3H), 3.60-3.45 (m, 5H), 3.26 (m, 1H), 3.13 (m,1H), 2.71 (s, 6H), 2.08 (m, 1H), 1.89 (m, 1H), 1.63-1.55 (m, 2H)
MS (ESI+) 555 (M⁺+1, 100%).

### Example 71

¹H NMR (300 MHz, DMSO-d₆) δ 8.13 (s, 1H), 7.83 (s, 1H), 7.60 (dd, J = 5.1, 8.0 Hz, 1H), 7.24-7.12 (m, 1H), 6.65 (dd, J = 2.9, 9.3 Hz, 1H), 5.59 (brs, 2H), 3.70 (s, 3H), 3.65-3.55 (m, 1H), 3.49-3.43 (m, 1H), 3.28-3.21 (m, 1H), 3.09-3.05 (m, 1H), 2.95-2.89 (m, 1H), 1.94 (m, 1H), 1.72-1.62 (m, 2H), 1.51-1.49 (m, 1H).
MS (ESI+) 528 (M⁺+1, 100%).

### Example 72

¹H NMR (300 MHz, DMSO-d₆) δ 8.14 (d, J = 1.3 Hz, 1H), 7.81 (d, J = 1.3 Hz, 1H), 7.58 (dd, J = 5.0, 9.3 Hz, 1H), 7.23-7.17 (m, 1H), 6.68 (dd, J = 2.9, 9.3 Hz, 1H), 5.62 (d, J = 17.4 HZ, 1H), 5.54 (d, J = 17.4 Hz, 1H), 3.96 (s, 3H), 3.92 (s, 3H), 3.70 (s, 3H), 3.67-3.63 (m, 1H), 3.55-3.45 (m, 1H), 3.24-3.17 (m, 1H), 3.03-2.99 (m, 1H), 2.83-2.77 (m, 1H), 1.92-1.78 (m, 2H), 1.60-1.54 (m, 2H).
MS (ESI+) 556 (M⁺+1, 100%).

The compounds of Examples 73 to 85 were synthesized from corresponding compounds of Reference Examples, respectively, by the same process as in Example 12.

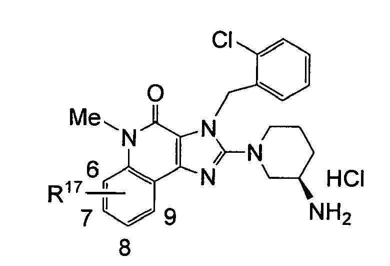

| Example number | R¹⁷ | Reference example number for starting material |
|---|---|---|
| Example 73 | 8-OCHF₂ | Reference Example 84 |
| Example 74 | 7-C(O)NH₂ | Reference Example 100 |
| Example 75 | 7-CN | Reference Example 99 |
| Example 76 | 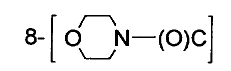 | Reference Example 102 |
| Example 77 | 8-C(O)NMe₂ | Reference Example 103 |
| Example 78 | 7-CH₂OMe | Reference Example 104 |
| Example 79 | 7-CO₂Et | Reference Example 106 |
| Example 80 | 7-CO₂(i-Pr) | Reference Example 108 |
| Example 81 | 7-CO₂(i-Bu) | Reference Example 109 |
| Example 82 | 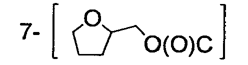 | Reference Example 110 |
| Example 83 | 7-CO₂CH(Me)CH(Me)₂ | Reference Example 111 |
| Example 84 | 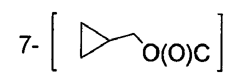 | Reference Example 112 |
| Example 85 | 7-CO₂(CH₂)₃OEt | Reference Example 113 |

### Example 73

¹H NMR (300 MHz, CD₃OD) δ 7.87 (d, J = 2.9 Hz, 1H), 7.66-7.59 (m, 1H), 7.50 (dd, J = 1.5, 8.0 Hz, 1H), 7.39 (dd, J = 2.9, 9.2 Hz, 1H), 7.35-7.10 (m, 3H), 6.67 (dd, J = 1.4, 7.7 Hz, 1H), 5.64 (brs, 2H), 3.69-3.58 (m, 1H), 3.61 (s, 3H), 3.38-3.20 (m, 2H), 3.10-3.06 (m, 1H), 2.88-2.81 (m, 1H), 1.95-1.89 (m, 1H), 1.75 (m, 1H), 1.61-1.51 (m, 2H).
MS (ESI+) 488 (M⁺+1, 100%).

### Example 74

¹H NMR (400 MHz, CD₃OD) δ 8.46 (d, J = 7.8 Hz, 1H), 8.16 (brs, 1H), 7.92 (d, J = 7.8 Hz, 1H), 7.51 (d, J = 7.8 Hz, 1H), 7.39-7.28 (m, 1H), 7.22-7.08 (m, 2H), 5.75 (brs, 2H), 4.01-3.93 (m, 2H), 3.83-2.97 (m, 6H), 2.17 (m, 1H), 1.88 (m, 1H), 1.74 (m, 2H).
MS (ESI+) 465 (M⁺+1, 100%).

### Example 75

¹H NMR (300 MHz, CD₃OD) δ 8.52-8.49 (m, 1H), 8.07 (brs, 1H), 7.72 (brd, J = 8.3 Hz, 1H), 7.49 (brd, J = 7.9 Hz, 1H), 7.35-7.15 (m, 2H), 6.99-6.97 (m, 1H), 5.78 (brs, 2H), 3.95-3.91 (m, 1H), 3.66 (s, 3H), 3.59-3.53 (m, 2H), 3.47-3.34 (m, 1H), 3.16 (m, 1H), 2.15 (m, 1H), 1.85-1.70 (m, 3H).
MS (ESI+) 447 (M⁺+1, 100%).

### Example 76

¹H NMR (300 MHz, CD₃OD) δ 8.67 (m, 1H), 7.78-7.75 (m, 2H), 7.52-7.44 (m, 1H), 7.36-7.27 (m, 2H), 7.06 (m, 1H), 5.68-5.58 (m, 2H), 4.14-4.10 (m, 1H), 3.86-3.24 (m, 12H), 3.65 (s, 3H), 2.15 (brs, 1H), 1.90-1.74 (m, 3H).
MS (ESI+) 535 (M⁺+1, 100%).

### Example 77

¹H NMR (300 MHz, CD₃OD) δ 8.60-8.53 (m, 1H), 7.80-7.66(m, 2H), 7.41 (d, J = 7.9 Hz, 1H), 7.27-7.13 (m, 2H), 7.10-6.98 (m, 1H), 5.63-5.42 (m, 2H), 4.01 (m, 1H), 3.64-3.55 (m, 2H), 3.55 (s, 3H), 3.49-3.46 (m, 1H), 3.31 (m, 1H), 3.09 (s, 3H), 3.05 (s, 3H), 2.05 (m, 1H), 1.84-1.68 (m, 3H).
MS (ESI+) 493 (M⁺+1, 100%).

### Example 78

¹H NMR (300 MHz, CD₃OD) 8.37(d, J = 8.1 Hz, 1H), 7.66 (brs, 1H), 7.50 (dd, J = 1.3, 8.1 Hz, 1H), 7.45 (brd, J = 8.0 Hz, 1H), 7.36-7.26 (m, 2H), 7.06 (d, J = 7.5 Hz, 1H), 5.80 (brs, 2H), 4.65 (brs, 2H), 3.99 (d, J = 9.9 Hz, 1H), 3.74 (s, 3H), 3.74-3.71 (m, 3H), 3.47 (s, 3H), 3.24-3.17 (m 1H), 2.16 (m, 1H), 1.88-1.72 (m, 3H).
MS (ESI+) 466 (M⁺+1, 000%).

### Example 79

¹H NMR (400 MHz, DMSO-d₆) δ 8.25 (d, J = 8.2 Hz, 1H), 8.08 (d, J = 1.2 Hz, 1H), 7.92 (dd, J = 1.2, 8.2 Hz, 1H), 7.53 (dd, J = 1.1, 7.9 Hz, 1H), 7.32-7.22 (m, 2H), 6.71 (d, J = 6.7 Hz, 1H), 5.65 (d, J = 17.1 Hz, 1H), 5.58 (d, J = 17.1 Hz, 1H), 4.39 (dd, J = 7.0, 14.1 Hz, 2H), 3.93-3.66 (m, 1H), 3.68 (s, 3H), 3.26 (brs, 1H), 3.25-3.22 (m, 1H), 3.09-3.06 (m, 1H), 2.89-2.82 (m, 1H), 1.96 (m, 1H), 1.79-1.75 (m, 1H), 1.64-1.52 (m, 2H), 1.38 (t, J = 7.0 Hz, 3H).
MS (ESI+) 494 (M⁺+1, 100%).

### Example 80

¹H NMR (300 MHz, DMSO-d₆) δ 8.31 (d, J = 8.0 Hz, 1H), 8.14 (d, J = 1.3 Hz, 1H), 7.97 (dd, J = 1.3, 8.0 Hz, 1H), 7.59 (dd, J = 1.3, 7.9 Hz, 1H), 7.40-7.28 (m, 2H), 6.77 (d, J = 6.2 Hz, 1H), 5.76 (d, J = 17.8 Hz, 1H), 5.65 (d, J = 17.8 Hz, 1H), 5.31-5.22 (m, 1H), 3.75 (s, 3H), 3.59-3.50 (m, 1H), 3.40-3.12 (m, 2H), 3.12 (m, 1H), 2.94-2.87 (m, 1H), 2.08-1.97 (m, 1H), 1.82 (m, 1H), 1.67-1.61 (m, 2H), 1.43 (d, J = 6.2 Hz, 6H).
MS (ESI+) 508 (M⁺+1, 100%).

### Example 81

¹H NMR (300 MHz, DMSO-d₆) δ 8.24 (d, J = 8.3 Hz, 1H), 8.08 (d, J = 1.1 Hz, 1H), 7.92 (dd, J = 1.1, 8.3 Hz, 1H), 7.51 (dd, J = 1.3, 8.0 Hz, 1H), 7.33-7.17 (m, 2H), 6.69 (d, J = 6.2 Hz, 1H), 5.65 (d, J = 17.8 Hz, 1H), 5.59 (d, J = 17.8 Hz, 1H), 4.12 (d, J = 6.6 Hz, 2H), 3.67 (s, 3H), 3.51-3.43 (m, 1H), 3.37-3.19 (m, 2H), 3.09-3.05 (m, 1H), 2.87-2.81 (m, 1H), 2.11-1.90 (m, 2H), 1.74 (m, 1H), 1.59-1.56 (m, 2H), 1.00 (d, J = 15.8 Hz, 6H).
MS (ESI+) 522 (M⁺+1, 100%).

### Example 82

¹¹H NMR (300 MHz, DMSO-d₆) δ 8.25 (d, J = 8.0 Hz, 1H), 8.07 (d, J = 1.3 Hz, 1H), 7.91 (dd, J = 1.3, 8.0 Hz, 1H), 7.51 (dd, J = 1.3, 8.1 Hz, 1H), 7.32-7.20 (m, 2H), 6.70 (d, J = 7.4 Hz, 1H), 5.65 (d, J = 17.4 Hz, 1H), 5.58 (d, J = 17.4 Hz, 1H), 4.37-4.18 (m, 3H), 3.83-3.76 (m, 1H), 3.72-3.64 (m, 2H), 3.67 (s, 3H), 3.32-3.19 (m, 2H), 3.05 (m, 1H), 2.83-2.80 (m, 1H), 2.07-1.54 (m, 8H).
MS (ESI+) 550 (M⁺+1, 100%).

### Example 83

¹H NMR (300 MHz, DMSO-d₆) δ 8.24 (d, J = 8.3 Hz, 1H), 8.07 (d, J = 1.1 Hz, 1H), 7.91 (dd, J = 1.1, 8.3 Hz, 1H), 7.51 (dd, J = 1.3, 7.9 Hz, 1H), 7.33-7.20 (m, 2H), 6.69 (d, J = 6.0 Hz, 1H), 5.69-5.56 (m, 2H), 4.99-4.92 (m, 1H), 3.67 (s, 3H), 3.73-3.63 (m, 1H), 3.51-3.42 (m, 1H), 3.37-3.19 (m, 2H), 2.84-2.81 (m, 1H), 2.01-1.91 (m, 2H), 1.75 (m, 1H), 1.54 (m, 2H), 1.27 (d, J = 6.4 Hz, 3H), 0.97 (dd, J = 3.5, 6.6 Hz, 6H).
MS (ESI+) 536 (M⁺+1, 100%).

### Example 84

¹H NMR (300 MHz, DMSO-d₆) δ 8.25 (d, J = 8.0 Hz, 1H), 8.07 (brs, 1H), 7.92 (dd, J = 0.7, 8.0 Hz, 1H), 7.51 (d, J = 7.9 Hz, 1H), 7.32-7.20 (m, 2H), 6.70 (d, J = 7.7 Hz, 1H), 5.64 (d, J = 17.0 Hz, 1H), 5.57 (d, J = 17.0 Hz, 1H), 4.17 (d, J = 7.1 Hz, 2H), 3.67 (s, 3H), 3.67 (m, 1H), 3.33-3.19 (m, 2H), 3.08-3.04 (m, 1H), 2.86-2.80 (m, 1H), 1.95 (m, 1H), 1.74 (m, 1H), 1.60-1.54 (m, 2H), 1.33-1.22 (m, 1H), 0.62-0.56 (m, 2H), 0.41-0.36 (m, 2H).
MS (ESI+) 520 (M⁺+1, 100%).

### Example 85

¹H NMR (300 MHz, DMSO-d₆) δ 8.19 (d, J = 8.1 Hz, 1H), 8.01 (d, J = 1.3 Hz, 1H), 7.86 (dd, J = 1.3, 8.1 Hz, 1H), 7.46 (dd, J = 1.1, 7.9 Hz, 1H), 7.28-7.14 (m, 2H), 6.64 (d, J = 6.4 Hz, 1H), 5.59 (d, J = 17.2 Hz, 1H), 5.53 (d, J = 17.2 Hz, 1H), 4.33 (m, 2H), 3.70-3.59 (m, 1H), 3.61 (s, 3H), 3.47 (t, J = 6.2 Hz, 2H), 3.37 (dd, J = 7.0, 14.1 Hz, 2H), 3.27-3.14 (m, 2H), 3.03-2.99 (m, 1H), 2.81-2.75 (m, 1H), 1.97-1.84 (m, 3H), 1.70 (m, 1H), 1.55-1.48 (m, 2H), 1.04 (t, J = 7.0Hz, 3H).
MS (ESI+) 552 (M⁺+1, 100%).

### Example 86

2-[(3R)-3-Aminopiperidin-1-yl]-3-(2-chlorobenzyl)-5-methyl-4-oxo-4,5-dihydro-3H-imidazo[4,5-c]quinoline-8-carboxylic acid methanesulfonate

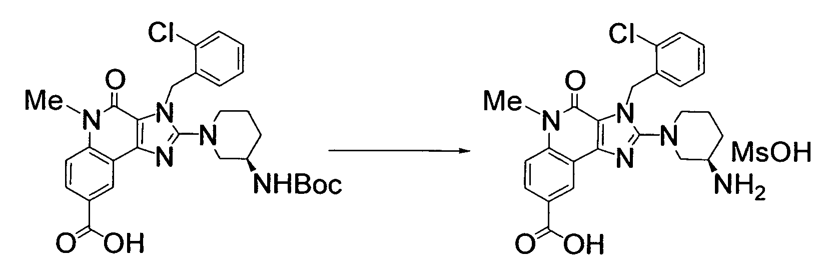

Methanesulfonic acid (770 µL) was added dropwise to a solution of 2-{(3R)-3-[(tert-butoxycarbonyl)amino]-piperidin-1-yl}-3-(2-chlorobenzyl)-5-methyl-4-oxo-4,5-dihydro-3H-imidazo[4,5-c]quinoline-8-carboxylic acid (1.5 g) in 1,4-dioxane (50 mL), and the resulting mixture was stirred at 90°C for 4 hours. After the reaction, the solid precipitated was filtered and the thus obtained solid was recrystallized from 2-propanol to obtain the title compound (780 mg) as a white solid.
¹H NMR (400 MHz, DMSO-d₆) δ 8.72 (s, 1H), 8.09 (m, 1H), 7.92 (bs, 3H), 7.68 (m, 1H), 7.53 (m, 1H), 7.32-7.24 (m, 2H), 6.70 (m, 1H), 5.62 (s, 2H), 3.70 (m, 1H), 3.67 (s, 3H), 3.38 (m, 1H), 3.19-3.16 (m, 2H), 2.90 (m, 1H), 2.33 (s, 3H), 1.95 (m, 1H), 1.75 (m, 1H), 1.53-1.47 (m, 2H)
MS (ESI+) 466(M⁺ +1, 100%).

### Example 87

2-[(3R)-3-Aminopiperidin-1-yl]-3-(2-chloro-5-fluorobenzyl)-5-methyl-4-oxo-4,5-dihydro-3H-imidazo[4,5-c]quinoline-8-carboxylic acid methanesulfonate

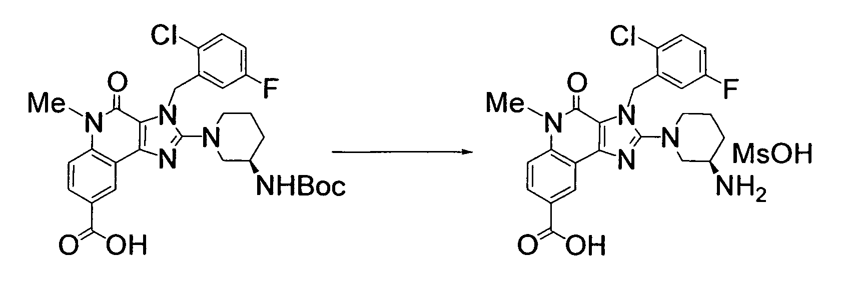

The title compound (447 mg) was obtained as a white solid by the same process as in Example 86.
¹H NMR (400 MHz, DMSO-d₆) δ 8.77 (s, 1H), 8.09 (m, 1H), 7.95 (bs, 3H), 7.70 (m, 1H), 7.61 (m, 1H), 7.23 (m, 1H), 6.64 (m, 1H), 5.57 (s, 2H), 3.70 (m, 1H), 3.66 (s, 3H), 3.46 (m, 1H), 3.24-3.19 (m, 2H), 2.93 (m, 1H), 1.95 (m, 1H), 1.75 (m, 1H), 1.58-1.53 (m, 2H)
MS (ESI+) 484(M⁺ +1, 100%).

### Example 88

2-[(3R)-3-Aminopiperidin-1-yl]-3-(2-chloro-5-fluorobenzyl)-8-fluoro-5-methyl-4-oxo-4,5-dihydro-3H-imidazo[4,5-c]quinoline-7-carboxylic acid methanesulfonate

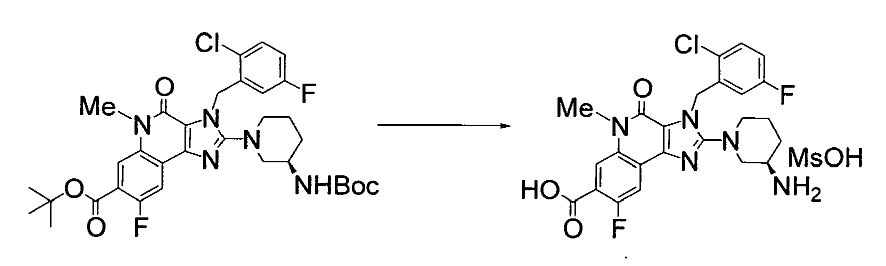

The title compound (1.2 g) was synthesized by the same process as in Example 86.
¹H NMR (400 MHz, CD₃OD) δ 8.15 (d, J=5.9Hz, 1H), 8.00 (d, J = 12 Hz, 1H), 7.53-7.49 (m, 1H), 7.13-7.06 (m, 1H), 6.68-6.63 (m, 1H), 5.74-5.65 (m, 2H), 3.85-3.78 (m, 1H), 3.75 (s, 3H), 3.62-3.52 (m, 1H), 3.40-3.21 (m, 2H), 3.12-3.01 (m, 1H), 2.70 (s, 3H), 2.17-2.10 (m, 1H), 1.90-1.80 (m, 1H), 1.78-1.63 (m, 2H).
MS (ESI+) 502(M⁺ +1, 100%).

### Example 89

2-[(3R)-3-Aminopiperidin-1-yl]-3-(2-chlorobenzyl)-5-methyl-4-oxo-4,5-dihydro-3H-imidazo[4,5-c]quinoline-8-carboxamide trifluoroacetate

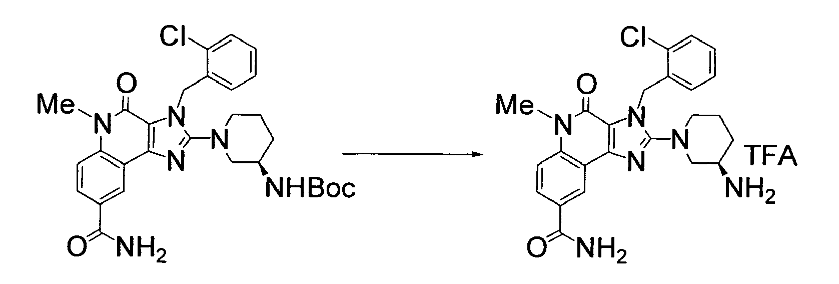

A 4N hydrochloric acid/1,4-dioxane solution (2 ml) was added to tert-butyl {(3R)-1-[8-(aminocarbonyl)-3-(2-chlorobenzyl)-5-methyl-4-oxo-4,5-dihydro-3H-imidazo[4,5-c]quinolin-2-yl]piperidin-3-yl}carbamate (23.9 mg), and the resulting mixture was stirred at 25°C for 2 hours. The solvent was removed by concentration under reduced pressure and the residue was purified by a preparative high-performance liquid chromatography to obtain the title compound (8.2 mg) as trifluoroacetate.
¹H NMR (300 MHz, CD₃OD) δ 8.73 (d, J = 2.2 Hz, 1H), 8.09 (dd, J = 2.2, 9.0 Hz, 1H), 7.66 (d, J = 9.0 Hz, 1H), 7.45 (dd, J = 1.1, 7.9 Hz, 1H), 7.28-7.09 (m, 2H), 6.65 (d, J = 6.4 Hz, 1H), 5.43 (d, J = 17.2 Hz, 1H), 5.35 (d, J = 17.2 Hz, 1H), 3.77-3.72 (m, 1H), 3.70 (s, 3H), 3.64-3.61 (m, 1H), 3.43-3.34 (m, 1H), 3.07-3.04 (m, 1H), 2.97-2.91 (m 1H), 2.09 (m, 1H), 1.85-1.63 (m, 3H).
MS (ESI+) 465 (M⁺+1, 100%).

### Example 90

2-[(3R)-3-Aminopiperidin-1-yl]-3-(2-chlorobenzyl)-5-methyl-5,9-dihydro-3H-furo[3,4-g]imidazo[4,5-c]quinoline-4,7-dione hydrochloride

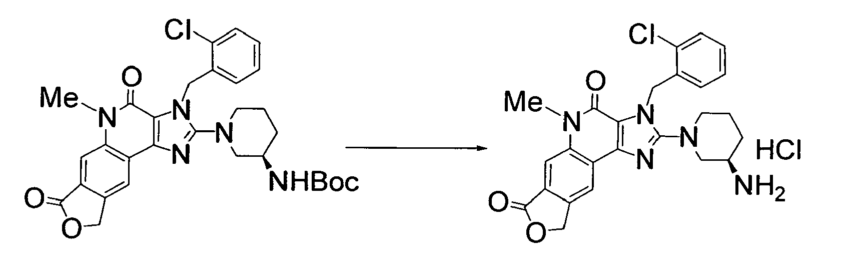

To a solution of tert-butyl {(3R)-1-[3-(2-chlorobenzyl)-5-methyl-4,7-dioxo-4,5,7,9-tetrahydro-3H-furo[3,4-g]imidazo[4,5-c]quinolin-2-yl]piperidin-3-yl}carbamate (3.7 mg) in chloroform (1 mL) was added dropwise 4N hydrochloric acid/1,4-dioxane (1 mL), and the resulting mixture was stirred at room temperature for 4 hours. After the reaction, the solvent was removed under reduced pressure and the resulting solid was washed with acetonitrile and collected by filtration to obtain the title compound (3.8 mg) as a white solid.
MS (ESI+) 496 (M⁺+1, 100%).

### Example 91

(5-Methyl-2-oxo-1,3-dioxol-4-yl)methyl 2-[(3R)-3-aminopiperidin-1-yl]-3-(2-chloro-5-fluorobenzyl)-5-methyl-4-oxo-4,5-dihydro-3H-imidazo[4,5-c]quinoline-7-carboxylate hydrochloride

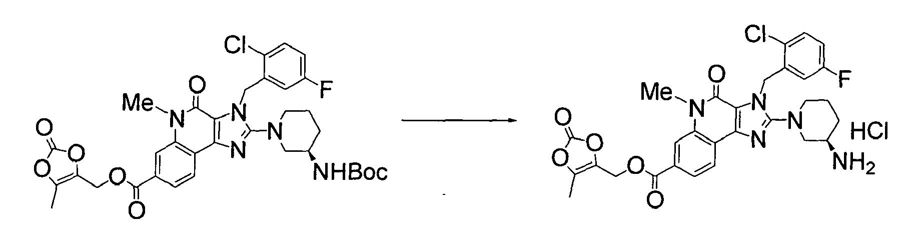

To a solution of (5-methyl-2-oxo-1,3-dioxol-4-yl)methyl 2-[(3R)-3-(tert-butoxycarbonyl)amino]-piperidin-1-yl}-3-(2-chloro-5-fluorobenzyl)-5-methyl-4-oxo-4,5-dihydro-3H-imidazo[4,5-c]quinoline-7-carboxylate (53 mg) in chloroform (2 mL) was added dropwise 4N hydrochloric acid/1,4-dioxane (2 mL), and the resulting mixture was stirred at room temperature for 4 hours. After the reaction, the solvent was removed under reduced pressure and the resulting solid was washed with acetonitrile and collected by filtration to obtain the title compound (24.4 mg) as a light-yellow solid.
¹H NMR (400 MHz, CH₃OD) δ 8.95 (s, 1H), 8.26 (m, 1H), 7.75 (m, 1H), 7.52 (m, 1H), 7.10 (m, 1H), 6.62 (m, 1H), 5.69 (s, 2H), 5.00 (s, 2H), 3.81 (m, 1H), 3.78 (s, 3H), 3.56 (m, 1H), 3.10 (m, 1H), 2.29 (s, 3H), 2.15 (m, 2H), 1.86 (m, 1H), 1.73-1.71 (m, 3H)
MS (ESI+) 596(M⁺ +1, 100%).

### Example 92

2-[(3R)-3-Aminopiperidin-1-yl]-3-(2-chloro-5-fluorobenzyl)-5-methyl-3,5-dihydro-4H-imidazo[4,5-c]-1,6-naphthylidin-4-one trifluoroacetate

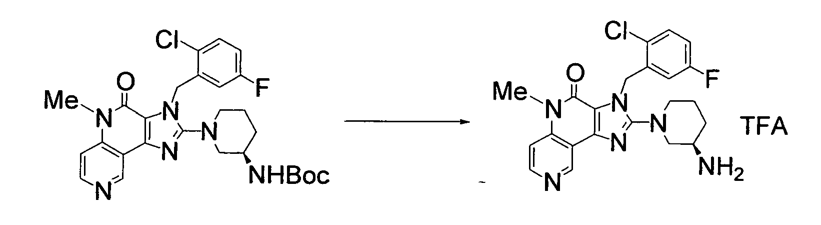

The title compound (1.0 mg) was synthesized by the same process as in Example 89.
¹H NMR (400 MHz, CD₃OD) δ 9.30 (s, 1H), 8.46-8.58 (m, 1H), 7.75-7.66 (m, 1H), 7.46-7.35 (m, 1H), 7.04-6.97 (m, 1H), 6.56-6.48 (m, 1H), 5.65-5.53 (m, 2H), 3.68 (s, 3H), 3.61-2.90 (m, 5H), 2.08-1.95 (m, 1H), 1.85-1.51 (m, 3H).
MS (ESI+) 441(M⁺ +1, 100%).

### Example 93

2-[(3R)-3-Aminopiperidin-1-yl]-3-(2-chlorobenzyl)-6-methoxy-5-methyl-4-oxo-4,5-dihydro-3H-imidazo[4,5-c]quinoline-7-carboxylic acid hydrochloride

2-[(3R)-3-Aminopiperidin-1-yl]-3-(2-chlorobenzyl)-6-hydroxy-5-methyl-4-oxo-4,5-dihydro-3H-imidazo[4,5-c]quinoline-7-carboxylic acid hydrochloride

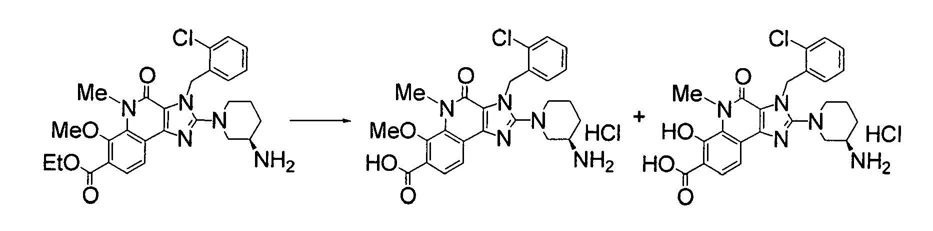

Ethyl 2-[(3R)-3-aminopiperidin-1-yl]-3-(2-chlorobenzyl)-6-methoxy-5-methyl-4-oxo-4,5-dihydro-3H-imidazo[4,5-c]quinoline-7-carboxylate (84.1 mg) was dissolved in 36% hydrochloric acid, and the reaction solution was stirred with heating under reflux for 3 hours. The reaction solution was cooled and then filtered. The white solid thus obtained was dried under reduced pressure to obtain 2-[(3R)-3-aminopiperidin-1-yl]-3-(2-chlorobenzyl)-6-hydroxy-5-methyl-4-oxo-4,5-dihydro-3H-imidazo[4,5-c]quinoline-7-carboxylic acid hydrochloride (40.1 mg). In addition, the filtrate was concentrated to obtain 2-[(3R)-3-aminopiperidin-1-yl]-3-(2-chlorobenzyl)-6-methoxy-5-methyl-4-oxo-4,5-dihydro-3H-imidazo[4,5-c]quinoline-7-carboxylic acid hydrochloride (28.3 mg) as a white solid.

2-[(3R)-3-Aminopiperidin-1-yl]-3-(2-chlorobenzyl)-6-methoxy-5-methyl-4-oxo-4,5-dihydro-3H-imidazo[4,5-c]quinoline-7-carboxylic acid hydrochloride:
¹H NMR (400 MHz, CD₃OD) δ 8.17 (d, J = 8.2 Hz, 1H), 7.74 (d, J = 8.2 Hz, 1H), 7.51 (d, J = 7.9 Hz, 1H), 7.37-7.29 (m, 2H), 7.08 (brs, 1H), 5.77 (brs, 2H), 4.02 (brs, 1H), 3.90 (s, 3H), 3.81 (s, 3H), 3.59-3.40 (m, 3H), 3.22 (brs, 1H), 2.18 (brs, 1H), 1.88 (brs, 1H), 1.74 (brs, 2H).
MS (ESI⁺) 496 (M⁺ + 1, 25%), 372 (59%), 125 (100%).

2-[(3R)-3-Aminopiperidin-1-yl]-3-(2-chlorobenzyl)-6-hydroxy-5-methyl-4-oxo-4,5-dihydro-3H-imidazo[4,5-c]quinoline-7-carboxylic acid hydrochloride:
¹H NMR (400 MHz, CD₃OD) δ 7.88 (d, J = 8.2 Hz, 1H), 7.80 (d, J = 8.2 Hz, 1H), 7.51 (dd, J = 7.9 and 1.3 Hz, 1H), 7.33 (ddd, J = 7.9, 7.9 and 1.3 Hz, 1H), 7.27 (ddd, J = 7.9, 7.9 and 1.3 Hz, 1H), 6.96 (d, J = 7.9, 1H), 5.77 (s, 2H), 4.00 (s, 3H), 3.94 (brs, 1H), 3.56-3.53 (m, 1H), 3.45-3.35 (m, 2H), 3.16-3.11 (m, 1H), 2.16-2.14 (m, 1H), 1.92-1.85 (m, 1H), 1.74-1.68 (m, 2H).
MS (ESI⁺) 482 (M⁺ + 1, 25%), 358 (50%), 340 (32%), 125 (100%).

### Example 94

Methyl 7-[(3R)-3-aminopiperidin-1-yl]-6-(2-chlorobenzyl)-2-(4-methoxybenzyl)-4-methyl-5-oxo-2,4,5,6-tetrahydroimidazo[4,5-d]pyrazolo[4,3-b]pyridine-3-carboxylate hydrochloride

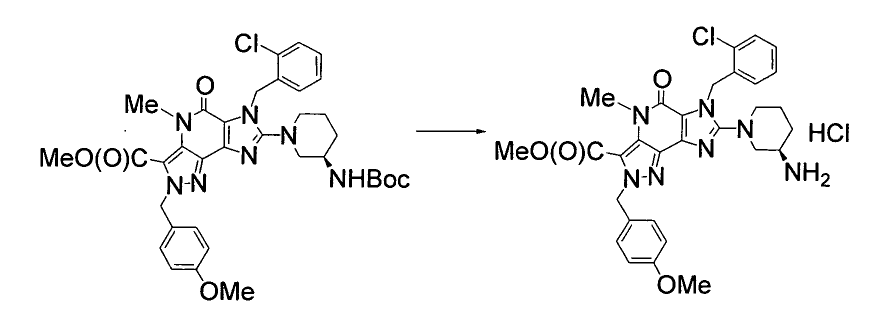

A solution (3 mL) of methyl 7-{(3R)-3-[(tert-butoxycarbonyl)amino]piperidin-1-yl}-6-(2-chlorobenzyl)-2-(4-methoxybenzyl)-4-methyl-5-oxo-2,4,5,6-tetrahydro-imidazo[4,5-d]pyrazolo[4,3-b]pyridine-3-carboxylate (40.0 mg) in 4N hydrochloric acid/1,4-dioxane was allowed to stand for 48 hours. The solvent was removed and the resulting solid was suspended in diethyl ether. The resulting suspension was filtered and the precipitate was dried to obtain the title compound (26.7 mg) as a white solid.
¹H NMR (400 MHz, CD₃OD) δ ppm 7.49 (ddd, J = 7.9, 7.9 and 1.3 Hz, 1H), 7.44 (d, J = 8.6 Hz, 2H), 7.34-7.19 (m, 2H), 6.87 (d, J = 8.6 Hz, 2H), 6.75 (d, J = 7.9 Hz, 1H), 5.85 (s, 2H), 5.81 (s, 2H), 3.98-3.95 (m, 6H), 3.90 (s, 3H), 3.78-3.75 (m, 3H), 3.54-3.46 (m, 1H), 3.20-2.95 (m, 3H), 2.13-2.10 (m, 1H), 1.83 (brs, 1H), 1.72-1.65 (m, 2H).
MS (ESI⁺) 590 (M⁺ + 1, 100%).

### Example 95

Methyl 7-[(3R)-3-aminopiperidin-1-yl]-6-(2-chlorobenzyl)-4-methyl-5-oxo-2,4,5,6-tetrahydroimidazo[4,5-d]pyrazolo[4,3-b]pyridine-3-carboxylate hydrochloride

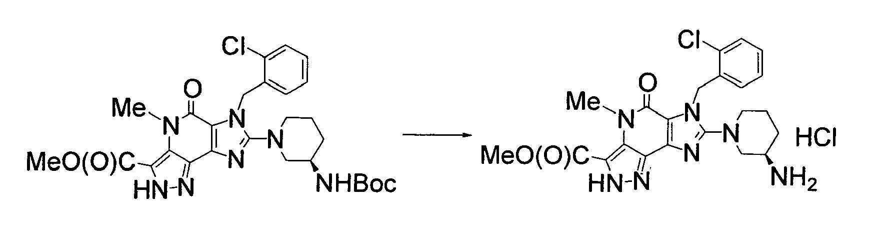

A solution (3 mL) of methyl 7-{(3R)-3-[(tert-butoxycarbonyl)amino]piperidin-1-yl}-6-(2-chlorobenzyl)-4-methyl-5-oxo-2,4,5,6-tetrahydroimidazo[4,5-d]pyrazolo[4,3-b]pyridine-3-carboxylate (94.6 mg) in 4N hydrochloric acid/1,4-dioxane was allowed to stand for 48 hours. The solvent was removed and the resulting solid was suspended in diethyl ether. The resulting suspension was filtered and the precipitate was dried to obtain the title compound (72.4 mg) as a white solid.
¹H NMR (400 MHz, CD₃OD) δ 7.49 (dd, J = 7.9 and 1.3 Hz, 1H), 7.31 (ddd, J = 7.9, 7.9 and 1.3 Hz, 1H), 7.24 (ddd, J = 7.9, 7.9 and 1.3 Hz, 1H), 6.86 (d, J = 7.9 Hz, 1H), 5.74 (s, 2H), 3.98 (s, 3H), 3.87 (s, 3H), 3.85-3.73 (m, 1H), 3.58-3.47 (m, 2H), 3.26-3.23 (m, 1H), 3.08-3.04 (m, 1H), 2.12 (brs, 1H), 1.87-1.83 (m, 1H), 1.74-1.67 (m, 2H).
MS (ESI⁺) 470 (M⁺ + 1, 100%).

### Example 96

2-[(3R)-3-Aminopiperidin-1-yl]-3-(2-chlorobenzyl)-6,8-difluoro-5-methyl-4-oxo-4,5-dihydro-3H-imidazo[4,5-c]quinoline-7-carboxylic acid hydrochloride

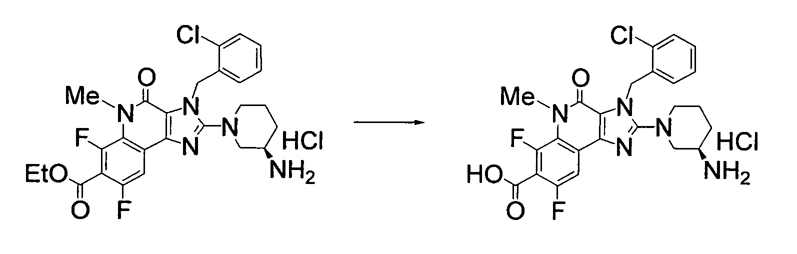

Ethyl 2-[(3R)-3-aminopiperidin-1-yl]-3-(2-chlorobenzyl)-6,8-difluoro-5-methyl-4-oxo-4,5-dihydro-3H-imidazo[4,5-c]quinoline-7-carboxylate hydrochloride (14.7 mg) was dissolved in 36% hydrochloric acid, and the reaction solution was stirred with heating under reflux for 2 hours. The reaction solution was cooled and the solvent was removed under reduced pressure to obtain the title compound (9.7 mg) as a white solid.
¹H NMR (400 MHz, CD₃OD) δ 7.90 (dd, J = 8.8 and 1.6 Hz, 1H), 7.49 (dd, J = 7.9 and 1.3 Hz, 1H), 7.31 (ddd, J = 7.9, 7.9 and 1.3 Hz, 1H), 7.24 (ddd, J = 7.9, 7.9 and 1.3 Hz, 1H), 6.84 (d, J = 7.9 Hz, 1H), 5.74 (s, 2H), 3.89-3.86 (m, 3H), 3.79 (dd, J = 11.8 and 2.5 Hz, 1H), 3.55-3.53 (m, 1H), 3.36-3.30 (m, 1H), 3.26-3.23 (m, 1H), 3.07-3.02 (m, 1H), 2.15-2.10 (m, 1H), 1.82 (s, 1H), 1.70-1.66 (m, 2H).
MS (ESI⁺) 502 (M⁺ + 1, 86%), 378 (68%), 125 (100%).

### Example 97

2-[(3R)-3-aminopiperidin-1-yl]-3-(2-chlorobenzyl)-8-fluoro-5-methyl-4-oxo-4,5-dihydro-3H-imidazo[4,5-c]quinoline-9-carboxylic acid hydrochloride

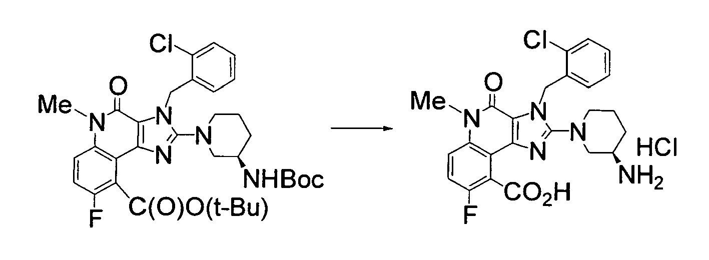

A solution (3 mL) of tert-butyl 2-{(3R)-3-[(tert-butoxycarbonyl)amino]piperidin-1-yl}-3-(2-chlorobenzyl)-8-fluoro-5-methyl-4-oxo-4,5-dihydro-3H-imidazo[4,5-c]quinoline-9-carboxylate (7.7 mg) in 4N hydrochloric acid/1,4-dioxane was allowed to stand for 12 hours. The solvent was removed and the resulting solid was suspended in diethyl ether. The resulting suspension was filtered and the precipitate was dried to obtain the title compound (5.2 mg) as a white solid.
¹H NMR (400 MHz, CD₃OD) δ 7.75-7.71 (m, 1H), 7.49-7.42 (m, 2H), 7.28 (ddd, J = 7.9, 7.9 and 1.3 Hz, 1H), 7.20 (ddd, J = 7.9, 7.9 and 1.3 Hz, 1H), 6.73 (d, J = 7.9 Hz, 1H), 5.77 (d, J = 16.5 Hz, 1H), 5.70 (d, J = 16.5 Hz, 1H), 3.75 (s, 3H), 3.70-3.57 (m, 4H), 3.21-3.09 (m, 2H), 1.82-1.57 (m, 3H).
MS (ESI⁺) 484 (M⁺ + 1, 100%).

### Example 98

2-[(3R)-3-Aminopiperidin-1-yl]-3-(2-chlorobenzyl)-8-fluoro-5-methyl-4-oxo-4,5-dihydro-3H-imidazo[4,5-c]quinoline-7-carboxylic acid hydrochloride

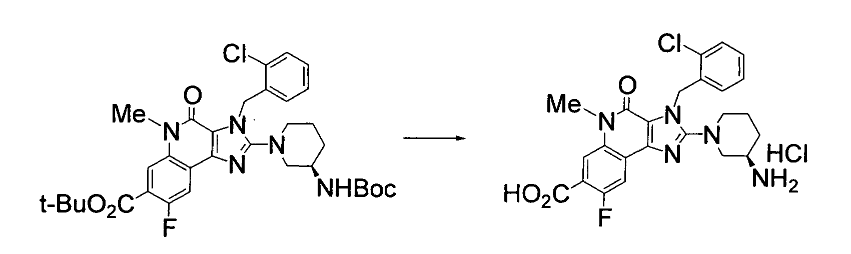

A solution (6 mL) of tert-butyl 2-{(3R)-3-[(tert-butoxycarbonyl)amino]piperidin-1-yl}-3-(2-chlorobenzyl)-8-fluoro-5-methyl-4-oxo-4,5-dihydro-3H-imidazo[4,5-c]quinoline-7-carboxylate (57.2 mg) in 4N hydrochloric acid/1,4-dioxane was allowed to stand for 72 hours. The solvent was removed and the resulting solid was suspended in diethyl ether. The resulting suspension was filtered and the precipitate was dried to obtain the title compound (46.1 mg) as a white solid.
¹H NMR (400 MHz, CD₃OD) δ 8.14 (d, J = 5.9 Hz, 1H), 8.04 (d, J = 10.6 Hz, 1H), 7.50 (dd, J = 7.9 and 1.3 Hz, 1H), 7.32 (ddd, J = 7.9, 7.9 and 1.3 Hz, 1H), 7.24 (ddd, J = 7.9, 7.9 and 1.3 Hz, 1H), 6.86 (d, J = 7.9 Hz, 1H), 5.76 (s, 2H), 3.84 (dd, J = 12.2 and 3.4 Hz, 1H), 3.74 (s, 3H), 3.56-3.53 (m, 1H), 3.39-3.25 (m, 1H), 3.09-3.04 (m, 2H), 2.14 (brs, 1H), 1.86-1.81 (m, 1H), 1.73-1.64 (m, 2H).
MS (ESI⁺) 484 (M⁺ + 1, 100%).

### Example 99

2-[(3R)-3-Aminopiperidin-1-yl]-3-(2-chlorobenzyl)-9-methoxy-5-methyl-4-oxo-4,5-dihydro-3H-imidazo[4,5-c]quinoline-7-carboxylic acid hydrochloride

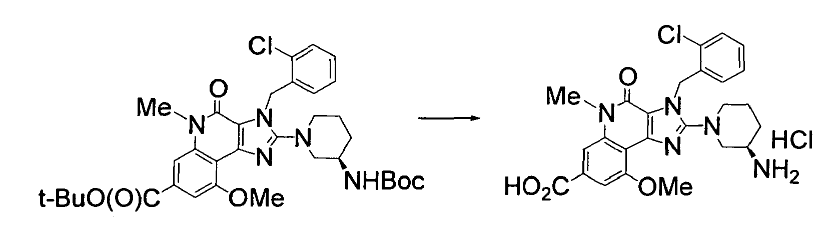

To a solution (6 mL) of tert-butyl 2-{(3R)-3-[(tert-butoxycarbonyl)amino]piperidin-1-yl}-3-(2-chlorobenzyl)-9-methoxy-5-methyl-4-oxo-4,5-dihydro-3H-imidazo[4,5-c]quinoline-7-carboxylate (122.1 mg) in 4N hydrochloric acid/1,4-dioxane was added 36% hydrochloric acid (3 ml), and the mixture was stirred at 90°C for 1 hour. After the reaction solution was cooled, the solvent was removed and the residue was dried under reduced pressure to obtain the title compound (68.2 mg) as a white solid.
¹H NMR (400 MHz, CD₃OD) δ 7.96 (s, 1H), 7.66 (s, 1H), 7.53 (dd, J = 7.9 and 1.3 Hz, 1H), 7.36 (ddd, J = 7.9, 7.9 and 1.3 Hz, 1H), 7.29 (ddd, J = 7.9, 7.9 and 1.3 Hz, 1H), 7.04 (d, J = 7.9 Hz, 1H), 5.91 (d, J = 16.5 Hz, 1H), 5.79 (d, J = 16.5 Hz, 1H), 4.22 (s, 3H), 3.94-3.91 (m, 1H), 3.79 (s, 3H), 3.76-3.73 (m, 1H), 3.68-3.64 (m, 2H), 3.59-3.57 (m, 1H), 2.17 (brs, 1H), 1.90 (brs, 1H), 1.77-1.69 (m, 2H).
MS (ESI⁺) 496 (M⁺ + 1, 38%), 372 (100%).

### Example 100

2-[(3R)-3-Aminopiperidin-1-yl]-3-(5-fluoro-2-methylbenzyl)-5-methyl-4-oxo-4,5-dihydro-3H-imidazo[4,5-c]quinoline-6-carboxylic acid hydrochloride

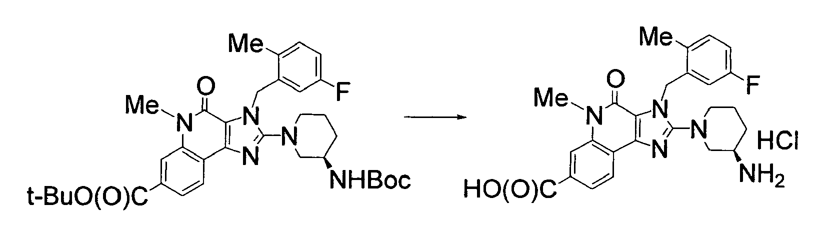

In 4N hydrochloric acid/1,4-dioxane (10 mL) was dissolved tert-butyl 2-{(3R)-3-[(tert-butoxycarbonyl)amino]-piperidin-1-yl}-3-(5-fluoro-2-methylbenzyl)-5-methyl-4-oxo-4,5-dihydro-3H-imidazo[4,5-c]quinoline-7-carboxylate (62 mg), and the resulting solution was stirred with heating at 80°C for 10 hours in a sealed tube. The reaction solution was cooled to 25°C and then concentrated under reduced pressure, and toluene was added thereto, followed by azeotropic distillation, whereby the title compound (37 mg) was obtained as a light-yellow solid.
¹H NMR (300 MHz, DMSO-d₆) δ ppm 8.40 (brs, 3H), 8.26 (d, J = 8.3Hz, 1H), 8.07 (s, 1H), 7.90 (d, J = 8.1Hz, 1H), 7.29-7.23 (m, 1H), 7.00-6.95 (m, 1H), 6.34-6.30 (m, 1H), 5.58 (d, J = 16.8Hz, 1H), 5.50 (d, J = 16.8Hz, 1H), 3.67 (s, 3H), 3.41-3.27 (m, 2H), 3.07-3.05 (m, 1H), 2.93-2.71 (m, 2H), 2.34 (s, 3H), 1.94-1.51 (m, 4H) .
MS (ESI+) 464 (M⁺ +1, 100%).

### Example 101

Methyl 2-[(3R)-3-Aminopiperidin-1-yl]-3-(2-chlorobenzyl)-5-methyl-4-oxo-4,5-dihydro-3H-imidazo[4,5-d]thieno[3,4-b]pyridine-6-carboxylate hydrochloride

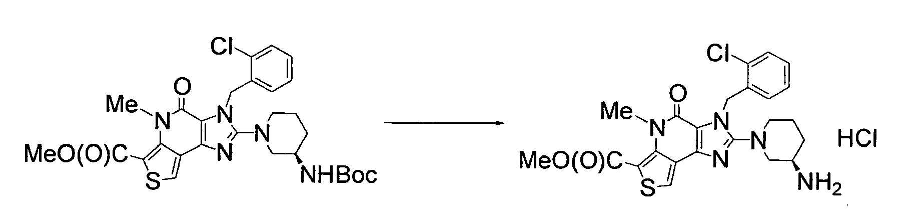

A 4N hydrochloric acid/1,4-dioxane solution (3 mL) was added to methyl 2-{(3R)-3-[(tert-butoxycarbonyl)amino]-piperidin-1-yl}-3-(2-chlorobenzyl)-5-methyl-4-oxo-4,5-dihydro-3H-imidazo[4,5-d]thieno[3,4-b]pyridine-6-carboxylate (112 mg), and the resulting mixture was stirred at 25°C for 20 hours. Toluene was added to the reaction solution and the solvent was removed under reduced pressure to obtain the title compound (112 mg) as a yellow solid.
¹H NMR (300 MHz, DMSO-d₆) δ 8.41 (s, 1H), 7.50 (dd, J = 1.3, 7.9 Hz, 1H), 7.32-7.15 (m, 2H), 6.72 (d, J = 7.7 Hz, 1H), 5.59 (d, J = 17.2 Hz, 1H), 5.53 (d, J = 17.2 Hz, 1H), 3.82 (s, 3H), 3.51 (s, 3H), 3.49-3.42 (m, 1H), 3.30-3.16 (m, 2H), 3.06-3.02 (m, 1H), 2.84-2,78 (m, 1H), 1.93 (m, 1H), 1.74 (m, 1H), 1.60-1.54 (m, 2H).
MS (ESI+) 486 (M⁺+1, 100%).

### Example 102

2-[(3R)-3-Aminopiperidin-1-yl]-3-(2-chlorobenzyl)-5-methyl-4-oxo-4,5-dihydro-3H-imidazo[4,5-d]thieno[3,4-b]pyridine-6-carboxylic acid hydrochloride

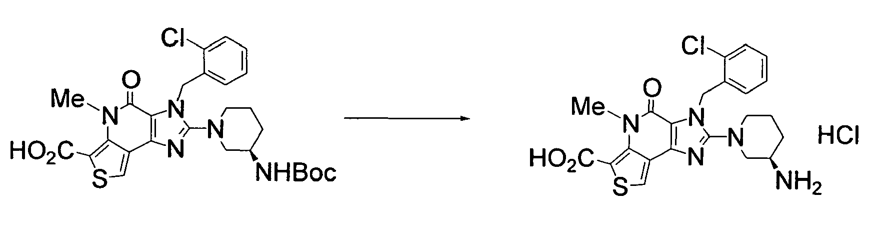

The title compound (110 mg) was synthesized by the same process as in Example 101.
¹H NMR (300 MHz, DMSO-d₆) δ 8.30 (s, 1H), 7.50 (dd, J = 1.3, 7.9 Hz, 1H), 7.32-7.20 (m, 2H), 6.69 (d, J = 7.9 Hz, 1H), 5.56 (brs, 2H), 3.73-3.42 (m, 1H), 3.55 (s, 3H), 3.31 (brs, 1H), 3.20-3.13 (brs, 1H), 3.02 (m, 1H), 2.82 (m, 1H), 1.92-1.90 (m, 1H), 1.77 (m, 1H), 1.54-1.51 (m, 2H).
MS (ESI+) 472 (M⁺+1, 100%).

### Example 103

2-[(3R)-3-Aminopiperidin-1-yl]-3-(2-chloro-5-fluorobenzyl)-5-methyl-5,8-dihydro-3H-furo[3,4-b]imidazo[4,5-d]pyridine-4,6-dione hydrochloride

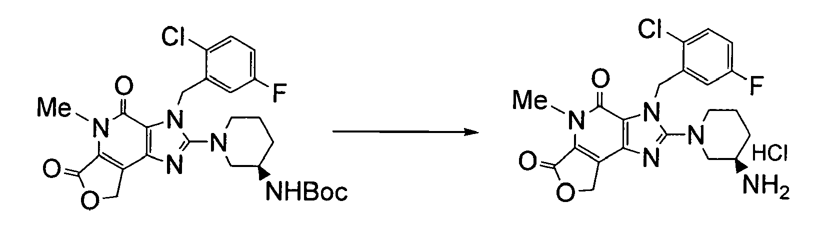

A 4N hydrochloric acid/1,4-dioxane solution (10 mL) was added to tert-butyl {(3R)-1-[3-(2-chloro-5-fluorobenzyl)-5-methyl-4,6-dioxo-4,5,6,8-tetrahydro-3H-furo[3,4-b]imidazo[4,5-d]pyridin-3-yl]carbamate (10 mg), and the resulting mixture was stirred at 25°C for 1 hour. After the reaction solution was concentrated under reduced pressure, toluene was added thereto, followed by azeotropic distillation. Thus, the 1,4-dioxane was completely removed to obtain the title compound.

### Reference Example 1

tert-Butyl {(3R)-1-[3-(2-chloro-5-fluorobenzyl)-5-methyl-4-oxo-4,5-dihydro-3H-imidazo[4,5-c]pyridin-2-yl]piperidin-3-yl}carbamate

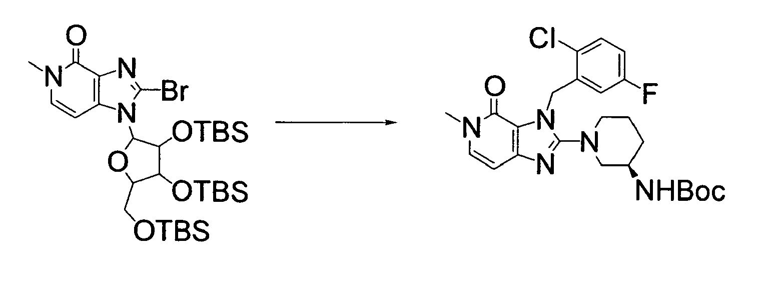

Acetic anhydride (5 mL) and phosphoric acid (0.2 mL) were added to the compound of Reference Example 2 (650 mg), and the resulting mixture was stirred with heating at 80°C for 2 hours. The precipitate formed was collected by filtration, washed with chloroform and then dried in a desiccator to obtain a product (210 mg) as a white solid. This product was dissolved in N,N-dimethylformamide (10 mL), followed by adding thereto 2-chloro-5-fluorobenzyl bromide (150 µL) and potassium carbonate (256 mg), and the resulting mixture was stirred at room temperature for 16 hours. Water was added to the reaction solution, followed by extraction with ethyl acetate. The organic layer was washed with water and a saturated aqueous sodium chloride solution, dried over sodium sulfate and then filtered, and the filtrate was concentrated under reduced pressure. The resulting residue was subjected to isolation and purification by a silica gel column chromatography (developing solvent: ethyl acetate) to obtain a product (72 mg) as a white amorphous substance. This product was dissolved in ethanol (3 ml), followed by adding thereto (R)-tert-3-butylpiperidin-3-ylcarbamate (227 mg), and the resulting mixture was stirred with heating at 100°C for 28 hours in a sealed tube. The reaction solution was cooled to 25°C and then concentrated under reduced pressure, and chloroform was added thereto, followed by washing with a 10% aqueous potassium hydrogensulfate solution. The organic layer was dried over sodium sulfate and filtered, and the filtrate was concentrated under reduced pressure. The residue was purified by a preparative thin-layer silica gel chromatography (developing solvent: chloroform/methanol = 10/1) to obtain the title compound (68 mg) as a white solid.
MS (ESI+) 490 (M⁺+1, 100%).

### Reference Example 2

2-Bromo-5-methyl-1-(2,3,5-tri-O-t-butyldimethyl-silyl-β-D-ribofuranosyl)imidazo[4,5-c]pyridin-4(5H)-one

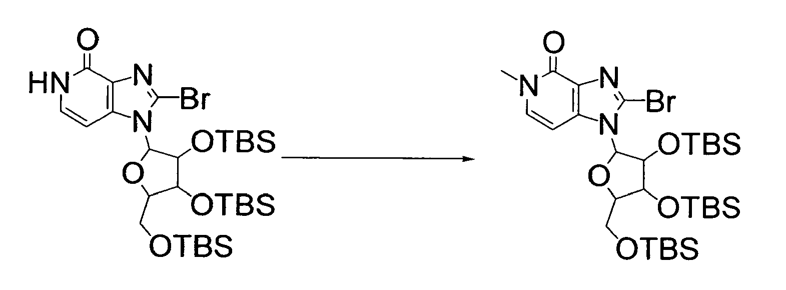

Potassium carbonate (334 mg), 18-crown-6 (43 mg) and methyl iodide (224 µL) were added to a solution of the compound of Reference Example 3 (830 mg) in N,N-dimethylformamide (20 mL), and the resulting mixture was stirred at 25°C for 6 hours. Water was added to the reaction solution, followed by extraction with ethyl acetate. The organic layer was washed with water and a saturated aqueous sodium chloride solution, dried over sodium sulfate and then filtered, and the filtrate was concentrated under reduced pressure. The resulting residue was purified by a silica gel column chromatography (developing solvent: chloroform/methanol = 100/1) to obtain the title compound (650 mg) as a light-yellow solid.
¹H NMR(400MHz, CDCl₃) δ 7.05 (m, 2H), 6.03-6.00 (m, 1H), 4.40-4.33 (m, 1H), 4.28-4.23 (m, 1H), 4.21-4.15 (m, 1H), 3.95-3.90 (m, 1H), 3.87-3.81 (m, 1H), 3.64 (s, 3H), 0.99 (s, 9H), 0.95 (s, 9H), 0.78 (s, 9H), 0.18 (s, 3H), 0.17 (s, 3H), 0.13 (s, 3H), 0.10 (s, 3H),0.00 (s, 3H), -0.15 (s, 3H).
MS (ESI+) 704(M⁺ +3, 100%).

### Reference Example 3

2-Bromo-1-(2,3,5-tri-O-t-butyldimethylsilyl-β-D-ribofuranosyl)imidazo[4,5-c]pyridin-4(5H)-one

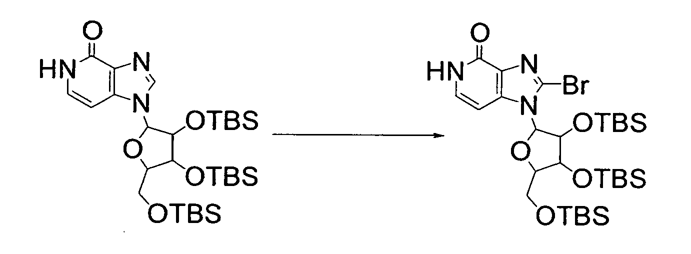

A solution of the compound of Reference Example 4 (1.78 g) in tetrahydrofuran (15 mL) was cooled to 0°C and n-butyllithium (a 1.58M hexane solution, 6.1 mL) was added dropwise thereto. After completion of the dropwise addition, the resulting mixture was stirred at 0°C. After 1.5 hours, 1,2-dibromotetrafluoroethane (1.1 mL) was added dropwise to the reaction solution. After completion of the dropwise addition, the resulting mixture was stirred at room temperature for 2 hours. After the reaction, a saturated aqueous ammonium chloride solution was added to the reaction solution, followed by extraction with chloroform. The organic layer was washed with a saturated aqueous sodium chloride solution and then dried over anhydrous sodium sulfate. The dried organic layer was filtered and the filtrate was concentrated under reduced pressure. The residue was purified by a silica gel column chromatography (developing solvent: chloroform/methanol = 20/1) to obtain the title compound (940 mg) as a light-yellow solid.
¹H NMR(400MHz, CDCl₃) δ 7.23-7.13 (m, 2H), 6.06-6.00 (m, 1H), 4.46-4.39 (m, 1H), 4.22-4.16 (m, 1H), 4.15-4.10 (m, 1H), 3.99-3.92 (m, 1H), 3.90-3.82 (m, 1H), 0.98 (s, 9H), 0.95(s, 9H), 0.78 (s, 9H), 0.18 (s, 3H), 0.17 (s, 3H), 0.13 (s, 3H), 0.11 (s, 3H), 0.00 (s, 3H), -0.15 (s, 3H).
MS (ESI+) 690(M⁺+3, 100%).

### Reference Example 4

1-(2,3,5-Tri-O-t-butyldimethylsilyl-β-D-ribofuranosyl)imidazo[4,5-c]pyridin-4(5R)-one

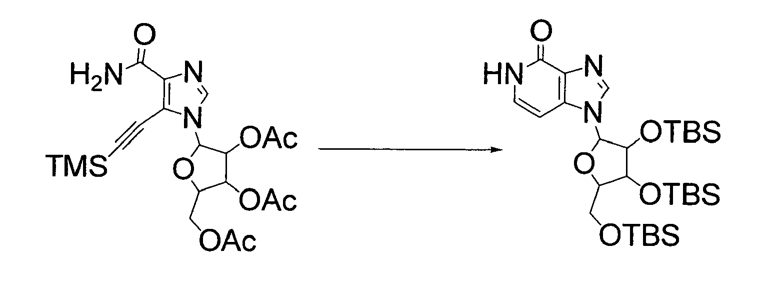

A solution consisting of the compound of Reference Example 5 (1.81 g), dimethylamine (a 40% aqueous solution, 10 mL) and ethanol (20 mL) was stirred at 80°C in an autoclave. After 6 hours, the reaction mixture was concentrated under reduced pressure. To the resulting residue were added ethanol (10 mL) and a 50% aqueous acetic acid solution (10 mL), and the resulting mixture was stirred at 25°C. After 16 hours, the reaction solution was concentrated under reduced pressure. The resulting residue was dissolved in N,N-dimethylformamide (50 mL), followed by adding thereto t-butyldimethylsilyl chloride (3.3 g) and imidazole (3.8 g), and the resulting mixture was stirred at 25°C for 72 hours. Water was added to the reaction mixture, followed by extraction with ethyl acetate. The organic layer was washed with water and then dried over anhydrous sodium sulfate. The dried organic layer was filtered and the filtrate was concentrated under reduced pressure. The residue was purified by a silica gel column chromatography (developing solvent: chloroform/methanol = 20/1) to obtain the title compound (1.79 g) as a white solid.
¹H NMR(400MHz, CDCl₃) δ 8.02 (s, 1H), 7.24-7.20 (m, 1H), 6.81 (d, J = 7.1 Hz, 1H), 5.75-5.71 (m, 1H), 4.35-4.30 (m, 1H), 4.20-4.17 (m, 1H), 4.13-4.11 (m, 1H), 3.96-3.92 (m, 1H), 3.84-3.81 (m, 1H), 0.97 (s, 9H), 0.94 (s, 9H), 0.76 (s, 9H), 0.17 (s, 3H), 0.15 (s, 3H), 0.12 (s, 3H), 0.11 (s, 3H), 0.00 (s, 3H), -0.12 (s, 3H).
MS (ESI+) 610(M⁺ +1, 100%).

### Reference Example 5

5-(Trimethylsilylethyn-1-yl)-1-(2,3,5-tri-O-acetyl-β-D-ribofuranosyl)imidazole-4-carboxamide

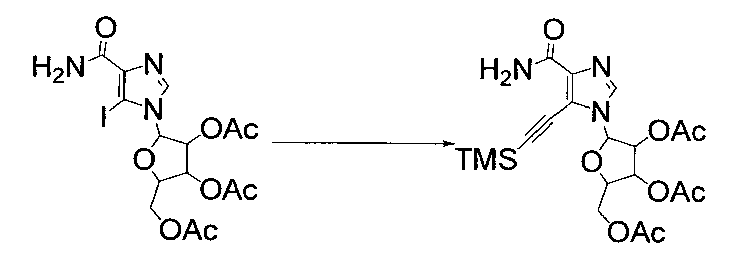

Under a nitrogen atmosphere, trimethyl[(tributyltin)ethynyl]silane (2.9 g) and bis(benzonitrile)palladium(II) chloride (243 mg) were added to a solution of the compound of Reference Example 6 (3.14 g) in acetonitrile (25 mL), and the resulting mixture was stirred at 100°C for 10 hours in an autoclave. After the reaction, the reaction solution was filtered through Celite and washed with ethanol. The filtrate was concentrated under reduced pressure and the resulting residue was purified by a silica gel column chromatography (developing solvent: hexane/ethyl acetate = 1/2 to 0/1) to obtain the title compound (2.17 g) as a brown amorphous substance.
¹H NMR(400MHz, CDCl₃) δ 7.73 (s, 1H), 6.03-6.01 (m, 1H), 5.53-5.50 (m, 1H), 5.41-5.37 (m, 1H), 4.45-4.42 (m, 1H), 4.40-4.37 (m, 2H), 2.17 (s, 3H), 2.12 (s, 3H), 2.11 (s, 3H), 0.30 (s, 9H).
MS (ESI+) 466(M⁺ +1, 100%).

### Reference Example 6

5-Iodo-1-(2,3,5-tri-O-acetyl-β-D-ribofuranosyl)-imidazole-4-carboxamide

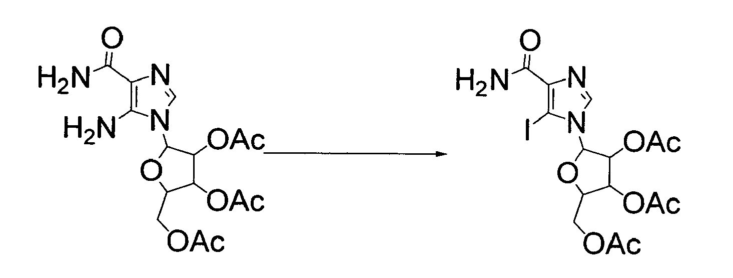

A solution consisting of isopentyl nitrite (3.5 mL) and diiodomethane (25 mL) was heated at 100°C, followed by adding dropwise thereto a solution of 5-amino-1-(2,3,5-tri-O-acetyl-β-D-ribofuranosyl)imidazole-4-carboxamide (2.0 g) in dichloromethane (10 mL), and the resulting mixture was stirred at 100°C for 1.5 hours. After the reaction mixture was allowed to cool, the diiodomethane was removed by a silica gel column chromatography (developing solvent: chloroform/methanol = 100/0 to 100/5), whereby the title compound (1.75 g) was purified and isolated as a light-yellow solid.
¹H NMR(400MHz, CDCl₃) δ 7.97 (s, 1H), 6.02-5.98 (m, 1H), 5.53-5.50 (m, 1H), 5.41-5.33 (m, 1H), 4.46-4.34 (m, 3H), 2.17 (s, 3H), 2.14 (s, 3H), 2.11 (s, 3H).
MS (ESI+) 496(M⁺ +1, 67%).

### Reference Example 7

tert-Butyl {(3R)-1-[3-(2-chlorobenzyl)-5-methyl-4-oxo-4,5-dihydro-3H-imidazo[4,5-c]pyridin-2-yl]piperidin-3-yl}carbamate

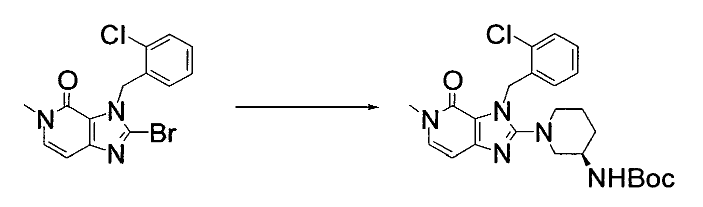

(R)-tert-3-Butylpiperidin-3-yl carbamate (116 mg) was added to a solution of 2-bromo-3-(2-chlorobenzyl)-5-methyl-3,5-dihydro-4H-imidazo[4,5-c]pyridin-4-one (51 mg) in ethanol (3 mL), and the resulting mixture was stirred with heating at 100°C for 28 hours in a sealed tube. The reaction solution was cooled to 25°C and then concentrated under reduced pressure, and chloroform was added thereto, followed by washing with a 10% aqueous potassium hydrogensulfate solution. The organic layer was dried over sodium sulfate and filtered, and the filtrate was concentrated under reduced pressure. The residue was purified by a preparative thin-layer silica gel chromatography (developing solvent: chloroform/methanol = 10/1) to obtain the title compound (49 mg) as a white solid.
¹H NMR (400 MHz, CDCl₃) δ 7.40-7.38 (m, 1H), 7.19-7.12 (m, 2H), 7.09 (d,J = 7.2 Hz, 1H), 6.69-6.67 (m, 1H), 6.58 (d, J = 7.2 Hz, 1H), 5.78 (d, J = 17.0 Hz, 1H), 5.62 (d, J = 17.0 Hz, 1H), 4.94-4.92 (m, 1H), 3.78-3.73 (m, 1H), 3.57 (s, 3H), 3.40-3.37 (m, 1H), 3.03-2.98 (m, 3H), 1.76-1.46 (m, 4H), 1.42 (s, 9H).
MS (ESI+) 472 (M⁺+1, 100%).

### Reference Example 8

2-Bromo-3-(2-chlorobenzyl)-5-methyl-3,5-dihydro-4H-imidazo[4,5-c]pyridin-4-one

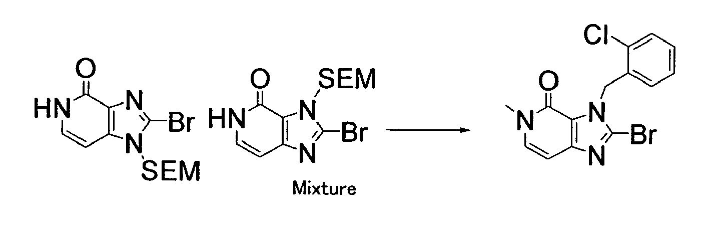

The compound of Reference Example 9 and the compound of Reference Example 10 were mixed and the mixture (171 mg) was dissolved in N,N-dimethylformamide (5 mL). Potassium carbonate (103 mg), 18-crown-6 (15 mg) and methyl iodide (92 µL) were added thereto and the resulting mixture was stirred at room temperature for 2 hours. Water was added thereto, followed by extraction with ethyl acetate. The organic layer was washed with water, dried over sodium sulfate and then filtered, and the filtrate was concentrated under reduced pressure. The resulting residue was dissolved in ethanol (18 mL), followed by adding thereto 4N hydrochloric acid (24 mL), and the resulting mixture was stirred at 80°C for 1.5 hours. After the mixture was allowed to cool, the precipitate formed was collected by filtration, washed with chloroform and then dried under reduced pressure to obtain a crude product (90 mg), 2-bromo-5-methyl-3,5-dihydro-4H-imidazo[4,5-c]pyridin-4-one as a brown solid. The spectrum of this compound is as follows:
¹H NMR (400 MHz, DMSO-d₆) δ 7.51 (d, J = 7.2 Hz, 1H), 6.56 (d, J = 7.2 Hz, 1H), 3.55 (s, 3H).
MS (ESI+) 228 (M⁺+1, 100%).

Subsequently, potassium carbonate (152 mg) and 2-chlorobenzyl bromide (88 µL) were added to a solution of the above-mentioned product in N,N-dimethylformamide (5 mL), and the resulting mixture was stirred at 25°C for 14 hours. Water was added to the reaction solution, followed by extraction with ethyl acetate. The organic layer was washed with water and a saturated aqueous sodium chloride solution, dried over sodium sulfate and then filtered, and the filtrate was concentrated under reduced pressure. The residue was purified by a silica gel column chromatography (developing solvent: ethyl acetate ~ chloroform/methanol = 10/1) to obtain the title compound (51 mg) as a white solid.
¹H NMR (400 MHz, CDCl₃) δ 7.43-7.41 (m, 1H), 7.24-7.13 (m, 2H), 7.13 (d,J = 7.2 Hz, 1H), 6.65 (d, J = 7.2 Hz, 1H), 6.47-6.45 (m, 1H), 5.92 (s, 2H), 3.59 (s, 3H).
MS (ESI+) 352 (M⁺+1, 85%).

### Reference Example 9

2-Bromo-3-{[2-(trimethylsilyl)ethoxy]methyl}-3,5-dihydro-4H-imidazo[4,5-c]pyridin-4-one

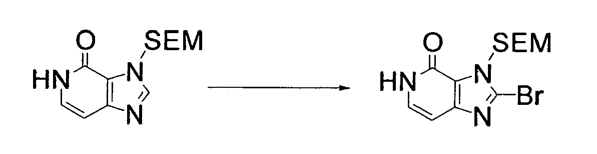

Under a nitrogen atmosphere, a solution of 3-{[2-(trimethylsilyl)ethoxy]methyl}-3,5-dihydro-4H-imidazo[4,5-c]pyridin-4-one (119 mg) in tetrahydrofuran (5 mL) was cooled to 0°C, followed by adding dropwise thereto n-butyllithium (0.8 mL, a 1.58M hexane solution), and the resulting mixture was stirred at 0°C for 1.5 hours. Then, 1,1,2,2-dibromotetrafluoroethane (0.16 mL) was added thereto and the resulting mixture was stirred at 25°C for 5 hours. A saturated aqueous ammonium chloride solution was added to the reaction solution, followed by extraction with chloroform. The organic layer was washed with a saturated aqueous sodium chloride solution, dried over sodium sulfate and then filtered, and the filtrate was concentrated under reduced pressure. The resulting residue was purified by a silica gel column chromatography (developing solvent: chloroform/methanol = 20/1) to obtain the title compound (76 mg) as a light-yellow solid.
¹H NMR (400 MHz, CDCl₃) δ 11.05 (bs, 1H), 7.13 (m, 1H), 6.70 (d, J = 7.0 Hz, 1H), 5.93 (s, 2H), 3.72 (t, J = 8.2 Hz, 2H), 0.93 (t, J = 8.2Hz, 2H), -0.04 (s, 9H).
MS (ESI+) 344 (M⁺+1, 100%).

### Reference Example 10

2-Bromo-1-{[2-(trimethylsilyl)ethoxy]methyl}-1,5-dihydro-4H-imidazo[4,5-c]pyridin-4-one

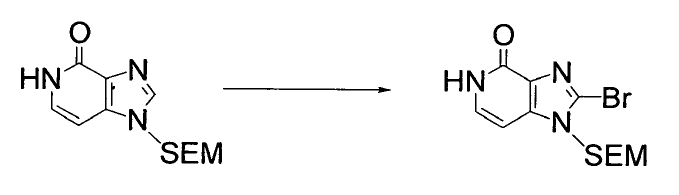

Using the compound of Reference Example 11 as a starting material, the title compound (95 mg) was obtained as a light-yellow solid by the same process as in Reference Example 9.
¹H NMR (400 MHz, CDCl₃) δ 12.63 (bs, 1H), 7.33 (m, 1H), 6.54 (d, J = 7.0 Hz, 1H), 5.48 (s, 2H), 3.57 (t, J = 8.2Hz, 2H), 0.91 (t, J = 8.2 Hz, 2H), -0.04 (s, 9H).
MS (ESI+) 344 (M⁺+1, 100%).

### Reference Example 11

3-{[2-(Trimethylsilyl)ethoxy]methyl}-3,5-dihydro-4H-imidazo[4,5-c]pyridin-4-one

1-{[2-(Trimethylsilyl)ethoxy]methyl}-1,5-dihydro-4H-imidazo[4,5-c]pyridin-4-one

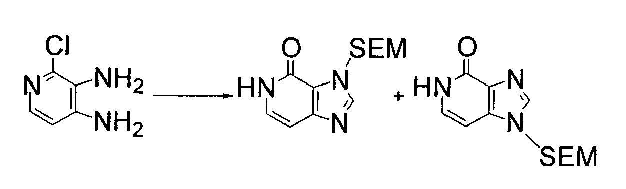

Diethoxymethyl acetate (15 mL) was added to 2-chloropyridine-3,4-diamine (480 mg) and the resulting mixture was stirred at room temperature for 12 hours. To the mixture was added 1N hydrochloric acid and the precipitate formed was filtered, washed with diethyl ether and then dried to obtain a crude product (400 mg), 4-chloro-1H-imidazo[4,5-c]pyridine as a brown solid. The spectrum of this compound is as follows:
¹H NMR (400 MHz, CD₃OD) δ 9.45 (s, 1H), 8.51 (d, J = 5.9 Hz, 1H), 7.94 (d, J = 5.9 Hz, 1H).
MS (ESI+) 154 (M⁺ +1, 100%).

A hydrochloric acid/methanol solution (25 mL, methanol component 80-90%) was added to this solid (220 mg) and the resulting mixture was heated under reflux for 30 hours. The reaction solution was cooled to room temperature and concentrated under reduced pressure, and the resulting residue was washed with diethyl ether and dried to obtain a crude product (150 mg), 3,5-dihydro-4H-imidazo[4,5-c]pyridin-4-one as a brown solid. The spectrum of this compound is as follows:
¹H NMR (400 MHz, CD₃OD) δ 9.40 (s, 1H), 7.58 (d, J = 7.2 Hz, 1H), 6.87 (d, J = 7.2 Hz, 1H).
MS (ESI+) 136 (M⁺+1, 100%).

Under a nitrogen atmosphere, sodium hydride (134 mg, a 60% oil dispersion) was added to N,N-dimethylformamide (15 mL) and the resulting suspension was cooled to -15°C. To the suspension was added 3,5-dihydro-4H-imidazo[4,5-c]pyridin-4-one (360 mg) and the resulting mixture was stirred at room temperature for 30 minutes. Then, chloro-2-(trimethylsilyl)ethoxymethane (0.550 mL) was added dropwise thereto, followed by stirring at room temperature for 20 hours. Water was added to the reaction solution, followed by extraction with chloroform. The organic layer was washed with water and a saturated aqueous sodium chloride solution, dried over sodium sulfate and then filtered, and the filtrate was concentrated under reduced pressure. The resulting residue was purified by a silica gel column chromatography (developing solvent: chloroform/methanol = 20/1 to 10/1) to obtain 3-{[2-(trimethylsilyl)ethoxy]methyl}-3,5-dihydro-4H-imidazo[4,5-c]pyridin-4-one (119 mg) and 1-{[2-(trimethyl-silyl)ethoxy]methyl}-1,5-dihydro-4H-imidazo[4,5-c]pyridin-4-one (113 mg) each as a white solid.

3-{[2-(Trimethylsilyl)ethoxy]methyl}-3,5-dihydro-4H-imidazo[4,5-c]pyridin-4-one
¹H NMR (400 MHz, CDCl₃) δ 11.49 (bs, 1H), 8.03 (s, 1H), 7.16 (m, 1H), 6.77 (d, J = 7.0 Hz, 1H), 5.91 (s, 2H), 3.66 (t, J = 8.2 Hz, 2H), 0.93 (t, J = 8.2 Hz, 2H), - 0.04 (s, 9H).
MS (ESI+) 266 (M⁺+1, 100%).

1-{[2-(Trimethylsilyl)ethoxy]methyl}-1,5-dihydro-4H-imidazo[4,5-c]pyridin-4-one
¹H NMR (400 MHz, CDCl₃) δ 10.97 (bs, 1H), 7.84 (s, 1H), 7.21 (m, 1H), 6.56 (d, J = 7.1 Hz, 1H), 5.45 (s, 2H), 3.51 (t, J = 8.2Hz, 2H), 0.90 (t, J = 8.2 Hz, 2H), - 0.03 (s, 9H).
MS (ESI+) 266 (M⁺+1, 100%).

### Reference Example 12

2-Chloropyridine-3,4-diamine

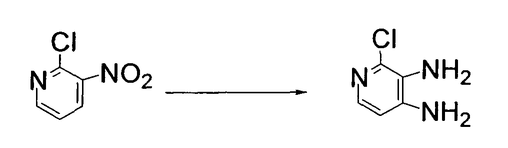

Under a nitrogen atmosphere, potassium t-butoxide (28 g) was added to a solution of zinc(II) chloride (8.6 g) in dimethoxyethane (200 mL) in small portions while cooling the solution in an ice bath. To this solution was added dropwise a solution of 2-chloro-3-nitropyridine (10 g) and O-methylhydroxylamine hydrochloride (7.9 g) in dimethyl sulfoxide (25 ml)/dimethoxyethane (25 ml), and the resulting mixture was stirred at room temperature for 50 hours. A saturated aqueous ammonium chloride solution was added to the reaction solution, followed by extraction with ethyl acetate. The extract solution was washed with water and a saturated aqueous sodium chloride solution, dried over magnesium sulfate and then filtered, and the filtrate was concentrated under reduced pressure. The residue was purified by a silica gel column chromatography (hexane/ethyl acetate = 5/1 to 2/1). A solution of the thus obtained crude product (2.43 g) in methanol (50 mL) was added dropwise to a solution of titanium(III) chloride (65 g, a 20% aqueous solution) in methanol (50 mL), and the resulting mixture was stirred at room temperature for 2 hours. The reaction solution was poured into water and sodium hydrogencarbonate was added thereto until no more carbon dioxide production was observed. The resulting solution was diluted with water and extracted with ethyl acetate. The organic layer was washed with a saturated aqueous sodium chloride solution, dried over magnesium sulfate and then filtered, and the filtrate was concentrated under reduced pressure. The residue was purified by a silica gel column chromatography (developing solvent: chloroform/methanol = 30/1 to 10/1) to obtain the title compound (1.4 g) as a brown solid.
¹H NMR (400 MHz, CD₃OD) δ 7.41 (d, J = 5.4 Hz, 1H), 6.56 (d, J = 5.4 Hz, 1H).
MS (ESI+) 144 (M⁺ +1, 100%).

### Reference Example 13

Methyl 2-{(3R)-3-[(tert-butoxycarbonyl)amino]-piperidin-1-yl}-3-(2-chlorobenzyl)-5-methyl-4-oxo-4,5-dihydro-3H-imidazo[4,5-c]pyridine-6-carboxylate

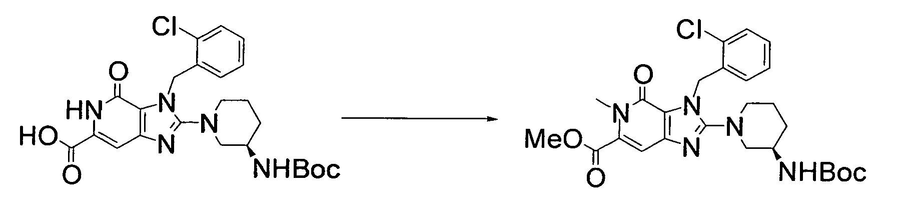

Methyl iodide (1.25 mL) was added to a solution of the compound of Reference Example 17 (2.00 g) and potassium carbonate (1.38 g) in N,N-dimethylformamide (30 mL), and the resulting mixture was stirred at 25°C for 16 hours. Water was added to the reaction mixture, followed by extraction with ethyl acetate. The organic layer was washed with water and a saturated aqueous sodium chloride solution, dried over magnesium sulfate and then filtered, and the filtrate was concentrated under reduced pressure. The residue was purified by a silica gel column chromatography (developing solvent: hexane/ethyl acetate = 1/1) to obtain the title compound (1.3 g) as a white amorphous substance.
¹H NMR (400 MHz, CDCl₃) δ 7.42-7.38 (m, 1H), 7.34 (s, 1H), 7.23-7.12 (m, 2H), 6.70-6.64(m, 1H), 5.79 (d, J = 17.0 Hz, 1H), 5.63 (d, J = 17.0 Hz, 1H), 3.93 (s, 3H), 3.84-3.72 (m, 1H), 3.72 (s, 3H), 3.45-3.38 (m, 1H), 3.09-2.95 (m, 3H), 1.84-1.52 (m, 4H), 1.42 (s, 9H).
MS (ESI+) 530(M⁺ +1, 100%).

### Reference Example 14

3-(Diethylamino)propyl 2-{(3R)-3-[(tert-butoxycarbonyl)amino]piperidin-1-yl}-3-(2-chlorobenzyl)-4-oxo-4,5-dihydro-3H-imidazo[4,5-c]pyridine-6-carboxylate

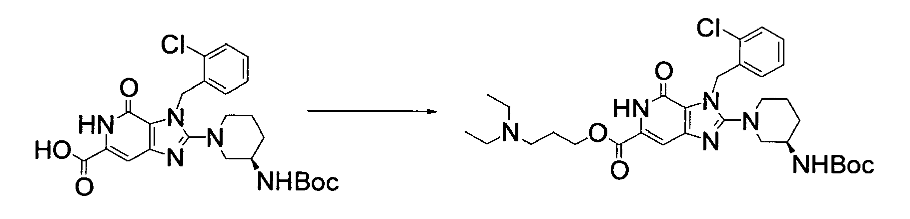

A solution of the compound of Reference Example 17 (100 mg), 3-diethylamino-1-propanol (45 µL), 1-hydroxybenzotriazole (40 mg), 1-ethyl-3-(dimethylaminopropyl)carbo-diimide hydrochloride (50 mg) and triethylamine (84 µL) in N,N-dimethylformamide (2 mL) was stirred at 25°C for 16 hours. Water was added to the reaction mixture, followed by extraction with ethyl acetate. The organic layer was washed with water and a saturated aqueous sodium chloride solution, dried over magnesium sulfate and then filtered, and the filtrate was concentrated under reduced pressure. The residue was purified by a silica gel column chromatography (developing solvent: chloroform/methanol = 20/1) to obtain the title compound (27 mg) as a white amorphous substance.
MS (ESI+) 615 (M⁺+1, 100%).

### Reference Example 15

2-Morpholin-4-yl ethyl 2-{(3R)-3-[(tert-butoxycarbonyl)amino]piperidin-1-yl}-3-(2-chlorobenzyl)-4-oxo-4,5-dihydro-3H-imidazo[4,5-c]pyridine-6-carboxylate

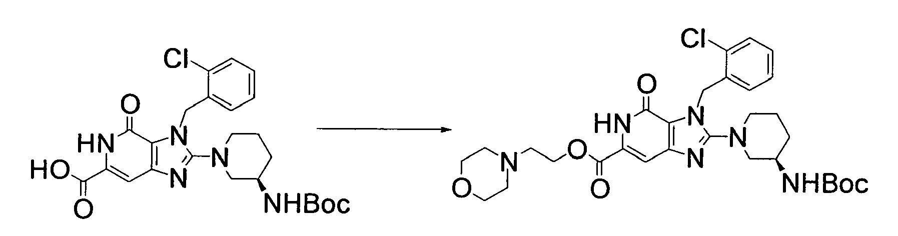

A solution of the compound of Reference Example 17 (100 mg), N-(2-hydroxyethyl)morpholine (36 µL), 1-hydroxybenzotriazole (40 mg), 1-ethyl-3-(dimethylaminopropyl)carbodiimide hydrochloride (50 mg) and triethylamine (84 µL) in dimethylformamide (2 mL) was stirred at 25°C for 16 hours. Water was added to the reaction mixture, followed by extraction with ethyl acetate. The organic layer was washed with water and a saturated aqueous sodium chloride solution, dried over magnesium sulfate and then filtered, and the filtrate was concentrated under reduced pressure. The residue was purified by a silica gel column chromatography (developing solvent: chloroform/methanol = 20/1) to obtain the title compound (35 mg) as a white amorphous substance.
MS (ESI+) 615 (M⁺+1, 100%).

### Reference Example 16

Methyl 2-{(3R)-3-[(tert-butoxycarbonyl)amino]-piperidin-1-yl}-3-(2-chlorobenzyl)-4-oxo-4,5-dihydro-3H-imidazo[4,5-c]pyridine-6-carboxylate

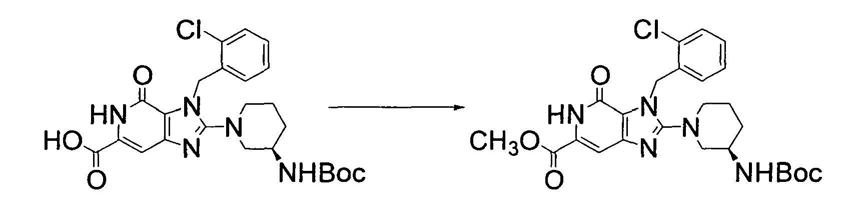

To a solution of the compound of Reference Example 17 (1.4 g) in methanol (25 mL) were added 1-hydroxybenzotriazole (555 mg) and 1-ethyl-3-(dimethylaminopropyl)carbodiimide hydrochloride (695 mg), and the resulting mixture was stirred at 25°C for 14 hours. Water was added to the reaction mixture, followed by extraction with ethyl acetate. The organic layer was washed with water and a saturated aqueous sodium chloride solution, dried over magnesium sulfate and then filtered, and the filtrate was concentrated under reduced pressure. The residue was purified by a silica gel column chromatography (developing solvent: hexane/ethyl acetate = 1/1) to obtain the title compound (1.01 g) as a white amorphous substance.
¹H NMR (400 MHz, CDCl₃) δ 7.61 (s, 1H), 7.45-7.38 (m, 1H), 7.26-7.13 (m, 2H), 6.76-6.69(m, 1H), 5.85 (d, J = 17.0 Hz, 1H), 5.68 (d, J = 17.0 Hz, 1H), 3.82-3.70 (m, 1H), 3.49 (s, 3H), 3.49-3.40 (m, 1H), 3.10-2.98 (m, 3H), 1.87-1.53 (m, 4H), 1.43 (s, 9H).
MS (ESI+) 516(M⁺ +1, 31%).

### Reference Example 17

2-{(3R)-3-[(tert-butoxycarbonyl)amino]piperidin-1-yl}-3-(2-chlorobenzyl)-4-oxo-4,5-dihydro-3H-imidazo[4,5-c]pyridine-6-carboxylic acid

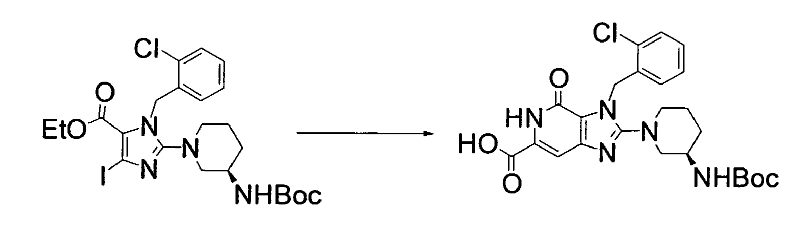

Palladium(II) acetate (974 mg) was added to a solution of the compound of Reference Example 18 (12.7 g), methyl 2-acetamidoacrylate (4.7 g), benzyltriethylammonium chloride (4.9 g) and sodium hydrogencarbonate (3.6 g) in dimethylformamide (65 mL), and the resulting mixture was stirred with heating at 80°C for 4 hours. The reaction mixture was allowed to cool and water was added thereto, followed by extraction with ethyl acetate. The organic layer was washed with a saturated aqueous sodium chloride solution, dried over magnesium sulfate and then filtered, and the filtrate was concentrated under reduced pressure. The residue was purified by a silica gel column chromatography (developing solvent: hexane/ethyl acetate = 3/1 to 0/1) to obtain a product (10.3 g) as a brown amorphous substance [MS (ESI+) 604 (M⁺, 52%)].

A solution consisting of this product (10.3 g), ethanol (30 mL) and sodium ethoxide (a 21% ethanol solution, 29 mL) was stirred with heating at 80°C. After 4 hours, the solution was cooled to 25°C and a 1N aqueous sodium hydroxide solution (15 mL) was added thereto, followed by stirring at 50°C for 1 hour. The reaction mixture was cooled to 25°C, adjusted to pH 7-8 with a saturated aqueous ammonium chloride solution, and then extracted with ethyl acetate. The organic layer was washed with a saturated aqueous sodium chloride solution, dried over anhydrous magnesium sulfate and then filtered, and the filtrate was concentrated under reduced pressure. The residue was recrystallized from ethyl acetate to obtain the title compound (6.87 g) as an orange amorphous substance.
¹H NMR (400 MHz, CDCl₃) δ 7.61 (s, 1H), 7.47-7.40 (m, 1H), 7.29-7.11 (m, 2H), 6.75-6.66(m, 1H), 5.85 (d, J = 17.0 Hz, 1H), 5.68 (d, J = 17.0 Hz, 1H), 3.82-3.70 (m, 1H), 3.50-3.39 (m, 1H), 3.11-2.98 (m, 3H), 1.87-1.53 (m, 4H), 1.43 (s, 9H).
MS (ESI+) 502(M⁺ +1, 38%).

### Reference Example 18

Ethyl 2-{(3R)-3-[(tert-butoxycarbonyl)amino]-piperidin-1-yl)-1-(2-chlorobenzyl)-4-iodo-1H-imidazole-5-carboxylate

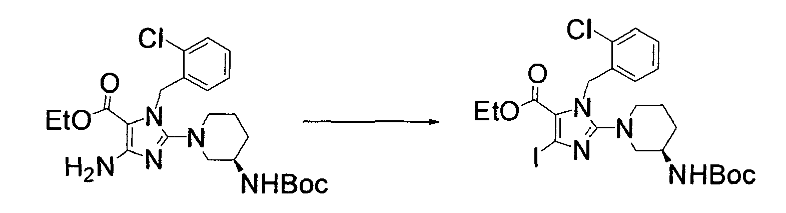

A solution of the compound of Reference Example 19 (20 g), isopentyl nitrite (28 mL) and diiodomethane (33 mL) in toluene (200 mL) was stirred with heating at 80°C for 3 hours.
After the reaction, the reaction mixture was concentrated under reduced pressure and the residue was subjected to isolation and purification by a silica gel column chromatography (developing solvent: hexane/ethyl acetate = 5/1 to 1/1) to obtain the title compound (18 g).
¹H NMR (400 MHz, CDCl₃) δ 7.42-7.35 (m, 1H), 7.23-7.13 (m, 2H), 6.62-6.55 (m, 1H), 5.51-5.37 (m, 2H), 4.18 (q, J = 7.1 Hz, 2H), 3.80-3.69 (m, 1H), 3.32-3.23 (m, 1H), 2.97-2.84 (m, 3H), 1.80-1.45 (m, 4H), 1.42 (s, 9H), 1.18 (t, J = 7.1 Hz, 3H).
MS (ESI+) 589(M⁺ +1, 46%).

### Reference Example 19

Ethyl 4-amino-2-{(3R)-3-[(tert-butoxycarbonyl)-amino]piperidin-1-yl}-1-(2-chlorobenzyl)-1H-imidazole-5-carboxylate

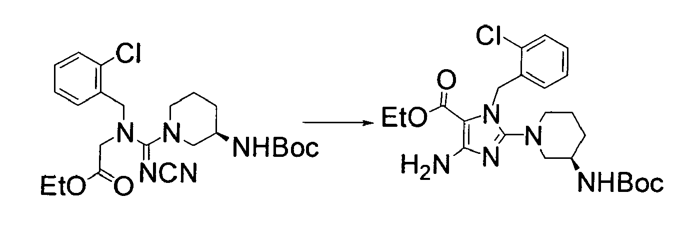

Sodium hydride (60%, 2.01 g) was added to tetrahydrofuran (223 mL) at room temperature and stirred for 30 minutes. A solution (100 mL) of ethyl N-[(Z)-{(3R)-3-[(tert-butoxycarbonyl)amino]piperidin-1-yl}(cyanoimino)methyl]-N-(2-chlorobenzyl)glycinate (16.0 g) in tetrahydrofuran was added to the reaction solution at 80°C and stirred at room temperature for 2 hours. The reaction solution was cooled to 0°C and water (1.8 mL) was carefully added thereto, followed by adding thereto a saturated aqueous ammonium chloride solution (10 mL). The reaction solution was concentrated under reduced pressure and water and potassium carbonate were added to the residue to obtain an alkaline solution, followed by two runs of extraction with ethyl acetate. The combined organic layer was dried over anhydrous sodium sulfate and filtered, and the filtrate was concentrated under reduced pressure to obtain the title compound as a crude product (16.7 g).
¹H NMR (400 MHz, CDCl₃) δ 7.39 (dd, J = 1.6, 7.7Hz, 1H), 7.23-7.18 (m, 2H), 6.81-6.76 (m, 1H), 5.31 (s, 2H), 5.23-5.03 (m, 1H), 4.12 (q, J = 7.1 Hz, 2H), 3.82-3.77 (m, 1H), 3.38-3.33 (m, 1H), 3.05-3.00 (m, 3H), 1.80-1.75 (m, 2H), 1.62-1.57 (m, 2H), 1.41 (s, 9H), 1.02 (t, J = 7.1 Hz, 3H).
MS (ESI+) 478(M⁺ +1, 100%).

### Reference Example 20

Ethyl N-[(Z)-{(3R)-3-[(tert-butoxycarbonyl)-amino]piperidin-1-yl}(cyanoimino)methyl]-N-(2-chlorobenzyl)glycinate

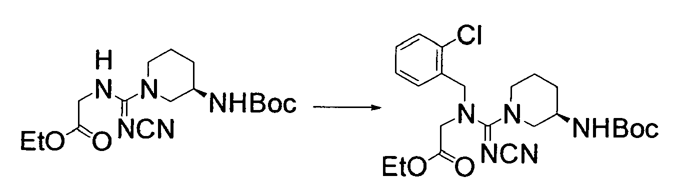

2-Chlorobenzyl bromide (18.3 g) and potassium carbonate (24.6 g) were added to a solution (113 mL) of ethyl N-[(E)-{(3R)-3-[(tert-butoxycarbonyl)amino]piperidin-1-yl}(cyanoimino)methyl]glycinate (21.0 g) in acetonitrile at room temperature, and the resulting mixture was stirred at 70°C for 2 hours. The reaction mixture was allowed to cool and then filtered, and the filtrate was concentrated under reduced pressure. The resulting residue was purified by a silica gel column chromatography (developing solvent: hexane/ethyl acetate = 2/1 to 2/3) to obtain the title compound (16.3 g).
¹H NMR (400 MHz, CDCl₃) δ 7.45-7.40 (m, 1H), 7.34-7.29 (m 3H), 4.63-4.58 (m, 2H), 4.22 (q, J = 7.1 Hz, 2H), 4.03-3.98 (m, 2H), 3.76-3.71 (m, 2H), 3.54-3.25 (m, 4H), 1.95-1.90 (m, 2H), 1.71-1.59 (m, 2H), 1.44 (s, 9H), 1.29 (t, J = 7.1 Hz, 3H).
MS (ESI+) 478 (M⁺+1, 82%).

### Reference Example 21

Ethyl N-[(E)-{(3R)-3-[(tert-butoxycarbonyl)-amino]piperidin-1-yl}(cyanoimino)methyl]glycinate

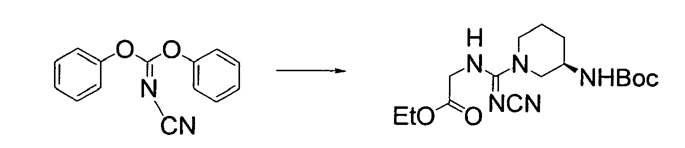

(R)-tert-3-Butyl piperidin-3-ylcarbamate (73.0 g) was added to a suspension (1.46 L) of diphenyl cyanoimidocarbonate (86.8 g) in 2-propanol at room temperature, and the reaction mixture was stirred at room temperature for 30 minutes. The reaction mixture was heated to 50°C, followed by adding thereto glycine ethyl ester hydrochloride (254 g) and triethylamine (254 mL), and the reaction mixture was further heated and then stirred at 80°C for 6 hours. The reaction mixture was allowed to cool to room temperature and the precipitate was collected by filtration and washed with ethyl acetate. The filtrate was concentrated under reduced pressure and water and potassium carbonate were added to the residue to obtain an alkaline solution, followed by two runs of extraction with chloroform. The combined organic layer was dried over anhydrous sodium sulfate and filtered, and the filtrate was concentrated under reduced pressure. The resulting residue was purified by a silica gel column chromatography (developing solvent: hexane/ethyl acetate = 1/1 to 0/1) to obtain the title compound (133 g) as an amorphous substance.
¹H NMR (400 MHz, CDCl₃) δ 5.61 (brs, 1H), 4.66 (brs, 1H), 4.24 (q, J = 7.1 Hz, 2H), 4.25-4.20 (m, 1H), 3.78-3.37 (m, 5H), 1.98-1.93 (m, 1H), 1.85-1.80 (m, 1H), 1.71-1.66 (m, 2H), 1.45 (s, 9H), 1.30 (t, J = 7.1 Hz, 3H).
MS (ESI+) 354(M⁺ +1, 20%).

### Reference Example 22

tert-Butyl {(3R)-1-[3-(2-chlorobenzyl)-4-oxo-5-(2-oxo-2-phenylethyl)-4,5-dihydro-3H-imidazo[4,5-c]pyridin-2-yl]piperidin-3-yl}carbamate

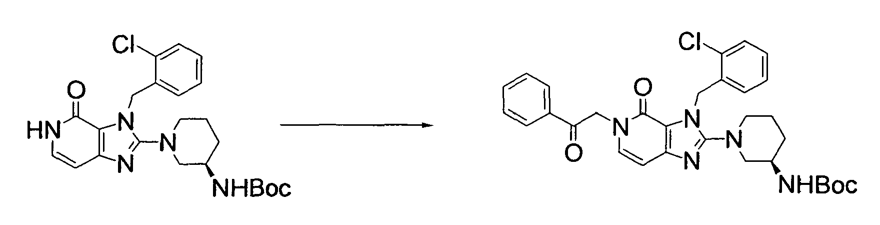

A solution of Reference Example 29 (53 mg), phenacyl bromide (26 mg) and potassium carbonate (50 mg) in N,N-dimethylformamide (1.5 mL) was stirred at room temperature for 6 hours. After the reaction, water was added to the reaction mixture, followed by extraction with ethyl acetate. The organic layer was washed with water and a saturated aqueous sodium chloride solution, dried over anhydrous sodium sulfate and then filtered, and the filtrate was concentrated under reduced pressure. The residue was purified by a silica gel column chromatography (developing solvent: hexane/ethyl acetate = 1/1 to 0/1) to obtain the title compound (52 mg) as a white amorphous substance.
¹H NMR (400 MHz, CDCl₃) δ 8.05-7.98 (m, 2H), 7.63-7.57 (m, 1H), 7.52-7.43 (m, 2H), 7.40-7.32 (m, 1H), 7.21-7.10 (m, 2H), 7.03 (d, J = 7.2 Hz, 1H), 6.75-6.70 (m, 1H), 6.68 (d,J = 7.2 Hz, 1H), 5.74 (d, J = 17.0 Hz, 1H), 5.61 (d, J = 17.0 Hz, 1H), 5.42 (s, 2H), 3.86-3.71 (m, 1H), 3.41-3.32 (m, 1H), 3.09-2.91 (m, 3H), 1.82-1.53 (m, 4H), 1.43 (s, 9H).
MS (ESI+) 576(M⁺ +1, 100%).

### Reference Example 23

Ethyl [2-{(3R)-3-[(tert-butoxycarbonyl)amino]-piperidin-l-yl}-3-(2-chlorobenzyl)-4-oxo-3,4-dihydro-5H-imidazo[4,5-c]pyridin-5-yl]acetate

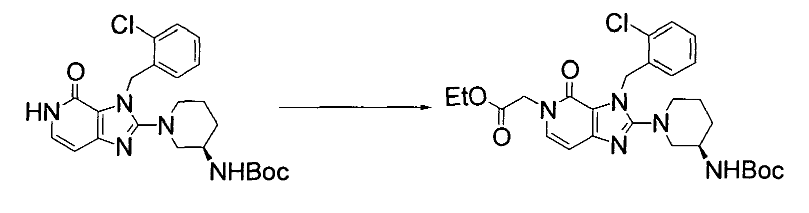

The title compound (121 mg) was synthesized by the same process as in Reference Example 22.
¹H NMR (400 MHz, CDCl₃) δ 7.42-7.35 (m, 1H), 7.22-7.10 (m, 2H), 7.02 (d, J = 7.2 Hz, 1H), 6.71-6.68 (m, 1H), 6.63 (d, J = 7.2 Hz, 1H), 5.75 (d, J = 17.0 Hz, 1H), 5.61 (d, J = 17.0 Hz, 1H), 4.68 (s, 2H), 4.21 (q, J = 7.2 Hz, 2H), 3.86-3.71 (m, 1H), 3.41-3.32 (m, 1H), 3.06-2.94 (m, 3H), 1.80-1.49 (m, 4H), 1.43 (s, 9H), 1.25 (t, J = 7.2 Hz, 3H).
MS (ESI+) 544 (M⁺+1, 100%).

### Reference Example 24

tert-Butyl ((3R)-1-{3-(2-chlorobenzyl)-5-[2-(2-methoxyphenyl)-2-oxoethyl]-4-oxo-4,5-dihydro-3H-imidazo[4,5-c]pyridin-2-yl}piperidin-3-yl)carbamate

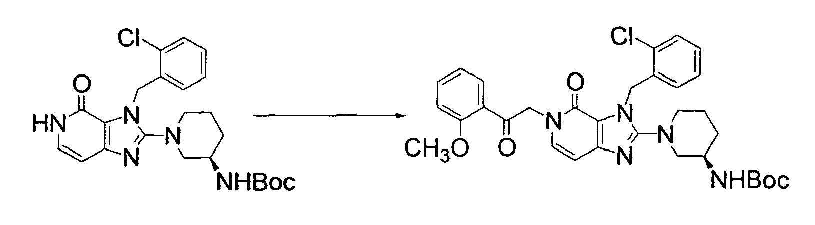

The title compound (86 mg) was synthesized by the same process as in Reference Example 22.
¹H NMR (400 MHz, CDCl₃) δ 7.95-7.89 (m, 1H), 7.66-7.57 (m, 1H), 7.40-7.32 (m, 1H), 7.20-7.11 (m, 2H), 7.05-6.95 (m, 3H), 6.74-6.69 (m, 1H), 6.66 (d, J = 7.2 Hz, 1H), 5.75 (d,J = 17.0 Hz, 1H), 5.61 (d, J = 17.0 Hz, 1H), 5.34 (s, 2H), 3.96 (s, 3H), 3.85-3.73 (m, 1H), 3.41-3.32 (m, 1H), 3.08-2.95 (m, 3H), 1.83-1.55 (m, 4H), 1.43 (s, 9H).
MS (ESI+) 606 (M⁺+1, 100%).

### Reference Example 25

tert-Butyl ((3R)-1-{3-(2-chlorobenzyl)-5-[2-(3-methoxyphenyl)-2-oxoethyl]-4-oxo-4,5-dihydro-3H-imidazo[4,5-c]pyridin-2-yl}piperidin-3-yl)carbamate

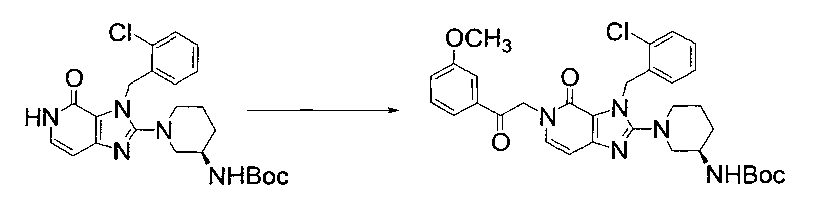

The title compound (71 mg) was synthesized by the same process as in Reference Example 22.
¹H NMR (400 MHz, CDCl₃) δ 7.63-7.53 (m, 1H), 7.53-7.51 (m, 1H), 7.42-7.34 (m, 2H), 7.23-7.10 (m, 3H), 7.02 (d, J = 7.2 Hz, 1H), 6.74-6.69 (m, 1H), 6.68 (d, J = 7.2 Hz, 1H), 5.74 (d, J = 17.0 Hz, 1H), 5.60 (d, J = 17.0 Hz, 1H), 5.40 (s, 2H), 3.83 (s, 3H), 3.83-3.72 (m, 1H), 3.43-3.35 (m, 1H), 3.08-2.93 (m, 3H), 1.75-1.49 (m, 4H), 1.43 (s, 9H).
MS (ESI+) 606(M⁺ +1, 100%).

### Reference Example 26

tert-Butyl {(3R)-1-[3-(2-chlorobenzyl)-5-(1-methyl-2-oxo-2-phenylethyl)-4-oxo-4,5-dihydro-3H-imidazo[4,5-c]pyridin-2-yl]piperidin-3-yl}carbamate

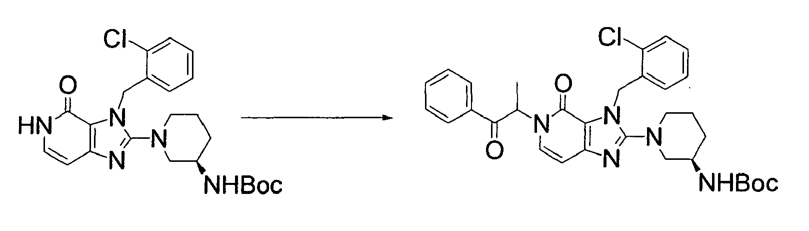

The title compound (78 mg) was synthesized by the same process as in Reference Example 22.
¹H NMR (400 MHz, CDCl₃) δ 7.97-7.92 (m, 2H), 7.56-7.49 (m, 1H), 7.45-7.32 (m, 3H), 7.28-7.18 (m, 1H), 7.17-7.09 (m, 1H), 7.07-7.00 (m, 1H), 6.67-6.58 (m, 2H), 5.85-5.72 (m, 1H), 5.69-5.58 (m, 1H), 4.99-4.88 (m, 1H), 3.82-3.71 (m, 1H), 3.41-3.31 (m, 1H), 3.04-2.93 (m, 3H), 1.79-1.48 (m, 4H), 1.62-1.61 (m, 3H), 1.42 (s, 9H).
MS (ESI+) 590 (M⁺+1, 100%).

### Reference Example 27

tert-Butyl {(3R)-1-[3-(2-chlorobenzyl)-4-oxo-5-(2-phenoxyethyl)-4,5-dihydro-3H-imidazo[4,5-c]pyridin-2-yl]piperidin-3-yl}carbamate

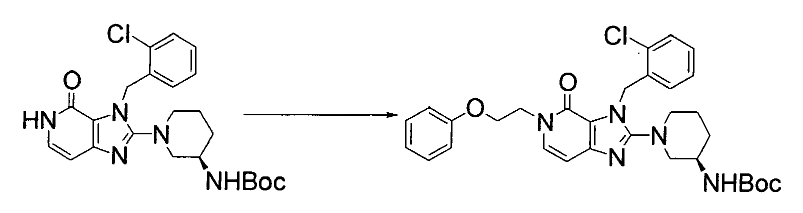

The title compound (47 mg) was synthesized by the same process as in Reference Example 22.
¹H NMR (400 MHz, CDCl₃) δ 7.42-7.35 (m, 1H), 7.31-7.08 (m, 5H), 6.95-6.88 (m, 1H), 6.86-6.78 (m, 2H), 6.70-6.63 (m, 1H), 6.60-6.57 (m, 1H), 5.75 (d, J = 17.0 Hz, 1H), 5.61 (d, J = 17.0 Hz, 1H), 4.37-4.34 (m, 1H), 4.25-4.22 (m, 2H), 3.82-3.71 (m, 2H), 3.41-3.32 (m, 1H), 3.03-2.90 (m, 3H), 1.78-1.49 (m, 4H), 1.42 (s, 9H).
MS (ESI+) 578 (M⁺+1, 100%).

### Reference Example 28

[2-{(3R)-3-[(tert-Butoxycarbonyl)amino]piperidin-1-yl}-3-(2-chlorobenzyl)-4-oxo-3,4-dihydro-5H-imidazo[4,5-c]pyridin-5-yl]acetic acid

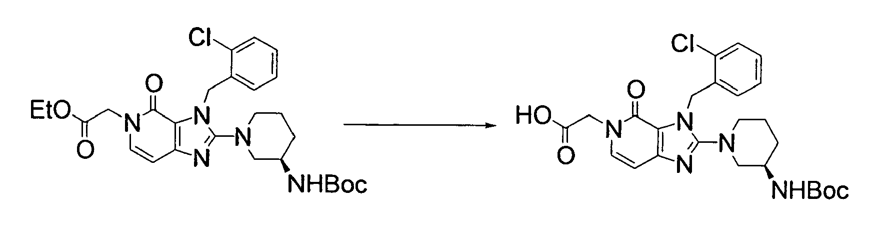

The compound of Reference Example 23 (73 mg) was dissolved in ethanol (2 mL), followed by adding thereto a 1N aqueous sodium hydroxide solution (0.5 mL), and the resulting mixture was stirred at 80°C for 1 hour. The reaction mixture was cooled to 25°C and concentrated under reduced pressure, and a saturated aqueous ammonium chloride solution was added thereto, followed by extraction with chloroform. The organic layer was washed with a saturated aqueous sodium chloride solution, dried over anhydrous sodium sulfate and filtered, and the filtrate was concentrated under reduced pressure to obtain the title compound (32 mg) as a white amorphous substance.
MS (ESI+) 516 (M⁺+1, 100%).

### Reference Example 29

tert-Butyl {(3R)-1-[3-(2-chlorobenzyl)-4-oxo-4,5-dihydro-3H-imidazo[4,5-c]pyridin-2-yl]piperidin-3-yl}carbamate

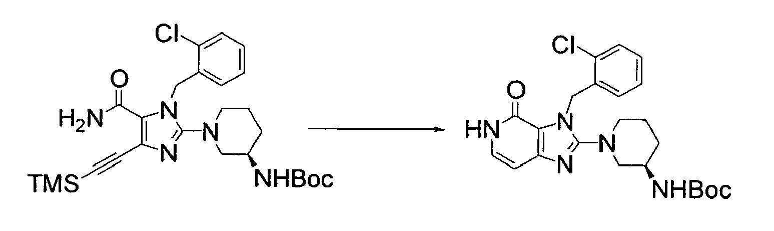

A solution consisting of the compound of Reference Example 30 (1.8 g), dimethylamine (a 40% aqueous solution, 17 mL) and ethanol (25 mL) was stirred at 80°C for 4 hours in an autoclave. The reaction mixture was cooled to 25°C and concentrated under reduced pressure, and the resulting residue was purified by a silica gel column chromatography (developing solvent: ethyl acetate) to obtain the title compound (1.3 g) as a white solid.
¹H NMR (400 MHz, CDCl₃) δ 7.42-7.38 (m, 1H), 7.22-7.10 (m, 2H), 7.01 (d, J = 7.1 Hz, 1H), 6.73-6.68 (m, 1H), 6.61 (d, J = 7.1 Hz, 1H), 5.76 (d, J = 17.0 Hz, 1H), 5.62 (d, J = 17.0 Hz, 1H), 3.83-3.72 (m, 1H), 3.43-3.35 (m, 1H), 3.08-2.94 (m, 3H), 1.82-1.49 (m, 4H), 1.4 (s, 9H).
MS (ESI+) 458(M⁺ +1, 100%).

### Reference Example 30

tert-Butyl ((3R)-1-(5-(aminocarbonyl)-1-(2-chlorobenzyl)-4-[(trimethylsilyl)ethynyl]-1H-imidazol-2-yl}piperidin-3-yl)carbamate

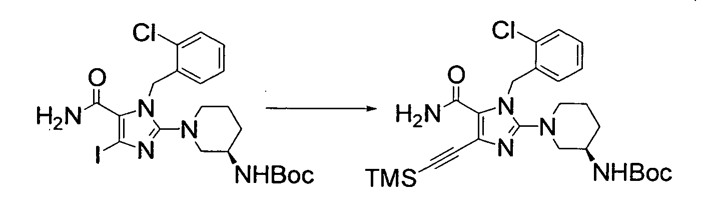

Under a nitrogen atmosphere, bis(benzonitrile)-palladium(II) chloride (38 mg) was added to a solution (3 mL) of the compound of Reference Example 31 (368 mg) and trimethyl[(tributyltin)ethynyl]silane (382 mg) in acetonitrile, and the resulting mixture was stirred at 80°C for 3 hours. The reaction mixture was cooled to 25°C and filtered through Celite and the filtrate was subjected to isolation and purification by a silica gel column chromatography (developing solvent: hexane/ethyl acetate = 3/1 to 1/1) to obtain the title compound (257 mg) as a white solid.
¹H NMR (400 MHz, CDCl₃) δ 7.40-7.34 (m, 1H), 7.23-7.12 (m, 2H), 6.60-6.54 (m, 1H), 5.65(d, J = 17.0 Hz, 1H), 5.55 (d, J = 17.0 Hz, 1H), 3.81-3.70 (m, 1H), 3.40-3.32 (m, 1H), 2.91-2.78 (m, 3H), 1.79-1.47 (m, 4H), 1.42 (s, 9H), 0.27 (s, 9H).
MS (ESI+) 530 (M⁺+1, 86%).

### Reference Example 31

tert-Butyl {(3R)-1-[5-(aminocarbonyl)-1-(2-chlorobenzyl)-4-iodo-1H-imidazol-2-yl]piperidin-3-yl}carbamate

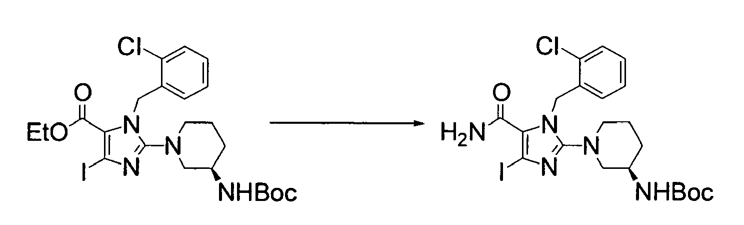

A solution consisting of the compound of Reference Example 18 (7.0 g), 1N sodium hydroxide (20 mL) and ethanol (50 mL) was stirred at 80°C for 1 hour. The reaction mixture was concentrated under reduced pressure and a saturated aqueous ammonium chloride solution was added thereto, followed by extraction with ethyl acetate. The organic layer was washed with a saturated aqueous sodium chloride solution, dried over anhydrous sodium sulfate and then filtered, and the filtrate was concentrated under reduced pressure. The resulting residue was dissolved in N,N-dimethylformamide (100 mL), followed by adding thereto 1-hydroxybenzotriazole (3.1 g), 1-ethyl-3-(dimethylaminopropyl)carbodiimide hydrochloride (3.8 g), triethylamine (8.8 mL) and ammonium chloride (1.2 g), and the resulting mixture was stirred at 25°C for 24 hours. Water was added to the reaction mixture, followed by extraction with ethyl acetate. The organic layer was washed with water and a saturated aqueous sodium chloride solution, dried over anhydrous sodium sulfate and then filtered, and the filtrate was concentrated under reduced pressure to obtain the title compound (6.54 g) as a white amorphous substance.
¹H NMR (400 MHz, CDCl₃) δ 7.40-7.34 (m, 1H), 7.22-7.13 (m, 2H), 6.71-6.65 (m, 1H), 5.58(d, J = 17.0 Hz, 1H), 5.51 (d, J = 17.0 Hz, 1H), 3.80-3.71 (m, 1H), 3.31-3.23 (m, 1H), 2.92-2.81 (m, 3H), 1.81-1.49 (m, 4H), 1.42 (s, 9H).
MS (ESI+) 560(M⁺ +1, 32%).

### Reference Example 32

tert-Butyl {(3R)-1-[6-acetyl-3-(2-chlorobenzyl)-5-methyl-4-oxo-4,5-dihydro-3H-imidazo[4,5-c]pyridin-2-yl]piperidin-3-yl}carbamate

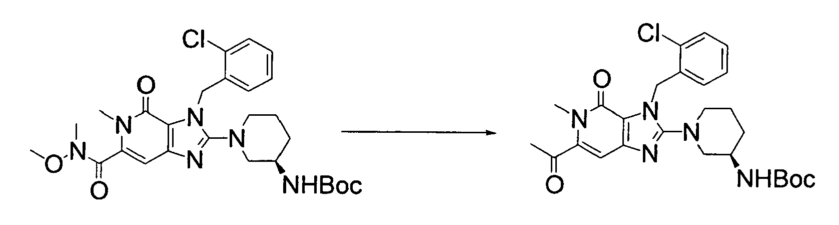

A solution of the compound of Reference Example 46 (90 mg) in tetrahydrofuran (2 mL) was cooled to 0°C, followed by adding dropwise thereto methylmagnesium bromide (0.68 mL), and the resulting mixture was stirred at 0°C. After 1 hour, the mixture was heated to 25°C and stirred for 2 hours. Water was added to the reaction mixture, followed by extraction with ethyl acetate. The organic layer was washed with a saturated aqueous ammonium chloride solution and a saturated aqueous sodium chloride solution, dried over anhydrous sodium sulfate and then filtered, and the filtrate was concentrated under reduced pressure. The resulting residue was purified by a silica gel column chromatography (developing solvent: hexane/ethyl acetate = 1/1) to obtain the title compound (53 mg) as a white amorphous substance.
¹H NMR (400 MHz, CDCl₃) δ 7.42-7.36 (m, 1H), 7.29 (s, 1H), 7.24-7.11 (m, 2H), 6.70-6.62 (m, 1H), 5.80 (d, J = 17.0 Hz, 1H), 5.64 (d, J = 17.0 Hz, 1H), 3.84-3.72 (m, 1H), 3.56 (s, 3H), 3.49-3.39 (m, 1H), 3.08-2.92 (m, 3H), 2.61 (s, 3H), 1.83-1.48 (m, 4H), 1.43 (s, 9H).
MS (ESI+) 514(M⁺ +1, 100%).

### Reference Example 33

tert-Butyl {(3R)-1-[6-benzoyl-3-(2-chlorobenzyl)-5-methyl-4-oxo-4,5-dihydro-3H-imidazo[4,5-c]pyridin-2-yl]piperidin-3-yl}carbamate

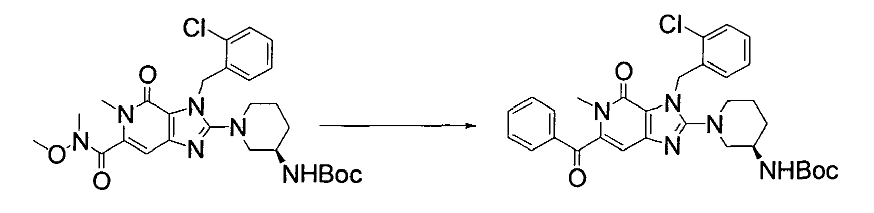

The title compound (53 mg) was synthesized by the same process as in Reference Example 32.
¹H NMR (400 MHz, CDCl₃) δ 7.95-7.90 (m, 2H), 7.70-7.63 (m, 1H), 7.55-7.48 (m, 2H), 7.44-7.38 (m, 1H), 7.26-7.15 (m, 2H), 6.82 (s, 1H), 6.75-6.69 (m, 1H), 5.82 (d, J = 17.0 Hz, 1H), 5.66 (d, J = 17.0 Hz, 1H), 3.82-3.71 (m, 1H), 3.57 (s, 3H), 3.43-3.35 (m, 1H), 3.07-2.94 (m, 3H), 1.81-1.47 (m, 4H), 1.42 (s, 9H).
MS (ESI+) 576 (M⁺+1, 100%).

### Reference Example 34

tert-Butyl ((3R)-1-{3-(2-chlorobenzyl)-6-[(3-methoxyphenyl)acetyl]-5-methyl-4-oxo-4,5-dihydro-3H-imidazo[4,5-c]pyridin-2-yl}piperidin-3-yl)carbamate

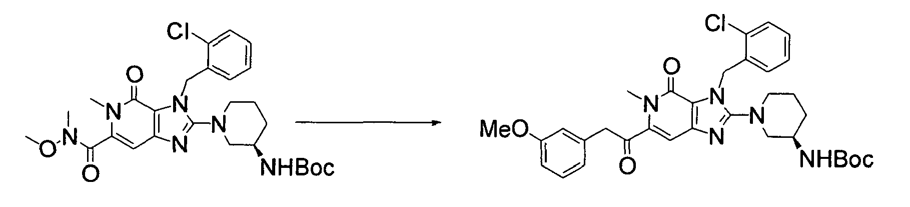

Cerium trichloride hexahydrate (381 mg) was dehydrated and dried at 140°C for 3 hours with a vacuum pump. The dried compound was suspended in tetrahydrofuran at 0°C and 3-methoxybenzylmagnesium bromide (1.0 M, 1.06 mL) was added dropwise thereto. After 30 minutes, a solution of the compound of Reference Example 46 (200 mg) in tetrahydrofuran (2 mL) was added and the resulting mixture was stirred at 0°C. After 1 hour, the mixture was heated to 25°C and stirred for 2 hours. Water was added to the reaction mixture, followed by extraction with ethyl acetate. The organic layer was washed with a saturated aqueous ammonium chloride solution and a saturated aqueous sodium chloride solution, dried over anhydrous sodium sulfate and then filtered, and the filtrate was concentrated under reduced pressure. The resulting residue was purified by a silica gel column chromatography (developing solvent: hexane/ethyl acetate = 1/1) to obtain the title compound (121 mg) as a white amorphous substance.
¹H NMR (400 MHz,CDCl₃) δ 7.41-7.38 (m, 1H), 7.28-7.15 (m, 4H), 6.86-6.82 (m, 2H), 6.81 (s, 1H), 6.68-6.65 (m, 1H), 5.79-5.60 (m, 2H), 4.82-4.80 (m, 1H), 4.15 (s, 2H), 3.81-3.80 (m, 1H), 3.79 (s, 3H), 3.49 (s, 3H), 3.44-3.39 (m, 1H), 3.03-2.96 (m, 3H), 1.80-1.76 (m, 4H), 1.43 (s, 9H).
MS (ESI+) 620 (M⁺+1, 100%).

### Reference Example 35

tert-Butyl {(3R)-1-[3-(2-chlorobenzyl)-5-methyl-4-oxo-6-(phenylacetyl)-4,5-dihydro-3H-imidazo[4,5-c]pyridin-2-yl]piperidin-3-yl}carbamate

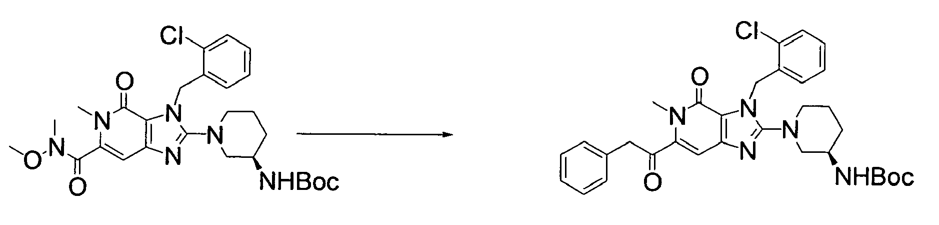

The title compound (54 mg) was synthesized by the same process as in Reference Example 34.
¹H NMR (400 MHz,CDCl₃) δ 7.40-7.27 (m, 7H), 7.15-7.14 (m, 2H), 6.67 (d, J= 7.2 Hz, 1H), 5.80-5.59 (m, 2H), 4.84-4.82 (m, 1H), 4.19 (s, 2H), 3.79-3.75 (m, 1H), 3.48 (s, 3H), 3.44-3.40 (m, 1H), 3.04-2.96 (m, 3H), 1.80-1.75 (m, 4H), 1.43 (s, 9H).
MS (ESI+) 590 (M⁺+1, 100%).

### Reference Example 36

tert-Butyl {(3R)-1-[3-(2-chlorobenzyl)-6-(3-methoxybenzoyl)-5-methyl-4-oxo-4,5-dihydro-3H-imidazo[4,5-c]pyridin-2-yl]piperidin-3-yl}carbamate

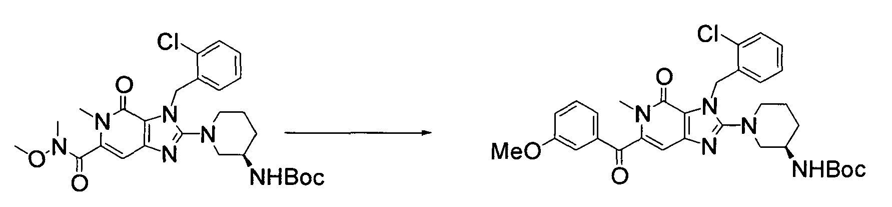

The title compound (91 mg) was synthesized by the same process as in Reference Example 34.
¹H NMR (400 MHz,CDCl₃) δ 7.49-7.39 (m, 4H), 7.26-7.18 (m, 3H), 6.83 (s, 1H), 6.72 (d, J= 7.3 Hz, 1H), 5.84-5.64 (m, 2H), 4.86-4.84 (m, 1H), 3.88 (s, 3H), 3.85-3.82 (m, 1H), 3.56 (s, 3H), 3.42-3.39 (m, 1H), 3.04-2.97 (m, 3H), 1.79-1.74 (m, 4H), 1.42 (s, 9H).
MS (ESI+) 606 (M⁺+1, 100%).

### Reference Example 37

tert-Butyl {(3R)-1-[3-(2-chlorobenzyl)-6-isobutyryl-5-methyl-4-oxo-4,5-dihydro-3H-imidazo[4,5-c]pyridin-2-yl]piperidin-3-yl}carbamate

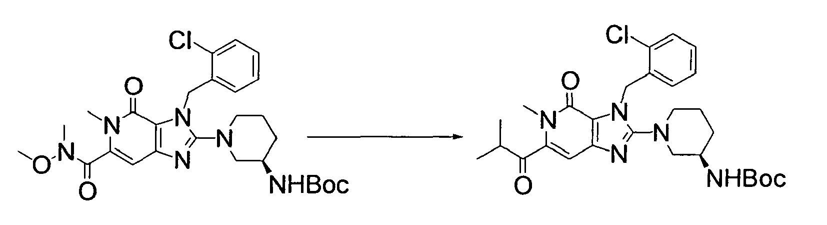

The title compound (10 mg) was synthesized by the same process as in Reference Example 32.
¹H NMR (400 MHz,CDCl₃) δ 7.41-7.39 (m, 1H), 7.21-7.16 (m, 2H), 7.08 (s, 1H), 6.69 (d, J=7.2 Hz, 1H), 5.81-5.61 (m, 2H), 4.82 (d, J=8.2 Hz, 1H), 3.81-3.71 (m, 1H), 3.55 (s, 3H), 3.41 (dd, J= 3.3 Hz, 8.2 Hz, 1H), 3.38-3.31 (m, 1H), 3.04-2.96 (m, 3H), 1.79-1.52 (m, 4H), 1.42 (s, 9H), 1.24-1.22 (m, 6H).
MS (ESI+) 542 (M⁺+1, 100%).

### Reference Example 38

tert-Butyl {(3R)-1-[3-(2-chlorobenzyl)-5-methyl-6-(1,3-oxazol-5-yl)-4-oxo-4,5-dihydro-3H-imidazo[4,5-c]pyridin-2-yl]piperidin-3-yl}carbamate

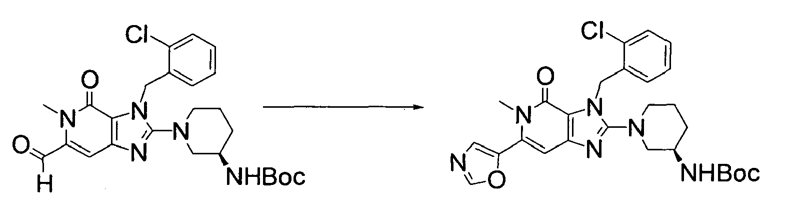

Potassium carbonate (21 mg) and p-toluenesulfonylmethyl isocyanide (30 mg) were added to a solution of the compound of Reference Example 39 (70 mg) in methanol, and the resulting mixture was heated under reflux for 4 hours. The reaction mixture was cooled to 25°C and concentrated under reduced pressure, and water was added thereto, followed by extraction with ethyl acetate. The organic phase was washed with a saturated aqueous sodium chloride solution, dried over sodium sulfate and then filtered, and the filtrate was concentrated under reduced pressure. The resulting residue was purified by a silica gel column chromatography (ethyl acetate) to obtain the title compound (60 mg) as a colorless amorphous substance.
¹H NMR (400 MHz, CDCl₃) δ 8.03 (s, 1H), 7.43-7.36 (m, 1H), 7.25-7.13 (m, 2H), 6.86 (s, 1H), 6.75-6.68 (m, 1H), 5.79 (d, J = 17 Hz, 1H), 5.64 (d, 17 Hz, 1H), 3.85-3.74 (m, 1H), 3.54 (s, 3H), 3.45-3.35 (m, 1H), 3.08-2.95 (m, 3H), 1.83-1.52 (m, 4H), 1.43 (s, 9H).
MS (ESI+) 539(M⁺ +1, 100%).

### Reference Example 39

tert-Butyl {(3R)-1-[3-(2-chlorobenzyl)-6-formyl-5-methyl-4-oxo-4,5-dihydro-3H-imidazo[4,5-c]pyridin-2-yl]piperidin-3-yl}carbamate

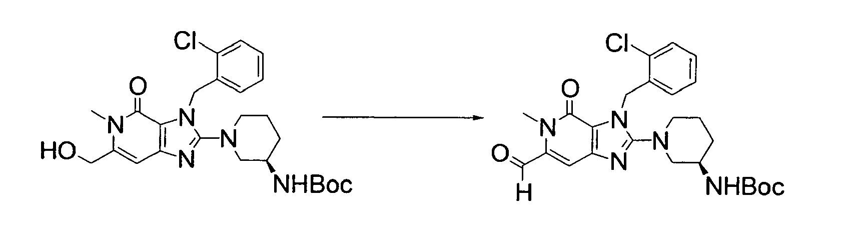

Manganese(IV) oxide (210 mg) was added to a solution of the compound of Reference Example 64 (300 mg) in chloroform, and the resulting mixture was stirred at 50°C for 6 hours and then at room temperature for 16 hours. The reaction mixture was filtered through Celite and the filtrate was concentrated under reduced pressure. The resulting residue was purified by a silica gel column chromatography (hexane/ethyl acetate = 1/1) to obtain the title compound (262 mg) as a white solid.
¹H NMR (400 MHz, CDCl₃) δ 9.55 (s, 1H), 7.45-7.38 (m, 1H), 7.27-7.13 (m, 3H), 6.70-6.63 (m, 1H), 5.82 (d, J = 17 Hz, 1H), 5.65 (d, J = 17 Hz, 1H), 3.88 (s, 3H), 3.82-3.72 (m, 1H), 3.50-3.41 (m, 1H), 3.12-2.94 (m, 3H), 1.86-1.45 (m, 4H), 1.43 (s, 9H).
MS (ESI+) 500(M⁺ +1, 100%).

### Reference Example 40

Methyl 2-{(3R)-3-[(tert-butoxycarbonyl)amino]-piperidin-1-yl}-3-(2-chlorobenzyl)-5,7-dimethyl-4-oxo-4,5-dihydro-3H-imidazo[4,5-c]pyridine-6-carboxylate

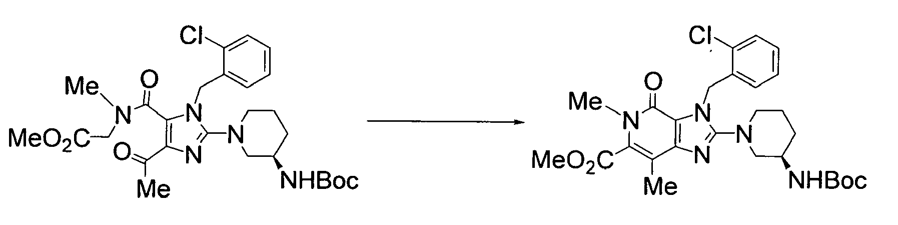

A solution of the compound of Reference Example 41 (1.0 g) and 1,8-diazabicyclo[5,4,0]-7-undecene (532 µL) in toluene (20 mL) was heated under reflux for 10 hours by the use of a Dean-Stark trap to remove water azeotropically. The reaction mixture was cooled to 25°C and concentrated under reduced pressure, and water was added thereto, followed by extraction with ethyl acetate. The organic phase was washed with a saturated aqueous sodium chloride solution, dried over sodium sulfate and then filtered, and the filtrate was concentrated under reduced pressure. The resulting residue was purified by a silica gel chromatography (hexane/ethyl acetate = 1/1) to obtain the title compound (310 mg) as a colorless amorphous substance.
¹H NMR (400 MHz, CDCl₃) δ 7.40-7.37 (m, 1H), 7.21-7.10 (m, 2H), 6.68-6.60 (m, 1H), 5.73 (d, J = 17 Hz, 1H), 5.60 (d, J = 17 Hz), 3.98 (s, 3H), 3.81-3.71 (m, 1H), 3.49 (s, 3H), 3.40-3.32 (m, 1H), 3.28-3.19 (m, 1H), 3.10-2.95 (m, 2H), 2.34 (s, 3H), 1.78-1.60 (m, 3H), 1.52-1.43 (m, 1H), 1.42 (s, 9H).
MS (ESI+) 544 (M⁺+1, 100%).

### Reference Example 41

Methyl N-{[4-acetyl-2-{(3R)-3-[(tert-butoxycarbonyl)amino]piperidin-1-yl}-1-(2-chlorobenzyl)-1H-imidazol-5-yl]carbonyl}-N-methylglycinate

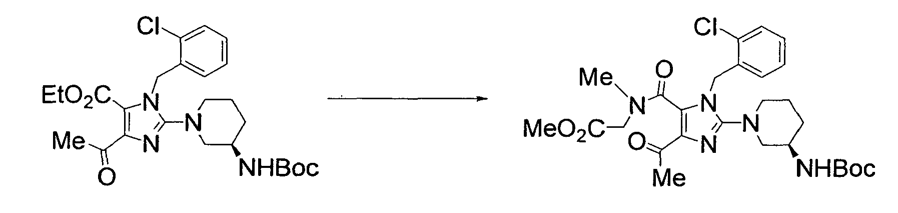

A mixed solution consisting of the compound of Reference Example 42 (2.2 g), a 1N aqueous sodium hydroxide solution (10 mL) and ethanol (20 ml) was stirred at 80°C for 2 hours. After the reaction solution was cooled to 25°C, a saturated aqueous ammonium chloride solution was added thereto, followed by extraction with ethyl acetate. The organic phase was washed with a saturated aqueous sodium chloride solution, dried over sodium sulfate and then filtered, and the filtrate was concentrated under reduced pressure. The resulting residue was dissolved in N,N-dimethylform-amide (45 mL), followed by adding thereto 1-hydroxybenzotriazole (1.1 g), 1-ethyl-3-(dimethylaminopropyl)carbodiimide hydrochloride (1.3 g), triethylamine (3.2 mL) and sarcosine methyl ester hydrochloride (1.0 g), and the resulting mixture was stirred at 25°C for 20 hours. A saturated aqueous ammonium chloride solution was added to the reaction mixture, followed by extraction with ethyl acetate. The organic phase was washed with water and a saturated aqueous sodium chloride solution, dried over sodium sulfate and then filtered, and the filtrate was concentrated under reduced pressure. The resulting residue was purified by a silica gel chromatography (hexane/ethyl acetate = 1/1→1/3) to obtain the title compound (1.0 g) as a colorless amorphous substance.
MS (ESI+) 562 (M⁺+1, 60%).

### Reference Example 42

Ethyl 4-acetyl-2-{(3R)-3-[(tert-butoxycarbonyl)-amino]piperidin-1-yl}-1-(2-chlorobenzyl)-1H-imidazole-5-carboxylate

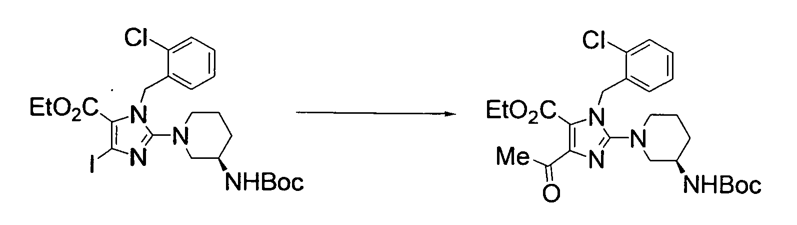

Under a nitrogen atmosphere, tributyl(1-ethoxyvinyl)tin (4.0 mL) and dichlorobis(benzonitrile)-palladium(II) (460 mg) were added to a solution of the compound of Reference Example 18 (4.7 g) in acetonitrile (40 mL), and the resulting mixture was stirred at 80°C for 9 hours. The reaction mixture was cooled to 25°C and filtered through Celite, and the filtrate was concentrated under reduced pressure. The resulting residue was purified by a silica gel chromatography (hexane/ethyl acetate = 2/1→1/1) to obtain a brown amorphous substance (2.5 g). To this compound were added a 5% aqueous potassium hydrogensulfate solution (50 mL) and tetrahydrofuran (50 mL), and the resulting mixture was stirred at 25°C for 110 hours. A saturated aqueous sodium hydrogencarbonate solution was added to the reaction mixture, followed by extraction with ethyl acetate. The organic phase was washed with a saturated aqueous sodium chloride solution, dried over sodium sulfate and then filtered, and the filtrate was concentrated under reduced pressure to obtain the title compound (2.2 g) as a colorless amorphous substance.
¹H NMR (400 MHz, CDCl₃) δ 7.39-7.37 (m, 1H), 7.25-7.16 (m, 2H), 6.72-6.70 (m, 1H), 5.33 (s, 2H), 4.16 (q, J = 7.2 Hz, 2H), 3.80-3.72 (m, 1H), 3.32-3.22 (m, 1H), 3.04-2.87 (m, 3H), 2.59 (s, 3H), 1.80-1.46 (m, 4H), 1.52 (s, 9H), 1.15 (t, J = 7.2 Hz, 3H).
MS (ESI+) 505(M⁺ +1, 29%).

### Reference Example 43

tert-Butyl {(3R)-1-[3-(2-chlorobenzyl)-5,7-dimethyl-4-oxo-4,5-dihydro-3H-imidazo[4,5-c]pyridin-2-yl]piperidin-3-yl}carbamate

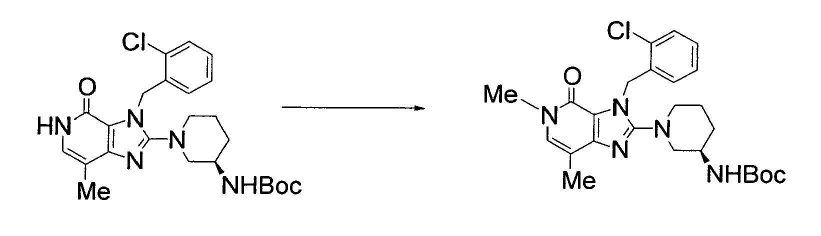

A solution of the compound of Reference Example 44 (28 mg), potassium carbonate (22 mg) and methyl iodide (8 µL) in N,N-dimethylformamide (1 mL) was stirred at room temperature for 12 hours. Water was added to the reaction mixture, followed by extraction with ethyl acetate. The organic phase was washed with water and a saturated aqueous sodium chloride solution, dried over sodium sulfate and then filtered, and the filtrate was concentrated under reduced pressure. The resulting residue was purified by a silica gel column chromatography (ethyl acetate) to obtain the title compound (15 mg) as a light-yellow amorphous substance.
MS (ESI+) 486 (M⁺+1, 100%).

### Reference Example 44

tert-Butyl {(3R)-1-[3-(2-chlorobenzyl)-7-methyl-4-oxo-4,5-dihydro-3H-imidazo[4,5-c]pyridin-2-yl]piperidin-3-yl}carbamate

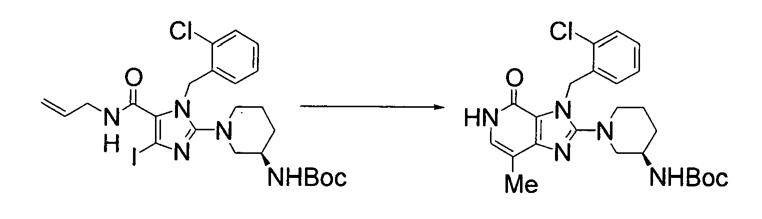

Under a nitrogen atmosphere, palladium(II) acetate (3.8 mg), triphenylphosphine (13 mg), potassium acetate (67 mg) and tetrabutylammonium bromide (55 mg) were added to a solution of the compound of Reference Example 45 (100 mg) in N,N-dimethylformamide (2 mL), and the resulting mixture was stirred at 80°C for 4 hours. After the reaction mixture was cooled to 25°C, water was added thereto, followed by extraction with ethyl acetate. The organic phase was washed with water and a saturated aqueous sodium chloride solution, dried over sodium sulfate and then filtered, and the filtrate was concentrated under reduced pressure. The resulting residue was purified by a silica gel column chromatography (ethyl acetate) to obtain the title compound (40 mg) as a light-yellow amorphous substance.
¹H NMR (400 MHz, CDCl₃) δ 7.42-7.35 (m, 1H), 7.22-7.10 (m, 2H), 6.83-6.75 (s, 1H), 6.73-6.66 (m, 1H), 5.73 (d, J = 17.0 Hz, 1H), 5.62 (d, J = 17.0 Hz, 1H), 3.81-3.71 (m, 1H), 3.41-3.32 (m, 1H), 3.28-3.17 (m, 1H), 3.11-2.97 (m, 2H), 2.30 (s, 3H), 1.77-1.38 (m, 4H), 1.44 (s, 9H).
MS (ESI+) 472(M⁺ +1, 100%).

### Reference Example 45

tert-Butyl {(3R)-1-[5-[(allylamino)carbonyl]-1-(2-chlorobenzyl)-4-iodo-1H-imidazol-2-yl]piperidin-3-yl}carbamate

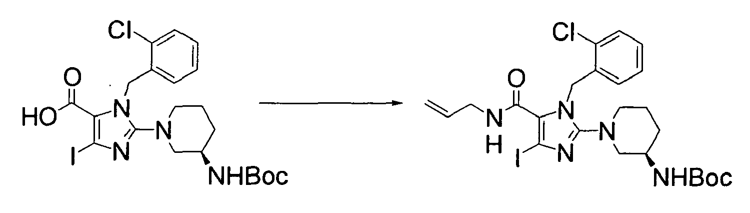

Allylamine (260 µL) was added to a solution of the compound of Reference Example 173 (1.5 g), N,N-bis(2-oxo-3-oxazolidinyl)phosphinyl chloride (1.0 g) and triethylamine (1.3 mL) in dichloromethane (25 mL), and the resulting mixture was stirred at 25°C for 2 hours. Water was added to the reaction mixture, followed by extraction with chloroform. The organic phase was washed with a saturated aqueous sodium chloride solution, dried over sodium sulfate and then filtered, and the filtrate was concentrated under reduced pressure. The resulting residue was purified by a silica gel chromatography (hexane/ethyl acetate = 1/1) to obtain the title compound (1.0 g) as a brown amorphous substance.
¹H NMR (400 MHz, CDCl₃) δ 7.38-7.31 (m, 1H), 7.23-7.13 (m, 2H), 6.79-6.69 (m, 1H), 5.88-5.75 (m, 1H), 5.55-5.40 (m, 2H), 5.21-5.08 (m, 2H), 3.96-3.90 (m, 2H), 3.80-3.68 (m, 1H), 3.40-3.28 (m, 1H), 2.95-2.79 (m, 3H), 1.80-1.45 (m, 4H), 1.42 (s, 9H).
MS (ESI+) 600(M⁺ +1, 70%).

### Reference Example 46

tert-Butyl [(3R)-1-[3-(2-chlorobenzyl)-6-{[methoxy(methyl)amino]carbonyl}-5-methyl-4-oxo-4,5-dihydro-3H-imidazo[4,5-c]pyridin-2-yl]piperidin-3-yl]carbamate

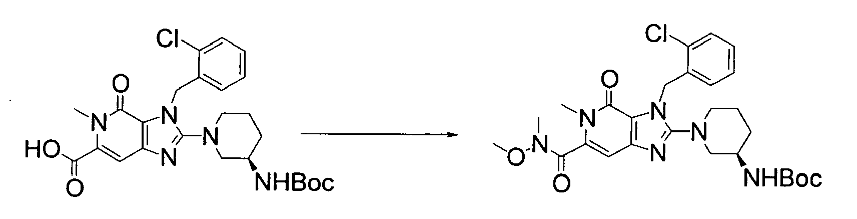

1-Hydroxybenzotriazole (251 mg), 1-ethyl-3-(dimethylaminopropyl)carbodiimide hydrochloride (316 mg), triethylamine (0.73 mL) and N,O-dimethylhydroxylamine hydrochloride (160 mg) were added to a solution of the compound of Reference Example 47 (456 mg) in N,N-dimethylformamide (10 mL), and the resulting mixture was stirred at 25°C for 24 hours. Water was added to the reaction mixture, followed by extraction with ethyl acetate. The organic layer was washed with water and a saturated aqueous sodium chloride solution, dried over anhydrous sodium sulfate and then filtered, and the filtrate was concentrated under reduced pressure. The residue was purified by a silica gel column chromatography (developing solvent: hexane/ethyl acetate = 1/1 to 0/1) to obtain the title compound (197 mg) as a white amorphous substance.
¹H NMR (400 MHz, CDCl₃) δ 7.42-7.36 (m, 1H), 7.24-7.12 (m, 2H), 6.71-6.65 (m, 1H), 6.65 (s, 1H), 5.77 (d, J = 17.0 Hz, 1H), 5.62 (d, J = 17.0 Hz, 1H), 3.85-3.73 (m, 1H), 3.61 (brs, 3H), 3.50 (s, 3H), 3.46-3.38 (m, 1H), 3.36 (s, 3H), 3.07-2.95 (m, 3H), 1.80-1.49 (m, 4H), 1.42 (s, 9H).
MS (ESI+) 559(M⁺ +1, 55%).

### Reference Example 47

2-{(3R) -3- [(tert-Butoxycarbonyl)amino]piperidin-1-yl}-3-(2-chlorobenzyl)-5-methyl-4-oxo-4,5-dihydro-3H-imidazo[4,5-c]pyridine-6-carboxylic acid

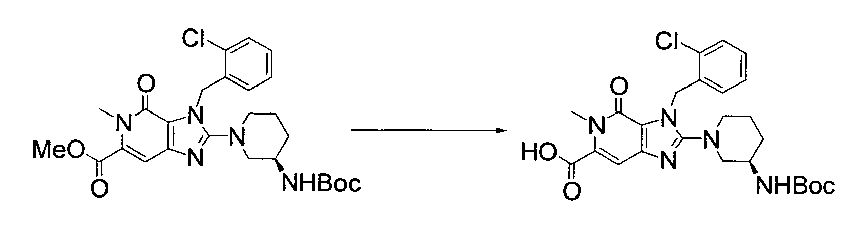

A solution consisting of the compound of Reference Example 13 (970 mg), 1N sodium hydroxide (4 mL) and ethanol (10 mL) was stirred at 80°C for 1 hour. After the reaction mixture was concentrated under reduced pressure, a saturated aqueous ammonium chloride solution was added thereto, followed by extraction with ethyl acetate. The organic layer was washed with a saturated aqueous sodium chloride solution, dried over anhydrous sodium sulfate and then filtered, and the filtrate was concentrated under reduced pressure to obtain the title compound (920 mg) as a white solid.
MS (ESI+) 516 (M⁺+1, 100%).

### Reference Example 48

Ethyl 2-{(3R)-3-[(tert-butoxycarbonyl)amino]-piperidin-1-yl}-3-(2-chlorobenzyl)-4-oxo-4,5-dihydro-3H-imidazo[4,5-c]pyridine-6-carboxylate

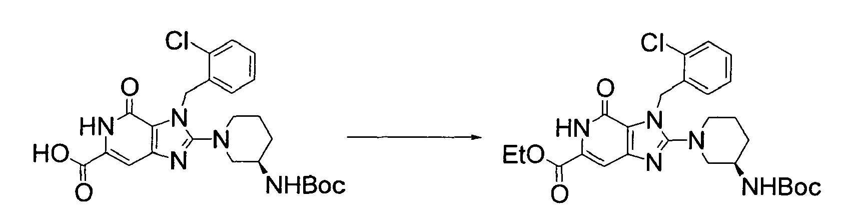

The title compound (17 mg) was synthesized by the same process as in Reference Example 16.
MS (ESI+) 530 (M⁺+1, 27%).

### Reference Example 49

Isopropyl 2-{(3R)-3-[(tert-butoxycarbonyl)amino]-piperidin-1-yl}-3-(2-chlorobenzyl)-4-oxo-4,5-dihydro-3H-imidazo[4,5-c]pyridine-6-carboxylate

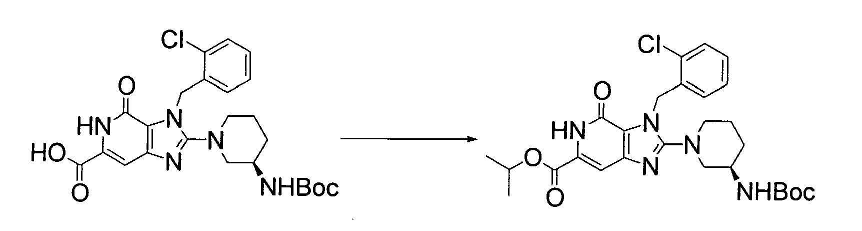

The title compound (50 mg) was synthesized by the same process as in Reference Example 16.
¹H NMR (400 MHz, CDCl₃) δ 7.44 (s, 1H), 7.42-7.38 (m, 1H), 7.24-7.12 (m, 2H), 6.73-6.68(m, 1H), 5.80 (d, J = 17.0 Hz, 1H), 5.65 (d, J = 17.0 Hz, 1H), 5.30-5.20 (m, 1H), 3.83-3.72 (m, 1H), 3.45-3.35 (m, 1H), 3.08-2.92 (m, 3H), 1.82-1.45 (m, 4H), 1.43 (s, 9H), 1.39(s, 3H), 1.37 (s, 3H).
MS (ESI+) 544(M⁺ +1, 44%).

### Reference Example 50

Benzyl 2-{(3R)-3-[(tert-butoxycarbonyl)amino]-piperidin-1-yl}-3-(2-chlorobenzyl)-4-oxo-4,5-dihydro-3H-imidazo[4,5-c]pyridine-6-carboxylate

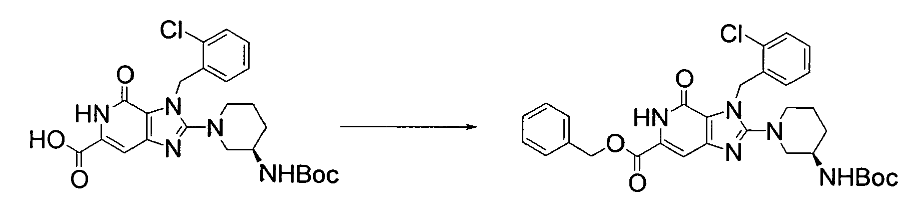

The title compound (55 mg) was synthesized by the same process as in Reference Example 16.
¹H NMR (400 MHz, CDCl₃) δ 7.50 (s, 1H), 7.45-7.32 (m, 6H), 7.25-7.12 (m, 2H), 6.72-6.68 (m, 1H), 5.80 (d, J = 17.0 Hz, 1H), 5.64 (d, J = 17.0 Hz, 1H), 5.38 (s, 2H), 3.82-3.70 (m, 1H), 3.45-3.35 (m, 1H), 3.08-2.95 (m, 3H), 1.82-1.49 (m, 4H), 1.42 (s, 9H).
MS (ESI+) 592 (M⁺+1, 67%).

### Reference Example 51

tert-Butyl {(3R)-1-[6-(aminocarbonyl)-3-(2-chlorobenzyl)-4-oxo-4,5-dihydro-3H-imidazo[4,5-c]pyridin-2-yl]piperidin-3-yl}carbamate

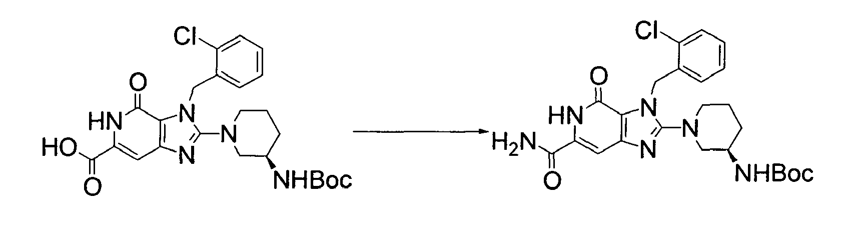

The compound of Reference Example 47 (150 mg) was dissolved in N,N-dimethylformamide (3 mL), followed by adding thereto 1-hydroxybenzotriazole (55 mg), 1-ethyl-3-(dimethylaminopropyl)carbodiimide hydrochloride (69 mg), triethylamine (0.18 mL) and ammonium chloride (21 mg), and the resulting mixture was stirred at 25°C for 13 hours. Water was added to the reaction mixture, followed by extraction with ethyl acetate. The organic layer was washed with water and a saturated aqueous sodium chloride solution, dried over anhydrous sodium sulfate and then filtered, and the filtrate was concentrated under reduced pressure. The resulting residue was purified by a silica gel column chromatography (developing solvent: chloroform/methanol = 10/1) to obtain the title compound (83 mg) as a light-yellow amorphous substance.
¹H NMR (400 MHz, CDCl₃) δ 7.46-7.38 (m, 1H), 7.29-7.15 (m, 3H), 6.79-6.71 (m, 1H), 5.75 (d, J = 17.0 Hz, 1H), 5.61 (d, J = 17.0 Hz, 1H), 3.87-3.73 (m, 1H), 3.51-3.39 (m, 1H), 3.11-2.94 (m, 3H), 1.86-1.50 (m, 4H), 1.42 (s, 9H).
MS (ESI+) 501(M⁺ +1, 50%).

### Reference Example 52

tert-Butyl {(3R)-1-[6-(aminocarbonyl)-3-(2-chlorobenzyl)-5-methyl-4-oxo-4,5-dihydro-3H-imidazo[4,5-c]pyridin-2-yl]piperidin-3-yl}carbamate

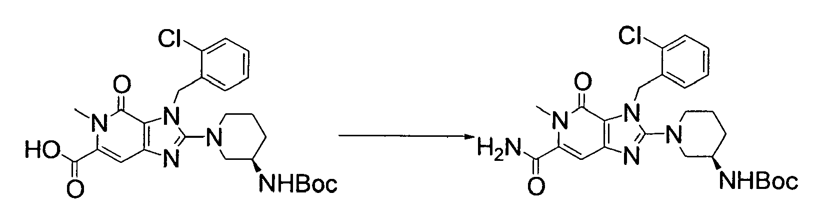

The title compound (85 mg) was synthesized by the same process as in Reference Example 51.
MS (ESI+) 515 (M⁺+1, 100%).

### Reference Example 53

tert-Butyl ((3R)-1-{3-(2-chlorobenzyl)-6-[(dimethylamino)carbonyl]-4-oxo-4,5-dihydro-3H-imidazo[4,5-c]pyridin-2-yl}piperidin-3-yl)carbamate

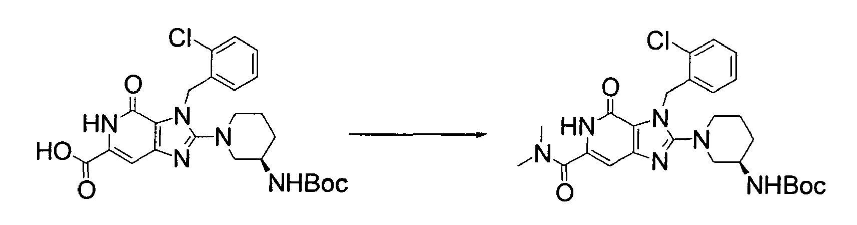

N,N-bis(2-oxo-3-oxazolidinyl)-phosphinyl chloride (21 mg), triethylamine (29 µL) and dimethylamine hydrochloride (7.8 mg) were added to a solution of the compound of Reference Example 17 (28 mg) in dichloromethane (1 mL), and the resulting mixture was stirred at 25°C for 2 hours. After the reaction, water was added to the reaction mixture, followed by extraction with chloroform. The organic layer was washed with a saturated aqueous sodium chloride solution, dried over anhydrous sodium sulfate and then filtered, and the filtrate was concentrated under reduced pressure. The resulting residue was subjected to isolation and purification by a preparative thin-layer silica gel chromatography (developing solvent: chloroform/methanol = 10/1) to obtain the title compound (16 mg) as a white amorphous substance.
¹H NMR (400 MHz, CDCl₃) δ 7.42-7.38 (m, 1H), 7.25-7.13 (m, 2H), 6.87 (s, 1H), 6.79-6.71(m, 1H), 5.79 (d, J = 17.0 Hz, 1H), 5.64 (d, J = 17.0 Hz, 1H), 3.84-3.71 (m, 1H), 3.49-3.39 (m, 1H), 3.20 (m, 6H), 3.10-2.91 (m, 3H), 1.83-1.50 (m, 4H), 1.43 (s, 9H).
MS (ESI+) 529 (M⁺+1, 44%).

### Reference Example 54

tert-Butyl ((3R)-1-{3-(2-chlorobenzyl)-6-[(dimethylamino)carbonyl]-5-methyl-4-oxo-4,5-dihydro-3H-imidazo[4,5-c]pyridin-2-yl}piperidin-3-yl)carbamate

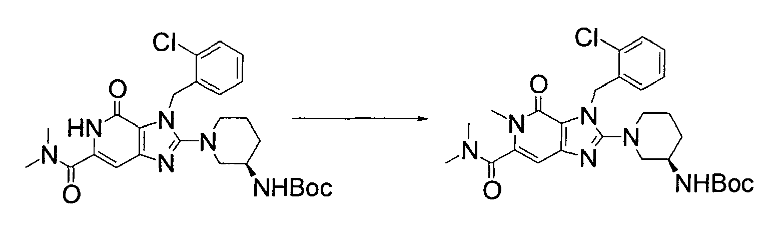

Potassium carbonate (3 mg) and methyl iodide (4 µL) were added to a solution of the compound of Reference Example 53 (11 mg) in N,N-dimethylformamide (0.5 mL), and the resulting mixture was stirred at 25°C for 13 hours. After the reaction, water was added to the reaction mixture, followed by extraction with chloroform. The organic layer was washed with a saturated aqueous sodium chloride solution, dried over anhydrous sodium sulfate and then filtered, and the filtrate was concentrated under reduced pressure to obtain the title compound (11 mg) as a light-yellow amorphous substance.
MS (ESI+) 543 (M⁺+1, 62%).

### Reference Example 55

Methyl 2-{(3R)-3-[(tert-butoxycarbonyl)amino]-piperidin-1-yl}-3-(2-chloro-5-fluorobenzyl)-4-oxo-4,5-dihydro-3H-imidazo[4,5-c]pyridine-6-carboxylate

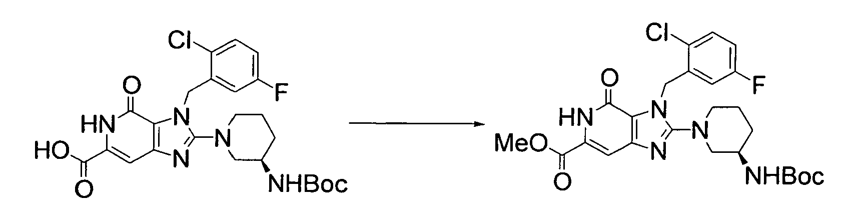

The title compound (265 mg) was synthesized by the same process as in Reference Example 16.
MS (ESI+) 534 (M⁺+1, 75%).

### Reference Example 56

2-{(3R)-3-[(tert-Butoxycarbonyl)amino]piperidin-1-yl}-3-(2-chloro-5-fluorobenzyl)-4-oxo-4,5-dihydro-3H-imidazo[4,5-c]pyridine-6-carboxylic acid

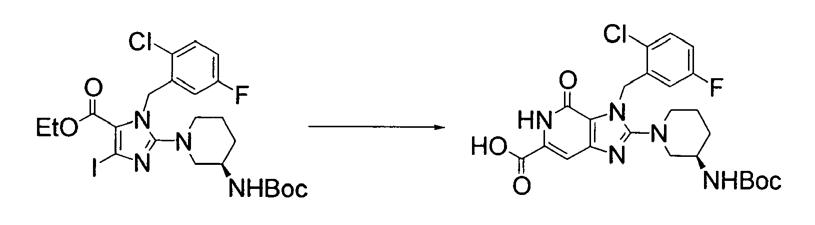

The title compound (907 mg) was synthesized by the same process as in Reference Example 17.
MS (ESI+) 520 (M⁺+1, 58%).

### Reference Example 57

Methyl 2-{(3R)-3-[(tert-butoxycarbonyl)amino]-piperidin-1-yl}-3-(3-methylbut-2-en-1-yl)-4-oxo-4,5-dihydro-3H-imidazo[4,5-c]pyridine-6-carboxylate

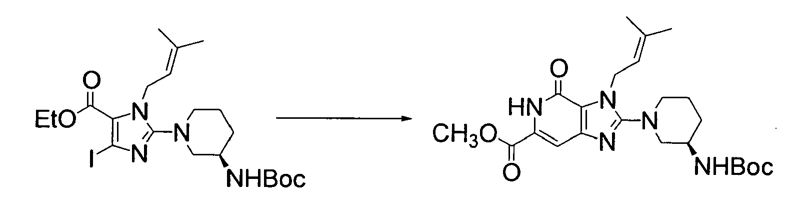

The title compound (680 mg) was synthesized by adopting the same process as in Reference Example 17 and then the same process as in Reference Example 16.
MS (ESI+) 460 (M⁺+1, 38%).

### Reference Example 58

Ethyl 2-{(3R)-3-[(tert-butoxycarbonyl)amino]-piperidin-1-yl}-1-(2-chloro-5-fluorobenzyl)-4-iodo-1H-imidazole-5-carboxylate

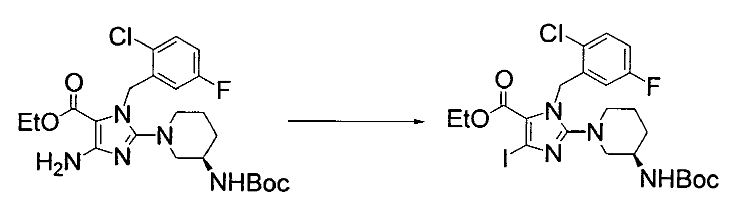

The title compound (3.3 g) was synthesized by the same process as in Reference Example 18.
MS (ESI+) 607 (M⁺+1, 30%).

### Reference Example 59

Ethyl 2-{(3R)-3-[(tert-butoxycarbonyl)amino]-piperidin-1-yl}-4-iodo-1-(3-methylbut-2-en-1-yl)-1H-imidazole-5-carboxylate

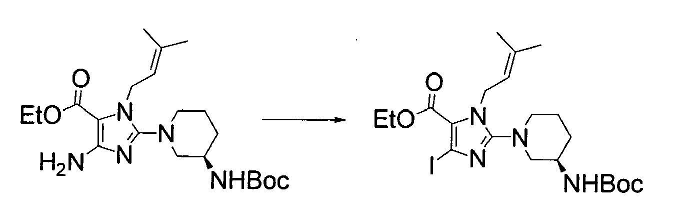

The title compound (2.8 g) was synthesized by the same process as in Reference Example 18.
MS (ESI+) 533 (M⁺+1, 33%).

### Reference Example 60

Ethyl 4-amino-2-{(3R)-3-[(tert-butoxycarbonyl)-amino]piperidin-1-yl}-1-(2-chloro-5-fluorobenzyl)-1H-imidazole-5-carboxylate

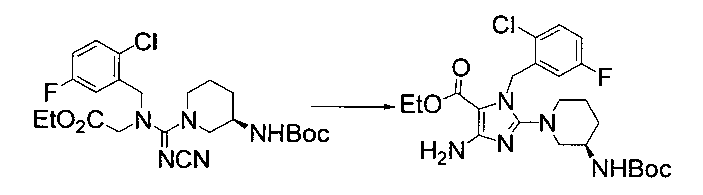

Sodium hydride (60%, 1.42 g) was added to tetrahydrofuran (260 mL) at room temperature and stirred for 30 minutes. A solution (110 mL) of ethyl N-[(E)-{(3R)-3-[(tert-butoxycarbonyl)amino]piperidin-1-yl}(cyanoimino)methyl]-N-(2-chloro-5-fluorobenzyl)glycinate (19.5 g) in tetrahydrofuran was added to the reaction solution at 0°C and stirred at room temperature for 2 hours. The reaction solution was cooled to 0°C and water (2.0 mL) was carefully added thereto, followed by adding thereto a saturated aqueous ammonium chloride solution (10 mL). The reaction solution was concentrated under reduced pressure and water and potassium carbonate were added to the residue to obtain an alkaline solution, followed by two runs of extraction with ethyl acetate. The combined organic layer was dried over anhydrous sodium sulfate and filtered, and the filtrate was concentrated under reduced pressure to obtain the title compound as a crude product (19.5 g).
¹H NMR (300 MHz, CDCl₃) δ 7.33 (dd, J =5.0, 8.7 Hz, 1H), 6.90 (dt, J = 3.0, 8.4 Hz, 1H), 6.54-6.52 (m, 1H), 5.21 (s, 2H), 5.12-4.97 (m, 3H), 4.15-4.10 (m, 2H), 3.79-3.70 (m, 1H), 3.30 (dd, J = 3.2, 12.1 Hz, 1H), 2.99-2.91 (m, 1H), 2.90-2.82 (m, 2H), 1.79-1.51 (m, 4H), 1.41 (s, 9H), 1.10-1.05 (m, 3H).
MS (ESI+) 496 (M⁺+1, 100%).

### Reference Example 61

Ethyl 4-amino-2-{(3R)-3-[(tert-butoxycarbonyl)-amino]piperidin-1-yl}-1-(3-methylbut-2-en-1-yl)-1H-imidazole-5-carboxylate

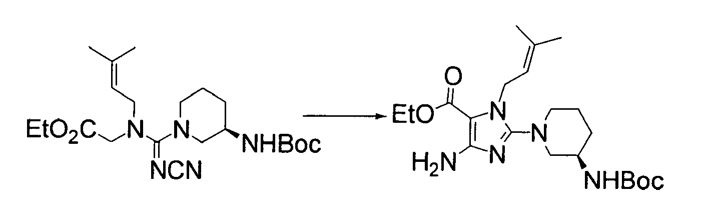

Sodium hydride (1.24 g, a 60% oil dispersion) was added to tetrahydrofuran (130 mL) at room temperature and stirred for 30 minutes. A solution (50 mL) of ethyl N-[(E)-{(3R)-3-[(tert-butoxycarbonyl)amino]piperidin-1-yl}(cyanoimino)methyl]-N-(3-methylbut-2-en-1-yl)glycinate (8.69 g) in tetrahydrofuran was added to the reaction solution at 0°C and stirred at room temperature for 2 hours. The reaction solution was cooled to 0°C and water (1.0 mL) was carefully added thereto, followed by adding thereto a saturated aqueous ammonium chloride solution (5 mL). The reaction solution was concentrated under reduced pressure and water and potassium carbonate were added to the residue to obtain an alkaline solution, followed by two runs of extraction with ethyl acetate. The combined organic layer was dried over anhydrous sodium sulfate and filtered, and the filtrate was concentrated under reduced pressure to obtain the title compound (8.71 g) as a crude product.
¹H NMR (300 MHz, CDCl₃) δ 5.24-5.21 (m, 1H), 5.06-4.96 (m, 1H), 4.92-4.82 (m, 2H), 4.57-4.55 (m, 2H), 4.27 (q, J = 7.1 Hz, 2H), 3.81-3.79 (m, 1H), 3.33-3.30 (m, 1H), 3.06-2.05 (m, 1H), 3.00-2.98 (m, 1H), 2.88 (dd, J = 6.8, 12.0 Hz, 1H), 1.84-1.78 (m, 2H), 1.81-1.40 (m, 2H), 1.73 (s, 3H), 1.70 (s, 3H), 1.44 (s, 9H), 1.33 (t, J = 7.1 Hz, 3H).
MS (ESI+) 422 (M⁺+1, 100%).

### Reference Example 62

Ethyl N-[(E)-{(3R)-3-[(tert-butoxycarbonyl)-amino]piperidin-1-yl}(cyanoimino)methyl]-N-(2-chloro-5-fluorobenzyl)glycinate

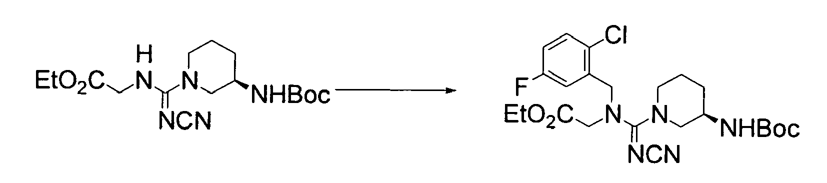

2-Chloro-5-fluorobenzyl bromide (21.9 g) and potassium carbonate (27.6 g) were added to a solution (133 mL) of ethyl N-[(E)-{(3R)-3-[(tert-butoxycarbonyl)amino]piperidin-1-yl}(cyanoimino)methyl]glycinate (23.4 g) in acetonitrile at room temperature and stirred overnight. The reaction mixture was filtered and the filtrate was concentrated under reduced pressure. The resulting residue was purified by a silica gel column chromatography (developing solvent: hexane/ethyl acetate = 2/1 to 2/3) to obtain the title compound (19.9 g).
¹H NMR (300 MHz, CDCl₃) δ 7.37 (dd, J = 5.0, 8.8 Hz, 1H), 7.08-7.06, (m, 1H), 7.01 (dt, J = 2.9, 8.3 Hz, 1H), 4.88-4.68 (m, 1H), 4.62-4.53 (m, 2H), 4.23 (q, J = 7.1 Hz, 2H), 4.03-3.89 (m, 2H), 3.74-3.70 (m, 2H), 3.59-3.51 (m, 1H), 3.45-3.35 (m, 1H), 3.22-3.14 (m, 1H), 1.95-1.71 (m, 2H), 1.71-1.66 (m, 1H), 1.59-1.56 (m, 1H), 1.43 (s, 9H), 1.29 (t, J = 7.1 Hz, 3H).
MS (ESI+) 496 (M⁺+1, 52%).

### Reference Example 63

Ethyl N-[(E)-{(3R)-3-[(tert-butoxycarbonyl)-amino]piperidin-1-yl}(cyanoimino)methyl]-N-(3-methylbut-2-en-1-yl)glycinate

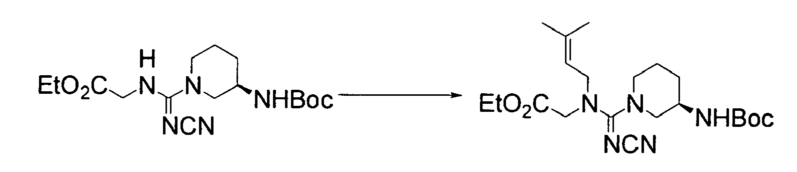

1-Bromo-3-methyl-2-butene (7.59 g) and potassium carbonate (14.1 g) were added to a solution (68 mL) of ethyl N-[(E)-{(3R)-3-[(tert-butoxycarbonyl)amino]piperidin-1-yl}(cyanoimino)methyl]glycinate (12.0 g) in acetonitrile at room temperature and stirred overnight. The reaction mixture was filtered and the filtrate was concentrated under reduced pressure. The resulting residue was purified by a silica gel column chromatography (developing solvent: hexane/ethyl acetate = 2/1 to 1/2) to obtain the title compound (8.89 g).
¹H NMR (300 MHz, CDCl₃) δ 5.18-5.14 (m, 1H), 4.81 (brs, 1H), 4.20 (q, J = 7.1 Hz, 2H), 4.03 (s, 2H), 3.91-3.89 (m, 2H), 3.69-3.67 (m, 2H), 3.55-3.50 (m, 1H), 3.40-3.30 (m, 1H), 3.20-3.15 (m, 1H), 1.94-1.86 (m, 2H), 1.74 (s, 3H), 1.64 (s, 3H), 1.81-1.40 (m, 2H), 1.44 (s, 9H), 1.27 (t, J = 7.1 Hz, 3H).
MS (ESI+) 422 (M⁺+1, 39%).

### Reference Example 64

tert-Butyl {(3R)-1-[3-(2-chlorobenzyl)-6-(hydroxymethyl)-5-methyl-4-oxo-4,5-dihydro-3H-imidazo[4,5-c]pyridin-2-yl]piperidin-3-yl}carbamate

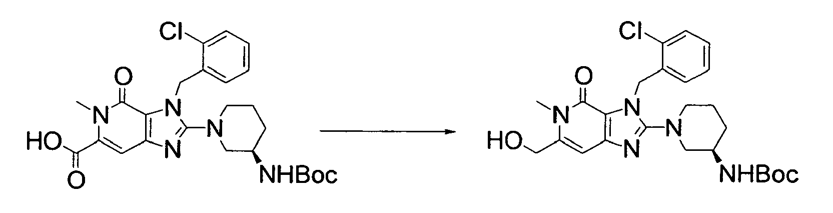

A solution of the compound of Reference Example 47 (2.11 g) and triethylamine (0.68 mL) in tetrahydrofuran (20 mL) was cooled to 0°C, followed by adding dropwise thereto isopropyl chlorocarbonate (0.68 mL), and the resulting mixture was stirred at 0°C for 1 hour. The precipitate formed was collected by filtration and washed with tetrahydrofuran, and the filtrate was cooled to 0°C. An aqueous solution (2 mL) of sodium tetrahydroborate (309 mg) was added dropwise thereto and the resulting mixture was stirred at 0°C for 30 minutes. After the reaction, a saturated aqueous ammonium chloride solution was added to the reaction mixture, followed by extraction with ethyl acetate. The organic layer was washed with a saturated aqueous sodium chloride solution, dried over sodium sulfate and then filtered, and the filtrate was concentrated under reduced pressure. The resulting residue was purified by a silica gel column chromatography (developing solvent: hexane/ethyl acetate = 1/1 ~ chloroform/methanol = 10/1) to obtain the title compound (1.9 g) as a white amorphous substance.
¹H NMR (400 MHz, CDCl₃) δ 7.42-7.35 (m, 1H), 7.22-7.08 (m, 2H), 6.65-6.58 (m, 1H), 6.56(s, 1H), 5.74 (d, J = 17.0 Hz, 1H), 5.60 (d, J = 17.0 Hz, 1H), 4.61(s, 2H), 3.82-3.72 (m, 1H), 3.65 (s, 3H), 3.44-3.32 (m, 1H), 3.06-2.90 (m, 3H), 1.81-1.50 (m, 4H), 1.42 (s, 9H).
MS (ESI+) 502 (M⁺+1, 100%).

### Reference Example 65

2-{(3R)-3-[(tert-Butoxycarbonyl)amino]piperidin-1-yl}-3-(3-methylbut-2-en-1-yl)-4-oxo-4,5-dihydro-3H-imidazo[4,5-c]pyridine-6-carboxylic acid

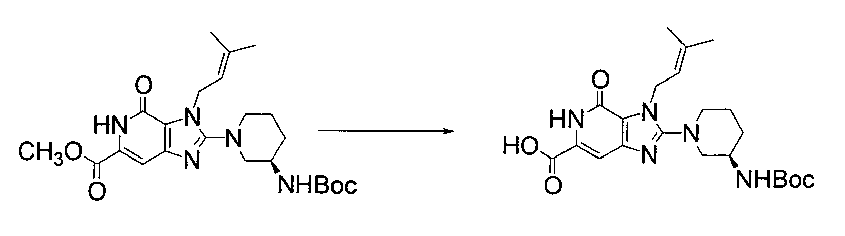

The title compound (590 mg) was synthesized by the same process as in Reference Example 47.
¹H NMR (400 MHz, CDCl₃) δ 7.63 (s, 1H), 5.44-5.37 (m, 1H), 5.05-4.94 (m, 2H), 3.94-3.80(m, 1H), 3.60-3.51 (m, 1H), 3.33-3.05 (m, 3H), 1.97-1.65 (m, 4H), 1.81 (s, 3H), 1.75 (s, 3H), 1.44 (s, 9H).
MS (ESI+) 446 (M⁺+1, 35%).

### Reference Example 66

tert-Butyl {(3R)-1-[3-(2-chlorobenzyl)-5-(3-methoxyphenyl)-4-oxo-4,5-dihydro-3H-imidazo[4,5-c]pyridin-2-yl]piperidin-3-yl}carbamate

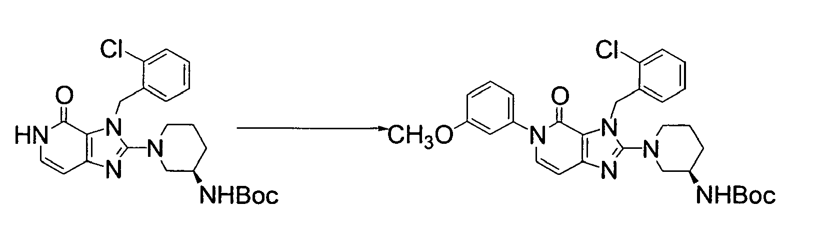

Under a nitrogen atmosphere, molecular sieve 4A (400 mg, Wako Pure Chemical Industries, Ltd.), triethylamine (59 µL) and copper acetate (80 mg) were added to a solution of the compound of Reference Example 29 (100 mg) and 3-methoxyphenylboric acid (66 mg) in dichloromethane (5 mL), and the resulting mixture was stirred at room temperature for 21 hours. The reaction mixture was filtered through Celite, followed by washing with chloroform, and the filtrate was concentrated under reduced pressure. The resulting residue was purified by a silica gel column chromatography (developing solvent: hexane/ethyl acetate = 1/1 to 0/1) to obtain the title compound (43 mg).
¹H NMR (400 MHz, CDCl₃) δ 7.39-7.28 (m, 2H), 7.21-7.09 (m, 3H), 6.97-6.87 (m, 3H), 6.75-6.71 (m, 1H), 6.67 (d, J = 7.3 Hz, 1H), 5.78 (d, J = 17.0 Hz, 1H), 5.63 (d, J = 17.0 Hz, 1H), 3.84-3.73 (m, 1H), 3.81 (s, 3H), 3.46-3.36 (m, 1H), 3.06-2.94 (m, 3H), 1.81-1.49(m, 4H), 1.43 (s, 9H).
MS (ESI+) 564(M⁺ +1, 100%).

### Reference Example 67

tert-Butyl {(3R)-1-[3-(2-chlorobenzyl)-5-(3-fluorophenyl)-4-oxo-4,5-dihydro-3H-imidazo[4,5-c]pyridin-2-yl]piperidin-3-yl}carbamate

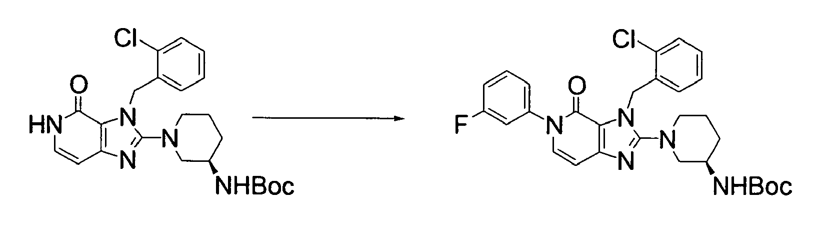

The title compound (36 mg) was synthesized by the same process as in Reference Example 66.
¹H NMR (400 MHz, CDCl₃) δ 7.49-7.32 (m, 2H), 7.20-7.02 (m, 6H), 6.77-6.73 (m, 1H), 6.70 (d, J = 7.2 Hz, 1H), 5.76 (d, J = 17.0 Hz, 1H), 5.61 (d, J = 17.0 Hz, 1H), 3.85-3.70 (m, 1H), 3.45-3.36 (m, 1H), 3.07-2.92 (m, 3H), 1.80-1.52 (m, 4H), 1.43 (s, 9H).
MS (ESI+) 552(M⁺ +1, 100%).

### Reference Example 68

tert-Butyl {(3R)-1-[3-(2-chlorobenzyl)-4-oxo-5-phenyl-4,5-dihydro-3H-imidazo[4,5-c]pyridin-2-yl]piperidin-3-yl}carbamate

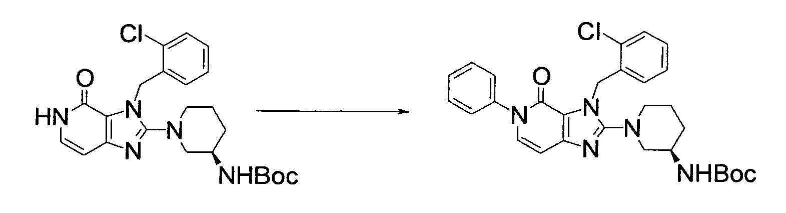

The title compound (73 mg) was synthesized by the same process as in Reference Example 66.
¹H NMR (400 MHz, CDCl₃) δ 7.48-7.41 (m, 2H), 7.40-7.32 (m, 4H), 7.24-7.10 (m, 3H), 6.80-6.72 (m, 1H), 6.68 (d, J = 7.3 Hz, 1H), 5.78 (d, J = 17.0 Hz, 1H), 5.63 (d, J = 17.0 Hz, 1H), 3.85-3.74 (m, 1H), 3.48-3.38 (m, 1H), 3.10-2.95 (m, 3H), 1.82-1.57 (m, 3H), 1.43(s, 9H), 0.91-0.73 (m, 1H).
MS (ESI+) 534(M⁺ +1, 100%).

### Reference Example 69

tert-Butyl {(3R)-1-[3-(2-chlorobenzyl)-5-(4-fluorophenyl)-4-oxo-4,5-dihydro-3H-imidazo[4,5-c]pyridin-2-yl]piperidin-3-yl}carbamate

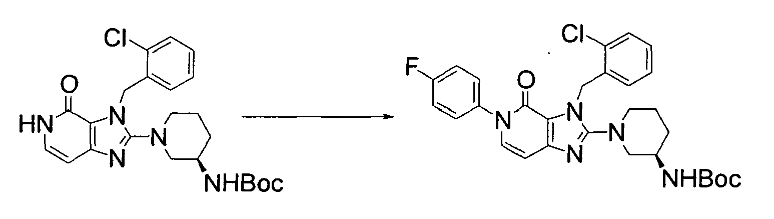

The title compound (58 mg) was synthesized by the same process as in Reference Example 66.
¹H NMR (400 MHz, CDCl₃) δ 7.40-7.33 (m, 3H), 7.18-7.11 (m, 5H), 6.77-6.71 (m, 1H), 6.69 (d, J = 7.3 Hz, 1H), 5.76 (d, J = 17.0 Hz, 1H), 5.61 (d, J = 17.0 Hz, 1H), 3.83-3.76 (m, 1H), 3.48-3.40 (m, 1H), 3.08-3.01 (m, 3H), 1.77-1.64 (m, 3H), 1.42 (s, 9H), 0.85-0.80 (m, 1H).
MS (ESI+) 552(M⁺ +1, 100%).

### Reference Example 70

tert-Butyl {(3R)-1-[3-(2-chlorobenzyl)-5-methyl-4-oxo-6-(phenoxymethyl)-4,5-dihydro-3H-imidazo[4,5-c]pyridin-2-yl]piperidin-3-yl}carbamate

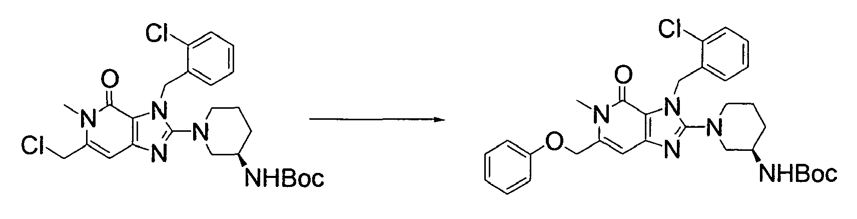

A solution of the compound of Reference Example 73 (100 mg), cesium carbonate (163 mg) and phenol (25 mL) in N,N-dimethylformamide (3 mL) was stirred at 25°C for 16 hours. After the reaction, water was added to the reaction mixture, followed by extraction with ethyl acetate. The organic layer was washed with a saturated aqueous sodium chloride solution, dried over sodium sulfate and then filtered, and the filtrate was concentrated under reduced pressure. The resulting residue was purified by a silica gel column chromatography (developing solvent: hexane/ethyl acetate = 1/1) to obtain the title compound (71 mg) as a white amorphous substance.
MS (ESI+) 578 (M⁺+1, 100%).

### Reference Example 71

tert-Butyl ((3R)-1-{3-(2-chlorobenzyl)-6-[(3-methoxyphenoxy)methyl]-5-methyl-4-oxo-4,5-dihydro-3H-imidazo[4,5-c]pyridin-2-yl}piperidin-3-yl)carbamate

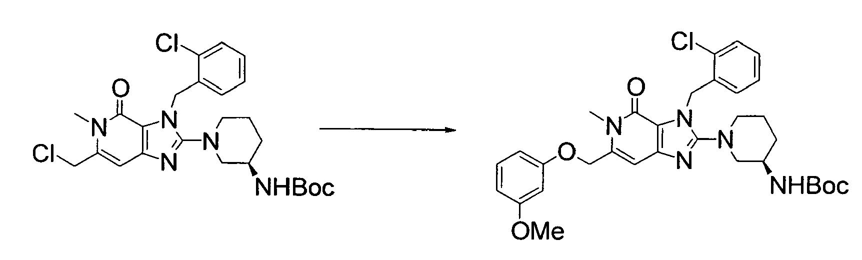

The title compound (57 mg) was obtained as a white amorphous substance by the same process as in Reference Example 70.
¹H NMR (400 MHz, CDCl₃) δ 7.43-7.37 (m, 1H), 7.26-7.15 (m, 3H), 6.75 (s, 1H), 6.74-6.68(m, 1H), 6.60-6.50 (m, 3H), 5.78 (d, J = 17.0 Hz, 1H), 5.63 (d, J = 17.0 Hz, 1H), 5.00 (s, 2H), 3.80 (s, 3H), 3.80-3.72 (m,1H), 3.62 (s, 3H), 3.40-3.37 (m, 1H), 3.02-2.89 (m, 3H), 1.76-1. 67 (m, 4H), 1.42 (s, 9H).
MS (ESI+) 608 (M⁺+1, 100%).

### Reference Example 72

tert-Butyl {(3R)-1-[3-(2-chlorobenzyl)-6-(cyanomethyl)-5-methyl-4-oxo-4,5-dihydro-3H-imidazo[4,5-c]pyridin-2-yl]piperidin-3-yl}carbamate

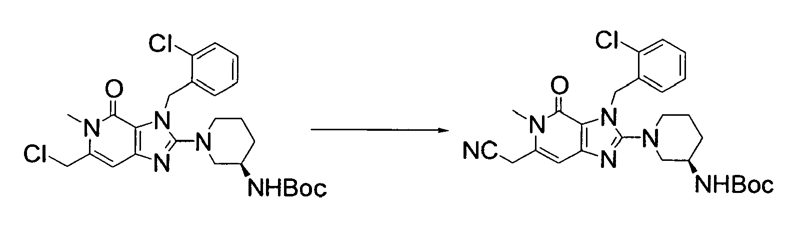

The compound of Reference Example 73 (200 mg), potassium cyanide (27 mg) and potassium iodide (3 mg) were dissolved in dimethylformamide (5 mL), and the resulting solution was stirred at 25°C for 24 hours. After the reaction, water was added to the reaction mixture, followed by extraction with ethyl acetate. The organic layer was washed with a saturated aqueous sodium chloride solution, dried over sodium sulfate and then filtered, and the filtrate was concentrated under reduced pressure. The resulting residue was purified by a silica gel column chromatography (developing solvent: ethyl acetate) to obtain the title compound (105 mg) as a brown amorphous substance.
MS (ESI+) 511 (M⁺+1, 100%).

### Reference Example 73

tert-Butyl {(3R)-1-[3-(2-chlorobenzyl)-6-(chloromethyl)-5-methyl-4-oxo-4,5-dihydro-3H-imidazo[4,5-c]pyridin-2-yl]piperidin-3-yl}carbamate

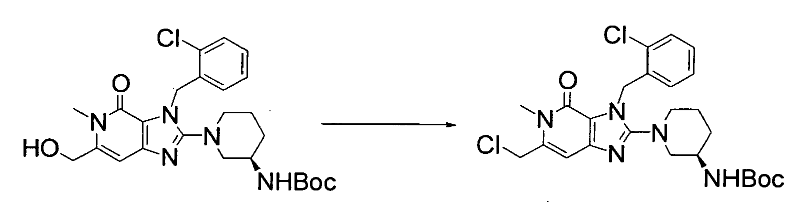

Under a nitrogen atmosphere, N-chlorosuccinimide (339 mg) and triphenylphosphine (656 mg) were added to a solution (10 mL) of the compound of Reference Example 64 (501 mg) in tetrahydrofuran, and the resulting mixture was stirred at 25°C for 2 hours. After the reaction, the reaction mixture was concentrated under reduced pressure and the resulting residue was purified by a silica gel column chromatography (developing solvent: hexane/ethyl acetate = 1/1) to obtain the title compound (485 mg) as a light-yellow amorphous substance.
MS (ESI+) 520 (M⁺+1, 100%).

### Reference Example 74

tert-Butyl {(3R)-1-[3-(2-chlorobenzyl)-4-oxo-4,5-dihydro-3H-imidazo[4,5-c]quinolin-2-yl]piperidin-3-yl}carbamate

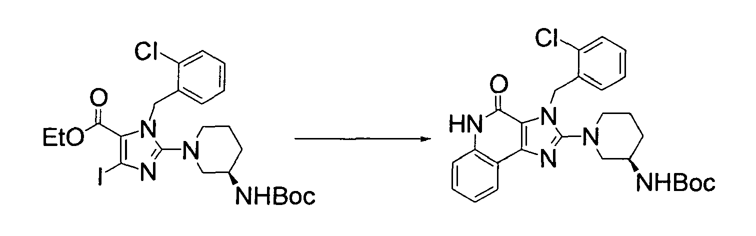

A solution of tetrakis(triphenylphosphine)-palladium(0) (20 mg), 2-aminobenzeneboric acid (23 mg) and sodium carbonate (36 mg) in water (0.7 mL) was added to a solution of the compound of Reference Example 18 (100 mg) in ethylene glycol dimethyl ether, and the resulting mixture was stirred at 80°C. After 6 hours, the reaction solution was allowed to cool and water was added thereto, followed by extraction with ethyl acetate. The organic layer was washed with water and a saturated aqueous sodium chloride solution, dried over sodium sulfate and then filtered, and the filtrate was concentrated under reduced pressure. The resulting residue was dissolved in ethanol (2 mL), followed by adding thereto sodium ethoxide (1 mL) (a 21% ethanol solution), and the resulting mixture was stirred at 80°C. After 1 hour, the reaction solution was allowed to cool and water was added thereto, followed by extraction with ethyl acetate. The organic layer was washed with an aqueous sodium chloride solution, dried over sodium sulfate and then filtered, and the filtrate was concentrated under reduced pressure. The resulting residue was purified by a silica gel column chromatography (developing solvent: hexane/ethyl acetate = 1/1) to obtain the title compound (57 mg) as a white solid.
¹H NMR (400 MHz, CDCl₃) δ 8.24-8.22 (m, 1H), 7.47-7.37 (m, 2H), 7.31-7.10 (m, 4H), 6.78-6.70 (m, 1H), 5.78 (d, J = 17 Hz, 1H), 5.68 (d, J = 17 Hz, 1H), 3.87-3.76 (m, 1H), 3.48-3.39 (m, 1H), 3.31-3.20 (m, 1H), 3.16-3.03 (m, 2H), 1.80-1.48 (m, 4H), 1.47 (s, 9H).
MS (ESI+) 508(M⁺ +1, 100%).

### Reference Example 75

Methyl 2-{(3R)-3-[(tert-butoxycarbonyl)amino]-piperidin-1-yl}-3-(2-chlorobenzyl)-4-oxo-4,5-dihydro-3H-imidazo[4,5-c]quinoline-8-carboxylate

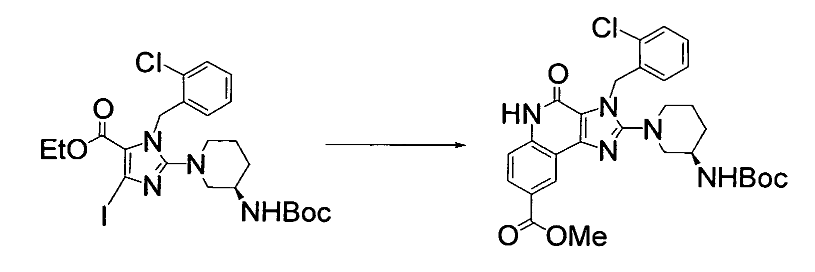

A solution of tetrakis(triphenylphosphine)-palladium(0) (129 mg), methyl 4-amino-3-(4,4,5,5-tetramethyl-1,3,2-dioxaborolan-2-yl)benzoate (344 mg) and sodium carbonate (240 mg) in water (4.7 mL) was added to a solution (9.4 mL) of ethyl 2-{(3R)-3-[(tert-butoxycarbonyl)-amino]piperidin-1-yl}-1-(2-chlorobenzyl)-4-iodo-1H-imidazole-5-carboxylate (665 mg) in ethylene glycol dimethyl ether, and the resulting mixture was stirred at 80°C. After 18.5 hours, the reaction solution was allowed to cool and a saturated aqueous ammonium chloride solution was added thereto, followed by extraction with ethyl acetate. The organic layer was washed with a saturated aqueous sodium chloride solution, dried over sodium sulfate and then filtered, and the filtrate was concentrated under reduced pressure. The resulting residue was purified by a silica gel column chromatography (developing solvent: hexane/ethyl acetate = 1/2). The compound thus obtained was dissolved in ethanol (2 mL), followed by adding thereto sodium ethoxide (1 mL) (a 21% ethanol solution), and the resulting mixture was stirred at 80°C. After 1.5 hours, the reaction solution was allowed to cool and water was added thereto, followed by extraction with ethyl acetate. The extract solution was dried over sodium sulfate and filtered, and the filtrate was concentrated under reduced pressure. The resulting residue was purified by a liquid chromatography to obtain the title compound (5.8 mg) as a white solid.
¹H NMR (300 MHz, CDCl₃) δ 8.81 (d, J = 1.5 Hz, 1H), 7.97 (dd, J = 1.8, 8.4 Hz, 1H), 7.39 (dd, J = 0.9, 7.9 Hz, 1H), 7.14-7.03 (m, 3H), 6.67 (d, J = 7.9 Hz, 1H), 5.72 (d, J = 16.8 Hz, 1H), 5.57 (d, J = 16.8 Hz, 1H), 5.29 (m, 1H), 3.90 (s, 3H), 3.76-3.02 (m, 5H), 1.98-1.53 (m, 4H), 1.37 (s, 9H).
MS (ESI+) 566 (M⁺+1, 100%).

### Reference Example 76

Methyl 2-{(3R)-3-[(tert-butoxycarbonyl)amino]-piperidin-1-yl}-3-(2-chlorobenzyl)-5-methyl-4-oxo-4,5-dihydro-3H-imidazo[4,5-c]quinoline-6-carboxylate

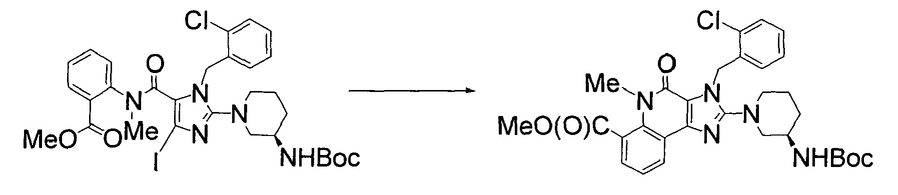

Palladium acetate (25 mg), triphenylphosphine (58 mg) and silver carbonate (59 mg) were added to a solution (10 mL) of methyl 2[{[2-{(3R)-3-[(tert-butoxycarbonyl)amino]-piperidin-1-yl}-1-(2-chlorobenzyl)-4-iodo-1H-imidazol-5-yl]carbonyl} (methyl)amino]benzoate (237 mg) in N,N-dimethylformamide, and the resulting mixture was stirred at 160°C. After one and a half hours, the reaction solution was allowed to cool and filtered through Celite, and a saturated aqueous sodium chloride solution was added to the filtrate, followed by extraction with ethyl acetate. The organic layer was dried over sodium sulfate and filtered, and the filtrate was concentrated under reduced pressure. The resulting residue was purified by a silica gel column chromatography (developing solvent: hexane/ethyl acetate = 2/1) to obtain the title compound (168 mg) as a white solid.
¹H NMR (300 MHz, CDCl₃) δ 8.43 (dd, J = 1.7, 7.9 Hz, 1H), 7.69 (dd, J = 1.7, 7.5 Hz, 1H), 7.40 (dd, 1.5, 7.7 Hz, 1H), 7.32 (dd, J = 7.5, 7.9 Hz, 1H), 7.23-7.12 (m, 2H), 6.72 (dd, J = 1.3, 7.4 Hz, 1H), 6.19 (m, 1H), 5.76 (d, J = 17.0 Hz, 1H), 5.64 (d, J = 17.0 Hz, 1H), 3.97 (s, 3H), 3.83 (brs, 1H), 3.57 (s, 3H), 3.44 (dd, J = 3.3, 13.0 Hz, 1H), 3.29-3.24 (m, 1H), 3.10-3.09 (m, 2H), 1.83-1.51 (m, 4H), 1.43 (s, 9H).
MS (ESI+) 580 (M⁺+1, 100%).

The compounds of Reference Examples 77 to 91 were synthesized from corresponding compounds of Reference Examples, respectively, by the same process as in Reference Example 76.

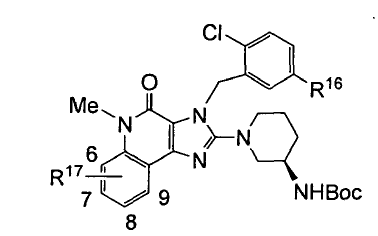

| Reference example number | R¹⁶ | R¹⁷ | Reference example number for starting material |
|---|---|---|---|
| Reference Example 77 | H | 7-CO₂(t-Bu) | Reference Example 131 |
| Reference Example 78 | H | 7-CO₂Me | Reference Example 133 |
| Reference Example 79 | H | 8-CO₂Me | Reference Example 134 |
| Reference Example 80 | H | 7,9-CO₂Me | Reference Example 135 |
| Reference Example 81 | H | 6-MeO/7-CO₂Et | Reference Example 136 |
| Reference Example 82 | H | 6,8-F/7-CO₂Et | Reference Example 137 |
| Reference Example 83 | F | 8-CO₂Me | Reference Example 138 |
| Reference Example 84 | H | 8-OCHF₂ | Reference Example 139 |
| Reference Example 85 | H | 9-OMe/7-CO₂(t-Bu) | Reference Example 141 |
| Reference Example 86 | F | 7-CO₂(t-Bu) | Reference Example 142 |
| Reference Example 87 | H | 8-CH₂CO₂Et | Reference Example 143 |
| Reference Example 88 | F | 7-MeO/8-CO₂Me | Reference Example 144 |
| Reference Example 89 | F | 6-MeO/8-CO₂Me | Reference Example 145 |
| Reference Example 90 | F | 8-F/7-CO₂(t-Bu) | Reference Example 146 |
| Reference Example 91 | F | 7,9-CO₂Me | Reference Example 132 |

### Reference Example 77

¹H NMR (300 MHz, CDCl₃) δ 8.31 (d, J = 8.3 Hz, 1H), 8.12 (d, J = 1.1 Hz, 1H), 7.91 (dd, J = 1.1, 8.3 Hz, 1H), 7.41 (dd, J = 1.5, 7.9 Hz, 1H), 7.23-7.11 (m, 2H), 6.69 (d, J = 6.2 Hz, 1H), 6.07-6.05 (m, 1H), 5.79 (d, J = 16.8 Hz, 1H), 5.66 (d, J = 16.8 Hz, 1H), 3.79 (m, 1H), 3.79 (s, 3H), 3.45 (dd, J = 3.5, 13.0 Hz, 1H), 3.27-3.21 (m, 1H), 3.09-3.07 (m, 2H), 1.74-1.52 (m, 4H), 1.65 (s, 9H), 1.47 (s, 9H).
MS (ESI+) 622 (M⁺+1, 100%).

### Reference Example 78

¹H NMR (300 MHz, CDCl₃) δ 8.35 (d, J = 8.1 Hz, 1H), 8.16 (d, J = 1.3 Hz, 1H), 7.97 (dd, J = 1.3, 8.1 Hz, 1H), 7.41 (dd, J = 1.5, 7.9 Hz, 1H), 7.23-7.10 (m, 2H), 6.68 (d, J = 6.4 Hz, 1H), 6.11 (m, 1H), 5.79 (d, J = 16.8 Hz, 1H), 5.66 (d, J = 16.8 Hz, 1H), 3.99 (s, 3H), 3.80 (s, 3H), 3.80-3.76 (m, 1H), 3.44 (dd, J = 3.1, 12.6 Hz, 1H), 3.25-3.23 (m, 1H), 3.10-3.08 (m, 2H), 1.74-1.55 (4H, m), 1.47 (s, 9H) . m, 3H), 1.83-1.53 (m, 4H), 1.43 (s, 9H).
MS (ESI+) 580 (M⁺+1, 100%).

### Reference Example 79

¹H NMR (300 MHz, CDCl₃) δ 8.95 (d, J = 2.0 Hz, 1H), 8.17 (dd, J = 2.0, 8.8 Hz, 1H), 7.46 (d, J = 8.8 Hz, 1H), 7.41 (dd, J = 1.3, 7.7 Hz, 1H), 7.23-7.11 (m, 2H), 6.70 (d, J = 7.1 Hz, 1H), 5.78 (d, J = 17.0 Hz, 1H), 5,63 (d, J = 17.0 Hz, 1H), 5.36-5.34 (m, 1H), 3.98 (s, 3H), 3.83 (brs, 1H), 3.76 (s, 3H), 3.46 (dd, J = 3.1, 12.4 Hz, 1H), 3.26-3.16 (m, 1H), 3.10(m, 2H), 1.83-1,61 (m, 4H), 1,44 (s, 9H).
MS (ESI+) 580 (M⁺+1, 100%).

### Reference Example 80

¹H NMR (300 MHz, CDCl₃) δ 8.21 (d, J = 1.3 Hz, 1H), 7.99 (brs, 1H), 7.41 (dd, J = 1.5, 7.9 Hz, 1H), 7.24-7.13 (m, 2H), 6.72 (d, J = 7.0 Hz, 1H), 5.81 (d, J = 17.0 Hz, 1H), 5.65 (d, J = 17.0 Hz, 1H), 4.66 (m, 1H), 4.04 (s, 3H), 3.99 (s, 3H), 3.81 (s, 3H), 3.75-3.73 (m, 1H), 3.45-3.42 (m, 1H), 3.10-2.98 (m, 3H), 1.76-1.51 (m, 4H), 1.42 (s, 9H).
MS (ESI+) 638 (M⁺+1, 100%).

### Reference Example 81

¹H NMR (400 MHz, CDCl₃) δ 8.08 (d, J = 8.2 Hz, 1H), 7.67 (d, J = 8.2 Hz, 1H), 7.41 (dd, J = 7.9 and 1.3 Hz, 1H), 7.21 (ddd, J = 7.9, 7.9 and 1.3 Hz, 1H), 7.15 (ddd, J = 7.9, 7.9 and 1.3 Hz, 1H), 6.73 (d, J = 7.9 Hz, 1H), 6.15 (brs, 1H), 5.78 (d, J = 17.0 Hz, 1H), 5.64 (d, J = 17.0 Hz, 1H), 4.44 (q, J = 7.1 Hz, 2H), 3.93 (s, 3H), 3.81 (s, 1H), 3.79 (s, 3H), 3.43 (dd, J = 12.0 and 3.3 Hz, 1H), 3.27-3.23 (m, 1H), 3.09-3.07 (m, 2H), 1.73 (brs, 2H), 1.56-1.50 (m, 2H), 1.46 (s, 9H), 1.45 (t, J = 7.1 Hz, 3H).
MS (ESI+) 624 (M⁺+1, 100%).

### Reference Example 82

¹H NMR (400 MHz, CDCl₃) δ 7.82 (dd, J = 8.8 and 1.6 Hz, 1H), 7.42 (dd, J = 7.9 and 1.3 Hz, 1H), 7.24 (ddd, J = 7.9, 7.9 and 1.3 Hz, 1H), 7.15 (ddd, J = 7.9, 7.9 and 1.3 Hz, 1H), 6.69 (d, J = 7.9 Hz, 1H), 6.06 (brs, 1H), 5.75 (d, J = 16.5 Hz, 1H), 5.62 (d, J = 16.5 Hz, 1H), 4.48 (q, J = 7.1 Hz, 2H), 3.91-3.89 (m, 3H), 3.81 (brs, 1H), 3.43 (dd, J = 12.1 and 3.4 Hz, 1H), 3.25-3.21 (m, 1H), 3.08 (brs, 3H), 1.74 (brs, 2H), 1.53 (brs, 1H), 1.46 (s, 9H), 1.43 (t, J = 7.1 Hz, 3H).
MS (ESI+) 630 (M⁺+1, 100%).

### Reference Example 83

¹H NMR (400 MHz, CD₃OD) δ 8.95 (s, 1H), 8.19 (m, 1H), 7.46 (m, 1H), 7.36 (m, 1H), 6.94 (m, 1H), 6.42 (m, 1H), 5.72-5.56 (m, 2H), 5.33 (bs, 1H), 4.03 (s, 3H), 3.93 (bs, 1H), 3.76 (s, 3H), 3.45 (m, 1H), 3.16-3.09 (m, 3H), 1.81-1.75 (m, 2H), 1.66-1.64 (m, 2H), 1.48 (s, 9H).
MS (ESI+) 598 (M⁺+1, 100%).

### Reference Example 84

¹H NMR (300 MHz, CDCl₃) δ 8.04 (m, 1H), 7.43-7.40 (m, 2H), 7.30 (dd, J = 2.6, 9.2 Hz, 1H), 7.22-7.10 (m, 2H), 6.67 (d, J = 7.7 Hz, 1H), 6.60 (t, J = 73.8 Hz, 1H), 5.79 (d, J = 16.8 Hz, 1H), 5,67 (d, J = 16.8 Hz, 1H), 3.81 (m, 1H), 3.73 (s, 3H), 3.46-3.42 (m, 1H), 3.21-3.18 (m, 1H), 3.10-3.08 (m, 1H), 1.74-1.59 (m, 4H), 1.45 (s, 9H).
MS (ESI+) 588 (M⁺+1, 100%).

### Reference Example 85

¹H NMR (400 MHz, CDCl₃) δ 7.77 (s, 1H), 7.45 (s, 1H), 7.41 (dd, J = 7.9 and 1.3 Hz, 1H), 7.19 (ddd, J = 7.9, 7.9 and 1.3 Hz, 1H), 7.10 (ddd, J = 7.9, 7.9 and 1.3 Hz, 1H), 6.43 (d, J = 7.9 Hz, 1H), 5.88 (d, J = 16.5 Hz, 1H), 5.70 (d, J = 16.5 Hz, 1H), 5.22 (d, J = 7.9 Hz, 1H), 4.14 (s, 3H), 3.86 (brs, 1H), 3.79 (s, 3H), 3.47 (dd, J = 12.1 and 3.4 Hz, 1H), 3.31-3.27 (m, 1H), 3.19-3.09 (m, 2H), 1.75-1.70 (m, 3H), 1.60 (s, 9H), 1.58-1.48 (m, 1H), 1.43 (s, 9H).
MS (ESI+) 652 (M⁺+1, 100%).

### Reference Example 86

¹H NMR (400 MHz, CDCl₃) δ 8.32 (d, 1H, J = 8.2 Hz), 8.13 (s, 1H), 7.91 (d, J = 8.2 Hz, 1H), 7.40 (m, 1H), 6.92 (m, 1H), 6.43 (d, J = 9.0 Hz, 1H), 6.01 (bs, 1H), 5.65-5.62 (m, 2H), 3.85 (m, 1H), 3.81 (s, 3H), 3.45 (m, 1H), 3.18-3.03 (m, 3H), 1.75-1.70 (m, 4H), 1.65 (s, 9H), 1.46 (s, 9H)
MS (ESI+) 640 (M⁺+1, 100%).

### Reference Example 87

¹H NMR (300 MHz, CDCl₃) δ ppm 8.17 (d, J = 2.0Hz, 1H), 7.49-7.39 (m, 3H), 7.22-7.09 (m, 2H), 6.65 (d, J = 7.1Hz, 1H), 5.81 (d, J = 17.9Hz, 1H), 5.66 (d, J = 17.9Hz, 1H), 5.50-5.48 (m, 1H), 4.17 (dd, J = 7.1, 14.3Hz, 2H), 3.82-3.80 (m, 1H), 3.76 (s, 2H), 3.74 (s, 3H), 3.45-3.41 (m, 1H), 3.18-3.06 (m, 3H), 1.72-1.58 (m, 4H), 1.45 (s, 9H), 1.27 (d, J = 7.1Hz, 3H).
MS (ESI+) 608 (M⁺+1, 100%).

### Reference Example 88

MS (ESI+) 628 (M⁺+1, 100%).

### Reference Example 89

¹H NMR (300 MHz, CDCl₃) δ ppm 8.59 (d, J = 1.8 Hz, 1H), 7.66 (d, J = 1.8 Hz, 1H), 7.37 (dd,J = 5.1 and 8.8 Hz, 1H), 6.92 (td, J = 3.1 and 8.3 Hz, 1H), 6.44 (dd, J = 3.1 and 8.8 Hz, 1H), 5.65 (dd, J = 17.2 and 38.3 Hz, 2H), 5.33 (d, J = 6.8 Hz, 1H), 3.98 (s, 3H), 3.97 (s, 3H), 3.95 (s, 3H), 3.91-3.76 (m, 1H), 3.50-3.40 (m, 1H), 3.20-3.00 (m, 3H), 1.85-1.65 (m, 2H), 1.65-1.45 (m, 2H), 1.43 (s, 9H).
MS (ESI+) 628 (M⁺+1, 100%).

### Reference Example 90

¹H NMR (400 MHz, CDCl₃) δ 8.01-7.93 (m, 2H), 7.41-7.35 (m, 1H), 6.96-6.89 (m, 1H), 6.47-6.38 (m, 1H), 5.70 (d, J = 17 Hz, 1H), 5.60 (d, J = 17 Hz, 1H), 3.88-3.75 (m, 1H), 3.77 (s, 3H), 3.50-3.40 (m, 1H), 3.25-3.00 (m, 3H), 1.86-1.50 (m, 4H), 1.64 (s, 9H), 1.46 (s, 9H).
MS (ESI+) 658(M⁺ +1, 100%).

### Reference Example 91

¹H NMR (300 MHz, DMSO-d₆) δ 8.14 (d, J = 1.3 Hz, 1H), 7.81 (d, J = 1.3 Hz, 1H), 7.58 (dd, J = 5.0, 9.3 Hz, 1H), 7.23-7.17 (m, 1H), 6.68 (dd, J = 2.9, 9.3 Hz, 1H), 5.62 (d, J = 17.4 HZ, 1H), 5.54 (d, J = 17.4 Hz, 1H), 3.96 (s, 3H), 3.92 (s, 3H), 3.70 (s, 3H), 3.67-3.63 (m, 1H), 3.55-3.45 (m, 1H), 3.24-3.17 (m, 1H), 3.03-2.99 (m, 1H), 2.83-2.77 (m, 1H), 1.92-1.78 (m, 2H), 1.60-1.54 (m, 2H).
MS (ESI+) 556 (M⁺+1, 100%).

### Reference Example 92

tert-Butyl {(3R)-1-[3-(2-chloro-5-fluorobenzyl)-5-methyl-4-oxo-4,5-dihydro-3H-imidazo[4,5-c]-1,6-naphthylidin-2-yl]piperidin-3-yl}carbamate

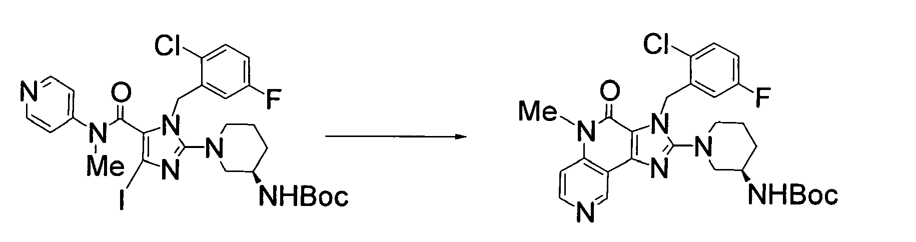

The title compound (15 mg) was synthesized by the same process as in Reference Example 76.
MS (ESI+) 541 (M⁺+1, 100%).

### Reference Example 93

Methyl 7-{(3R)-3-[(tert-butoxycarbonyl)amino]-piperidin-1-yl]-6-(2-chlorobenzyl)-2-(4-methoxybenzyl)-4-methyl-4-oxo-2,4,5,6-tetrahydroimidazo[4,5-d]pyrazolo[4,3-b]pyridine-3-carboxylate

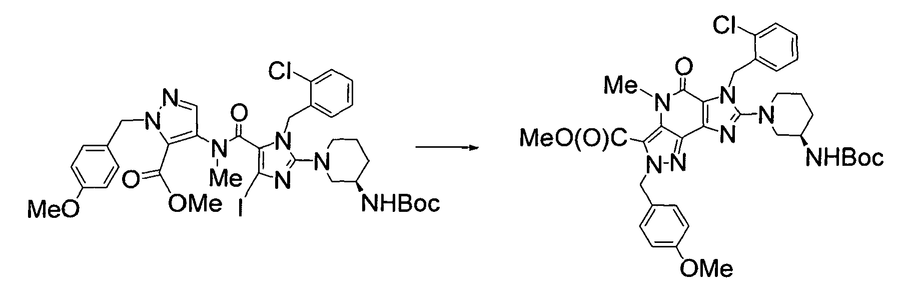

The title compound (179 mg) was synthesized by the same process as in Reference Example 76.
¹H NMR (400 MHz, CDCl₃) δ 7.50 (d, J = 8.6 Hz, 2H), 7.40 (dd, J = 7.9 and 1.3 Hz, 1H), 7.20 (ddd, J = 7.9, 7.9 and 1.3 Hz, 1H), 7.14 (ddd, J = 7.9, 7.9 and 1.3 Hz, 1H), 6.83 (d, J = 8.6 Hz, 2H), 6.73 (d, J = 7.9 Hz, 1H), 5.93 (d, J = 16.5 Hz, 1H), 5.86 (d, J = 16.5 Hz, 1H), 5.81 (d, J = 16.5 Hz, 1H), 5.71 (brd, J = 4.9 Hz, 1H), 5.59 (d, J = 16.5 Hz, 1H), 4.00 (s, 3H), 3.91 (brs, 1H), 3.86 (s, 3H), 3.76 (s, 3H), 3.47 (dd, J = 12.0 and 3.3 Hz, 1H), 3.23-3.18 (m, 1H), 3.07 (brs, 2H), 1.74 (brs, 4H), 1.44 (s, 9H).
MS (ESI+) 690(M⁺ +1, 100%).

### Reference Example 94

Methyl 2-{(3R)-3-[(tert-butoxycarbonyl)amino]-piperidin-1-yl}-3-(2-chlorobenzyl)-5-methyl-4-oxo-4,5-dihydro-3H-imidazo[4,5-d]thieno[3,4-b]pyridine-6-carboxylate

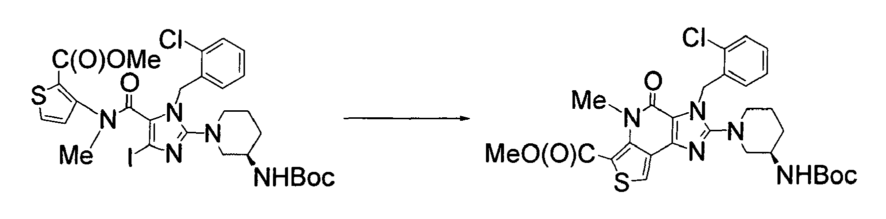

The title compound (238 mg) was synthesized by the same process as in Reference Example 76.
¹H NMR (300 MHz, CDCl₃) δ 8.05 (s, 1H), 7.40 (dd, J= 1.5, 7.7 Hz, 1H), 7.23-7.11 (m, 2H), 6.68 (d, J = 7.3 Hz, 1H), 5.72 (d, J = 16.9 Hz, 1H), 5.60 (d, J = 16.9 Hz, 1H), 5.39 (m, 1H), 3.91 (s, 3H), 3.82 (brs, 1H), 3.72 (s, 3H), 3.42 (dd, J = 3.5, 12.5 Hz, 1H), 3.12-3.03 (m, 3H), 1.74-1.59 (m, 4H), 1.44 (s, 9H).
MS (ESI+) 586 (M⁺+1, 100%).

### Reference Example 95

tert-Butyl {(3R)-1-[3-(2-chloro-5-fluorobenzyl)-5-methyl-4,6-dioxo-4,5,6,8-tetrahydro-3H-furo[3,4-b]imidazo[4,5-d]pyridin-3-yl]carbamate

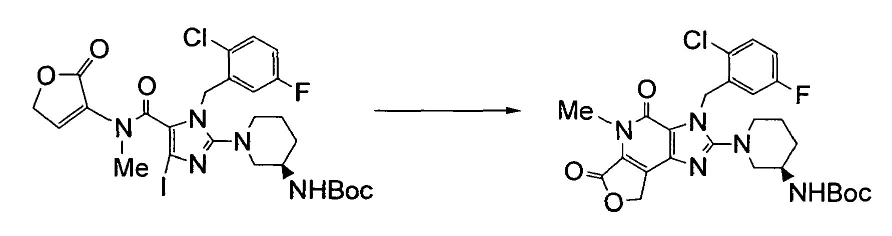

Palladium acetate (76 mg), triphenylphosphine (1688 mg) and silver carbonate (179 mg) were added to a solution (30 mL) of tert-butyl [(3R)-1-[1-(2-chloro-5-fluorobenzyl)-4-iodo-5-{[methyl(2-oxo-2,5-dihydrofuran-3-yl)amino]carbonyl}-1H-imidazo-2-yl]piperidin-3-yl]carbamate (722 mg) in N,N-dimethylformamide, and the resulting mixture was stirred at 160°C. After one and a half hours, the reaction solution was allowed to cool and filtered through Celite, and a saturated aqueous sodium chloride solution was added to the filtrate, followed by extraction with ethyl acetate. The organic layer was dried over sodium sulfate and filtered, and the filtrate was concentrated under reduced pressure. The resulting residue was purified by a silica gel column chromatography (developing solvent: hexane/ethyl acetate = 2/1) to obtain the title compound.

### Reference Example 96

tert-Butyl 2-{(3R)-3-[(tert-butoxycarbonyl)-amino]piperidin-1-yl}-3-(2-chlorobenzyl)-8-fluoro-5-methyl-4-oxo-4,5-dihydro-3H-imidazo[4,5-c]quinoline-7-carboxylate

tert-Butyl 2-{(3R)-3-[(tert-butoxycarbonyl)-amino]piperidin-1-yl}-3-(2-chlorobenzyl)-8-fluoro-5-methyl-4-oxo-4,5-dihydro-3H-imidazo[4,5-c]quinoline-9-carboxylate

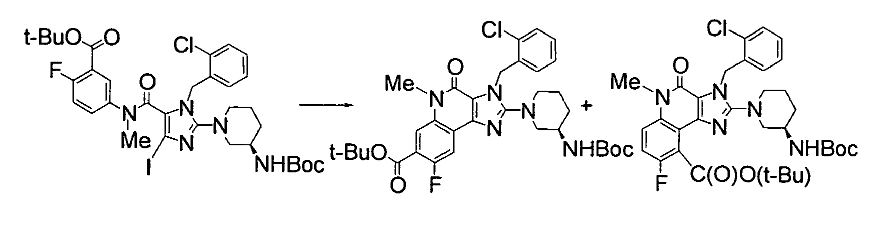

Palladium acetate (10 mg), triphenylphosphine (23 mg) and sodium carbonate (24 mg) were added to a solution (10 mL) of tert-butyl 5-[{[2-{(3R)-3-[(tert-butoxycarbonyl)-amino]piperidin-1-yl}-1-(2-chlorobenzyl)-4-iodo-1H-imidazol-5-yl]carbonyl}(methyl)amino]-2-fluorobenzoate (112 mg) in dimethyl sulfoxide, and the resulting mixture was stirred with heating at 100°C for 30 minutes. The solid was removed by filtration and the residue was concentrated under reduced pressure. The resulting residue was purified by a silica gel column chromatography (developing solvent: hexane/ethyl acetate = 4/1 to 2/1) to obtain the title compounds in amounts of 57 mg and 8 mg, respectively.

tert-Butyl 2-{(3R)-3-[(tert-butoxycarbonyl)-amino]piperidin-1-yl}-3-(2-chlorobenzyl)-8-fluoro-5-methyl-4-oxo-4,5-dihydro-3H-imidazo[4,5-c]quinoline-7-carboxylate: ¹H NMR (400 MHz, CDCl₃) δ 7.98-7.95 (m, 2H), 7.42 (dd, J = 7.9 and 1.3 Hz, 1H), 7.21 (ddd, J = 7.9, 7.9 and 1.3 Hz, 1H), 7.14 (ddd, J = 7.9, 7.9 and 1.3 Hz, 1H), 6.69 (d, J = 7.9 Hz, 1H), 6.10 (brs, 1H), 5.77 (d, J = 16.5 Hz, 1H), 5.65 (d, J = 16.5 Hz, 1H), 3.81 (brs, 1H), 3.76 (s, 3H), 3.43 (dd, J = 12.1 and 3.4 Hz, 1H), 3.26-3.21 (m, 1H), 3.07 (brs, 3H), 1.74 (brs, 3H), 1.65 (s, 9H), 1.47 (s, 9H).
MS (ESI+) 640(M⁺ +1, 100%).

tert-Butyl 2-{(3R)-3-[(tert-butoxycarbonyl)-amino]piperidin-1-yl}-3-(2-chlorobenzyl)-8-fluoro-5-methyl-4-oxo-4,5-dihydro-3H-imidazo[4,5-c]quinoline-9-carboxylate: ¹H NMR (400 MHz, CDCl₃) δ 7.43-7.39 (m, 2H), 7.24 (dd, J = 7.9 and 1.3 Hz, 1H), 7.20 (ddd, J = 7.9, 7.9 and 1.3 Hz, 1H), 7.13 (ddd, J = 7.9, 7.9 and 1.3 Hz, 1H), 6.69 (d, J = 7.9 Hz, 1H), 5.83 (d, J = 16.5 Hz, 1H), 5.66 (d, J = 16.5 Hz, 1H), 4.65 (d, J = 6.6 Hz, 1H), 3.78 (brs, 1H), 3.73 (s, 3H), 3.38 (brs, 1H), 3.26-3.20 (m, 1H), 2.99 (brs, 3H), 1.71 (s, 9H), 1.66-1.63 (m, 3H), 1.43 (s, 9H).
MS (ESI+) 640(M⁺ +1, 100%).

### Reference Example 97

tert-Butyl 2-{(3R)-3-[(tert-butoxycarbonyl)-amino]piperidin-1-yl}-3-(5-fluoro-2-methylbenzyl)-5-methyl-4-oxo-4,5-dihydro-3H-imidazo[4,5-c]quinoline-7-carboxylate

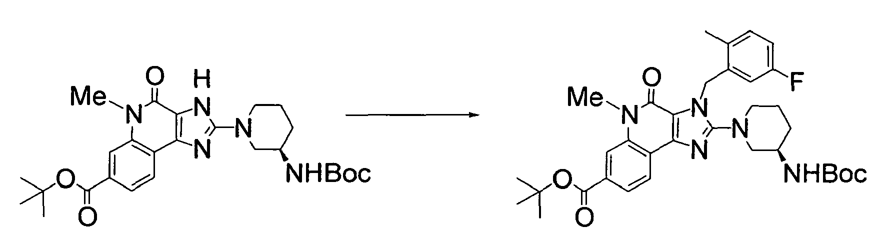

2-Methyl-5-fluorobenzyl bromide (108 mg) and potassium carbonate (98 mg) were added to a solution (6 mL) of tert-butyl 2-{(3R)-3-[(tert-butoxycarbonyl)amino]piperidin-1-yl}-5-methyl-4-oxo-4,5-dihydro-3H-imidazo[4,5-c]quinoline-7-carboxylate (177 mg) in N,N-dimethylformamide, and the resulting mixture was stirred at room temperature for 7 hours. After completion of the reaction, water was added to the reaction mixture, followed by extraction with ethyl acetate (100 mL). The organic layer was washed with a saturated aqueous sodium hydrogencarbonate solution and a saturated aqueous sodium chloride solution. The organic layer was dried over sodium sulfate and filtered, and the filtrate was concentrated under reduced pressure. The resulting residue was purified by a silica gel column chromatography (developing solvent: ethyl acetate/hexane = 1/4 to 1/1) to obtain the title compound (62 mg) as a white solid.
¹H NMR (300 MHz, CDCl₃) δ 8.31 (d, J = 8.0Hz, 1H), 8.12 (s, 1H), 7.92 (d, J = 7.9Hz, 1H), 7.14 (d, J = 5.8, 8.3Hz, 1H), 6.86-6.80 (m, 1H), 6.25 (dd, J = 2.4, 9.7Hz, 1H), 5.99-5.97 (m, 1H), 5.63 (d, J = 16.7Hz, 1H), 5.48 (d, J = 16.7Hz, 1H), 3.82-3.78 (m, 1H), 3.78 (s, 3H), 3.45 (dd, J = 3.2, 12.7Hz, 1H), 3.19-3.11 (m, 3H), 2.39 (s, 3H), 1.86-1.55 (m, 4H), 1.65 (s, 9H), 1.46 (s, 9H).
MS (ESI+) 620 (M⁺+1, 100%).

### Reference Example 98

2-[(3R)-3-(tert-Butoxycarbonyl)amino]piperidin-1-yl}-3-(2-chloro-5-fluorobenzyl)-5-methyl-4-oxo-4,5-dihydro-3H-imidazo[4,5-c]quinoline-7-carboxylic acid

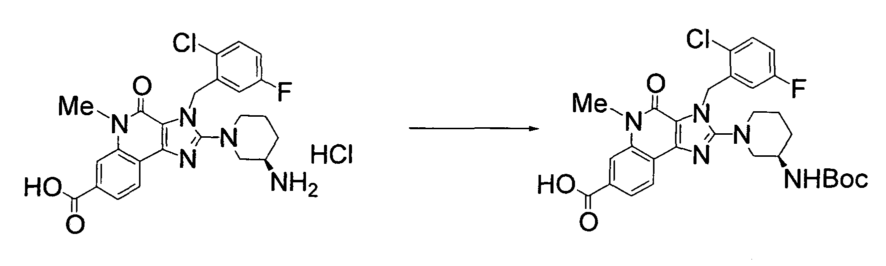

Di-tert-butyl dicarbonate (420 mg) was added to a solution of 2-[(3R)-3-aminopiperidin-1-yl]-3-(2-chloro-5-fluorobenzyl)-5-methyl-4-oxo-4,5-dihydro-3H-imidazo[4,5-c]quinoline-8-carboxylic acid hydrochloride (830 mg) in a mixture of 1,4-dioxane (10 mL) and a saturated aqueous sodium hydrogencarbonate solution (10 mL), and the resulting mixture was stirred overnight at room temperature. The reaction solution was adjusted to pH 2 by pouring thereto a 10% aqueous potassium hydrogensulfate solution and was extracted with ethyl acetate. The organic layer was dried over sodium sulfate and filtered, and the filtrate was concentrated under reduced pressure to obtain the title compound (620 mg) as a white solid.
MS (ESI+) 584 (M⁺+1, 100%).

### Reference Example 99

tert-Butyl {(3R)-1-[3-(2-chloro-5-fluorobenzyl)-7-cyano-5-methyl-4-oxo-4,5-dihydro-3H-imidazo[4,5-c]quinolin-2-yl]piperidin-3-yl}carbamate

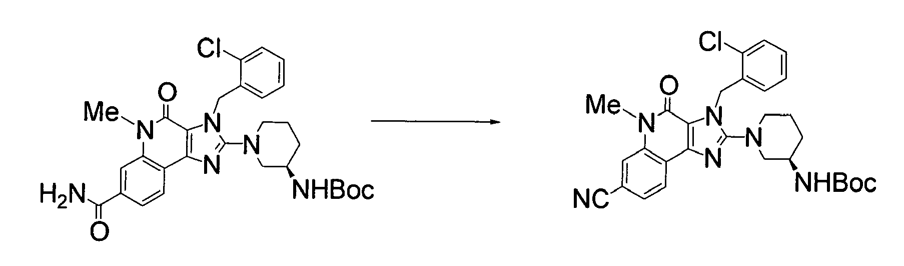

Trifluoroacetic anhydride (44 µL) was added dropwise to a solution of tert-butyl {(3R)-1-[7-(aminocarbonyl)-3-(2-chloro-5-fluorobenzyl)-5-methyl-4-oxo-4,5-dihydro-3H-imidazo[4,5-c]quinolin-2-yl]piperidin-3-yl}carbamate (44.2 mg) in tetrahydrofuran (1.1 mL), and the resulting mixture was stirred at room temperature for 2 hours. After the reaction, the reaction mixture was concentrated under reduced pressure and the residue was dissolved in methanol (1.1 mL). Potassium carbonate (33.9 mg) and water (20 µL) were added thereto and the resulting mixture was stirred at room temperature. After 12 hours, the reaction mixture was concentrated under reduced pressure and the resulting residue was purified by a thin-layer chromatography (developing solvent: hexane/ethyl acetate = 1/1) to obtain the title compound (26.3 mg) as a white solid.
MS (ESI+) 547 (M⁺+1, 100%).

### Reference Example 100

tert-Butyl {(3R)-1-[7-(aminocarbonyl)-3-(2-chloro-5-fluorobenzyl)-5-methyl-4-oxo-4,5-dihydro-3H-imidazo[4,5-c]quinolin-2-yl]piperidin-3-yl}carbamate

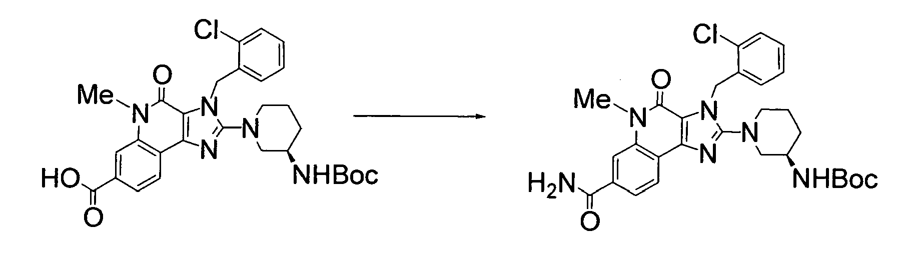

The title compound (71.8 mg) was synthesized by the same process as in Reference Example 51.
MS (ESI+) 565 (M⁺+1, 100%).

### Reference Example 101

tert-Butyl {(3R)-1-[8-(aminocarbonyl)-3-(2-chlorobenzyl)-5-methyl-4-oxo-4,5-dihydro-3H-imidazo[4,5-c]quinolin-2-yl]piperidin-3-yl}carbamate

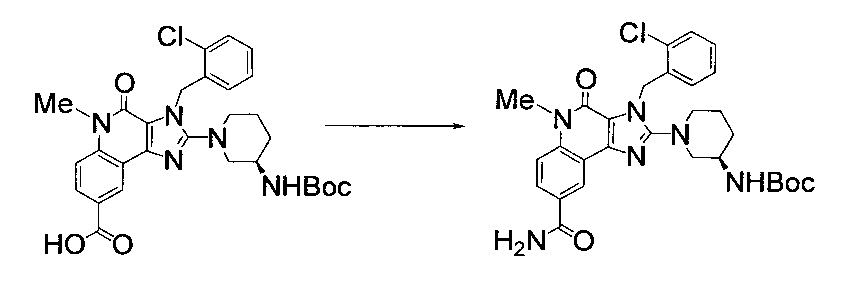

The title compound (13.5 mg) was synthesized by the same process as in Reference Example 51.
MS (ESI+) 565 (M⁺+1, 100%).

### Reference Example 102

tert-Butyl {(3R)-1-[3-(2-chlorobenzyl)-5-methyl-8-(morpholin-4-ylcarbonyl)-4-oxo-4,5-dihydro-3H-imidazo[4,5-c]quinolin-2-yl]piperidin-3-yl}carbamate

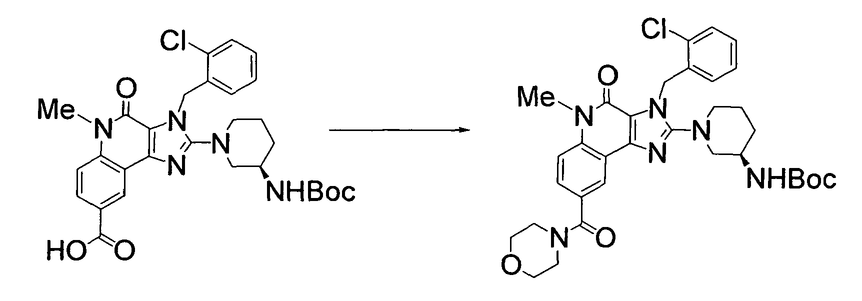

2-{(3R)-3-[(tert-butoxycarbonyl)amino]piperidin-1-yl}-3-(2-chlorobenzyl)-5-methyl-4-oxo-4,5-dihydro-3H-imidazo[4,5-c]quinoline-8-carboxylic acid (81.3 mg) was dissolved in N,N-dimethylformamide (1.2 mL), followed by adding thereto 1-hydroxybenzotriazole (35 mg), 1-ethyl-3-(dimethylaminopropyl)carbodiimide hydrochloride (47 mg), triethylamine (100 µL) and morpholine (19 µL), and the resulting mixture was stirred at 25°C for 18 hours. A saturated aqueous ammonium chloride solution was added to the reaction mixture, followed by extraction with ethyl acetate. The organic layer was dried over anhydrous sodium sulfate and filtered, and the filtrate was concentrated under reduced pressure to obtain the title compound (61.6 mg) as a white solid.
MS (ESI+) 635 (M⁺+1, 100%).

### Reference Example 103

tert-Butyl ((3R)-1-(3-(2-chlorobenzyl)-8-[(dimethylamino)carbonyl]-5-methyl-4-oxo-4,5-dihydro-3H-imidazo[4,5-c]quinolin-2-yl}piperidin-3-yl)carbamate

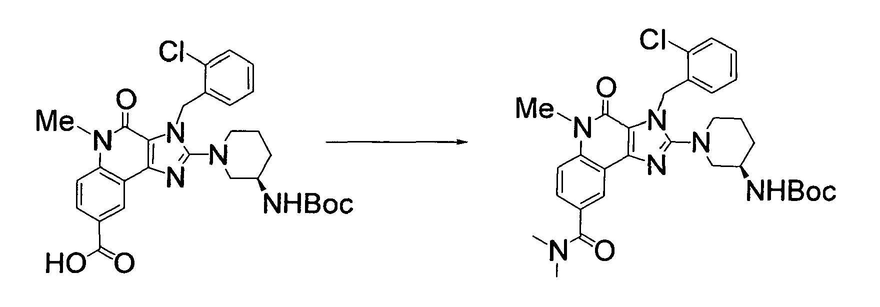

2-((3R)-3-[(tert-butoxycarbonyl)amino]piperidin-1-yl}-3-(2-chlorobenzyl)-5-methyl-4-oxo-4,5-dihydro-3H-imidazo[4,5-c]quinoline-8-carboxylic acid (82.4 mg) was dissolved in N,N-dimethylformamide (1.2 mL), followed by adding thereto 1-hydroxybenzotriazole (40 mg), 1-ethyl-3-(dimethylaminopropyl)carbodiimide hydrochloride (48 mg), triethylamine (102 µL) and a 40% aqueous dimethylamine solution (17 µL), and the resulting mixture was stirred at 25°C for 15 hours. A saturated aqueous ammonium chloride solution was added to the reaction mixture, followed by extraction with chloroform. The organic layer was dried over sodium sulfate and filtered, and the filtrate was concentrated under reduced pressure. The resulting residue was purified by a silica gel column chromatography (developing solvent: chloroform/methanol = 10/1) to obtain the title compound (80.0 mg) as a white solid.
MS (ESI+) 593 (M⁺+1, 100%).

### Reference Example 104

tert-Butyl {(3R)-1-[3-(2-chlorobenzyl)-7-(methoxymethyl)-5-methyl-4-oxo-4,5-dihydro-3H-imidazo[4,5-c]quinolin-2-yl]piperidin-3-yl}carbamate

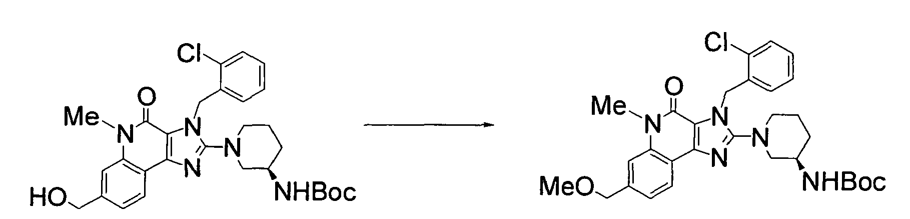

Sodium hydride (9 mg) was added to a solution of tert-butyl {(3R)-1-[3-(2-chlorobenzyl)-7-(hydroxymethyl)-5-methyl-4-oxo-4,5-dihydro-3H-imidazo[4,5-c]quinolin-2-yl]piperidin-3-yl}carbamate (84.5 mg) in tetrahydrofuran (1.0 mL) at 0°C and stirred for 20 minutes. Iodomethane (14 µL) was added dropwise thereto and the resulting mixture was stirred at room temperature for 18 hours. A saturated aqueous ammonium chloride solution was added to the reaction mixture, followed by extraction with ethyl acetate. The organic layer was dried over sodium sulfate and filtered, and the filtrate was concentrated under reduced pressure. The resulting residue was purified by a silica gel column chromatography (developing solvent: hexane/ethyl acetate = 1/1) to obtain the title compound (72 mg) as a white solid.
¹H NMR (300 MHz, CDCl₃) δ 8.28 (d, J = 8.0 Hz, 1H), 7.43 (brs, 1H), 7.40 (dd, J = 1.5, 8.0 Hz, 1H), 7.28 (d, J = 8.0 Hz, 1H), 7.22-7.09 (m, 2H), 6.68 (d, J = 6.4 Hz, 1H), 6.27 (m, 1H), 5.77 (d, J = 16.9 Hz, 1H), 5.66 (d, J = 16.9 Hz, 1H), 4.62 (s, 2H), 3.81-3.69 (m, 1H), 3.75 (s, 3H), 3.46 (s, 3H), 3.44-3.40 (m, 1H), 3.27-3.22 (m, 1H), 3.09-3.07 (m, 2H), 1.73 (m, 4H), 1.47 (s, 9H).
MS (ESI+) 566 (M⁺+1, 100%).

### Reference Example 105

tert-Butyl {(3R)-1-[3-(2-chlorobenzyl)-7-(hydroxymethyl)-5-methyl-4-oxo-4,5-dihydro-3H-imidazo[4,5-c]quinolin-2-yl]piperidin-3-yl]carbamate

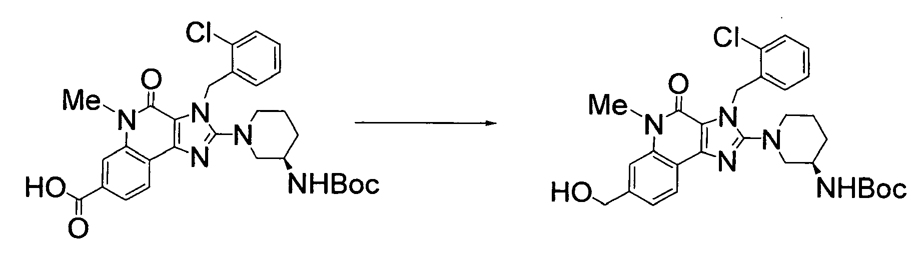

The title compound (86.5 mg) was synthesized by the same process as in Reference Example 64.
¹H NMR (300 MHz, CDCl₃) δ 8.22 (d, J = 8.1 Hz, 1H), 7.41-7.39 (m, 2H), 7.28-7.26 (m, 1H), 7.22-7.09 (m, 2H), 6.67 (d, J = 6.6 Hz, 1H), 6.14 (m, 1H), 5.76 (d, J = 16.9 Hz, 1H), 5.64 (d, J = 16.9 Hz, 1H), 4.83 (brs, 2H), 3.81 (m, 1H), 3.75-3.68 (m, 1H), 3.69 (s, 3H), 3.48-3.41 (m, 1H), 3.30-3.22 (m, 1H), 3.09-3.07 (m, 2H), 1.73-1.42 (m, 4H), 1.47 (s, 9H).
MS (ESI+) 552 (M⁺+1, 100%).

### Reference Example 106

Ethyl 2-{(3R)-3-[(tert-butoxycarbonyl)amino]-piperidin-1-yl}-3-(2-chlorobenzyl)-5-methyl-4-oxo-4,5-dihydro-3H-imidazo[4,5-c]quinoline-7-carboxylate

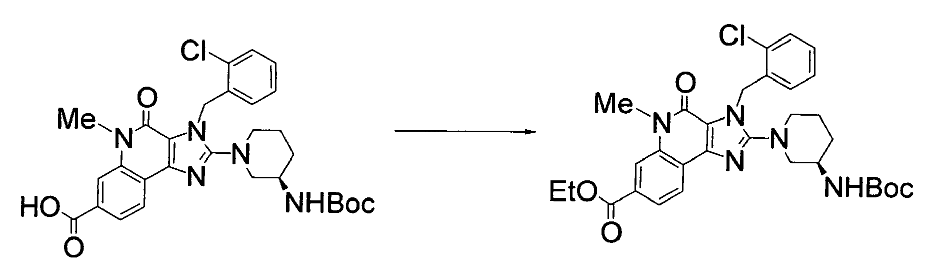

1-Hydroxybenzotriazole (67 mg), 1-ethyl-3-(dimethylaminopropyl)carbodiimide hydrochloride (103 mg) and ethanol (0.5 mL) were added to a solution of 2-{(3R)-3-[(tert-butoxycarbonyl)amino]piperidin-1-yl}-3-(2-chlorobenzyl)-5-methyl-4-oxo-4,5-dihydro-3H-imidazo[4,5-c]quinoline-7-carboxylic acid (125 mg) in N,N-dimethylformamide (2 mL), and the resulting mixture was stirred at 25°C for 21 hours. A saturated aqueous ammonium chloride solution was added to the reaction mixture, followed by extraction with ethyl acetate. The organic layer was dried over sodium sulfate and filtered, and the filtrate was concentrated under reduced pressure. The residue was purified by a silica gel column chromatography (developing solvent: hexane/ethyl acetate = 3/1) to obtain the title compound (117 mg) as a white solid.
¹H NMR (300 MHz, CDCl₃) δ 8.34 (d, J = 8.2 Hz, 1H), 8.16 (d, J = 1.1 Hz, 1H), 7.98 (dd, J = 1.1, 8.2 Hz, 1H), 7.41 (dd, J = 1.3, 7.7 Hz, 1H), 7.24-7.11 (m, 2H), 6.69 (d, J = 7.3 Hz, 1H), 6.11 (m, 1H), 5.79 (d, J = 16.8 Hz, 1H), 5.66 (d, J = 16.8 Hz, 1H), 4.45 (dd, J = 7.1, 14.1 Hz, 2H), 3.81 (s, 3H), 3.81-3.76 (m, 1H), 3.45 (dd, J = 3.3, 12.7 Hz, 1H), 3.27-3.23 (m, 1H), 3.10-3.08 (m, 2H), 1.74-1.66 (4H, m), 1.47 (s, 9H), 1.46 (t, J = 7.1 Hz, 3H).
MS (ESI+) 594 (M⁺+1, 100%).

The compounds of Reference Examples 107 to 113 were synthesized from corresponding compounds of Reference Examples, respectively, by the same process as in Reference Example 106.

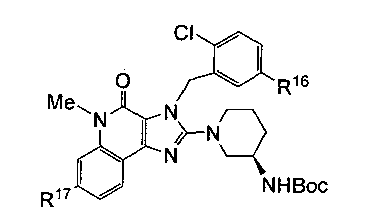

| Refence example number | R¹⁶ | R¹⁷ | Reference example number for starting material |
|---|---|---|---|
| Reference Example 107 | H | 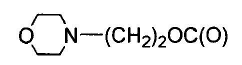 | Reference Example 120 |
| Reference Example 108 | H | CO₂(i-Pr) | Reference Example 120 |
| Reference Example 109 | H | CO₂(i-Bu) | Reference Example 120 |
| Reference Example 110 | H | 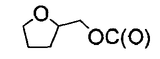 | Reference Example 120 |
| Reference Example 111 | H | CO₂CH(Me)CH(Me)₂ | Reference Example 120 |
| Reference Example 112 | H | 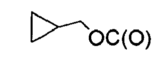 | Reference Example 120 |
| Reference Example 113 | H | CO₂(CH₂)₃OEt | Reference Example 120 |

### Reference Example 107

MS (ESI+) 679 (M⁺+1, 100%).

### Reference Example 108

¹H NMR (400 MHz, CDCl₃) δ 8.33 (d, J = 8.2 Hz, 1H), 8.15 (d, J = 1.1 Hz, 1H), 7.96 (d, J = 8.2 Hz, 1H), 7.41 (dd, J = 1.1, 7.9 Hz, 1H), 7.27-7.12 (m, 2H), 6.69 (d, J = 7.5 Hz, 1H), 6.06 (brs, 1H), 5.78 (d, J = 16.9 Hz, 1H), 5.66 (d, J = 16.9 Hz, 1H), 5.35-5.29 (m, 1H), 3.81 (s, 3H), 3.86-3.78 (m, 1H), 3.45 (dd, J = 3.3, 12.8 Hz, 1H), 3.26-3.23 (m, 1H), 3.10-3.08 (m, 2H), 1.77-1.49 (m, 4H), 1.47 (s, 9H), 1.43 (d, J = 6.3 Hz, 6H).
MS (ESI+) 608 (M⁺+1, 100%).

### Reference Example 109

¹H NMR (300 MHz, CDCl₃) δ 8.34 (d, J = 8.2 Hz, 1H), 8.17 (d, J = 1.3 Hz, 1H), 7.98 (dd, J = 1.3, 8.2 Hz, 1H), 7.41 (dd, J = 1.5, 7.9 Hz, 1H), 7.23-7.11 (m, 2H), 6.69 (d, J = 6.4 Hz, 1H), 6.08 (brd, J = 6.9 Hz, 1H), 5.79 (d, J = 16.8 Hz, 1H), 5.66 (d, J = 16.8 Hz, 1H), 4.17 (d, J = 6.6 Hz, 2H), 3.80 (s, 3H), 3.80-3.76 (m, 1H), 3.44 (dd, J = 3.3, 12.8 Hz, 1H), 3.23 (dd, J = 4.2, 12.6 Hz, 1H), 3.10-3.08 (m, 2H), 1.74-1.69 (m, 4H), 1.47 (s, 9H), 1.06 (d, J = 6.8 Hz, 6H).
MS (ESI+) 622 (M⁺+1, 100%).

### Reference Example 110

¹H NMR (300 MHz, CDCl₃) δ 8.34 (d, J = 8.2 Hz, 1H), 8.17 (d, J= 1.3 Hz, 1H), 8.00 (d, J = 8.2 Hz, 1H), 7.41 (dd, J = 1.3, 8.1 Hz, 1H), 7.23-7.11 (m, 2H), 6.69 (d, J = 7.3 Hz, 1H), 6.06 (m, 1H), 5.79 (d, J = 16.8 Hz, 1H), 5.66 (d, J= 16.8 Hz, 1H), 4.47-4.42 (m, 1H), 4.38-4.32 (m, 2H), 4.04-3.83 (m, 1H), 3.80 (s, 3H), 3.68 (dd, J = 3.3, 11.5 Hz, 1H), 3.54-3.42 (m, 2H), 3.25 (m, 1H), 3.08 (m, 2H), 2.14-1.63 (m, 8H), 1.47 (s, 9H).
MS (ESI+) 650 (M⁺+1, 100%).

### Reference Example 111

¹H NMR (300 MHz, CDCl₃) δ 8.34 (d, J = 8.2 Hz, 1H), 8.16 (d, J = 1.1 Hz, 1H), 7.97 (d, J = 8.2 Hz, 1H), 7.41 (dd, J = 1.3, 8.0 Hz, 1H), 7.24-7.11 (m, 2H), 6.69 (d, J = 6.6 Hz, 1H), 6.05 (m, 1H), 5.79 (d, J = 17.0 Hz, 1H), 5.66 (d, J = 17.0 Hz, 1H), 5.10-5.01 (m, 1H), 3.80 (s, 3H), 3.80 (m, 1H), 3.45 (dd, J = 3.3, 12.8 Hz, 1H), 3.25-3.22 (m, 1H), 3.08 (m, 2H), 2.02-1.94 (m, 1H), 1.74 (m, 4H), 1.42 (s, 9H), 1.35 (d, J = 6.2 Hz, 3H), 1.03 (dd, J = 3.1, 6.8 Hz, 6H).
MS (ESI+) 636 (M⁺+1, 100%).

### Reference Example 112

¹H NMR (300 MHz, CDCl₃) δ 8.34 (d, J = 8.2 Hz, 1H), 8.17 (d, J = 1.1 Hz, 1H), 8.01 (d, J = 8.2 Hz, 1H), 7.41 (dd, J = 1.5, 7.9 Hz, 1H), 7.24-7.11 (m, 2H), 6.69 (d, J = 6.6 Hz, 1H), 6.05 (m, 1H), 5.79 (d, J = 16.8 Hz, 1H), 5.65 (d, J = 16.8 Hz, 1H), 4.23 (d, J = 7.1 Hz, 2H), 3.81 (s, 3H), 3.81 (m, 1H), 3.45 (dd, J = 3.3, 12.8 Hz, 1H), 3.27-3.23 (m, 1H), 3.10-3.08 (m, 2H), 1.74-1.42 (m, 3H), 1.47 (s, 9H), 1.37-1.24 (m, 2H), 0.69-0.63 (m, 2H), 0.49-0.39 (m, 2H).
MS (ESI+) 620 (M⁺+1, 100%).

### Reference Example 113

¹H NMR (300 MHz, CDCl₃) δ 8.34 (d, J = 8.1 Hz, 1H), 8.16 (d, J = 1.3 Hz, 1H), 7.97 (dd, J = 1.3, 8.1 Hz, 1H), 7.41 (dd, J = 1.3, 7.9 Hz, 1H), 7.24-7.11 (m, 2H), 6.69 (d, J = 6.2 Hz, 1H), 6.07 (m, 1H), 5.79 (d, J = 17.0 Hz, 1H), 5.66 (d, J = 17.0 Hz, 1H), 4.50 (t, J = 6.4 Hz, 2H), 3.80-3.77 (m, 1H), 3.80 (s, 3H), 3.66-3.60 (m, 2H), 3.56-3.42 (m, 3H), 3.27-3.23 (m, 1H), 3.10-3.08 (m, 2H), 2.15-2.05 (m, 2H), 1.88-1.80 (m, 2H), 1.47 (s, 9H), 1.28-1.19 (m, 5H).
MS (ESI+) 652 (M⁺+1, 100%).

### Reference Example 114

(5-Methyl-2-oxo-1,3-dioxol-4-yl)methyl 2-[(3R)-3-(tert-butoxycarbonyl)amino]piperidin-1-yl}-3-(2-chloro-5-fluorobenzyl)-5-methyl-4-oxo-4,5-dihydro-3H-imidazo[4,5-c]quinoline-7-carboxylate

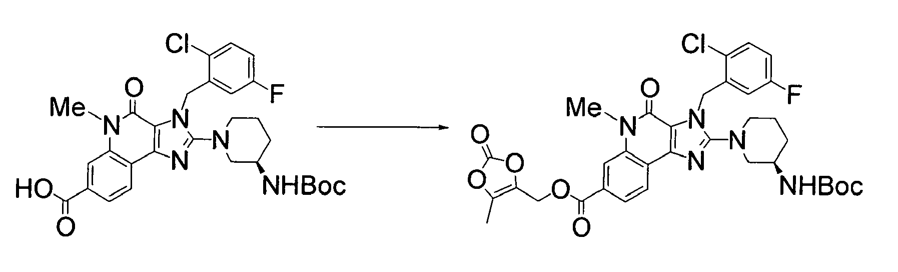

Potassium carbonate (47 mg) and 4-(bromomethyl)-5-methyl-1,3-dioxol-2-one (43 mg) were added to a solution of 2-[(3R)-3-(tert-butoxycarbonyl)amino]piperidin-1-yl}-3-(2-chloro-5-fluorobenzyl)-5-methyl-4-oxo-4,5-dihydro-3H-imidazo[4,5-c]quinoline-7-carboxylic acid (100 mg) in N,N-dimethylformamide (2 mL), and the resulting mixture was stirred overnight at room temperature. Water was added to the reaction solution, followed by extraction with ethyl acetate. The organic layer was dried over sodium sulfate and filtered, and the filtrate was concentrated under reduced pressure. The resulting residue was purified by a silica gel column chromatography (developing solvent: hexane/ethyl acetate = 2/1) to obtain the title compound (54 mg) as a light-yellow solid.
¹_{H} NMR (400 MHz, CDCl₃) δ 8.94 (s, 1H), 8.18 (m, 1H), 7.49 (m, 1H), 7.38 (m, 1H), 6.95 (m, 1H), 6.44 (m, 1H), 5.72-5.57 (m, 2H), 5.15 (s, 2H), 4.42-4.39 (m, 2H), 3.81 (m, 1H), 3.76 (s, 3H), 3.46 (m, 1H), 3.12-3.07 (m, 3H), 2.28 (s, 3H), 1.69 (m, 1H), 1.42 (s, 9H)
MS (ESI+) 696(M⁺ +1, 100%).

The compounds of Reference Examples 115 to 119 were synthesized from corresponding compounds of Reference Examples, respectively, by the same process as in Reference Example 114.

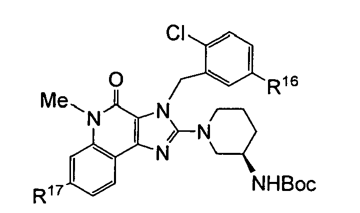

| Reference example number | R¹⁶ | R¹⁷ | Reference example number for starting material |
|---|---|---|---|
| Reference Example 115 | H | t-BuC(O)OCH₂O(O)C | Reference Example 120 |
| Reference Example 116 | F | EtOC(O)OCH(Me)O(O)C | Reference Example 98 |
| Reference Example 117 | F | 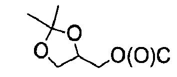 | Reference Example 98 |
| Reference Example 118 | F | Me₂N(CH₂)₂O(O)C | Reference Example 98 |
| Reference Example 119 | F | 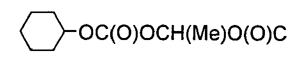 | Reference Example 98 |

### Reference Example 115

¹H NMR (300 MHz, CDCl₃) δ 8.34 (m, 1H), 8.17 (s, 1H), 8.00 (m, 1H), 7.40 (m, 1H), 7.23-7.11 (m, 2H), 6.67, (m, 1H), 6.06 (s, 2H), 5.81-5.63 (m, 2H), 3.80 (s, 3H), 3.47 (m, 1H), 3.25 (m, 1H), 3.08-3.03 (m, 2H), 2.96 (s, 1H), 2.88 (s, 1H), 1.75-1.73 (m, 2H), 1.55 (m, 1H), 1.46 (s, 9H), 1.24 (s, 9H)
MS (ESI+) 680 (M⁺+1, 100%).

### Reference Example 116

¹H NMR (400 MHz, CDCl₃) δ 8.35 (m, 1H), 8.16 (s, 1H), 7.98 (m, 1H), 7.38 (m, 1H), 7.13 (m, 1H), 6.94 (m, 1H), 6.44 (m, 1H), 6.01 (bs, 1H), 5.73-5.58 (m, 2H), 4.26 (q, J = 7.16 Hz, 2H), 3.85 (m, 1H), 3.80 (s, 3H), 3.46 (m, 1H), 3.25-3.05 (m, 3H), 1.82-1.95 (m, 2H), 1.78-1.76 (m, 3H), 1.59 (m, 1H), 1.46 (s, 9H), 1.33 (t, J = 7.16 Hz, 3H)
MS (ESI+) 700 (M⁺+1, 100%).

### Reference Example 117

MS (ESI+) 698.6 (M⁺+1, 100%).

### Reference Example 118

¹H NMR (400 MHz, CD₃OD) δ 8.34 (m, 1H), 8.17 (m, 1H), 7.98 (m, 1H), 7.36 (m, 1H), 6.92 (m, 1H), 6.43 (m, 1H), 6.04 (bs, 1H), 5.73-5.59 (m, 2H), 4.51 (t, J = 5.84 Hz, 2H), 3.82 (m, 1H), 3.80 (s, 3H), 3.43 (m, 1H), 3.22-3.07 (m, 3H), 2.78 (t, J = 5.84 Hz, 2H), 2.38 (s, 6H), 1.86-1.77 (m, 3H), 1.58 (m, 1H), 1.45 (s, 9H)
MS (ESI+) 655 (M⁺+1, 100%).

### Reference Example 119

¹H NMR (400 MHz, CD₃OD) δ 8.35 (m, 1H), 8.16 (s, 1H), 7.98 (m, 1H), 7.36 (m, 1H), 7.11 (m, 1H), 6.94 (m, 1H), 6.42 (m, 1H), 6.03 (bs, 1H), 5.69-5.63 (m, 2H), 4.66 (m, 1H), 3.85 (m, 1H), 3.81 (s, 3H), 3.44 (m, 1H), 3.28-3.08 (m, 3H), 1.95-1.93 (m, 2H), 1.77-1.73 (m, 4H), 1.70-1.68 (m, 3H), 1.61-1.53 (m, 4H), 1.46 (s, 9H), 1.41-1.26 (m, 4H)
MS (ESI+) 754 (M⁺+1, 100%).

### Reference Example 120

2-{(3R)-3-[(tert-Butoxycarbonyl)amino]piperidin-1-yl}-3-(2-chlorobenzyl)-5-methyl-4-oxo-4,5-dihydro-3H-imidazo[4,5-c]quinoline-7-carboxylic acid

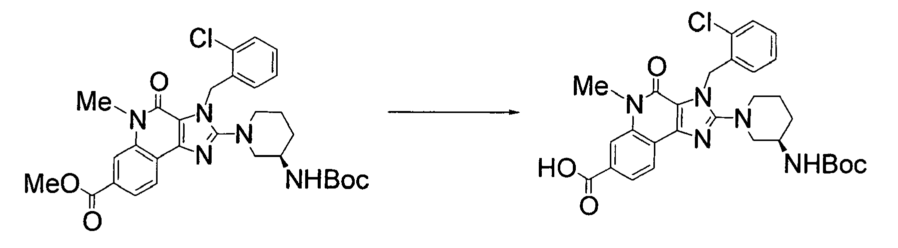

A solution consisting of methyl 2-{(3R)-3-[(tert-butoxycarbonyl)amino]piperidin-1-yl}-3-(2-chlorobenzyl)-5-methyl-4-oxo-4,5-dihydro-3H-imidazo[4,5-c]quinoline-7-carboxylate (313 mg), 2N sodium hydroxide (4 mL) and ethanol (7 mL) was stirred at 80°C for 2 hours. After the reaction mixture was concentrated under reduced pressure, a saturated aqueous ammonium chloride solution was added thereto, followed by extraction with ethyl acetate. The organic layer was dried over anhydrous sodium sulfate and filtered, and the filtrate was concentrated under reduced pressure to obtain the title compound (247 mg) as a white solid.
¹H NMR (300 MHz, CDCl₃) δ 8.34 (d, J = 8.3 Hz, 1H), 8.17 (brs, 1H), 8.01 (dd, J = 1.3, 8.3 Hz, 1H), 7.41 (dd, J = 1.3, 7.7 Hz, 1H), 7.28-7.11 (m, 2H), 6.71 (dd, J = 1.5, 7.5 Hz, 1H), 6.01 (d, J = 7.0 Hz, 1H), 5.79 (d, J = 17.0 Hz, 1H), 5.67 (d, J = 17.0 Hz, 1H), 3.79 (s, 3H), 3.79 (m, 1H), 3.49 (dd, J = 3.3, 12.6 Hz, 1H), 3.23 (dd, J = 5.5, 12.4 Hz, 1H), 3.09 (m, 2H), 1.76-1.58 (m, 4H), 1.48 (s, 9H).
MS (ESI+) 566 (M⁺+1, 100%).

The compounds of Reference Examples 121 to 128 were synthesized from corresponding compounds of Reference Examples, respectively, by the same process as in Reference Example 120.

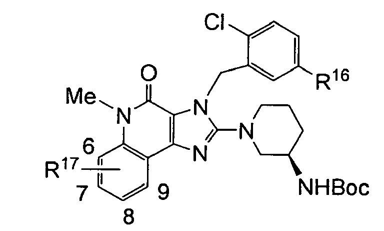

| Reference example number | R¹⁶ | R¹⁷ | Reference example number for starting material |
|---|---|---|---|
| Reference Example 121 | H | 8-CO₂H | Reference Example 79 |
| Reference Example 122 | F | 8-CO₂H | Reference Example 83 |
| Reference Example 123 | H | 6-CO₂H | Reference Example 76 |
| Reference Example 124 | H | 7,9-CO₂H | Reference Example 80 |
| Reference Example 125 | H | 8-CH₂CO₂H | Reference Example 87 |
| Reference Example 126 | F | 7-MeO/8-CO₂H | Reference Example 88 |
| Reference Example 127 | F | 6-MeO/8-CO₂H | Reference Example 89 |
| Reference Example 128 | F | 7,9-CO₂H | Reference Example 91 |

### Reference Example 121

¹H NMR (300 MHz, CDCl₃) δ 9.49 (brs, 1H), 8.21 (d, J = 2.9 Hz, 1H), 7.48-7.42 (m, 2H), 7.26-7.13 (m, 2H), 6.72 (d, J = 7.1 Hz, 1H), 5.85 (d, J = 16.7 Hz, 1H), 5,70 (d, J = 16.7 Hz, 1H), 4.94 (m, 1H), 3.85 (brs, 1H), 3.76 (s, 3H), 3.71-3.67 (m, 1H), 3.31-3.23 (m, 3H), 1.92 (brs, 1H), 1.69-1.63 (m, 3H), 1.43 (s, 9H).
MS (ESI+) 566 (M⁺+1, 100%).

### Reference Example 122

¹H NMR (400 MHz, CDCl₃) δ 9.49 (s, 1H), 8.25 (m, 1H), 7.52 (m, 1H), 7.40 (m, 1H), 6.95 (m, 1H), 6.46 (m, 1H), 5.80-5.64 (m, 2H), 4.91 (bs, 1H), 3.86 (bs, 1H), 3.78 (s, 3H), 3.65 (m, 1H), 3.25 (s, 3H), 1.93 (m, 1H), 1.79 (m, 1H), 1.70 (m, 1H), 1.59 (m, 1H), 1.42 (s, 9H).
MS (ESI+) 584 (M⁺+1, 100%).

### Reference Example 123

¹H NMR (300 MHz, CDCl₃) δ 8.36 (d, J = 7.9 Hz, 1H), 7.72 (m, 1H), 7.36 (d, J = 7.9 Hz, 1H), 7.26-7.04 (m, 3H), 6.67 (d, J = 7.3 Hz, 1H), 6.08 (m, 1H), 5.68 (d, J = 17.0 Hz, 1H), 5.55 (d, J = 17.0 Hz, 1H), 3.82 (m, 1H), 3.60 (s, 3H), 3.51-3.44 (m, 1H), 3.25-3.22 (m, 2H), 3.07 (m, 1H), 1.73-1.46 (m, 4H), 1.46 (s, 9H).
MS (ESI+) 566(M⁺ +1, 100%).

### Reference Example 124

MS (ESI+) 610 (M⁺+1, 100%).

### Reference Example 125

¹H NMR (300 MHz, CDCl₃) δ 8.25 (s, 1H), 7.49-7.37 (m, 3H), 7.18-7.10 (m, 2H), 6.61 (d, J = 7.9Hz, 1H), 5.77 (d, J = 16.7Hz, 1H), 5.63 (d, J = 16.7Hz, 1H), 5.22-5.19 (m, 1H), 3.80 (s, 2H), 3.77-3.73 (m, 1H), 3.71 (s, 3H), 3.46-3.42 (m, 1H), 3.09-3.03 (m, 3H), 1.74-1.60 (m, 4H), 1.43 (s, 9H).
MS (ESI+) 580 (M⁺+1, 100%).

### Reference Example 126

¹H NMR (300 MHz, DMSO-d₆) δ 8.44 (s, 1H), 7.54 (dd, J = 5.0 and 8.6 Hz, 1H), 7.16 (td, J = 2.9 and 8.4 Hz, 1H), 7.03 (s, 1H), 6.90 (d, J = 7.7 Hz, 1H), 6.58 (dd, J = 2.6 and 9.3 Hz, 1H), 5.46 (dd, J = 17.9 and 21.6 Hz, 2H), 3.96 (s, 3H), 3.63 (s, 3H), 3.55-3.20 (m, 3H), 2.90-2.78 (m, 1H), 2.76-2.64 (m, 1H), 1.80-1.60 (m, 2H), 1.60-1.40 (m, 2H), 1.31 (s, 9H).
MS (ESI+) 614 (M⁺+1, 100%).

### Reference Example 127

¹H NMR (300 MHz, DMSO-d₆) δ 8.33 (d, J = 1.8 Hz, 1H), 7.57 (d, J = 1.8 Hz, 1H), 7.53 (dd, J = 5.1 and 8.8 Hz, 1H), 7.15 (td, J = 2.9 and 8.4 Hz, 1H), 6.89 (d, J = 7.7 Hz, 1H), 6.62 (dd, J = 2.9 and 8.8 Hz, 1H), 5.48 (dd, J = 17.6 and 22.3 Hz, 2H), 3.93 (s, 3H), 3.79 (s, 3H), 3.55-3.20 (m, 3H), 2.90-2.78 (m, 1H), 2.76-2.64 (m, 1H), 1.80-1.60 (m, 2H), 1.60-1.40 (m, 2H), 1.25 (s, 9H).
MS (ESI+) 614 (M⁺+1, 100%).

### Reference Example 128

MS (ESI+) 628 (M⁺+1, 100%).

### Reference Example 129

2-{(3R)-3-[(tert-Butoxycarbonyl)amino]piperidin-1-yl}-3-(2-chlorobenzyl)-5-methyl-4-oxo-4,5-dihydro-3H-imidazo[4,5-d]thieno[3,4-b]pyridine-6-carboxylic acid

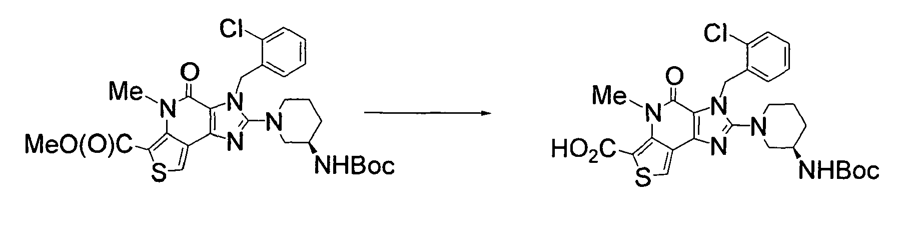

The title compound (120 mg) was obtained by the same process as in Reference Example 120.
¹H NMR (300 MHz, CDCl₃) δ 8.11 (s, 1H), 7.40 (d, J = 7.5 Hz, 1H), 7.22-7.12 (m, 2H), 6.71 (d, J = 7.3 Hz, 1H), 5.73 (d, J = 16.7 Hz, 1H), 5.59 (d, J = 16.7 Hz, 1H), 5.24 (d, J = 7.0 Hz, 1H), 3.82 (m, 1H), 3.74 (s, 3H), 3.51-3.48 (m, 1H), 3.06 (m, 3H), 1.80-1.44 (m, 4H), 1.44 (s, 9H).
MS (ESI+) 572 (M⁺+1, 100%).

### Reference Example 130

Methyl 2-[{[2-{(3R)-3-[(tert-butoxycarbonyl)-amino]piperidin-1-yl}-1-(2-chlorobenzyl)-4-iodo-1H-imidazol-5-yl]carbonyl}(methyl)amino]benzoate

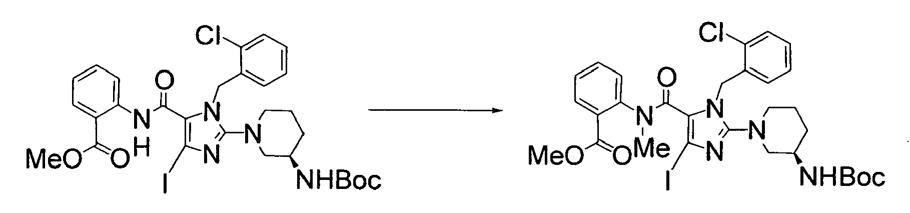

Potassium carbonate (227 mg) and methyl iodide (95 µL) were added to a solution of methyl 2-({[2-{(3R)-3-[(tert-butoxycarbonyl)amino]piperidin-1-yl}-1-(2-chlorobenzyl)-4-iodo-1H-imidazol-5-yl]carbonyl}amino)benzoate (425 mg) in N,N-dimethylformamide (4 mL), and the resulting mixture was stirred at 25°C for 86 hours. After the reaction, a saturated aqueous sodium chloride solution was added to the reaction mixture, followed by extraction with ethyl acetate. The organic layer was washed with a saturated aqueous sodium chloride solution, dried over anhydrous sodium sulfate and then filtered, and the filtrate was concentrated under reduced pressure. The resulting residue was purified by a silica gel column chromatography (developing solvent: ethyl acetate/hexane = 1/3) to obtain the title compound (237 mg) as a white solid.
MS (ESI+) 708 (M⁺+1, 100%).

The compounds of Reference Examples 131 to 146 were synthesized from corresponding compounds of Reference Examples, respectively, by the same process as in Reference Example 130.

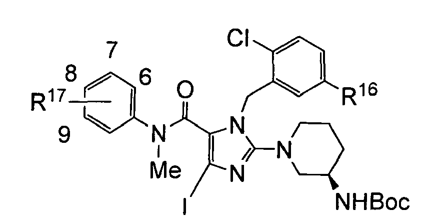

| Reference example number | R¹⁶ | R¹⁷ | Reference example number for starting material |
|---|---|---|---|
| Refence Example 131 | H | 7-CO₂(t-Bu) | Refence Example 153 |
| Refence Example 132 | F | 7,9-CO₂Me | Refence Example 154 |
| Refence Example 133 | H | 7-CO₂Me | Refence Example 155 |
| Refence Example 134 | H | 8-CO₂Me | Refence Example 156 |
| Refence Example 135 | H | 7,9-CO₂Me | Refence Example 157 |
| Refence Example 136 | H | 6-MeO/7-CO₂Et | Refence Example 158 |
| Refence Example 137 | H | 6,8-F/7-CO₂Et | Refence Example 159 |
| Refence Example 138 | F | 8-CO₂Me | Refence Example 160 |
| Refence Example 139 | H | 8-OCHF₂ | Refence Example 161 |
| Refence Example 140 | H | 8-F/7-CO₂(t-Bu) | Refence Example 162 |
| Refence Example 141 | H | 9-OMe/7-CO₂(t-Bu) | Refence Example 163 |
| Refence Example 142 | F | 7-CO₂(t-Bu) | Refence Example 168 |
| Refence Example 143 | H | 8-CH₂CO₂Et | Refence Example 164 |
| Refence Example 144 | F | 7-MeO/8-CO₂Me | Refence Example 165 |
| Refence Example 145 | F | 6-MeO/8-CO₂Me | Refence Example 166 |
| Refence Example 146 | F | 8-F/7-CO₂(t-Bu) | Refence Example 167 |

### Reference Example 131

MS (ESI+) 750 (M⁺+1, 100%).

### Reference Example 132

MS (ESI+) 784 (M⁺+1, 100%).

### Reference Example 133

MS (ESI+) 708 (M⁺+1, 100%).

### Reference Example 134

MS (ESI+) 708 (M⁺+1, 100%).

### Reference Example 135

MS (ESI+) 766 (M⁺+1, 100%).

### Reference Example 136

¹H NMR (400 MHz, CDCl₃) δ 7.74 (brs, 1H), 7.42-7.40 (m, 1H), 7.31-7.21 (m, 3H), 7.17-7.13 (m, 1H), 6.92 (brs, 1H), 5.26 (brs, 2H), 5.02 (brs, 1H), 4.34 (q, J = 7.1 Hz, 2H), 3.66 (brs, 4H), 3.29 (brs, 4H), 2.90 (brs, 3H), 1.77 (brs, 2H), 1.51 (brs, 2H), 1.42 (s, 9H), 1.26 (t, J = 7.1 Hz, 3H).
MS (ESI+) 752 (M⁺+1, 100%).

### Reference Example 137

¹H NMR (400 MHz, CDCl₃) δ 7.42 (d, J = 7.9 Hz, 1H), 7.32-7.21 (m, 3H), 7.14 (brs, 1H), 6.82 (brs, 1H), 5.22 (brs, 2H), 4.91 (brs, 1H), 4.40 (q, J = 7.1 Hz, 2H), 3.80 (brs, 1H), 3.32 (brs, 1H), 3.15 (brs, 3H), 2.99 (brs, 2H), 2.88 (brs, 2H), 1.78 (brs, 2H), 1.69-1.50 (m, 2H), 1.42 (s, 9H), 1.38 (t, J = 7.1 Hz, 3H).
MS (ESI+) 758 (M⁺+1, 100%).

### Reference Example 138

¹H NMR (400 MHz, CDCl₃) δ 7.90-7.84 (m, 2H), 7.42 (m, 1H), 7.03 (m, 1H), 6.95 (m, 1H), 6.70-6.68 (m, 2H), 5.20 (brs, 2H), 4.93 (brs, 1H), 3.90 (s, 3H), 3.79 (brs, 1H), 3.32 (m, 1H), 3.26 (s, 3H), 2.99 (m, 2H), 2.83 (m, 2H), 1.81 (m, 1H), 1.65-1.62 (m, 1H), 1.46 (s, 9H).
MS (ESI+) 726 (M⁺+1, 100%).

### Reference Example 139

MS (ESI+) 716 (M⁺+1, 100%).

### Reference Example 140

¹H NMR (400 MHz, CDCl₃) δ 7.55-7.53 (m, 1H), 7.44-7.42 (m, 1H), 7.35-7.29 (m, 3H), 7.20 (brs, 1H), 6.97 (brs, 1H), 5.28 (brs, 1H), 5.07 (brs, 1H), 4.89 (brs, 1H), 3.81 (brs, 1H), 3.35-3.32 (m, 1H), 3.11 (brs, 3H), 3.01 (brs, 3H), 2.89 (brs, 1H), 1.80 (brs, 3H), 1.59 (s, 9H), 1.42 (s, 9H).
MS (ESI+) 768 (M⁺+1, 100%).

### Reference Example 141

¹H NMR (400 MHz, CD₃OD) δ 7.42-7.37 (m, 2H), 7.34-7.25 (m, 3H), 7.14 (brs, 1H), 6.64 (brs, 1H), 5.20-4.81 (m, 3H), 3.81-3.75 (m, 4H), 3.28 (brs, 1H), 3.12 (brs, 3H), 2.96-2.78 (m, 3H), 1.77 (brs, 3H), 1.59 (s, 9H), 1.54-1.49 (m, 1H), 1.42 (s, 9H).
MS (ESI+) 780 (M⁺+1, 100%).

### Reference Example 142

MS (ESI+) 768 (M⁺+1, 100%).

### Reference Example 143

¹H NMR (400 MHz, CD₃OD) δ 8.17 (d, J = 2.0Hz, 1H), 7.49-7.39 (m, 3H), 7.22-7.09 (m, 2H), 6.65 (d, J = 7.1Hz, 1H), 5.81 (d, J = 17.9Hz, 1H), 5.66 (d, J = 17.9Hz, 1H), 5.50-5.48 (m, 1H), 4.17 (dd, J = 7.1, 14.3Hz, 2H), 3.82-3.80 (m, 1H), 3.76 (s, 2H), 3.74 (s, 3H), 3.45-3.41 (m, 1H), 3.18-3.06 (m, 3H), 1.72-1.58 (m, 4H), 1.45 (s, 9H), 1.27 (d, J = 7.1Hz, 3H).
MS (ESI+) 608 (M⁺+1, 100%).

### Reference Example 144

MS (ESI+) 756 (M⁺+1, 100%).

### Reference Example 145

MS (ESI+) 756 (M⁺+1, 100%).

### Reference Example 146

MS (ESI+) 786(M⁺+1, 100%).

### Reference Example 147

tert-Butyl [(3R)-1-(1-(2-chloro-5-fluorobenzyl)-4-iodo-5-{[methyl(pyridin-4-yl)amino]carbonyl}-1H-imidazol-2-yl)piperidin-3-yl]carbamate

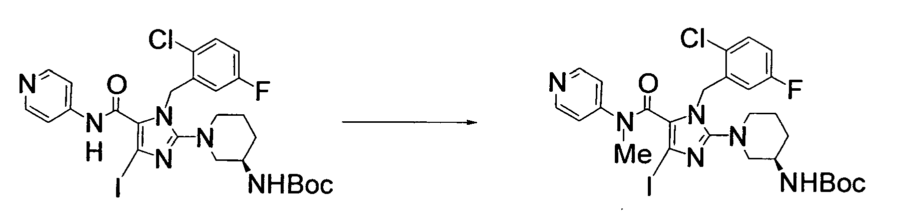

A solution of the compound of Reference Example 169 (250 mg), potassium carbonate (68 mg) and methyl iodide (28 µL) in N,N-dimethylformamide (1 mL) was stirred at 25°C for 3 hours. A saturated aqueous ammonium chloride solution was added to the reaction mixture, followed by extraction with ethyl acetate. The organic layer was washed with water and a saturated aqueous sodium chloride solution, dried over sodium sulfate and then filtered, and the filtrate was concentrated under reduced pressure. The resulting residue was purified by a silica gel chromatography (hexane/ethyl acetate = 2/1-1/2) to obtain the title compound (80 mg) as a colorless amorphous substance.
MS (ESI+) 669 (M⁺+1, 100%).

### Reference Example 148

Methyl 4-[{[2-{(3R)-3-[(tert-butoxycarbonyl)-amino]piperidin-1-yl}-1-(2-chlorobenzyl)-4-iodo-1H-imidazol-5-yl]carbonyl}(methyl)amino]-1-(4-methoxybenzyl)-1H-pyrazole-5-carboxylate

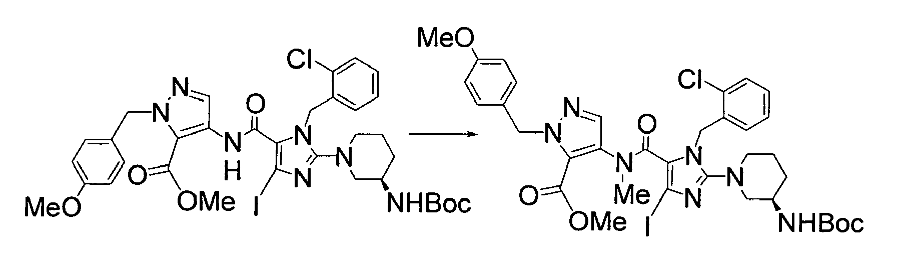

The title compound (265 mg) was synthesized by the same process as in Reference Example 147.
¹H NMR (400 MHz, CDCl₃) δ 7.25-7.12 (m, 6H), 6.92 (d, J = 8.6 Hz, 2H), 6.81 (brs, 1H), 5.17 (bs, 4H), 4.82 (brs, 1H), 3.87-3.72 (m, 10H), 3.22-2.82 (m, 4H), 1.70-1.47 (m, 4H), 1.42 (s, 9H).
MS (ESI+) 818 (M⁺+1, 100%).

### Reference Example 149

Methyl 3-[{[2-{(3R)-3-[(tert-butoxycarbonyl)-amino]piperidin-1-yl}-1-(2-chlorobenzyl)-4-iodo-1H-imidazol-5-yl]carbonyl}(methyl)amino]thiophene-2-carboxylate

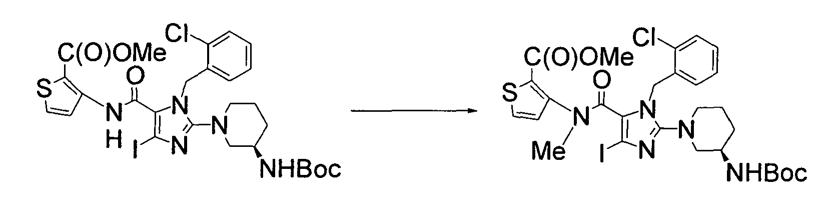

The title compound (526 mg) was synthesized by the same process as in Reference Example 147.
MS (ESI+) 714 (M⁺+1, 100%).

### Reference Example 150

tert-Butyl [(3R)-1-[1-(2-chloro-5-fluorobenzyl)-4-iodo-5-{[methyl(2-oxo-2,5-dihydrofuran-3-yl)amino]carbonyl}-1H-imidazo-2-yl]piperidin-3-yl]carbamate

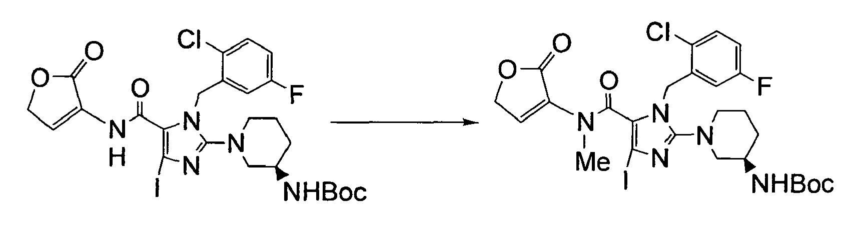

A solution of tert-butyl [(3R)-1-[1-(2-chloro-5-fluorobenzyl)-4-iodo-5-{[(2-oxo-2,5-dihydrofuran-3-yl)-amino]carbonyl}-1H-imidazo-2-yl]piperidin-3-yl]carbamate (750 mg), potassium carbonate (204 mg) and methyl iodide (84 µL) in N,N-dimethylformamide (5 mL) was stirred at 25°C for 3 hours. A saturated aqueous ammonium chloride solution was added to the reaction mixture, followed by extraction with ethyl acetate. The organic phase was washed with water and a saturated aqueous sodium chloride solution, dried over sodium sulfate and then filtered, and the filtrate was concentrated under reduced pressure. The resulting residue was purified by a silica gel chromatography (hexane/ethyl acetate = 2/1→1/2) to obtain the title compound.

### Reference Example 151

tert-Butyl {(3R)-1-[3-(2-chlorobenzyl)-5-methyl-4-oxo-4,5-dihydro-3H-imidazo[4,5-c]quinolin-2-yl]piperidin-3-yl}carbamate

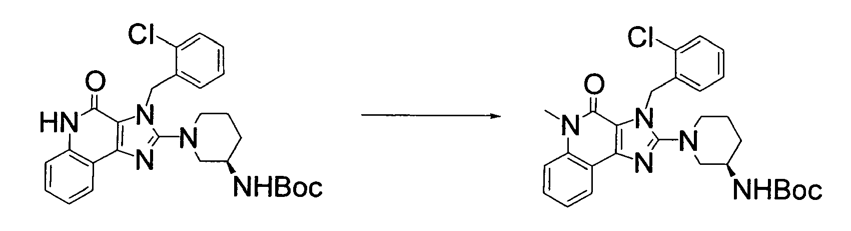

The title compound (16.5 mg) was synthesized by the same process as in Reference Example 147.
MS (ESI+) 522 (M⁺+1, 100%).

### Reference Example 152

Methyl 2-({[2-{(3R)-3-[(tert-butoxycarbonyl)-amino]piperidin-1-yl}-1-(2-chlorobenzyl)-4-iodo-1H-imidazol-5-yl]carbonyl}amino)benzoate

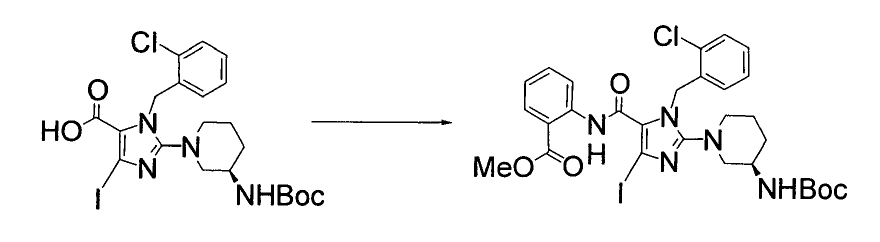

N,N-dimethylformamide (three drops) and oxalyl chloride (0.31 ml) were added to a solution of 2-[(3R)-3-(tert-butoxycarbonyl)amino]piperidin-1-yl}-1-(2-chlorobenzyl)-4-iodo-1H-imidazole-5-carboxylic acid (1.04 g) in dichloromethane (35 mL) at 0°C. After the resulting mixture was stirred at 25°C for 4 hours, the solvent was distilled off by concentration under reduced pressure. Toluene (20 mL), diisopropylethylamine (0.64 mL) and methyl anthranilate (0.36 mL) were added to the residue and the resulting mixture was stirred at 120°C for 8 hours. After the reaction solution was allowed to cool, a saturated aqueous ammonium chloride solution was added thereto, followed by extraction with ethyl acetate. The organic phase was dried over sodium sulfate and filtered, and the filtrate was concentrated under reduced pressure. The resulting residue was purified by a silica gel chromatography (hexane/ethyl acetate = 5/1→1/1) to obtain the title compound (425 mg) as a white solid.
¹H NMR (300 MHz, CDCl₃) δ 11.26 (s, 1H), 8.45 (dd, J = 1.1, 8.4 Hz, 1H), 8.02 (dd, J = 1.7, 8.1 Hz, 1H), 7.51 (ddd, 1.7, 7.6, 8.4 Hz, 1H), 7.34-7.31 (m, 1H), 7.17-7.08 (m, 3H), 6.82-6.79 (m, 1H), 5.46 (d, J = 16.5 Hz, 1H), 5.39 (d, J = 16.5 Hz, 1H), 4.92 (m, 1H), 3.93 (s, 3H), 3.75 (brs, 1H), 3.27 (dd, J = 3.3, 11.9 Hz, 1H), 2.90-2.86 (m, 3H), 1.71-1.52 (m, 4H), 1.43 (s, 9H).
MS (ESI+) 694(M⁺ +1, 100%).

The compounds of Reference Examples 153 to 168 were synthesized from corresponding compounds of Reference Examples, respectively, by the same process as in Reference Example 152.

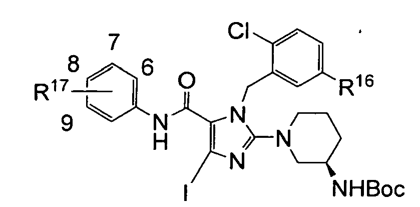

| Reference example number | R¹⁶ | R¹⁷ | Reference example number for starting material |
|---|---|---|---|
| Reference Example 153 | H | 7-CO₂(t-Bu) | Reference Example 173 |
| Reference Example 154 | F | 7,9-CO₂Me | Reference Example 174 |
| Reference Example 155 | H | 7-CO₂Me | Reference Example 173 |
| Reference Example 156 | H | 8-CO₂Me | Reference Example 173 |
| Reference Example 157 | H | 7,9-CO₂Me | Reference Example 173 |
| Reference Example 158 | H | 6-MeO/7-CO₂Et | Reference Example 173 |
| Reference Example 159 | H | 6,8-F/7-CO₂Et | Reference Example 173 |
| Reference Example 160 | F | 8-CO₂Me | Reference Example 174 |
| Reference Example 161 | H | 8-OCHF₂ | Reference Example 173 |
| Reference Example 162 | H | 8-F/7-CO₂(t-Bu) | Reference Example 173 |
| Reference Example 163 | H | 9-OMe/7-CO₂(t-Bu) | Reference Example 173 |
| Reference Example 164 | H | 8-CH₂CO₂Et | Reference Example 173 |
| Reference Example 165 | F | 7-MeO/8-CO₂Me | Reference Example 174 |
| Reference Example 166 | F | 6-MeO/8-CO₂Me | Reference Example 174 |
| Reference Example 167 | F | 8-F/7-CO₂(t-Bu) | Reference Example 174 |
| Reference Example 168 | F | 8-CO₂(t-Bu) | Reference Example 174 |

### Reference Example 153

¹H NMR (300 MHz, CDCl₃) δ 8.16 (brs, 1H), 7.92-7.88 (m, 2H), 7.75-7.71 (m, 1H), 7.40-7.35 (m, 2H), 7.22-7.13 (m, 2H), 6.82-6.79 (m, 1H), 5.58 (d, J = 15.9 Hz, 1H), 5.49 (d, J = 15.9 Hz, 1H), 4.91 (m, 1H), 3.77 (brs, 1H), 3.30 (dd, J = 3.3, 12.1 Hz, 1H), 2.90-2.84 (m, 3H), 1.80-1.59 (m, 4H), 1.59 (s, 9H), 1.43 (s, 9H).
MS (ESI+) 736 (M⁺+1, 100%).

### Reference Example 154

¹H NMR (300 MHz, CDCl₃) δ 8.46-8.37 (m, 3H), 7.34 (dd, J = 5.1, 8.8 Hz, 1H), 6.95-6.89 (m, 1H), 6.52 (dd, 2.4, 8.8 Hz, 1H), 5.57 (d, J = 16.3 Hz, 1H), 5.49 (d, 16.3 Hz, 1H), 4.87 (brd, J = 7.5 Hz, 1H), 3.95 (s, 6H), 3.76 (brs, 1H), 3.31 (dd, J = 2.9, 11.7 Hz, 1H), 2.94-2.81 (m, 3H), 1.83-1.64 (m, 4H), 1.42 (s, 9H).
MS (ESI+) 770 (M⁺+1, 100%).

### Reference Example 155

¹H NMR (300 MHz, CDCl₃) δ 8.19 (brs, 1H), 8.03-8.02 (m, 1H), 7.89-7.86 (m, 1H), 7.81-7.78 (m, 1H), 7.43-7.35 (m, 2H), 7.22-7.13 (m, 2H), 6.82-6.79 (m, 1H), 5.58 (d, J = 16.1 Hz, 1H), 5.51 (d, J = 16.1 Hz, 1H), 4.93-4.90 (m, 1H), 3.92 (s, 3H), 3.76 (brs, 1H), 3.30 (dd, J = 3.3, 12.1 Hz, 1H), 2.93-2.84 (m, 3H), 1.83-1.53 (m, 4H), 1.43 (s, 9H).
MS (ESI+) 694 (M⁺+1, 100%).

### Reference Example 156

H NMR (300 MHz, CDCl₃) δ 8.30 (brs, 1H), 8.05-7.97 (m, 2H), 7.65-7.60 (m, 2H), 7.38-7.35 (m, 1H), 7.21-7.08 (m, 2H), 6.81-6.79 (m, 1H), 5.58 (d, J = 16.3 Hz, 1H), 5.50 (d, J = 16.3 Hz, 1H), 4.91 (m, 1H), 3.93-3.91 (m, 1H), 3,90 (s, 3H), 3.76 (brs, 1H), 3.30 (d, J = 9.2 Hz, 1H), 2.94-2.83 (m, 2H), 1.83-1.74 (m, 2H), 1.55-1.52 (m, 2H), 1.43 (s, 9H).
MS (ESI+) 694 (M⁺+1, 100%).

### Reference Example 157

¹H NMR (300 MHz, CDCl₃) δ 8.44 (s, 1H), 8.35 (m, 2H), 8.27 (brs, 1H), 7.38-7.35 (m, 1H), 7.20-7.15 (m, 2H), 6.83-6.80 (m, 1H), 5.59 (d, J = 16.3 Hz, 1H), 5.51 (d, J = 16.3 Hz, 1H), 4.89-4.86 (m, 1H), 3.94 (s, 6H), 3.76 (m, 1H), 3.31 (dd, J = 3.5, 12.2 Hz, 1H), 2.94-2.84 (m, 3H), 1.79-1.56 (m, 4H), 1.43 (s, 9H).
MS (ESI+) 752 (M⁺+1, 000%).

### Reference Example 158

¹H NMR (400 MHz, CDCl₃) δ 8.94 (brs, 1H), 8.46 (dd, J = 7.9 and 1.7 Hz, 1H), 7.56 (dd, J = 7.9 and 1.7 Hz, 1H), 7.36 (dd, J = 7.6 and 2.1 Hz, 1H), 7.21-7.14 (m, 2H), 7.11 (dd, J = 7.9 and 7.9 Hz, 1H), 6.78 (dd, J = 7.6 and 2.1 Hz, 1H), 5.60 (d, J = 16.3 Hz, 1H), 5.53 (d, J = 16.3 Hz, 1H), 4.94 (d, J = 7.6 Hz, 1H), 4.40 (q, J = 7.1 Hz, 2H), 3.91 (s, 3H), 3.77 (brs, 1H), 3.30 (dd, J = 12.0 and 3.3 Hz, 1H), 2.94-2.87 (m, 3H), 1.76-1.63 (m, 2H), 1.59-1.48 (m, 2H), 1.43 (s, 9H), 1.41 (t, J = 7.1 Hz, 3H).
MS (ESI+) 738 (M⁺+1, 100%).

### Reference Example 159

¹H NMR (400 MHz, CDCl₃) δ 8.43 (brs, 1H), 8.35-8.29 (m, 1H), 7.37 (dd, J = 7.9 and 1.3 Hz, 1H), 7.22-7.15 (m, 2H), 6.90 (ddd, J = 7.9, 7.9 and 1.3 Hz, 1H), 6.75 (ddd, J = 7.9, 7.9 and 1.3 Hz, 1H), 5.58 (d, J = 16.5 Hz, 1H), 5.51 (d, J = 16.5 Hz, 1H), 4.92 (d, J = 7.9 Hz, 1H), 4.43 (q, J = 7.1 Hz, 2H), 3.77 (brs, 1H), 3.31 (dd, J = 12.1 and 3.4 Hz, 1H), 2.96-2.84 (m, 3H), 1.78-1.65 (m, 1H), 1.55-1.47 (m, 3H), 1.43 (s, 9H), 1.42 (t, J = 7.1 Hz, 3H).
MS (ESI+) 744 (M⁺+1, 100%).

### Reference Example 160

¹H NMR (400 MHz, CDCl₃) δ 8.41 (s, 1H), 8.02-7.99 (m, 2H), 7.64-7.58 (m, 2H), 7.34 (m, 1H), 6.93 (m, 1H), 6.50 (m, 1H), 5.53-5.46 (m, 2H), 4.85 (m, 1H), 3.91 (s, 3H), 3.76 (m, 1H), 3.31 (m, 1H), 2.99-2.81 (m, 3H), 1.80 (m, 1H), 1.68-1.58 (m, 3H), 1.42 (s, 9H).
MS (ESI+) 712 (M⁺+1, 100%).

### Reference Example 161

¹H NMR (300 MHz, CDCl₃) δ 8.09 (brs, 1H), 7.53-7.50 (m, 2H), 7.38-7.35 (m, 1H), 7.21-7.13 (m, 2H), 7.10-7.07 (m, 2H), 6.82-6.78 (m, 1H), 6.45 (t, J = 73.8 Hz, 1H), 5.67 (d, J = 16.5 Hz, 1H), 5.50 (d, J = 16.5 Hz, 1H), 4.91-4.90 (m, 1H), 3.75-3.73 (m, 1H), 3.29 (dd, J = 3.5, 12.2 Hz, 1H), 2.96-2.83 (m, 3H), 1.77-1.57 (m, 4H), 1.43 (s, 9H).
MS (ESI+) 702 (M⁺+1, 100%).

### Reference Example 162

¹H NMR (400 MHz, CDCl₃) δ 8.11 (brs, 1H), 7.88-7.72 (m, 2H), 7.37-7.34 (m, 1H), 7.21-7.10 (m, 2H), 7.10-7.04 (m, 1H), 6.81-6.78 (m, 1H), 5.56 (d, J = 16.5 Hz, 1H), 5.49 (d, J = 16.5 Hz, 1H), 4.92 (brd, J = 6.6 Hz, 1H), 3.77 (brs, 1H), 3.30 (dd, J = 12.1 and 3.4, 1H), 2.95-2.84 (m, 3H), 1.93-1.70 (m, 3H), 1.59 (s, 9H), 1.43 (s, 9H).
MS (ESI+) (M⁺+1, 100%).

### Reference Example 163

¹H NMR (400 MHz, CDCl₃) δ 8.12 (s, 1H), 7.58 (s, 1H), 7.47 (s, 1H), 7.36 (dd, J = 7.9 and 1.3 Hz, 1H), 7.28-7.26 (m, 1H), 7.21-7.14 (m, 2H), 6.79 (d, J = 7.9 Hz, 1H), 5.58 (d, J = 16.5 Hz, 1H), 5.51 (d, J = 16.5 Hz, 1H), 4.92 (d, J = 7.9 Hz, 1H), 3.83 (s, 3H), 3.78 (brs, 1H), 3.30 (dd, J = 12.1 and 3.4 Hz, 1H), 2.92-2.84 (m, 3H), 1.76-1.68 (m, 3H), 1.58 (s, 9H), 1.54-1.48 (m, 1H), 1.43 (s, 9H).
MS (ESI+) 766 (M⁺+1, 100%).

### Reference Example 164

¹H NMR (400 MHz, CDCl₃) δ 8.08 (s, 1H), 7.48 (d, J = 8.4Hz, 2H), 7.37-7.34 (m, 1H), 7.27-7.13 (m, 4H), 6.82-6.79 (m, 1H), 5.58 (d, J = 16.2Hz, 1H), 5.50 (d, J = 16.2Hz, 1H), 4.93-4.91 (m, 1H), 4.13 (dd, J = 7.1, 14.3Hz, 2H), 3.78-3.75 (m, 1H), 3.57 (s, 2H), 3.29 (dd, J = 3.3, 11.9Hz, 1H), 2.92-2.86 (m, 3H), 1.74-1.58 (m, 4H), 1.43 (s, 9H), 1.24 (d, J = 7.1Hz, 3H).
MS (ESI+) 722 (M⁺+1, 100%).

### Reference Example 165

MS (ESI+) 742 (M⁺+1, 100%).

### Reference Example 166

MS (ESI+) 742 (M⁺+1, 100%).

### Reference Example 167

MS (ESI+) 772(M⁺ +1, 100%).

### Reference Example 168

MS (ESI+) 754(M⁺ +1, 100%).

### Reference Example 169

tert-Butyl ((3R)-1-{1-(2-chloro-5-fluorobenzyl)-4-iodo-5-[(pyridin-4-ylamino)carbonyl]-1H-imidazol-2-yl}piperidin-3-yl)carbamate

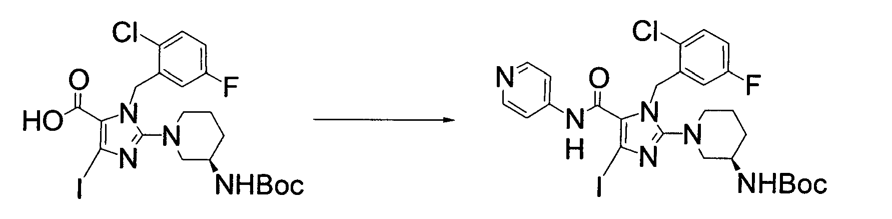

The title compound (250 mg) was synthesized by the same process as in Reference Example 152.
MS (ESI+) 655 (M⁺+1, 100%).

### Reference Example 170

Methyl 4-({[2-{(3R)-3-[(tert-butoxycarbonyl)-amino]piperidin-1-yl}-1-(2-chlorobenzyl)-4-iodo-1H-imidazol-5-yl]carbonyl}amino)-1-(4-methoxybenzyl)-1H-pyrazole-5-carboxylate

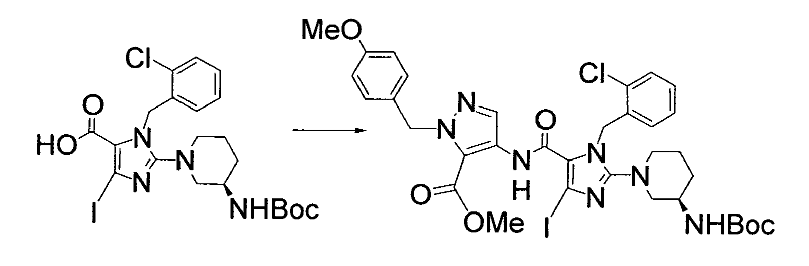

The title compound (421 mg) was synthesized by the same process as in Reference Example 152.
¹H NMR (400 MHz, CDCl₃) δ 9.74 (s, 1H), 8.16 (s, 1H), 7.35 (dd, J = 7.9 and 1.3 Hz, 1H), 7.22 (d, J = 8.6 Hz, 2H), 7.19-7.11 (m, 2H), 6.86 (d, J = 8.6 Hz, 2H), 6.69 (d, J = 7.9 Hz, 1H), 5.53 (d, J = 16.5 Hz, 1H), 5.46 (d, J = 16.5 Hz, 1H), 5.24 (s, 2H), 4.88 (d, J = 8.0 Hz, 1H), 3.99 (s, 3H), 3.79 (s, 3H), 3.74 (brs, 1H), 3.23 (dd, J = 12.0 and 3.3 Hz, 1H), 2.89-2.81 (m, 3H), 1.73-1.53 (m, 4H), 1.42 (s, 9H).
MS (ESI+) 804(M⁺ +1, 100%).

### Reference Example 171

Methyl 3-({[2-{(3R)-3-[(tert-butoxycarbonyl)-amino]piperidin-1-yl}-1-(2-chlorobenzyl)-4-iodo-1H-imidazol-5-yl]carbonyl}amino)thiophene-2-carboxylate

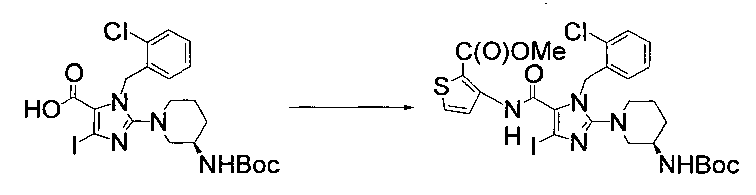

The title compound (770 mg) was synthesized by the same process as in Reference Example 152.
¹H NMR (300 MHz, CDCl₃) δ 10.61 (brs, 1H), 8.01 (d, J = 5.5 Hz, 1H), 7.44 (d, J = 5.5 Hz, 1H), 7.36-7.33 (m, 1H). 7.20-7.12 (m, 2H), 6.77-6.73 (m, 1H), 5.50 (d, J = 16.1 Hz, 1H), 5.43 (d, J = 16.1 Hz, 1H), 4.92-4.90 (m, 1H), 3.90 (s, 3H), 3.76 (brs, 1H), 3.28 (dd, J = 3.3, 12.3 Hz, 1H), 2.90-2.79 (m, 3H), 1.71-1.52 (m, 4H), 1.42 (s, 9H).
MS (ESI+) 700 (M⁺+1, 100%).

### Reference Example 172

tert-Butyl [(3R)-1-[1-(2-chloro-5-fluorobenzyl)-4-iodo-5-([(2-oxo-2,5-dihydrofuran-3-yl)amino]carbonyl}-1H-imidazo-2-yl]piperidin-3-yl]carbamate

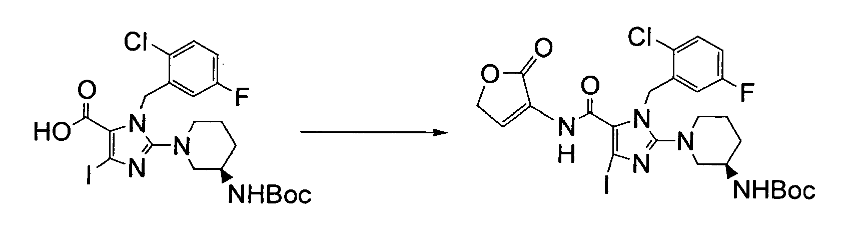

N,N-dimethylformamide (three drops) and oxalyl chloride (0.15 mL) were added to a solution of 2-[(3R)-3-(tert-butoxycarbonyl)amino]piperidin-1-yl}-1-(2-chloro-5-fluorobenzyl)-4-iodo-1H-imidazole-5-carboxylic acid (0.52 g) in dichloromethane (20 mL) at 0°C. After the resulting mixture was stirred at 25°C for 4 hours, the solvent was distilled off by concentration under reduced pressure. Toluene (10 mL), diisopropylethylamine (0.3 mL) and 3-aminofuran-2(5H)-one (0.3 mL) were added to the residue and the resulting mixture was stirred at 120°C for 8 hours. After the reaction solution was allowed to cool, a saturated aqueous ammonium chloride solution was added thereto, followed by extraction with ethyl acetate. The organic phase was dried over sodium sulfate and filtered, and the filtrate was concentrated under reduced pressure. The resulting residue was purified by a silica gel chromatography (hexane/ethyl acetate = 5/1-1/1) to obtain the title compound.

### Reference Example 173

2-{(3R)-3-[(tert-Butoxycarbonyl)amino]piperidin-1-yl}-1-(2-chlorobenzyl)-4-iodo-1H-imidazole-5-carboxylic acid

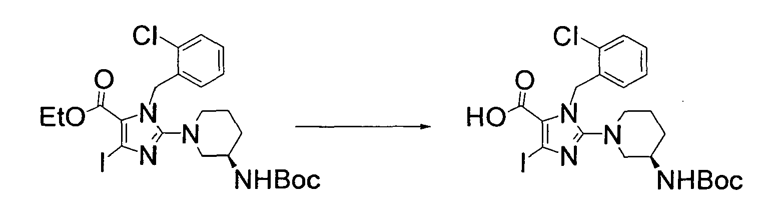

A solution consisting of ethyl 2-[(3R)-3-(tert-butoxycarbonyl)amino]piperidin-1-yl}-1-(2-chlorobenzyl)-4-iodo-1H-imidazole-5-carboxylate (7.0 g), 1N sodium hydroxide (20 mL) and ethanol (50 mL) was stirred at 80°C for 1 hour. After the reaction mixture was concentrated under reduced pressure, a saturated aqueous ammonium chloride solution was added thereto, followed by extraction with ethyl acetate. The organic layer was washed with a saturated aqueous sodium chloride solution, dried over anhydrous sodium sulfate and then filtered, and the filtrate was concentrated under reduced pressure to obtain the title compound (6.5 g) as a light-yellow amorphous substance.
MS (ESI+) 561 (M⁺+1, 100%).

### Reference Example 174

2-[(3R)-3-(tert-Butoxycarbonyl)amino]piperidin-1-yl}-1-(2-chloro-5-fluorobenzyl)-4-iodo-1H-imidazole-5-carboxylic acid

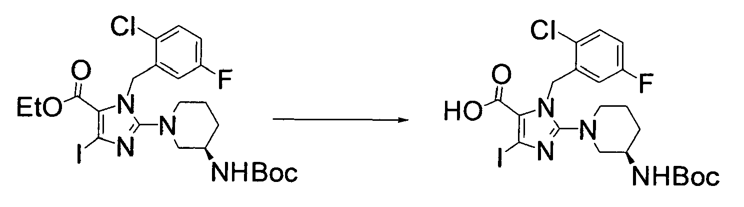

The title compound (30.3 g) was synthesized by the same process as in Reference Example 173.
¹H NMR (400 MHz, CDCl₃) δ 7.33 (m, 1H), 6.90 (m, 1H), 6.32 (d, J = 9.0 Hz, 1H), 5.45-4.34 (m, 2H), 4.84 (m, 1H), 3.73-3.66 (m, 1H), 3.33-3.31 (m, 1H), 2.912.77 (m, 4 H), 1.78-1.57 (m, 3H), 1.40 (s, 9H).
MS (ESI+) 579 (M⁺+1, 100%).

### Reference Example 175

2-Methoxy-3-nitrobenzoic acid

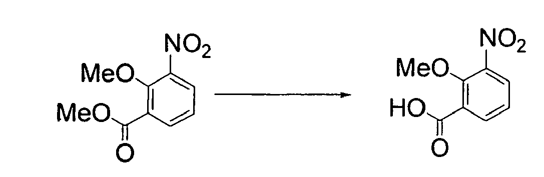

A 3N aqueous sodium hydroxide solution (155 mL) was added to a solution of methyl 2-methoxy-3-nitrobenzoate (9.83 g) in a mixture of tetrahydrofuran and methanol (1:1, 400 mL) and stirred for 48 hours. The organic solvent was removed under reduced pressure and water (400 mL) was added to the residue to obtain a solution. This solution was acidified (pH = 1) with 36% hydrochloric acid and extracted with ethyl acetate (3 x 200 mL). The combined organic layer was dried over anhydrous magnesium sulfate and filtered, and the filtrate was concentrated under reduced pressure to obtain the title compound (8.01 g).
¹H NMR (400 MHz, CDCl₃) δ 8.29 (dd, J = 7.9 and 1.7 Hz, 1H), 8.04 (dd, J = 7.9 and 1.7 Hz, 1H), 7.38 (dd, J = 7.9 and 7.9 Hz, 1H), 4.09 (s, 3H).
MS (ESI⁺) 198 (M⁺ + 1, 18%), 180 (100%).

### Reference Example 176

Ethyl 2-methoxy-3-nitrobenzoate

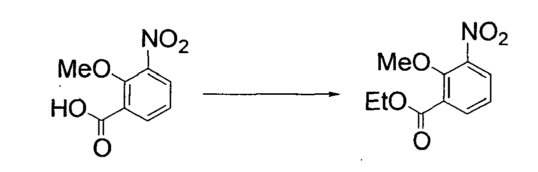

A reactor containing a solution (500 mL) of 2-methoxy-3-nitrobenzoic acid (8.01 g) in ethanol was cooled in an ice-water bath and thionyl chloride (6.10 g) was added dropwise to the solution. After completion of the dropwise addition, the reaction solution was stirred for 8 hours with heating under reflux. The reaction solution was cooled and then concentrated under reduced pressure, and the residue was dissolved in ethyl acetate (500 mL). The resulting solution was washed with a saturated aqueous sodium hydrogencarbonate solution (2 x 100 mL), dried over anhydrous magnesium sulfate and then filtered, and the filtrate was concentrated under reduced pressure to obtain the title compound (7.75 g) as a yellow oil.
¹H NMR (400 MHz, CDCl₃) δ 8.03 (dd, J = 7.9 and 1.7 Hz, 1H), 7.91 (dd, J = 7.9 and 1.7 Hz, 1H), 7.28 (dd, J = 7.9 and 7.9 Hz, 1H), 4.43 (q, J = 7.1 Hz, 2H), 4.01 (s, 3H), 1.42 (t, J= 7.1 Hz, 3H).
MS (ESI⁺) 226 (M⁺ + 1, 14%), 180 (100%).

### Reference Example 177

Ethyl 3-amino-2-methoxybenzoate

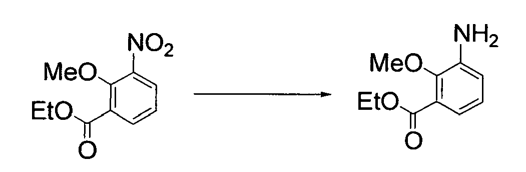

To a solution of ethyl 2-methoxy-3-nitrobenzoate (7.75 g) in a mixture of tetrahydrofuran and methanol (1:1, 400 mL) was added 10% palladium-active carbon carrier (containing 50% water, 1.4 g), and the resulting mixture was stirred for 4 hours under a hydrogen atmosphere. The reaction solution was filtered and the filtrate was concentrated under reduced pressure. The residue was purified by a silica gel column chromatography (developing solvent: hexane/ethyl acetate = 6/1 to 3/1) to obtain the title compound (6.69 g) as a light-yellow oil.
¹H NMR (400 MHz, CDCl₃) δ 7.19 (dd, J = 7.9 and 1.7 Hz, 1H), 6.95 (dd, J = 7.9 and 7.9 Hz, 1H), 6.90 (dd, J = 7.9 and 1.7 Hz, 1H), 4.37 (q, J = 7.1 Hz, 2H), 3.94 (brs, 2H), 3.85 (s, 3H), 1.40 (t, J = 7.1 Hz, 3H).
MS (ESI⁺) 196 (M⁺ + 1, 7%), 150 (100%).

### Reference Example 178

Ethyl 3-amino-2,6-difluorobenzoate

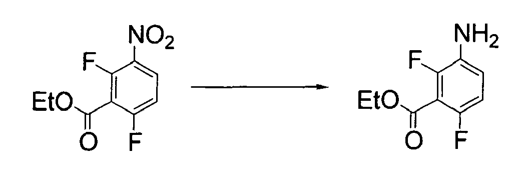

Tin chloride dihydrate (SnCl₂•2H₂O) (5.32 g) was added to a solution (50 mL) of ethyl 2,6-difluoro-3-nitrobenzoate (1.0 g) in ethanol, and the mixture was stirred for 2 hours with heating under reflux. After the reaction solution was cooled, the solvent was removed to obtain a yellow oil. This residue was dissolved in ethyl acetate (100 mL) and the resulting solution was made basic with a saturated aqueous sodium hydrogencarbonate solution and extracted with ethyl acetate (4 x 50 ml). The combined organic layer was dried over anhydrous magnesium sulfate and filtered, and the filtrate was concentrated under reduced pressure. The residue was purified by a silica gel column chromatography (developing solvent: hexane/ethyl acetate = 4/1) to obtain the title compound (819.7 mg) as a light-yellow oil.
¹H NMR (400 MHz, CDCl₃) δ 6. 85-6. 74 (m, 2H), 4.42 (q, J = 7.1 Hz, 2H), 3.68 (brs, 2H), 1.39 (t, J = 7.1 Hz, 3H).
MS(ESI⁺) 202 (M⁺ + 1, 100%).

### Reference Example 179

Methyl 4-amino-1-(4-methoxybenzyl)-1H-pyrazole-5-carboxylate

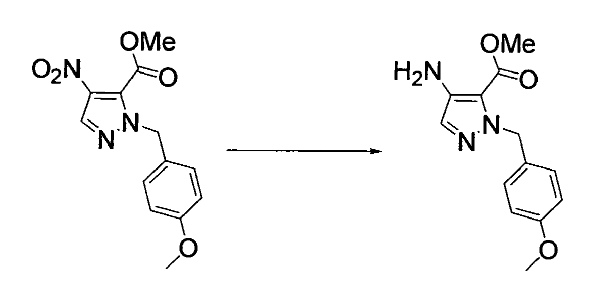

To a solution of methyl 1-(4-methoxybenzyl)-4-nitro-1H-pyrazole-5-carboxylate (6.54 g) in a mixture of tetrahydrofuran and methanol (1:3, 400 mL) was added 10% palladium-active carbon carrier (containing 50% water, 2.31 g), and the resulting mixture was stirred for 4 hours under a hydrogen atmosphere. The reaction solution was filtered and the filtrate was concentrated under reduced pressure. The residue was purified by a silica gel column chromatography (developing solvent: hexane/ethyl acetate = 1/1) to obtain the title compound (5.11 g) as a light-yellow oil.
¹H NMR (400 MHz, CDCl₃) δ 7.19 (d, J = 8.6 Hz, 2H), 6.87 (d, J = 8.6 Hz, 2H), 6.85 (s, 1H), 5.18 (s, 2H), 4.05 (brs, 2H), 3.93 (s, 3H), 3.80 (s, 3H).
MS (ESI⁺) 262 (M⁺ + 1, 100%).

### Reference Example 180

tert-Butyl 2-fluoro-5-nitrobenzoate

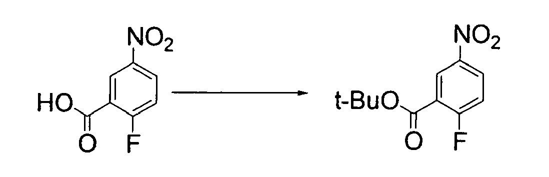

N,N-dimethylaminopyridine (801 mg) and di-tert-butyl dicarbonate (9.54 g) were added to a suspension of 2-fluoro-5-nitrobenzoic acid (4.04 g) in tetrahydrofuran (60 mL) and stirred for 11 hours. Then, tert-butanol (60 mL) was added thereto and stirred for 24 hours. The reaction solution was filtered and the filtrate was concentrated under reduced pressure. The residue was suspended in ethyl acetate (500 mL) and the suspension was filtered. The filtrate was concentrated under reduced pressure and the residue was purified by a silica gel column chromatography (developing solvent: hexane/ethyl acetate = 20/1) to obtain the title compound (4.652 g) as a light-yellow oil.
¹H NMR (400 MHz, CDCl₃) δ 8.77-8.75 (m, 1H), 8.39-8.35 (m, 1H), 7.31-7.27 (m, 1H), 1.53 (s, 9H).

### Reference Example 181

tert-Butyl 5-amino-2-fluorobenzoate

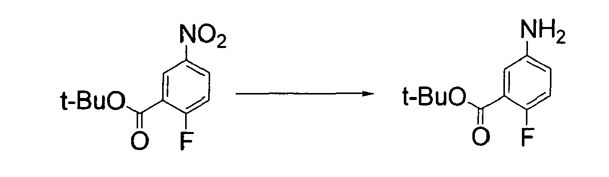

To a solution of tert-butyl 2-fluoro-5-nitrobenzoate (500 mg) in tetrahydrofuran (50 mL) was added 10% palladium-active carbon carrier (containing 50% water, 58 mg), and the resulting mixture was stirred for 6 hours under a hydrogen atmosphere. The reaction solution was filtered and the filtrate was concentrated under reduced pressure to obtain the title compound (447.2 mg) as a yellow oil.
¹H NMR (400 MHz, CDCl₃) δ 7.14-7.11 (m, 1H), 6.92-6.88 (m, 1H), 6.78-6.74 (m, 1H), 3.62 (brs, 2H), 1.53 (s, 9H).
MS (ESI⁺) 212 (M⁺ + 1, 67%), 156 (100%).

### Reference Example 182

3-Methoxy-5-nitrobenzoic acid

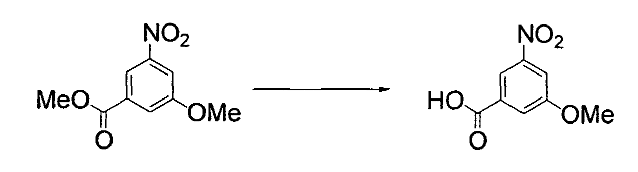

A 4N aqueous sodium hydroxide solution (56.9 ml) was added to a solution of methyl 3-methoxy-5-nitrobenzoate (9.60 g) in a mixture of tetrahydrofuran and methanol (1:1, 400 mL) and stirred for 48 hours. The organic solvent was removed under reduced pressure and water (150 mL) was added to the residue to obtain a solution. This solution was acidified with 36% hydrochloric acid and extracted with ethyl acetate (4 x 100 mL). The combined organic layer was dried over anhydrous magnesium sulfate and filtered, and the filtrate was concentrated under reduced pressure to obtain the title compound (8.96 g).
¹H NMR (400 MHz, CDCl₃) δ 8.54 (dd, J = 2.5 and 1.3 Hz, 1H), 7.98 (dd, J = 2.5 and 2.5 Hz, 1H), 7.94 (dd, J = 2.5 and 1.3 Hz, 1H), 3.97 (s, 3H).
MS (ESI⁺) 198 (M⁺ + 1, 100%).

### Reference Example 183

tert-Butyl 3-methoxy-5-nitrobenzoate

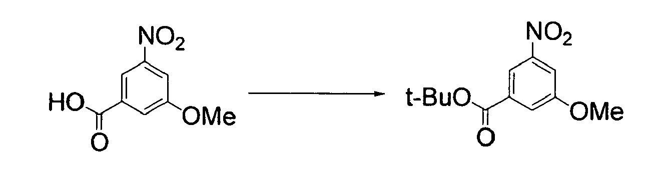

N,N-dimethylaminopyridine (1.66 g) and di-tert-butyl dicarbonate (19.86 g) were added to a solution of 3-methoxy-5-nitrobenzoic acid (8.96 g) in tetrahydrofuran (200 mL) and stirred for 24 hours. Then, tert-butanol (200 mL) was added thereto and stirred for 24 hours. The reaction solution was concentrated under reduced pressure and the residue was suspended in ethyl acetate (500 mL), followed by filtration. The filtrate was concentrated under reduced pressure and the residue was purified by a silica gel column chromatography (developing solvent: hexane/ethyl acetate = 50/1) to obtain the title compound (10.68 g) as a light-yellow oil.
¹H NMR (400 MHz, CDCl₃) δ 8.38 (dd, J = 2.5 and 1.3 Hz, 1H), 7.88 (dd, J = 2.5 and 2.5 Hz, 1H), 7.84 (dd, J = 2.5 and 1.3 Hz, 1H), 3.94 (s, 3H), 1.62 (s, 9H).
MS (ESI⁺) 198 (M⁺ -tBu, 100%).

### Reference Example 184

tert-Butyl 3-amino-5-methoxybenzoate

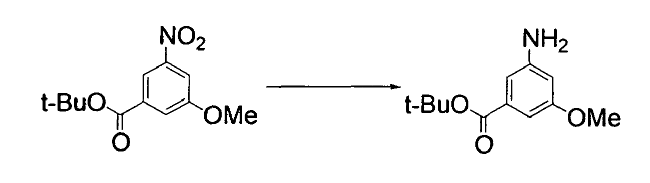

To a solution of tert-butyl 3-methoxy-5-nitrobenzoate (10.68 g) in tetrahydrofuran (500 mL) was added 10% palladium-active carbon carrier (containing 50% water, 2.0 g), and the resulting mixture was stirred for 6 hours under a hydrogen atmosphere. The reaction solution was filtered and the filtrate was concentrated under reduced pressure. The residue was purified by a silica gel column chromatography (developing solvent: hexane/ethyl acetate = 10/1 to 1/1) to obtain the title compound (9.7 g) as a yellow oil.
¹H NMR (400 MHz, CDCl₃) δ 6.94-6.92 (m, 2H), 6.38 (dd, J = 2.5 and 2.5 Hz, 1H), 3.79 (s, 5H), 1.57 (s, 9H).
MS (ESI⁺) 224 (M⁺ + 1, 100%).

### Reference Example 185

tert-Butyl 2-{(3R)-3-[(tert-butoxycarbonyl)-amino]piperidin-1-yl}-5-methyl-4-oxo-4,5-dihydro-3H-imidazo[4,5-c]quinoline-7-carboxylate

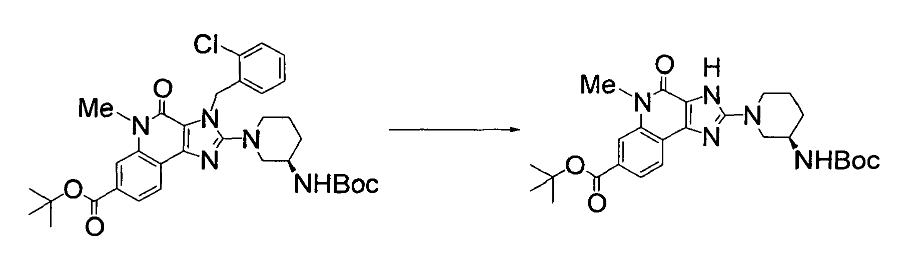

Ammonium formate (2.55 g) and 10% palladium-carbon (2.50 g) were added to a solution (100 mL) of tert-butyl 2-{(3R)-3-[(tert-butoxycarbonyl)amino]piperidin-1-yl}-3-(2-chlorobenzyl)-5-methyl-4-oxo-4,5-dihydro-3H-imidazo[4,5-c]quinoline-7-carboxylate (2.52 g) in methanol, and the resulting mixture was stirred with heating at 70°C for 3 hours in a nitrogen stream.
After completion of the reaction, the palladium-carbon was removed by filtration and the solvent was distilled off under reduced pressure. A saturated aqueous sodium hydrogencarbonate solution was added to the residue, followed by extraction with ethyl acetate (200 mL). The organic layer was dried over sodium sulfate and filtered, and the filtrate was concentrated under reduced pressure. The resulting residue was purified by a silica gel column chromatography (developing solvent: chloroform/methanol = 100/1 to 25/1) to obtain the title compound (1.63 g) as a light-yellow solid.
¹H NMR (300 MHz, CDCl₃) δ ppm 11.95 (bs, 1H), 8.24 (d, J = 7.9Hz, 1H), 7.92 (s, 1H), 7.91 (d, J = 7.9Hz, 1H), 5.00 (d, J = 7.0 Hz,), 3.88 (s, 3H), 3.75-3.65 (m, 5H), 1.92-1.52 (m, 4H), 1.65 (s, 9H), 1.40 (s, 9H).
MS (ESI+) 498 (M⁺+1, 100%).

### Reference Example 186

2-{(3R)-3-[(tert-Butoxycarbonyl)amino]piperidin-1-yl}-3-(2-chlorobenzyl)-4-oxo-4,5-dihydro-3H-imidazo[4,5-c]quinoline-8-carboxylic acid

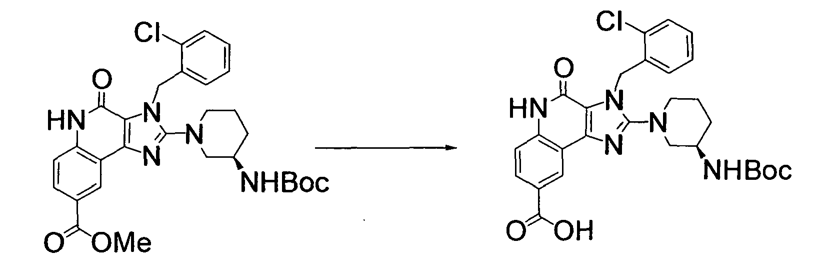

The title compound (5.0 mg) was synthesized by the same process as in Reference Example 120.
MS (ESI+) 552 (M⁺+1, 100%).

### Reference Example 187

Methyl 1-(4-methoxybenzyl)-4-nitro-1H-pyrazole-5-carboxylate

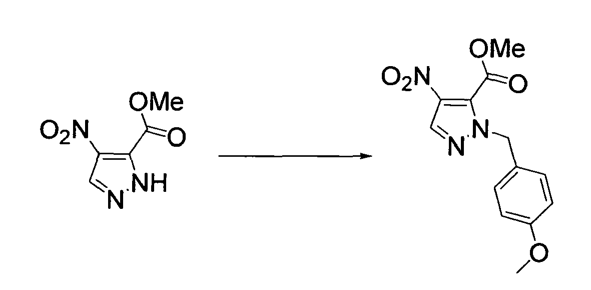

4-Methoxybenzyl chloride (5.04 g) and potassium carbonate (4.45 g) were added to a solution (60 mL) of methyl 4-nitro-1H-pyrazole-5-carboxylate (5.00 g) in N,N-dimethylformamide, and the resulting mixture was stirred with heating at 60°C for 12 hours. After completion of the reaction, the reaction mixture was cooled to room temperature and water was added to the reaction mixture, followed by extraction with ethyl acetate. The organic layer was washed with a saturated aqueous sodium hydrogencarbonate solution and a saturated aqueous sodium chloride solution. The organic layer was dried over sodium sulfate and filtered, and the filtrate was concentrated under reduced pressure. The resulting residue was purified by a silica gel column chromatography (developing solvent: ethyl acetate/hexane = 1/4 to 1/1) to obtain the title compound (6.54 g).
MS (ESI+) 292 (M⁺+1, 100%).

### Reference Example 188

Methyl 7-{(3R)-3-[(tert-butoxycarbonyl)amino]-piperidin-1-yl}-6-(2-chlorobenzyl)-4-methyl-5-oxo-2,4,5,6-tetrahydroimidazo[4,5-d]pyrazolo[4,3-b]pyridine-3-carboxylate

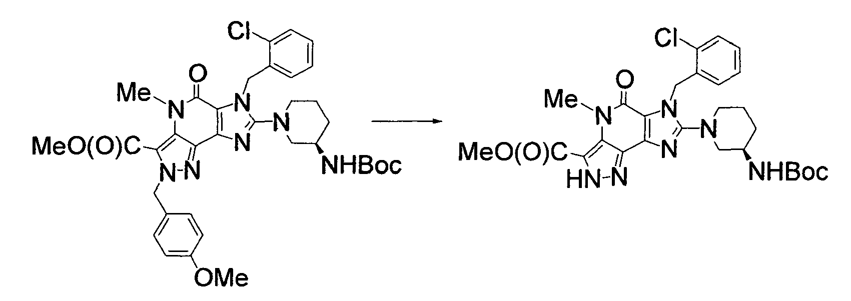

Trifluoroacetic acid (17 mL) and concentrated sulfuric acid (0.5 mL) were added to a solution of methyl 7-{(3R)-3-[(tert-butoxycarbonyl)amino]piperidin-1-yl}-6-(2-chlorobenzyl)-2-(4-methoxybenzyl)-4-methyl-5-oxo-2,4,5,6-tetrahydroimidazo[4,5-d]pyrazolo[4,3-b]pyridine-3-carboxylate (139 mg) in anisole (1 mL), and the resulting mixture was allowed to stand at room temperature for 5 days. The solvent for reaction was distilled off under reduced pressure and the residue was diluted with tetrahydrofuran (50 mL) and then adjusted to pH 10 with a saturated aqueous sodium hydrogencarbonate solution. To the resulting mixed solution was added di-tert-butyl dicarbonate (88 mg), and the resulting mixture was stirred at room temperature for 5 hours. The tetrahydrofuran was removed under reduced pressure, followed by two runs of extraction with ethyl acetate (30 mL). The organic layer was dried over anhydrous magnesium sulfate and filtered, and the filtrate was concentrated under reduced pressure. The resulting residue was purified by a silica gel column chromatography (developing solvent: ethyl acetate/hexane = 1/1 to 1/0) to obtain the title compound (94 mg).
¹H NMR (400 MHz, CD₃OD) δ 7.45 (dd, J = 7.9 and 1.3 Hz, 1H), 7.26 (ddd, J = 7.9, 7.9 and 1.3 Hz, 1H), 7.20 (ddd, J = 7.9, 7.9 and 1.3 Hz, 1H), 6.73 (d, J = 7.9 Hz, 1H), 5.71 (s, 2H), 3.97 (s, 3H), 3.85 (s, 3H), 3.64 (brs, 1H), 3.49-3.46 (m, 1H), 3.26-3.23 (m, 1H), 2.96-2.92 (m, 1H), 2.85-2.80 (m, 1H), 1.85-1.66 (m, 4H), 1.41 (s, 9H).
MS (ESI⁺) 570 (M⁺ + 1, 100%).

### Reference Example 189

tert-Butyl {(3R)-1-[3-(2-chlorobenzyl)-5-methyl-4,7-dioxo-4,5,7,9-tetrahydro-3H-furo[3,4-g]imidazo[4,5-c]quinolin-2-yl]piperidin-3-yl}carbamate

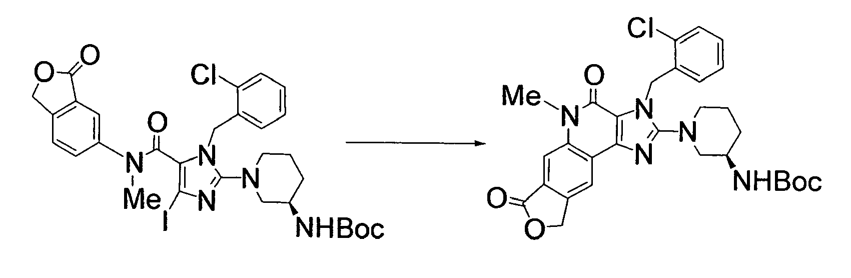

The title compound (3.7 mg) was synthesized by the same process as in Reference Example 76.
MS (ESI+) 596 (M⁺+1, 100%).

### Reference Example 190

tert-Butyl [(3R)-1-(1-(2-chlorobenzyl)-4-iodo-5-{[methyl(3-oxo-1,3-dihydro-2-benzofuran-5-yl)amino]-carbonyl}-1H-imidazol-2-yl)piperidin-3-yl]carbamate

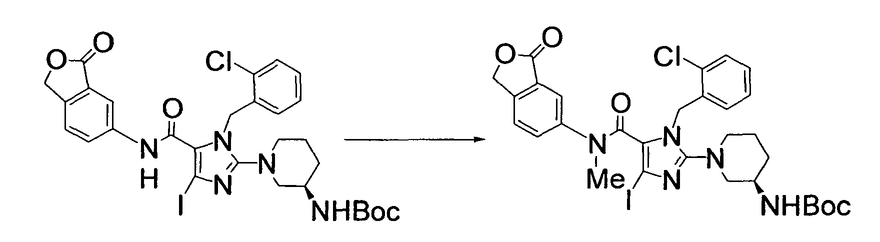

The title compound (25 mg) was synthesized by the same process as in Reference Example 130.
MS (ESI+) 724 (M⁺+1, 100%).

### Reference Example 191

tert-Butyl [(3R)-1-(1-(2-chlorobenzyl)-4-iodo-5-{[(3-oxo-1,3-dihydro-2-benzofuran-5-yl)amino]carbonyl}-1H-imidazol-2-yl)piperidin-3-yl]carbamate

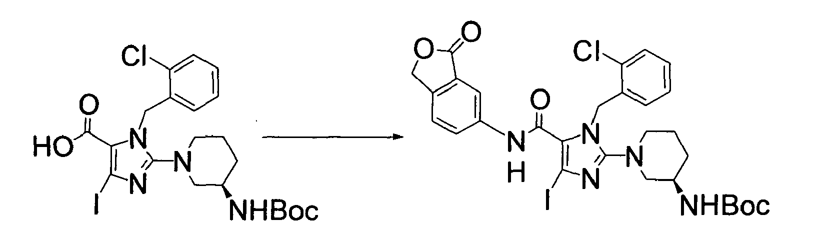

The title compound (64 mg) was synthesized by the same process as in Reference Example 152.
MS (ESI+) 710 (M⁺+1, 100%).

### Test Examples

### In vitro DPP-IV inhibitory effect measurement test (1)

Bovine plasma containing DPP-IV enzyme was diluted with assay buffer (25mM Tris-HCl, 140mM NaCl, 10mM KCl, pH = 7.9) and 50 µL of the dilution was added to a micro assay plate. After 1 µL of a solution of each compound was added, mixing was conducted, followed by incubation at room temperature. A substrate (Glycyl-L-Proline 4-Methyl-Coumaryl-7-Amide, Peptide Laboratories Co., Ltd.) was diluted to 0.2mM with the assay buffer, and 50 µL of this solution was added and then stirred, followed by incubation at room temperature. Thereafter, the reaction was terminated by the addition of 100 µL of a 25% aqueous acetic acid solution. The intensity of fluorescence at an excitation wavelength of 360 nm and a measuring wavelength of 460 nm was measured by the use of a fluorescent plate reader. The difference in intensity of fluorescence between a background well in which the reaction had been terminated by previously adding a 25% aqueous acetic acid solution before the addition of the substrate solution and a control well to which no compound had been added was taken as 100%. The intensity of fluorescence of a well containing the compound was interpolated and the residual enzyme activity in the case of the addition of the compound was calculated as a relative value. A compound concentration for 50% inhibition of the enzyme activity was calculated as an IC₅₀ value from relative residual enzyme activity values obtained by adding each compound to a plurality of concentrations.

Compounds of Examples were used in this test. The results obtained are shown in Table 1.

**Table 1**

| Compound | IC₅₀ (nM) |
|---|---|
| Compound of Example 2 | 11.0 |
| Compound of Example 4 | 68.0 |
| Compound of Example 7 | 1.4 |
| Compound of Example 11 | 44.0 |
| Compound of Example 13 | 5.0 |
| Compound of Example 30 | 203.0 |
| Compound of Example 38 | 112.0 |

### In vitro DPP-IV inhibitory effect measurement test (2)

Human serum containing DPP-IV enzyme was used in an experiment after being diluted with assay buffer (25mM Tris-HCl, 140mM NaCl, 10mM KCl, pH 7.9) (finally, diluted 10-fold). Each of solutions of each test compound having various concentrations was added to the diluted serum and the resulting mixture was incubated at room temperature. Then, a substrate (Glycyl-L-Proline 4-Methyl-Coumaryl-7-Amide, Peptide Laboratories Co., Ltd.) was added thereto to a final concentration of 100 µM and the reaction was carried out at room temperature. Acetic acid was added to the reaction mixture to a final concentration of 12.5% to terminate the reaction, and the intensity of fluorescence at an excitation wavelength of 360 nm and a measuring wavelength of 460 nm was measured by the use of a fluorescent plate reader. A compound concentration for 50% inhibition was calculated as an IC₅₀ value from enzyme inhibitory activity values obtained by adding each test compound to a plurality of concentrations.

Compounds of Examples were used in this test. The results obtained are shown in Table 2.

**Table 2**

| Compound | IC₅₀ (nM) |
|---|---|
| Compound of Example 46 | 418.0 |
| Compound of Example 47 | 51.0 |
| Compound of Example 48 | 45.0 |
| Compound of Example 49 | 271.0 |
| Compound of Example 50 | 21.0 |
| Compound of Example 51 | 12.0 |
| Compound of Example 52 | 72.0 |
| Compound of Example 53 | 16.0 |
| Compound of Example 54 | 3.6 |
| Compound of Example 55 | 6.6 |
| Compound of Example 56 | 4.6 |
| Compound of Example 57 | 103.0 |
| Compound of Example 58 | 2.4 |
| Compound of Example 59 | 47.0 |
| Compound of Example 60 | 38.0 |
| Compound of Example 61 | 11.0 |
| Compound of Example 65 | 3.5 |
| Compound of Example 66 | 5.4 |
| Compound of Example 67 | 5.8 |
| Compound of Example 68 | 130.0 |
| Compound of Example 71 | 2.3 |
| Compound of Example 72 | 4.0 |
| Compound of Example 73 | 60.0 |
| Compound of Example 74 | 12.0 |
| Compound of Example 75 | 12.0 |
| Compound of Example 76 | 13.0 |
| Compound of Example 77 | 13.0 |
| Compound of Example 78 | 17.0 |
| Compound of Example 79 | 17.0 |
| Compound of Example 80 | 24.0 |
| Compound of Example 81 | 94.0 |
| Compound of Example 87 | 6.6 |
| Compound of Example 88 | 0.9 |
| Compound of Example 89 | 14.0 |
| Compound of Example 90 | 11.0 |
| Compound of Example 92 | 7.0 |
| Compound of Example 94 | 42.0 |
| Compound of Example 95 | 98.0 |
| Compound of Example 97 | 14.0 |
| Compound of Example 98 | 1.2 |
| Compound of Example 99 | 1.5 |
| Compound of Example 100 | 0.5 |
| Compound of Example 102 | 12.0 |

### INDUSTRIAL APPLICABILITY

The present invention makes it possible to provide compounds having DPP-IV inhibitory activity and improved in safety, non-toxicity and the like.

The present inventive compounds are useful for the suppression of postprandial hyperglycemia in a prediabetic, the treatment of non-insulin-dependent diabetes mellitus, the treatment of autoimmune diseases such as arthritis and articular rheumatism, the treatment of intestinal mucosa diseases, growth acceleration, the inhibition of rejection of a transplantate, the treatment of corpulence, the treatment of eating disorder, the treatment of HIV infection, the suppression of cancer metastasis, the treatment of prostatomegaly, the treatment of periodontitis, and the treatment of osteoporosis.

## Claims

1. A compound represented by the formula (I): 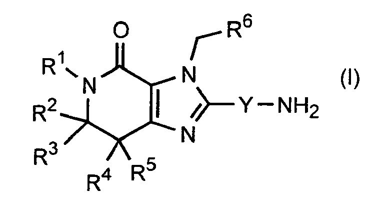 wherein R¹ is a hydrogen atom, an optionally substituted alkyl group, an optionally substituted cycloalkyl group, an optionally substituted aryl group, or an optionally substituted heteroaryl group;
R² and R³ are independently a hydrogen atom, a halogen atom, a cyano group, a formyl group, an optionally substituted alkyl group, an optionally substituted cycloalkyl group, an optionally substituted cycloalkyloxy group, an optionally substituted alkenyl group, an optionally substituted alkynyl group, an optionally substituted amino group, an optionally substituted carbamoyl group, a carboxyl group, an optionally substituted alkoxy group, an optionally substituted alkoxycarbonyl group, an optionally substituted aryl group, an optionally substituted aryloxy group, an optionally substituted aryloxycarbonyl group, an optionally substituted aralkyl group, an optionally substituted aralkyloxy group, an optionally substituted aroyl group, an optionally substituted arylthio group, an optionally substituted arylsulfinyl group, an optionally substituted arylsulfonyl group, an optionally substituted alkylthio group, an optionally substituted alkylsulfinyl group, an optionally substituted alkylsulfonyl group, an optionally substituted heteroaryl group, an optionally substituted heteroarylalkyl group, an optionally substituted heteroarylcarbonyl group, an optionally substituted heteroaryloxy group, an optionally substituted alkylcarbonyl group, an optionally substituted nitrogen-containing saturated heterocyclic group, an optionally substituted aralkyloxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein _{R}¹⁸ is a hydrogen atom, an alkyl group, an alkenyl group, a cycloalkyl group or an alkoxy group, and R¹⁹ is an optionally substituted alkyl group, an optionally substituted alkenyl group, a cycloalkyl group, a cycloalkyloxy group, an optionally substituted alkoxy group, an optionally substituted alkenyloxy group, a 2-indanyloxy group, a 5-indanyloxy group or an optionally substituted aryloxy group, or R² and R³ may be taken together to form an oxo group on the ring;
R⁴ and R⁵ are independently a hydrogen atom, a halogen atom, an optionally substituted alkyl group or an alkoxycarbonylmethyl group;
R³ and R⁵ may be taken together to form a double bond on the ring;
R², R³, R⁴ and R⁵ may form an optionally substituted benzene ring, an optionally substituted cycloalkene ring or an optionally substituted 5-or 6-membered heteroaromatic ring together with the adjacent carbon atoms;
R⁶ is a hydrogen atom, an optionally substituted alkyl group, an optionally substituted cycloalkyl group, an optionally substituted aryl group, an optionally substituted vinyl group, an optionally substituted nitrogen-containing saturated heterocyclic group or an optionally substituted heteroaryl group; and
-Y-NH₂ is a group represented by the following formula (A) or a group represented by the following formula (B): 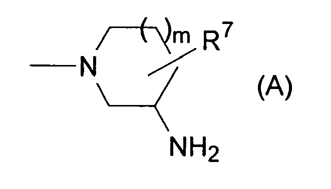 wherein m is 0, 1 or 2, and R⁷ is absent or one or two R⁷s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁷s, when taken together, represent methylene or ethylene and may bind to two carbon atoms constituting the ring, to form a new ring, or 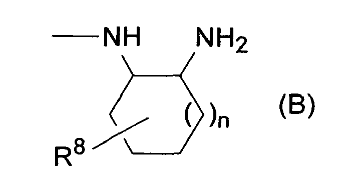 wherein n is 0, 1 or 2, and R⁸ is absent or one or two R⁸s are present and are independently a halogen atom, a hydroxyl group, an oxo group, an optionally substituted alkoxy group, an optionally substituted alkyl group, an optionally substituted aryl group, an optionally substituted aralkyl group, an optionally substituted amino group, a carboxyl group, an optionally substituted alkoxycarbonyl group or an optionally substituted carbamoyl group, or two R⁸s, when taken together, represent methylene or ethylene and may bind to two carbon atoms constituting the ring, to form a new ring,
a prodrug of said compound, or a pharmaceutically acceptable salt of said compound or prodrug.

2. A compound, a prodrug thereof or a pharmaceutically acceptable salt of the compound or prodrug according to claim 1, wherein -Y-NH₂ is a group represented by the formula (A) and m is 1 or 2, or -Y-NH₂ is a group represented by the formula (B) and n is 1 or 2.

3. A compound, a prodrug thereof or a pharmaceutically acceptable salt of the compound or prodrug according to claim 1 or 2, wherein R² and R³ are taken together to form an oxo group on the ring.

4. A compound, a prodrug thereof or a pharmaceutically acceptable salt of the compound or prodrug according to claim 1 or 2, wherein R³ and R⁵ are taken together to form a double bond on the ring.

5. A compound, a prodrug thereof or a pharmaceutically acceptable salt of the compound or prodrug according to claim 1 or 2, wherein R², R³, R⁴ and R⁵ form an optionally substituted benzene ring, an optionally substituted cycloalkene ring or an optionally substituted 5-or 6-membered heteroaromatic ring together with the adjacent carbon atoms.

6. A compound, a prodrug thereof or a pharmaceutically acceptable salt of the compound or prodrug according to claim 4, wherein R² is a hydrogen atom, a cyano group, an optionally substituted alkyl group, a carboxyl group, an optionally substituted alkoxy group, an optionally substituted alkoxycarbonyl group, an optionally substituted cycloalkyloxycarbonyl group, an optionally substituted aryl group, an optionally substituted heteroaryl group, an optionally substituted aryloxy group, an optionally substituted aryloxycarbonyl group, an optionally substituted aralkyl group, an optionally substituted aralkyloxy group, an optionally substituted aroyl group, an optionally substituted alkylcarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1.

7. A compound, a prodrug thereof or a pharmaceutically acceptable salt of the compound or prodrug according to claim 4, wherein R⁴ is a hydrogen atom or a methyl, ethyl or alkoxycarbonylmethyl group.

8. A compound, a prodrug thereof or a pharmaceutically acceptable salt of the compound or prodrug according to claim 4, wherein R² is a hydrogen atom, a cyano group, an optionally substituted alkyl group, a carboxyl group, an optionally substituted alkoxy group, an optionally substituted alkoxycarbonyl group, an optionally substituted cycloalkyloxycarbonyl group, an optionally substituted aryl group, an optionally substituted heteroaryl group, an optionally substituted aryloxy group, an optionally substituted aryloxycarbonyl group, an optionally substituted aralkyl group, an optionally substituted aralkyloxy group, an optionally substituted aroyl group, an optionally substituted alkylcarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1; and R⁴ is a hydrogen atom or a methyl, ethyl or alkoxycarbonylmethyl group.

9. A compound, a prodrug thereof or a pharmaceutically acceptable salt of the compound or prodrug according to any one of claims 1 to 8, wherein R⁶ is a group represented by the following formula (C), (D) or (E): 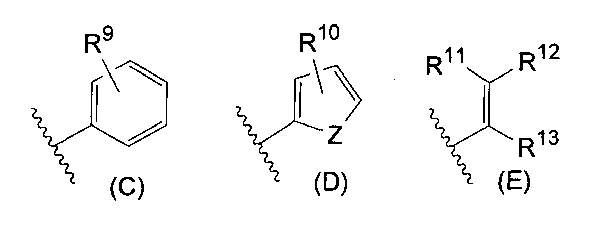 wherein Z is an oxygen atom, -S(O)p or -N(R¹⁴)-,
R⁹ is absent or one or two R⁹s are present and are independently a halogen atom, a hydroxyl group, a formyl group, a carboxyl group, a cyano group, an alkylthio group, an alkylsulfinyl group, an alkylsulfonyl group, an alkyl group, a haloalkyl group, a cycloalkyl group, an alkoxy group, a haloalkoxy group, an optionally substituted amino group, an optionally substituted carbamoyl group, an alkoxycarbonyl group, an optionally substituted alkylcarbonyl group, a cycloalkylcarbonyl group, an optionally substituted aryl group, or an optionally substituted heteroaryl group, or two R⁹s, when taken together, represent a C₁₋₃ alkylenedioxy group,
R¹⁰ is absent or one or two R¹⁰s are present and are independently a halogen atom, a cyano group, an alkyl group, a haloalkyl group, a cycloalkyl group, an alkoxy group or a haloalkoxy group,
R¹¹ is methyl, ethyl, a chlorine atom or a bromine atom,
R¹² is a hydrogen atom, methyl, ethyl, a chlorine atom or a bromine atom,
R¹³ is a hydrogen atom, methyl or ethyl,
p is 0, 1 or 2, and
R¹⁴ is a hydrogen atom or an alkyl group.

10. A compound, a prodrug thereof or a pharmaceutically acceptable salt of the compound or prodrug according to claim 9, wherein R⁶ is the formula (C) or the formula (E).

11. A compound, a prodrug thereof or a pharmaceutically acceptable salt of the compound or prodrug according to claim 10, wherein R⁶ is the formula (C), and R⁹ is absent or one or two R⁹s are present and are independently a halogen atom, a cyano group, an alkylthio group, an alkylsulfonyl group, a C₁₋₃ alkylenedioxy group, an alkyl group, a haloalkyl group, a cycloalkyl group, an alkoxy group, a haloalkoxy group, an alkoxycarbonyl group, an alkylcarbonyl group, a haloalkylcarbonyl group or a cycloalkylcarbonyl group.

12. A compound, a prodrug thereof or a pharmaceutically acceptable salt of the compound or prodrug according to any one of claims 1 to 8, wherein R⁶ is the following formula (F): 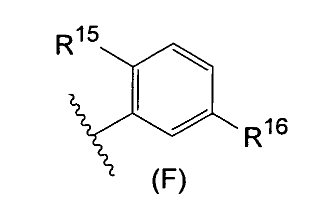 wherein R¹⁵ is a halogen atom, a cyano group, an alkyl group, a haloalkyl group, a cycloalkyl group, an alkoxy group or a haloalkoxy group, and R¹⁶ is a hydrogen atom or a fluorine atom.

13. A compound, a prodrug thereof or a pharmaceutically acceptable salt of the compound or prodrug according to any one of claims 1 to 12, wherein R¹ is a hydrogen atom or an optionally substituted alkyl group of 1 to 3 carbon atoms whose substituent(s) is selected from fluorine atom, optionally substituted aroyl groups, carboxyl group, optionally substituted alkoxycarbonyl groups, optionally substituted aryl groups and optionally substituted aryloxy groups.

14. A compound, a prodrug thereof or a pharmaceutically acceptable salt of the compound or prodrug according to any one of claims 1 to 12, wherein R¹ is a group represented by the formula: -Ra-Rb-Rc in which
Ra is an alkylene chain,
Rb is a single bond or a carbonyl group, and
Rc is an optionally substituted alkyl group, an optionally substituted alkoxy group, an optionally substituted aryl group, an optionally substituted heteroaryl group, an optionally substituted aryloxy group or an optionally substituted heteroaryloxy group.

15. A compound, a prodrug thereof or a pharmaceutically acceptable salt of the compound or prodrug according to any one of claims 1 to 12, wherein R¹ is a hydrogen atom, methyl or ethyl.

16. A compound according to claim 1, which is represented by the formula (II): 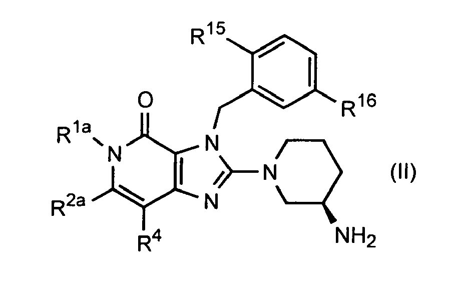 wherein R⁴ is as defined in claim 1; R¹⁵ and R¹⁶ are as defined in claim 12; R^{1a} is a hydrogen atom, methyl or the formula: -Ra-Rb-Rc wherein Ra, Rb and Rc are as defined in claim 14; and R^{2a} is a cyano group, a carboxyl group, an oxazolyl group, an optionally substituted alkoxycarbonyl group, an optionally substituted cycloalkyloxycarbonyl group, a tetrahydrofuranyloxycarbonyl group, an optionally substituted aryloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1,
a prodrug of said compound, or a pharmaceutically acceptable salt of said compound or prodrug.

17. A compound according to claim 1, which is represented by the formula (III): 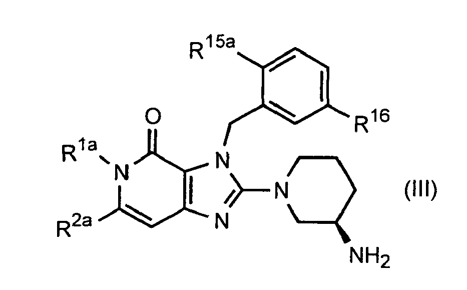 wherein R¹⁶ is as defined in claim 12; R^{1a} and R^{2a} are as defined in claim 16; and R^{15a} is a chlorine atom, a bromine atom, an iodine atom, a cyano group, methyl, difluoromethyl, trifluoromethyl, methoxy, fluoromethoxy, difluoromethoxy or trifluoromethoxy,
a prodrug of said compound, or a pharmaceutically acceptable salt of said compound or prodrug.

18. A compound, a prodrug thereof or a pharmaceutically acceptable salt of the compound or prodrug according to claim 17, wherein R^{1a} is a hydrogen atom.

19. A compound, a prodrug thereof or a pharmaceutically acceptable salt of the compound or prodrug according to claim 17 or 18, wherein R^{2a} is a carboxyl group, an optionally substituted alkoxycarbonyl group, or a group represented by the formula: - C(O)OCH(R¹⁸)OC(O)R¹⁹ wherein R¹⁸ and R¹⁹ are as defined in claim 1.

20. A compound according to claim 1, which is represented by the formula (IV): 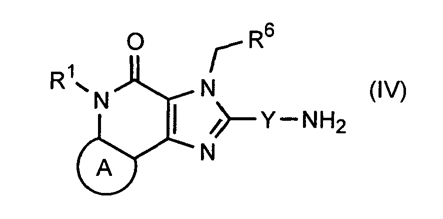 wherein R¹, R⁶ and Y are as defined in claim 1; and the ring A is an optionally substituted benzene ring, an optionally substituted cycloalkene ring or an optionally substituted 5-or 6-membered heteroaromatic ring,
a prodrug of said compound, or a pharmaceutically acceptable salt of said compound or prodrug.

21. A compound according to claim 1, which is represented by the formula (V): 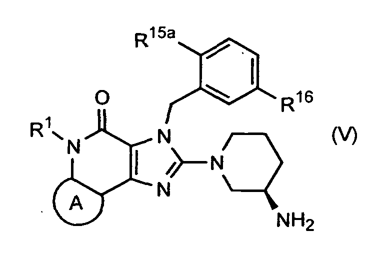 wherein R¹ is as defined in claim 1; R¹⁶ is as defined in claim 12; R^{15a} is as defined in claim 17; and A is as defined in claim 20,
a prodrug of said compound, or a pharmaceutically acceptable salt of said compound or prodrug.

22. A compound, a prodrug thereof or a pharmaceutically acceptable salt of the compound or prodrug according to claim 21, wherein R¹ is a hydrogen atom or methyl.

23. A compound according to claim 1, which is represented by the formula (VI): 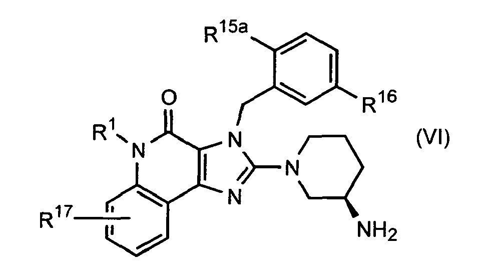 wherein R¹ is as defined in claim 1; R¹⁶ is as defined in claim 12; R^{15a} is as defined in claim 17; and R¹⁷ is absent or one to four R¹⁷s are present and are independently a hydroxyl group, a halogen atom, a cyano group, a carboxyl group, an optionally substituted alkyl group, an optionally substituted cycloalkyl group, an optionally substituted cycloalkyloxy group, an optionally substituted alkenyl group, an optionally substituted carbamoyl group, an optionally substituted alkoxy group, an optionally substituted alkoxycarbonyl group, an optionally substituted aryloxycarbonyl group, an optionally substituted alkylcarbonyl group, an optionally substituted cycloalkyloxycarbonyl group, an optionally substituted aralkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1,
a prodrug of said compound, or a pharmaceutically acceptable salt of said compound or prodrug.

24. A compound, a prodrug thereof or a pharmaceutically acceptable salt of the compound or prodrug according to claim 23, wherein R¹ is a hydrogen atom, methyl or the formula: -Ra-Rb-Rc wherein Ra, Rb and Rc are as defined in claim 14.

25. A compound, a prodrug thereof or a pharmaceutically acceptable salt of the compound or prodrug according to claim 23, wherein R¹ is methyl.

26. A compound, a prodrug thereof or a pharmaceutically acceptable salt of the compound or prodrug according to claim 25, wherein R¹⁷ is a fluorine atom, a chlorine atom, a cyano group, a carboxyl group, acetyl, dimethylcarbamoyl, diethylcarbamoyl, methyl, ethyl, isopropyl, cyclopropyl, difluoromethyl, trifluoromethyl, methoxy, ethoxy, isopropoxy, difluoromethoxy, trifluoromethoxy, an alkoxyalkyl group optionally substituted by a halogen atom or a hydroxyl group, an optionally substituted alkoxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1.

27. A compound, a prodrug thereof or a pharmaceutically acceptable salt of the compound or prodrug according to claim 25, wherein R¹⁷ is a fluorine atom, a cyano group, a carboxyl group, an alkoxymethyl group optionally substituted by a halogen atom, an optionally substituted alkoxycarbonyl group, an optionally substituted cycloalkyloxycarbonyl group, a tetrahydrofuranyloxycarbonyl group, a cinnamyloxycarbonyl group, or a group represented by the formula: -C(O)OCH(R¹⁸)OC(O)R¹⁹ wherein R¹⁸ and R¹⁹ are as defined in claim 1.

28. A dipeptidyl peptidase IV inhibitor comprising a compound, a prodrug thereof or a pharmaceutically acceptable salt of the compound or prodrug according to any one of claims 1 to 27 as an active ingredient.

29. A pharmaceutical composition for the treatment of diabetes comprising a compound, a prodrug thereof or a pharmaceutically acceptable salt of the compound or prodrug according to any one of claims 1 to 27 as an active ingredient.

30. Use of a compound, a prodrug thereof or a pharmaceutically acceptable salt of the compound or prodrug according to any one of claims 1 to 27 in the manufacture of a dipeptidyl peptidase IV inhibitor.

31. Use of a compound, a prodrug thereof or a pharmaceutically acceptable salt of the compound or prodrug according to any one of claims 1 to 27 in the manufacture of a pharmaceutical composition for the treatment of diabetes.

32. A method for treating diabetes comprising administering an effective amount of a compound, a prodrug thereof or a pharmaceutically acceptable salt of the compound or prodrug according to any one of claims 1 to 27 to a patient who needs treatment.
